# EUROPEAN PATENT APPLICATION

(11) **EP 1 413 626 A1**
(43) Date of publication of application: **28.04.2004**
(21) Application number: 02023597.4
(22) Date of filing: 23.10.2002
(51) Int. Cl.: C12N 15/31, C07K 14/36, C12P 1/06

(54) **Genes and proteins for the biosynthesis of the glycopeptide antibiotic A40926**

(71) Applicant: Vicuron Pharmaceuticals, Inc., King of Prussia, PA 19406 (US)
(72) Inventor: Donadio, Stefano, 21046 Malnate (VA) (IT); Sosio, Margherita, 20020 Solaro (MI) (IT); Beltrametti, Fabrizio, 20020 Seveso (MI) (IT)
(74) Representative: Sgarbi, Renato

(57) **Abstract**

The present invention relates to the field of antibiotics and more specifically to the isolation of nucleic acid molecules that code for the biosynthetic pathway of the glycopeptide antibiotic A40926. Disclosed are the functions of the gene products involved in A40926 production. The present invention provides biosynthetic genes that code for A40926 production, the encoded polypeptides, the recombinant vectors comprising the nucleic acid sequences that encode said polypeptides, the host cells transformed with said vectors and methods for producing glycopeptide antibiotics using said transformed host cells, including methods for producing A40926, a precursor thereof, a derivative thereof or a modified glycopeptide different from A40926 or a precursor thereof.

SEQUENCE LISTING

<110>  Biosearch Italia

<120>  Genes and proteins for the biosynthesis of the glycopeptide antibiotic
A40926

<130>  G68525
<160>  50

<170>  PatentIn version 3.1

<210>  1
<211>  71138
<212>  DNA
<213>  Nonomuria

<220>
<221>  misc_feature
<222>  (40)..(1140)
<223>  ORF1; negative strandedness


<220>
<221>  misc_feature
<222>  (1259)..(2329)
<223>  ORF2; negative strandedness


<220>
<221>  misc_feature<222>  (2558)..(5161)
<222>  (2558)..(5161)
<223>  ORF3; negative strandedness


<220>
<221>  misc_feature
<222>  (5266)..(6231)
<223>  ORF4; negative strandedness


<220>
<221>  misc_feature
<222>  (7183)..(8292)
<223>  ORF5; positive strandedness


<220>
<221>  misc_feature
<222>  (8320)..(8973)
<223>  ORF6; positive strandedness


<220>
<221>  misc_feature
<222>  (9069)..(9659)
<223>  ORF7; positive strandedness


<220>
<221>  misc_feature
<222>  (9708)..(10667)
<223>  ORF8; negative strandedness

<220>
<221> misc_feature
<222> (10670)..(11896)
<223> ORF9; negative strandedness

<220>
<221> misc_feature
<222> (11950)..(13419)
<223> ORF10; negative strandedness

<220>
<221> misc_feature
<222> (13479)..(14741)
<223> ORF11; negative strandedness

<220>
<221> misc_feature
<222> (14823)..(16019)
<223> ORF12; negative strandedness

<220>
<221> misc_feature
<222> (16009)..(17163)
<223> ORF13; negative strandedness

<220>
<221> misc_feature
<222> (17185)..(18366)
<223> ORF14; negative strandedness

<220>
<221> misc_feature
<222> (18462)..(18671)
<223> ORF15; negative strandedness

<220>
<221> misc_feature
<222> (18668)..(24259)
<223> ORF16; negative strandedness

<220>
<221> misc_feature
<222> (24278)..(36529)
<223> ORF17; negative strandedness

<220>
<221> misc_feature
<222> (36760)..(39021)
<223> ORF18; negative strandedness

<220>

<221> misc_feature
<222> (39153)..(39851)
<223> ORF19; negative strandedness

<220>
<221> misc_feature
<222> (40125)..(41732)
<223> ORF20; negative strandedness

<220>
<221> misc_feature
<222> (41772)..(42584)
<223> ORF21; negative strandedness

<220>
<221> misc_feature
<222> (42868)..(44130)
<223> ORF22; negative strandedness

<220>
<221> misc_feature
<222> (44226)..(46355)
<223> ORF23; negative strandedness

<220>
<221> misc_feature
<222> (46632)..(48578)
<223> ORF24; positive strandedness

<220>
<221> misc_feature
<222> (48575)..(54868)
<223> ORF25; positive strandedness

<220>
<221> misc_feature
<222> (54865)..(58056)
<223> ORF26; positive strandedness

<220>
<220>
<221> misc_feature
<222> (58152)..(58985)
<223> ORF27; positive strandedness

<220>
<221> misc_feature
<222> (59046)..(60641)
<223> ORF28; positive strandedness

<220>
<221> misc_feature
<222> (60874)..(62445)
<223> ORF29; negative strandedness

<220>
<221>  misc_feature
<222>  (62887)..(63312)
<223>  ORF30; positive strandedness


<220>
<221>  misc_feature
<222>  (63469)..(64587)
<223>  ORF31; positive strandedness


<220>
<221>  misc_feature
<222>  (64599)..(65240)
<223>  ORF32; positive strandedness


<220>
<221>  misc_feature
<222>  (65237)..(66541)
<223>  ORF33; positive strandedness


<220>
<221>  misc_feature
<222>  (66538)..(67335)
<223>  ORF34; positive strandedness


<220>
<221>  misc_feature
<222>  (67332)..(68618)
<223>  ORF35; positive strandedness


<220>
<221>  misc_feature
<222>  (68685)..(69423)
<223>  ORF36; negative strandedness


<220>
<221>  misc_feature
<222>  (69608)..(70894)
<223>  ORF37; positive strandedness


<220>
<221>  misc_feature
<222>  (71065)..(71138)
<223>  attL site, remnant


<400>  1
gggggctggg cctgctgcgg ctcgcgagcg ggctgacggt caggagacga accccgcgcc      60

ggggcgggtc gtcctgagtg cctgggctgc ggcgacgtcg ccgcagcctg ccaggccgag     120

cccgtcctcg atctcggcac ccaggaggcc gagcaccgta cggacccccc gttcgccgtc     180

cgcggccaga ccccagatca cggggcgtcc gacgagcaca cccgacgccc cgagcgccag 240
cgccttgagg acgtcggctc ccgaccggac gccaccatcg agcatgatct cgcagcggcc 300
cccgacgctc tccgccaccc ccggcagcgc gtcgagactg gccacggcgc cgtcgagctg 360
acgtccgccg tggttggaga ccacgatgcc gtcgatgccg aggtccgcgg cgcggcgggc 420
gtcctcgggg tgcagaatgc ccttgaccac cagcgggagc ccgctggcgg cccggagggt 480
ctcgaggtac gaccagtcca ccgcggcgga gagctccatg gccgtgtgcg ccgccagcgc 540
ggagccgccg gaggcacccc gatgagcctc ggtcccggag ttcgccgtca ggtgcacggg 600
ccgcacgtgc gggggcaggc ggaaccggtt gcggatgtca cgtggcctgc ggcccatcca 660
cggcacatcg agcgtgagca tcaacgcccg gcaccccgcg tcctcggccc ggcggatcag 720
gccgagggtg gcggcgtgct cgcgaaggca gtagagctgg aaccagacgt gtccccccag 780
ggcggtgacg tcctccaccg ggacgctgct caaggtgctg acggtgaacg ggaccccggc 840
gtcccgcgcc gcccgggccg tcgccagctc accgtcggga tgcacgagcc ggtggtaggc 900
gacgggggcc accgccaccg gcatcgtcgc ggggtggccc agcagcgtcg cacgggtgga 960
gcacgccgac acgtcctgga gcacccgcgg caccaggaac acccggtcga aggcggcccg 1020
attcgcacgg agggtctgct cgcggccgct cccgccgtcg atgaagtccc ggacgtcggc 1080
ggggaggacc ttggcggcga tctcctcgta ctcggcgagg cagacgggac tttcgtgcac 1140
gctgtcagga cgctcgggcc cgctgccggg acgctcgggc ccgctgccgg gacgctcggg 1200
cccgctgccg ggacgctcgg gcccgctgcc gggacgctcc cgcacgctgc tgggacgctc 1260
atgcacgctg ctgggacctc gccacctcga cggcctcgta gagggccttg atgttggcgc 1320
ctccgaaggt gcgggctccc tgccgctcga tgacctcgaa gaagagggtc tcgcgcggat 1380
gggtggacgc cgtgaagatc tggaagagct gtccgccgtg atcctcgtca gcgagcagtc 1440
ccgtcgcgcg caactggtcc accgtgtgac cccggatctg gatccgtgat tcgagcaggt 1500
cgtagtagct gcccggcgtg ctgaggaagc ggacgccccg ctcggacagg gtgttcacgg 1560
cgtgcacggc gtccgaggag gagaaggcga cgtgctgcac cccggcaccg gcgtgccgtt 1620
cgaggaacat gtcgatctgg ccggcctcgg ccatcgggtc gggttcgatg agtgtcagcg 1680
tgacggcgcc ggaggcgctc tgcaccacct tggactccat ggcctgggtg ccgacctcga 1740
tgcgttcctt gaaggtctcg ctgaagccga gggtggcgac gtagaagtcg gtgatgatgt 1800
cgaggtcacc cgtgggcagg cacacggcga agtggtcgat gtcgagcagc tccgccgcgt 1860
ccgcaccgga ctcggcggcg gacggagcct cggagaagcc gaccggcagg ccggggtcgt 1920
cgccggggtc ccgctggacg agggtgtgga ccacgtcgcc gaagccgccg atcgcggcgg 1980
agcaggccgg cccggggccg gggtgccggg acggggaccg tacggccggg gcgccggcgg 2040
ccacggcatg ggtgaagacg acgtcgacgt cgggggtccg cagggcgatg tcggcgaccc 2100

cgtcgccgtg cgtccgcaca taggccgaag ccggatggcc gtcggacgtg gcctgggtga 2160

ggacgagggt gatgcggccc tgccggagcg cgacgctgcg atggtcgctg gcgttcgccg 2220

tgcccacgac ggcgaagcgg tattcgtcgg tccaagggag agtggcgacc ttcagatccg 2280

ctacgtacat ttcgacgtaa tcaacggcga gaggcggaag cgattccata ttccgacgct 2340

acggccgggc ggggaggttc gcaccgtgtc cattggacgc gctcgcaggc cgcgctcaca 2400

gcagattccg gtacattccc gaggcctttt caggccggcg tggacggtcg gcggatcagg 2460

cttcataaaa agcctgccct ggcgtattct cgggttaatc aaccccgatg gatatcctgc 2520

ccgaggccgg cgaattcggc ttgtcgaact cttcgctcta cagccgcact gcctcacgcg 2580

gctcgcggcc ggctgtggcc gtcgccttat cggcgatgtc cgcggcgaag aggttgccca 2640

ggtcaccccg agtctgtacg tggagtttcc gatagatcct ggtgagatgc tgctcgaccg 2700

tgctgcgcgt gatgtagagc gcctcggcga tctcacgatt ggtgtgccca cgggcggcga 2760

gcacggcgac ccgccgttcg gcgccgctca acggtgcggt ctcaccatga tcgtgctgtg 2820

cggcgagcct cctcatcaag ggcttcgcgt tgcactcgcg ggccagctcc tgcgcccgca 2880

cccagtaggc ccgggcctcg tccttgccgc ccttgagctg tggggtcccg gcgaggtcgc 2940

agagggaaag ggccagctgg tagcgatcct gggcggcctc cagcgcgtcc acggactgca 3000

tcagcaaccg ctggcgctgt gcgggcttgc tcagctgcgc gtgcaggcgg agggcgaccc 3060

cgtacgtccg caggtcaccg gaggaggtgt gggcgatctg ggccgtgacc agatcggccg 3120

ccctgcgacg ccagcccagt tgcaggcacg ctcgcgccgc gccgaggcgc caggggacca 3180

cgtcggacag gctgctgccc cagcgctgga ccgcctgccc gcatgccagg aagccggcga 3240

aggcggcgcg aggctgctcc gtgaccaggt ggtagtgcgc cctcgcgagc tcgtagccga 3300

tcccgaaggc ggtctccgcc gtctcgcggg gcatcggcac cgccacggtc gccttcgcct 3360

cgtcgaggtg gcccatcgcg gtctgggcat ggagcagggt gctgagtggc gcgccgatcg 3420

cgacgcccca gccgctgggc tgcagtatgg tcagcgcctc ctgcgcatgg gcctcggccc 3480

ccgccaggtc gcccttgcgc cacgccgtct ccgcccggat ggcggagatg atcgccttcc 3540

aggtgggcgc cttcgtcacc ccgggctcct tgaggagcgt ctcgcaggag gccgccacct 3600

ccgacactcc gcccagcagc agtgccatca gagccgagat gatgctgtcc atcgcctcat 3660

ccgtcggctc cgcctgacgc aggatccggc gcgcgtcctc gactgtctgg cccatcgagc 3720
cgcgggcgga ccggggcaat ctgtcgagaa gcaccgggtg gacgtggcac atcgcgatca 3780
cgcgggcgga ccggggcaat ctgtcgagaa gcaccgggtg gacgtggcac atcgcgatca 3780

aggacgcgtc ggcgtccctg tcggcgacgc tcggcctcag ccgatcgatc agctccgccg 3840

cgtcggcgaa ccggccgtac cacagcagct ggcggaacag ctccatcccg tgagatccac 3900

gcaacgcacc cgagcgcgtg gcgtcgagca gatcgggcac gtgacgtgcc gccactgccg 3960

ggtcgacgcg ccactccgcc gcggcgagca tcaccttcac gtccagccgg cgaggcgtgc 4020

cccagccgga cgccagggcg agccgtaagc acttcatgac agcgacgaaa tcaccctcgt 4080

cgaacgcctg ccttcccgct tcgacgagga cgtcgaaagc ccactcctca cccgaccagc 4140

ccgcctccag caagcgcgtg gccacagccg acggtgggcc tccccgccga tggaggatct 4200

ccgcggcccg ccggaggatc tccatcctgc cgtaggaggt catgcgcccg agtacggcca 4260

ggcggcccgc ctcatgacga aagcgccccc cggccaggag cccggcgcgc tccagcatcc 4320

ccatcgagcg tgtcgcggca ggaggggcga tgcccacgag ctcgcccacc gcgtccggcg 4380

tggcgtgctc gccgaggacg gccaccgcct ccgcgacgcg gacggcctcc ggctcgcagc 4440

catgaacgca cgctgccacc gcgctcatga aggagtcgcc gaccacgagc ccgggcgcgc 4500

cggcctcctg atcctcgatc agcgcccgga ccagcagcgg gctgccgccg ctgaagcggt 4560

agaggtcgtc ggcgagctgg tcggccgtct cggccccgag gtgctcggtg gcgaactgcc 4620

tgaccgccgg acggggcagg agcggcagct cgaccagctc gatgccgggc aggcgcagca 4680

aggactcggc gacatggggg agagggccgg gcggccggtc ctggcagatc gtgacggcga 4740

tcatcatcct ggtgtccgtc agcaggggcg tcatggacag gatggccagc agggacggat 4800

cgtcggcgag atccacgtcg tcgatcgtca ggaggatcgg gttcgcctcg gccatctgga 4860

acagggagac gcagacgtcc tcgaccgcgt caccgcccct gcgcgcggtc agccgctccg 4920

ctgccccgcg gcccaggatc tggtcggcga ccccccagtc cagcgactgc tccgccggcg 4980

tgcagcgggc cgtgaccagc cggatgccgg ccgcgatgga tcgcatgccg agctcgtgga 5040

ggatggcggt cttgccgccg accacaggcc ccgtgatgac ggccactcca ccgcggccgg 5100

ccgcggtgga atcgagcaac ctcgtcagac tcttcagttc acgatctcgc ccgaacagca 5160

ctcttgctcg tcccccaagc ggttcgtcga cttggtttgc cgtgtgcctg atctggtcct 5220

ggtcccgtcg ctctacatac ggccgcccgg ctcatccact cgtgctcatc cagcggccag 5280

atcggtcgcc cgcccctcca ggcgatccgc gaacgctgcc cagatctggg catgatccct 5340

ggcgcatctc gcgacaacgg tgccccaatg cggcggcact ccgcgcaaga tcctttccca 5400

ttcctgcccg tcgatggagt gcaatgatag cattcgcaac aaaattcggc cggtttcggt 5460

cagacgtaat gcgggatcgg ccttgagcct ttccagcacg gcttggcggt ctcggccgtc 5520

gacgtggccg aaatcctgtt ctgcccgacg cagtaattcc ggcttggtcc ttaatctccg 5580

gctcccgtcc ggaatcgggc tctcaccgtg ctccaaccgg cctctcacat cccggaccgt 5640

ctccggggag atgccgacct gtttggcgac ctggcggagc gaaaggtccg gatggctgcg 5700

gatgagctcg gcggcgagtc tcctcccctc tgagctgtcc accggacgga tgcgcccgtc 5760

ccgcccgatt ctggcctcgt caccgccttg tcctctgcgc ctgcgcaggt cggccaccgt 5820

gccggcggag atgccggtcg ccgaggccac ccgtcgatca gaccactgcg gatgtgtccc 5880

gatgatccgg acggccgcac gcttgcggtc ggccagtgag agcggcagcc cgtgccgcac 5940

gttcgcctcg acggccagga cgaaggcgtc cgattcggtg ccgtcgatca gcctcaccga 6000

gattgttgtt tcacccctga cccgcgccac cttcaaccgg tgcaggccgt cgatcacccg 6060

catcgttgga cggtggacga gaatgggcgg aagctcccct tgtgccgaca acagggtctc 6120

gacgtgctct ggatcctcgc ccgaagtccg gggtgagtac acagaggaca gccgggacag 6180

ctcgatttcg acgacaggga gagtggctat gtcaactccc gtcgggtcca cctagcctcc 6240

gattcgatta gcgtcatatc ggagccgggg gcgttcaaaa aacaacccag ccgcgtgcgc 6300

cgcgcgcacc ttcgacgatt ccccgtcgcg cctgcagcat ctggtcccgg gcaagcctgg 6360

acttcccggc gcgagctgca taaatcgatc ggccaagtgc tctgtcgaga gaatgcgtcg 6420

catcctcttt tcttcggcaa ctccacgcgg caaagaattg gacgctgtcg ccgcgaatcc 6480

gtagccgtct acctcatcga attgcagaac gcttcccgtt agcattccga tcactccgac 6540

tttcggttag gccttcctcc ggaaggttaa aggaggctgt gcaggtcgaa ccacccccta 6600

tcccggacat ccacccccct agtttcggat aagaccgatg cgcggggttg cgcctctgtc 6660

gcgaagcgga gtatccggtg ctggaccgcc cgaatcgagc ggtcaccatg cgtgtcaatc 6720

cgtgtgtatt ggcatgcgcc gtcggcgcga gcccggcggg gccgcggcgg tcccacggtt 6780

tcgctcatga caccgtctcc aggtgagggt gatcgcggta gccggccacg ggcgcgctgc 6840

cgcagcggcg gccatgctga tctgcccatg gaccagcagg ccgacggtga ggtcccgccg 6900

gacggccggc tcccggtaca acgtacgtca gttcttctcg gcgatctagg ggagtgggcg 6960

gggtgccttc gccgggcatg cgggcggcct gtcctttggc aattgacagg cgtgaatgca 7020

gaaaggagcg cggccacctc tgacctgccg agtaagggaa tggattactc atcaatggcg 7080

ccggtggcca cggaactcgc ccggcgatcc ggcgtgtcca aatggcgcgg tgcccaggcc 7140

cgccgatgga caccgcccgg tgcgcgggct taagaagtag ccgtgaccct ggagaggacg 7200

ctcatcgtcg gcaccggtct gatcggcacc tccgccgcgc tcgcccttcg cgagaagggg 7260

gtggcggtct acctgtccga cgtcgacgca catgccgtac ggctggcgcg agcgctcggc 7320

gcgggccagg agtggaccgg tcagcgcgtg gacctggcat tgatcgccgt gcccccgccc 7380

agcgtggggc agcggctggc cgatctgcag cagcggcggg ccgcgcgggc gtacaccgat 7440

gtgaccagcg tcaaggtcga tccgatcgcc gacgcggagc ggctcggctg cgacctgacc 7500

tcctatgtcc ccggacaccc gctcgccggc cgggagcggt ccggcccggc cgccgcccgt 7560
gccgatctgt tcctgggacg tccctggcgg ctctgccccc gccctgagac gggtgcggat 7620

gccgtgcggc tggccaggga gctggtctcg atgtgcgggg cggagcccta caccgtgagt 7680

gcgggcgagc acgacacggc ggtggcgctg gtgtcgcacg ccccgcacgt ggccgcgtcc 7740

gcggtggcgg cgcggctgag ggacggcgac gacgtcgcgc tggccctggc ggggcagggg 7800

ctgcgcgacg tgacgcggat cgccgcaggg gaccccctgc tgtggcggat gattctcgcc 7860

gcgaacgccc tgccggtggc cggggtgctg gagcggatcg cggccgatct cgccgcggcg 7920

gcctcggcgc tgcggtccgg cgatctcgac gatgtgacgg atctgctgcg gcgcggcgtg 7980

gacggccacg gccggatccc cgacaagcac ggcggcccgg cgcgtgacta cacggtgatc 8040

caggtggtgc tgcaggatcg gccgggagag ctggcgaggc tcttcaacgc ggcggggctc 8100

gcggacgtca acatcgagga catccgcctg gagcactcgg ccggcctgcc ggtcggggtg 8160

gtcgaggtct ccgtgcgccc ggaggacacc ggccggctca ccgaggcact gcgcttccac 8220

ggctggcacg tcccgcccgt ccccgacggc aactcgagga tcgaccggac gcgagctatg 8280

gtgtcagact gacagccccc gatcgagacg gcgacacgaa tgcgcgttct ggtggtggag 8340

gaccaagtcg acctggccga ctcggtggcg cgggtgctgc gtcgcgaggg catggccgtc 8400

gatgtcagtc atgacggcga tgacgcacag gagcgcctct ccgtgatcga ctacgacgtc 8460

gtggtgcttg atcgggatat tcccggcgtc catggcgacg agctgtgcgc tgagatcgcc 8520

gtggacgatc gcaggacccg ggtgctgatg ctcaccgcgt ccgggacgac cgctgaccgg 8580

gtggcgggcc tgagcctggg cgccgacgac tatctgccga agccgttcgc cttcgccgag 8640

ctggtggcgc gcatccgcgc cctgggcagg cgcgcgcatc ctcccgcgcc gccgatcctg 8700

gtccacggcg acctgcggct cgatccggcg caacgggtgg cgatcagggg cggcatgcgg 8760

ctgccgctga ccaccaagga gctggcggtc ctggagcatc tgctgaccgc gcgcggccgg 8820

gtggtgtcgg ccgaggagct gctcgaacgg gtctgggacg agcaagccga cccgttcacc 8880

accaccgtga aggcgacgat caaccggctg cgctcgaagc tcggccagcc gccggtgatc 8940

gaaaccgtcc cgcgcgaggg atatcgcatc tgatccgcgc ggtcacagag cggtcacacg 9000

ttctctgacc ctcgtgtcac cttctgctcc gtagaactgg tgtcagatca ccagactgga 9060

ggagagggat gaggagaagc gagggtgacg acgaaccacg cactctcccg cctcgggccc 9120

gggaccgggt gtacaccgcg gtcacgcggg tgctcgccgt gctcctgctg cccgtggcgt 9180

tcgtccgtca gcccggccgc gcccgcgagc tggcctgcgg ctgggcgttg aggatgcgat 9240

tcccggcaga ggacctcacc gggctcaccg acggcgccag ggcggcgttc accgcggcgc 9300

gggccgaggc gctgtggcgt cacggccagc tcgtcggtct cacttccgga taccgcgatc 9360

cccgggtcca gcagcggatg ttcgaggagg aggtgcgccg ctcagggtcc gtggccgccg 9420

cacggatgtt cgtggcgccg ccggccgagt ccaaccacgt caagggcatg gcgctggacg 9480

tacgcccgca cgagggcgcg cgctggctgg aggcgcacgg cgcccgctac gacctctacc 9540

gcatctacga caacgagtgg tggcacttcg aacaccgccc ggagtgcggt ggcacgccac 9600

cacggcggct accccaccca ggcgcggcct gggcgagccg gaacgggggc cgggtctagc 9660

tagggcacgg ggtcgccgcg gggatcggtc cccggccggc ttcggcgcta gggcagctcg 9720

atgcggccgc tccgctgata ccagtgacgg cccgccagca aatgggtgac gaccgccttc 9780

tccagcgtcg agcgctgcgg aagctcctcc agcggctggc cgttgtagcg gaagacgaac 9840

tccaggatcg cgtcgtcccc gtcctgcggt tcggagtcga ggagcgtcca gccgcgtctg 9900

agctgcagga tgtgggagtc ctcggcgaga tactcggcca gctgcggatc gagcagccac 9960

gaggtgcctg tcgccgcccg ggcgccgtgc tcgggaaaat gccgttcgaa gaacgggcgg 10020

gcacgacgga gcgagtcgta atagatgtcg gggatcagcg gcccgcccac ttcggggatg 10080

tgcaggccga ggacgggcgt gccgtccttg gcgacggcca ggttgtactg gagccggccg 10140

agccggtaga ccaggccgcg cacgagcagg gtgagccacc acggcatgtt cgtgccgccc 10200

tcgccgtact tgcggcgatg gatggccacc gattccccca gctgcgtcag ggtctcccag 10260

gtggtcgcct cggggatgtc ccgtgtcgcg tggaagcgcc gcaacgccgg aagcgtcgcg 10320

aggaagacgt acacgtggaa gtagcgggcg gcggccccgg tctcgtacgg cagggtcggc 10380

ccaccccgta ccttcacctt gtagtcgccc atgtgccgga cgagctcgtg gtgggcgcgt 10440

tcgagcagcc accacagggc cgggtcgcga tccgggccgg gggtggccgc cacgatctcc 10500

tcgacgtcgg gagccggcac ctccagccgg tgtaagagat cacgagcctc atcgccctga 10560

ggcaggcgca ccggctcggg gggcggtccg agctcctcga gccgggagag ccacgccgtg 10620

gcgttctctc ccagccgcag ctgcctgcgc acgctctcag catccatcgt cactccgttc 10680

tgttccgccc ggccccggcg gccgtgtcga gcaggagttt cgcggccacg gccgccccgt 10740

cggcgcggat cttcccggcc acgtcgatcg cccgcgcgcg ggtctcggga gccagcgccg 10800

tggtgagcgc ggccgacagg ctctccacgg tcggcacccg cccgtcgtgt gccacgccga 10860

tgcccagctc ggccacccgg ccggcgtggt acggctggtc ggtcatctgg ggcaccacga 10920

cctggggagc gcccgcccgg gtgaccgcgg tcgtgatgcc cgcgctgccg gcgtggacga 10980

cggcggccac ccggccgaac aacacctggt ggttcacctc gccgacggtc aggcagtcgc 11040

tccggtcgtc gggcggggct aggccggccc agccacggga gacgatcacc cggtggccat 11100

gggcccggat cgcctcgatg gccaccctcg cggcgtcggt gggggcgggc ccgctgccga 11160

actccacgtg caccggtggc gggccggcct ccaggaacgc ctccacctcg gcgggcaggg 11220

gccgttcgtc gggcatgatc cacgcaccgg tctgcacgac gtcgaggtcc gtccgctgca 11280

gcggggccag gaccgggtcc gcggccagga aggggcgatc ggtgtagccg tagctgaaga 11340

tgtcgtccac cggcggcagg ccgatcgagg cccgccggct gttgagcgcg gcaccgaacc 11400
gctggtaggc gccctggttg ttgcggtccc acagcacccg gttgtcggtc acgtcccgcg 11460

cgggctgctc accgaggggt ggcggcggcg ggtagtacgg cgacggcaca tagatggggc 11520

agtagaagac gtagacgtag gggatgccga gcttctcggc caccgaccgg acggcgaccg 11580

ccgccgacag cacgccgctc accaccatca cctcgcaccc ctcggcggcc ggcaggacct 11640

ggtcgagctg cgtggcgatg gcctcggcgt cgagccgggg cacgtcctcg agcgagggcg 11700

gcctcttccc gtgcagcttc gcgcgcatcg aggtgccgac cggcaccagc ggcaccccgg 11760

cctcggccag tctctccgcg cagtccggcg gggcgcacat ccgtgtctcc gcgcctagct 11820

cacgcagctg gaccgccagg cccagcagcg gttcgacgtc cccgcgtgat ccggacgtcg 11880

acaacaacac gcgcatgtcg tatccctgtt ccgtggattc tggtgcggat cgatcggaag 11940

gccggagcct caggggtgat gtgtcagcca tctcatcccg tcgggtgagg tcaccagccc 12000

gccggggaac agtggctgct cgggctcggc gtccgcaccg agcaccgccc tcatctgctc 12060

ctgcccgccc tcctgcatca cctgtttgac cacctgtgac ttgaacagcg gcaccatgct 12120

ggagtcgtcg ccgtcggcca tctgatcgac ggccgcggcg aactcggcgc tgcgctcggc 12180

gatccgccct gaggtcgcca gcgccgtctc gccggaggac agaccgccca ccaggtccac 12240

gaacgactcc agctcggtgt actccttgtt gttggtgacc ttcttggcgt gccagaaata 12300

cgactcctcg ttcacgttca tctcgtagaa cgccagcagg aactcgtagt acacgctgta 12360

ctcgcggcga tatcgcgcct cgaactcatg cagcgcgatc ttctcctcga cgtcaccggc 12420

caggacgctg ttgatcgacc gggccgccag gaggccgctg taggtggcca ggtgcacccc 12480

ggaggagaac accgggtcca cgaagcacgc ggcatcgccc accaggatca tcccgggccg 12540

ccagaacttc gtctggtggt aggagtagtc cttgcggacc cgcagctgcc cgtacttgcc 12600

ggtcgtcacc cggcgcgccg gcgcgaggta ctccgagatc agcgggcact cggcgatcag 12660

cgcggccagc gccttctccc gatcgccctg gatcttctcc gccatctccc ggcgcaccac 12720

cgcgcccacg ctggtcagcg tgtcgctcag cgggatgtac cagaaccagc cgctgtcgaa 12780

ggccacgctc aggatgttgc ccgagtacgg ctccgccagc cgcttgccgc cctcgaagta 12840

accgaacagc gccaggctgc ggaagaactc cgaatagttc cgcgtgccac cgacgctgga 12900

atacaaccgg ctcttgttgc ccgacgcgtc gatcacgaaa cgcgcggaca ccgcgtgctc 12960

gccgccgtca ggatcgacgt aacgcaggcc ggtgacccga tcgccgtcct cgatcacctc 13020

ggtgaccgag catccctcac gcaccaccac gcccttgcgt ctggcgttgc cgagcaggat 13080

ctcgtcgaag cgtgcccgct ccacctggta ggcgaaagtc gtcggacccg tgatccgcgg 13140

agagacggag aaggagaacg tccacggctc cggccgcgcc ccccaccgga aggtgccccc 13200

gcgcttcacg ggaaaccccg ccgccgcgag ctcgtccgtc accccgagca tccggcacac 13260

cccgtgcacg gtcgagggca gcaacgactc gccgatctga taccgcggaa agacttcctt 13320

ctccaccagc agcacccgat gaccctgcat ggccaccagt gtcgccacgg tcgaaccgcc 13380

agggccgccg ccggcgacca ccacatcgaa ctcttccacg gacttctcct ttttcgttgt 13440

ggtcatgcga agtcgcccgg catttcggcc gcaggccgct accaggtgac cggcaactcg 13500

tccgggcaat cgatgaacgc gttacggaac ttcacttcct cggcggacac cgccaggcgc 13560

agcccgggaa accggcgcca caaactctga tacgccatgc gcagcagcgt cctggctatc 13620

gccgcgccta tgcagtaatg gatgccgtgc ccgaacccga cgtgcgagcc gcagtcacgg 13680

cgcacgtcga ggacgttggc attcggcgtc agcgcctcgt cgcgattcgc catcagaatc 13740

gagcacagga cgtaatcccc ggccttgatc agctggccgt cgacgaccac gtcccggacg 13800

gcgagccgtg gattcggctg ctgcacgggc gacaggaacc gcagcagctc accgaccacc 13860

cggtcggcct cctcgcgtcc ggcgaagaga gactgtcgct ggtcgggatg atccagcagc 13920

gcgagaaccc cgaagccgat cgaccccgcg acggtttcga caccacccag gatcagcgcc 13980

gtgagtacgc ccttcagctc ctcgtccgtg acatcgtctc cgtgctcccg caccagcatc 14040

ccgatgaacc cctcgtcagg gtccttccgc tgccggatga tgaggccgtt cagataccgg 14100

ttgaacgccg cgctgtcggc cgcccgggcc ttgaacccgc ggctgagatc gacgttctgc 14160

ctgacacgcc ggatgaactc gatccgatca tcacgcggga tgccgagcag ctcacacagc 14220

actcctccgc cgaccggatc ggcgaacagc gcctggacgt ccgcgggcgg ccccgcggcc 14280

tccagctcgt cgatgcggtc atcgatgagg tcctgcatgg cgggctccag ccggcggatc 14340

cggcgggccg tgaactccgg ggtcagcatc ccgcgcagcc gcgtgtgctc gggcggatca 14400

tagaccgaca gctgaccgac cagattcggc ggtatgggct ctccggcgat cgatggcgcc 14460

gaactccacc gggggcgcgt cgtgaagttc tcgtgatcgc cgagtattct gcgcacgacg 14520

tcgtatccca aagcctgcca gacatagtcg acacgcagct gagtggccgc gtcaccgcct 14580

atccggacca gtgggccatg cgccctgagc gcgaacatgt cctcatgcgg atcacagtgc 14640

gtccgcatca tgtagttcgc cgtcggctgc aggaccggcg cgcccgcatc gatatcgtca 14700

tccatacccg ggtcgaaact ccattcgctg tcgatccgca cgctcggctg atccgatcct 14760

ggtcgcgggc ggaagatatt tccagcgtcg tcaaatggac gatgggaacg ggaattccgc 14820

gatcaccagg cgaccatcag actggtcaat ccatacgcgg gagtggtcaa ccggaacgat 14880

ggctcccgat cgggatccgc gagcctcagc gtgggaaaac gccgccacag ggcggtgtag 14940

accgtgcgca gttcgaggcg ggccagagcg gctcccaggc aatgatggac gccgtgcccg 15000

aacgcgacat gggggacggg ctcgcgccgg acatcgaggc ggccggcatc cggcagcagg 15060

gcagggtcac ggttggccat gggcagagag caggagacgg tctctccctc cttgatcacc 15120

tggcccccga tggtgacgtc ctccatggcg acccggggcg tcggcgcata ggggacggtc 15180

aggtagcgga tcagctcgtc gaccgcccga tccgccgact ggtcgtcgcc ctgcaacgcg 15240
gcgatctgct cggggtgtct gagcagggcc agcacgccga gcccgatcat gccggagatg 15300

ttgtcgtcgc cggccagcat cacctgaacg cagaagcccc gcagctcctc gtccgtggcc 15360

gtgtcaccgt actcggcgag gacggctccg agcagcccct cgccgggatc cttccgctcc 15420

ctggcgatca tggccagcag gtagcgggag aacgccgcgc cggcggccgc ccgcctcttc 15480

tggctgcgcg aggcgtcgag atggccgtga cacagctgca tgaacatggc gcggtcgtcc 15540

cgtggcaccc cgatcagctc gcacagcacg gcccctggca cctcgtcggc gacgagttcg 15600

accagatccg caggggggcc cgcccgttcc agggcgtcga gccgttcggt cacgatctgt 15660

tcgatgtacg gcttcagccg ccggatccgg cgcagggtga atcccggggt cagcttctgg 15720

cggagccgcg tgtgctcagg cgggtcgtag tccatcaggt tcccgaccag ctcacgcggc 15780

cggaagttgc ctcttccgcc gatctcgtcc cgttcgttcc agcggcgccg ggtgctgaac 15840

cgccggtgat cgccgagcac ctgccgcacg acggtgtacc ccgtggccag ccaggtggtc 15900

tccgcgtccg ctcctgatcc gatggtgatc ctcgtcagcg ttccggcggc gcgcagttcg 15960

tccgccggat ccaggtcctg ccgccgagta tggaggggcc gcgcgccgtc accactcaac 16020

gggaagctcc tccacggcga aggggggcgg cttccccggt ttgaaccgca ggtcctctgc 16080

cgggaccgcc agccgcagcg acgggaaccg gcggaccaac gccggcaacg ccacttgcgc 16140

ctggagcctg gccagcggtg ccccgaggca gaagtggacg ccgtgcccga acgcgaggtg 16200

ttcggggttg ccccgggtga ggtcgaagcg gtcctcgcgc gcacgattgc ccgccaggat 16260

cgagcaggtc aggacatcac cggcgtggat gtcccgtccg gccaggcgcg tgtcgaccag 16320

tgcggtccgg ggagaggggg tctcgacgat cgacgcgtag cggaacacct cctcggccgc 16380

gctgtccgcg agctccggac gctcgcgcag cagcgccatc tggtcggggt gggtcacgag 16440

gaggagcacc gcgatcgcca gctgcgaggc catctgctcg accgccccga tcatgatccc 16500

ctcgacgagc cccgccagct cctcgtcggt gacctcgcct ccgtgctcgc gcacgatgcc 16560

gccgatcatc ccggtgccgg gatcgaggcg ttcgcgagcc gccagtttcc tggcgtagtc 16620

gacgatgccc aggcccgaca cgttccgctg cctggggact cggctctccc ggctgtcccg 16680

gaacatcctc gacagctcgg cctggtcgtc acgggggacg ccgaggaagt cgcaggcgat 16740

cagcgccggg atgggccagg cggcgttcct gacgaagtcg accggcgacc ccatgctctc 16800

cagatcggcc aagcagtcct cgacggtctc ttcgacgaca ggccgcaggc tctcgatccg 16860

ccgggcggtg ttcgccctgg tcacggtcct gcgcaaccgg gtgtgatcgg gcgcgtcgta 16920

cgactgcagg attcccggca gccaggcgcg ctccgcctcg tcctcgaccg ggcgcatcga 16980

gctgaaccgg ttggcgtcgg cgagaatctc tctgatctcg ccatatccgg tgacgagcca 17040

ctgcttgtgg ccgtccagcc ccggctcggt gtcgtactcg tgcagcggcc cgtcctcttg 17100

caggtcgaag agcgccggca cgggatcgag cctcagccgc tgatgcggca gcggaaccac 17160

catgattctc ctcagcttcc ggcgttacca gtcgagcagc agtgctttca cgtcgaacgg 17220

cggcggcccc aatctgatct cttgttcggg ctcggccagc cggagtgcgg ggaagcggcg 17280

tgccagagcc gggatcgccg accggaagat gagctcggcc aggggtctgc caagacagtg 17340

atggatgccg tgcccgaacg cgacattcgg cgctctgtcg cgggtgaggt cgaactgatc 17400

gtccgggccg gggaagtgcc gccggttcgc tcccagcaac gagcacgtga cggtgtcgcc 17460

ggccttgacg atgcggtcac cgatgcgcac gtcctccagc gcgatccgcg gagtgagctt 17520

ctcgtcgatg gtgagatagc gcaccatctc ctccagccag tcaggcacga cgtccggctt 17580

gtcccgaagc agcgcgaact gctcggggac ctcggccatc agccacgcgc ccgccgcgag 17640

gaaacgagcc acctggtcac cgcccgcgcc catgacgaac gcggccagcc ccgtcagctc 17700

cgcgtcggtg atctcgtcac cgtgttcgcg caccacgacg ctgagcatgt cgtcgccggg 17760

atcacggcgc gtgcgagcca cgacctggcc catgtaggtc atgaacttgt tcccggcggc 17820

tccgcgtctg ctggccgccc gctgggaccg gctggcgtgc aggctgcgtg acagctccgc 17880

ctgatcatca cgtggaatgc cgaggaagtc gcaggtcgcc gtcgtcgcga tggaccaccc 17940

gaaatgcggg acgaagtcca gcgggccacc gatggactcg atggcgtcca ggcagtcctc 18000

gacgacctga tcgacctgcg ggcggaaccg ctccatccgc cggacggtga acgcgggcga 18060

gaccacccgg cgcagtcgcg tgtgctccgg cgggtcgtac tgggtgatga agccgggaaa 18120

gacgattccg gccgcggtcc cgccgtagag caacctggag ctgaacttgt ccgagcccag 18180

cacctgccgg acctcgtcga acccggtggc gagccacgcg gtccgccctc cagggccttc 18240

ctcggcgccc agctcggtca tcgggccctc ggccatgaag gaccgcagct gtggcaccgg 18300

atcgaaccgg tccctccagt gcagctcccc gggcaggacg acgttgagct cctcgaacac 18360

ttccacgtca caggtccttc cgcctcaacg gtggtctcag gccggtcgga cgggcgctgg 18420

gcccgtccaa ccgtccacat caagcggctg gacaccctcg ctcaggcgcc ggcttccgcg 18480

atgagactct tcggccggat gtccgtccag ttctcctcga cgtaggcgag gcattcctgg 18540

cgggtggcca cgccgtggac gcgggtccag ccaggcggca cctccgcgaa cgagggccag 18600

agcgagtgct gtccttcgtc gttgacgagc acgaggaagg agccgtcttc gttctcgaac 18660

gggttggtca tcgctgtgtc ctttcaccgt ccggccgggg ccggagtttc tcggcgacga 18720

cggccccgat ccgggccagc gccgcgggct gcagcatctg catgtggtcg atctcgatct 18780

cgtgaggctc gacggttccg gtggtgaggg gtcgccagct ggcgatggcg tccgcgacgg 18840

gcagatggga ggggcggttc actgtggcga cgaagagcag gatgtcgcag ccgaagctgc 18900

gggaggtgtg cagcggcccg acccgggcga ggtgctccat gacctcgtcc aggcgcttcc 18960

tggcgccggc ctccgtggcc accgcggcgg cgagctgcgc ctgctgctcc tgctgcctgt 19020

cgaagtccgc ggcctcctga tcggccgcgt cgccgcgcgg gcgccggagc ctgcccacgt 19080
cggtgggata ggcgtcgagc agggcgagca ggccgacctg ctccccttgc tcctccagca 19140

ccctggccat ctcctgcgcg atccgcccgc ccagtgacca cccgaggagg tggtacggcc 19200

cggtcggctg cacggcgcgg atctgctcga cgtaatcggc cgccatctcc tcgacgccgg 19260

ccgccagcgg ctccgtacga gccaggccgc gcgcctgcac gccgtagacc ggctggttgg 19320

ggggcaggct ccgcagcagg ggcgcgtagt tccagctcag ccctccgctc gcatggacgc 19380

agtacagcgg cggacggtta ccgccggctc gcagcggcag cagcacctcg aagtcgcccg 19440

tgccggtctt caccgagccg ctcgatcccc gccgctcgcc gacgacgacc agggtgcccc 19500

cggctgtcca gcgggcgagc aggcccgtgg gcagcatgcg ccgccccgtg gccccgaagg 19560

ggcaggccag ggtccggtcg cccggctccg cgtccaccgc cgcgccggtg aggtagagct 19620

cgccgacggc gacggcgggg cgcagccggt cgtcgagcac gagcgcgccg agcggaccgc 19680

cctcggccag gtcggcggcc accgggggca cggcctcgga ccacttcgcc ggcgcctcgg 19740

ccggacgttc gcgttcggcg tcgtccagca ggacgtccag gtcgctgacg cgccgctggg 19800

gatcctccgc cacctgctcc aggaagctga ccagccgccg tgccagcgac tcggccgtgg 19860

cctgatcgaa gaggtcggcg gcatagtgga gggtgccctc gatgccgtcc tcgtcgcggc 19920

gctcggtgag cctgaacgcc agatccagct cgatggcctc cggcccgaca ggttcgacgc 19980

tggtgcgcag ggcgggcagc tccgtcgcgt cccacgcgcc gaggtcctcc tcgtgcacct 20040

ccaaccccac ctggaacacg ggatggcggg agagcgagac cggcaggtcg agcagctcga 20100

cgatcctggc gaagggcacg tccaggtgct ggcgcgcgga ccggatcgcc tcctgcgccc 20160

gggtgacgac ctccaggaag gtggggtcgc ccgagaggtc cgtgcgcagg gcgagcggcc 20220

gggcgaaggg cccgatcatc ggctccaggt cgatgaggtc gtcgtcccgt ggcagcttcg 20280

tgccgatcac caggtcgtgg cccgccccga gcctggtcag cagcatggcg agcgcggcat 20340

gcacgatctg gaacgggtgc gcgccgatcg ggtccaccgc ctccaccagc ctggcgtgcg 20400

ggccggcgtc cagtcgcaac gacaccgtgc cggcccgccg cgacgcgacg gccgaccggg 20460

ggcggtcgaa cgggagcacc gtctccccgt ggatgcccgc cagattgtcg cgccagaaca 20520

ccagctgctc gttgatcagg ccgtccgcat cgcgctcgcc ttcgagcagg cgccgctccc 20580

agatcgcgta gtcggcgaac tgcagtgtca ggggcgcccg ctccggtgcc cggccggcac 20640

gccgcgcgcc gtacgccgcc gacaggtccc ggaggaacac atccagcgac tcgtcatcgg 20700

cgaggatccg gtgcaccatc aggtgcagga cgtgttcccc gtcggagagc cggaacaggt 20760

caccgcgcca cggcacctcc cgggtgaggt cgaagaccga ctcccgcagc tcggtgagca 20820

gcccgggcag gctctcctcg gtggcgggaa ccggcgtcag gtcaaccggc gaggcgtcgt 20880

gtacgtgctg gtgaacgctc tgcgcgtggc cggggaaggt cgtccggaga atctcgtgcc 20940

gcgccgcgac gtcgccgagc gccgcctcca gcgcgggcac gtccagccgg ccgcgcaagc 21000

gcagcgcgac cgagacgtgc aggccggcgg cctccccggg actcgccagc agccaggcgc 21060

tgagctgctg ggcggtgagc ggtacccggc ccggccgttc ggcgggctcc agcgcggggc 21120

gtgacttcgc ggccagcgcc cgggcgacac ccgcgggggt ggccgcggag aacagctgcc 21180

ggatgggcag gtccgcaccg agctcctcac ggatccgcgc gatgagccgc atggccagtg 21240

ccgaactgcc gccgagatca tggaaggcgt cgtcgacgcc cacccggtcg acgccgagga 21300

tctcggcgaa gagcgcgcac agcaccttct cggtctcgct ctcctgtgcc ctgtccggcg 21360

cacgtcccac gagatcgggg gcgggcagag ccaggcgatc caccttgccg ttgggcgtga 21420

caggcagcgc gggcagggcg acgaccgcca ggggaaccat gtacgcgggc aggaccaggg 21480

ccatctcccg ccggatctcc gccggccccg catcggttcc gtcggagatg aagtagccga 21540

ccaggcgctt ctcgcccggc tggtcctccc gcgccacgac gaccgcctcg accacgccgc 21600

gctgggccgc cagcacggcc tccacctcgc cgagctccac ccggtagccg cggatcttca 21660

cctggtcgtc ggtgcggccg aggaacacca cctcgccgtc gcggttccag cgcgccaggt 21720

cgccggtgcg gtacatgcgc tcaccaggag aagggtccac ggaggccgga acggccacga 21780

accgctctgc cgtcaggccc ggcccgccga gatacccgcg ggccagcccg gtgcccgcga 21840

tgtacagctc gcccgccacc cccggcgcga ccgggcgcag gaaggcgtcc aggatgtaga 21900

tcctgcggtt ggtcatggga cggccgatcg gcagctcccg cccgacctcc tcgccgggct 21960

cgatcggctt ccacgtcgcg cacagggtgg tctcggtcgg tccgtacgtg ttgcgcaccc 22020

gcaggccggg cacggcccgc cgcaggtgct ccacggactg cgccggaacc acgtcaccac 22080

cggtcccgac ctcgaccagg cccgcgaaac actccgggga cgactccgcg agggcccgga 22140

aggtaccggc ggtgagatgg acgaaggtca cgccccgttc gacggcctgt ctcatcccga 22200

gcgcgtccag cactcccggc tcggtgagca cgacccggcc acccatggcg agcggcaccc 22260

acatcgcgta gagcgaaggg tcgaagacgt gcgtcgcgtg catcagcacg gcgtcgcccg 22320

ggccgatccg ccagccctcg tcgcccgcca ggccggccac ggccccatgg gggacgccca 22380

cccccttcgg caggccggtg gagccggagg tgtacatcac gtacgccagg tcgtcggcgc 22440

tcagccggat ctgcggcgcc gtggcggcac ccgcgtcgat ggccgcgcgg gtctccgggg 22500

cgtcgatgac gatcgcgtcc gccggcgcca cttccctggt ggcccgggtg cacaggacgg 22560

ccgagacacc ggagtcggcg agcacgaact cgatccgctc ggccgggtgc tcgacgtcca 22620

ccgggacgta cgcggcgccc gccttccagc tcgccaggaa cgcgatcagc aggtcaggcg 22680

acctgtccat gaccacgccg acgcggtcgc cacggccgat gccacgagcg gcgaggtggc 22740

gggccagccg gttcgccgcc tggtcgacct cggcgtaggt caggtccgcg ccgccggcat 22800

cggtgatcgc caccgcgtcc ggcgccgtgg ccacccgccg tccgaagaga tcgagcacgg 22860

actgccccgg cgtggggccc gccgtcgcgt tccagtcctc caccaccagg gcgcgctcgg 22920
cttcgctcag cagcgtcagc cggccgacga ggacgtcggg ctcggcgacc agccgctcaa 22980

gcacgcgagc cagcgcgccc acgaccgatt cggcggccgc ctcgtcgaag aggccgcggt 23040

cgtagtcgag gatgagcggc atctgcgcgc cgggcccggt gatcagggtg aacgggtagt 23100
gcgaggagtt gcgtccgcgc cgcaccggtc tcaggtccag gccgccgtcc tcttcggccc 23160
ggccgagtcc ctggcgcggg aagttctcgt agatgacgag cgtgtcgaag accgccccgg 23220
gcccgacggc ggcctgcatc tcctgcaggc cgagatgctg gtgcgccatg agtgccgact 23280
ggctgcgctg cagctcggcg agcagctcga cgacccgccg gccgccttcg agccggaccc 23340
gtaccggcag ggtgccgagc agctgcccga ccatcgactc cacaccggcc agctcggcgg 23400
gccgccccga ggcggtcgcg ccgaacacca cgtcggtgcg gccggcgagc tgcgccagga 23460
ccatggccca cgcaccctgg acgacggtgt tcaaggtgag gccgtggccg cgcgccagcc 23520
gcgccagccc gtccgtcagc tccgcggaga gctcgatcac cgcggtgccc atgtccggca 23580
cgcgggccgg atcggccggg gcgaccagcg tgggcgtgtc cagtccggcg agctcctgcc 23640
gccaggccgc ccgggccgcc tccttgtcct gccggcccag ccaggcgagg tagtcccggt 23700
aggacacggc ggcaggcagc ccggacgcgt cgccgcccgc cgcgtagatc gcggccagct 23760
cgcggtgcag gatcggcatc gaccagccgt ccagcaggac atggtgcagg gtgtgcacga 23820
gccggtggct ggccgggccg agccggatga ggtgcagctt catcagcggt gccgcatcga 23880
gggggagccg ctcggccagc tcgtccgccg ccagccggtc cacctcgctg tcgaggaggt 23940
cgtccggcag cccgtggaga tccgtttcac gccaggggat ctccgcctcc cgcgcgatga 24000
cctgcaccat ctgcgcgccg ctgacatagc ggaaataggc ccgcagcgcg gcatgccggt 24060
ccacgagcgc ctgccacgac gccctcagcc gcccggcgtc cagcgggccg tcgatgccgt 24120
acacggtctg caccgtgtag gtgtcgggcc cgtcgtcgtc gagggcggtg tgatagagca 24180
tgccctcctg cagcggcgag aggggccaga cgtcttccac gctggagcgc ggcttcgcac 24240
gagtgtcgtc aatggtcacg atctgctcct tatggagtca tccgccggcc ggtccggcct 24300
cgagttggtc cagctgatca ggcgaaagat ccacgagtgt gccggccggg ggctccggtt 24360
ccccggtgcc ctcgccgtcg gccgggagct ccttgacgac cgccgccagc cgttccgccg 24420
tcttctcgtc gaacacctgc cacggggtga ggtccagccc cttccggcgg gccagggccg 24480
acagctgcat cgaggtgatc gagtcgccgc cgagctcgaa gaagctgtcg ccggcgccca 24540
cctcctccag gccgagcacc tccgcgaaca gctcgcacaa cttcgcctcc atggccgagc 24600
gcgggtcgcg tcctgacgac gacctcgcga aatcgggggc ccgcagcgcc cgatgatcga 24660
ccttgccgtt cggggtcagg ggcatcgtgt ccagcgggac gaacgccgcc ggcctcatgt 24720
gctccggcag gcgtccggcc gcgctctcgc ggagggcgga gatcaacgcg ccgtcctgcc 24780
cggcctcgga gggagcgccg gccacctgct cggccgcggg gacgacatac gccaccaggt 24840
acttctggcc cggaccgtcc tcgcgggcga cgaccgccac ctgggcgacc cccggatggt 24900

ccgccagcac ggcctcgatc tcacccggct cgatccgata gccgcgcacc ttgacctgcg 24960

cgtcggcccg gccggtgaag accagctcac cgtcccgggt ccagcgggcc cggttgccgg 25020

tgcggtacat gcgctcacca ggacgggcag ggctcaccga ggccggcacc gcgacgaacc 25080

gctccgctgt caggcccgga cggccgaggt agccgcgggc gagcccggcc cccgccacat 25140

agagctcgcc ggtcacacct ggcggcacgg gctgcaggaa ggcgtcgagc acataggctc 25200

gcaggccggt gatcggccgg ccgatgggca ccacgtcgcg tcccggagac agcggggagc 25260

tcatcgtcgc gcagacggtc gtctcggtgg gcccgtaggc gttgatcatc cggcggcccg 25320

gcgaccagcg gtccaccagc gcgggcgggc aggcctcgcc ggccacgacc agggtctcca 25380

ggctgtccgg caggtcgtcc tcgacggccg gcacgctcgg cggcacggtc acgtgggtga 25440

tgccccaccg gcgtaccgcg tcgcccagcg acacccgggg cggcatgctc tccgcgtcgg 25500

ccagcaccac ggtcccgccc gacaacaggg ccatgcacag ctcggagacg gcggcgtcga 25560

agccgagagc ggcgaactgc aggatccgcg aggcggacgt gacgccgaag cgctcgatct 25620

gcgcgctcgc cagattgccg agcccggcat gggggacgag gactcccttg ggcacgcccg 25680

tcgaccccga ggtgtagatc acatacgccc cgtcacccgc ctccacccgg ggcagcgcag 25740

tgcgcggatc ggcggcgagc ggcgcgtcca gcgccaccac cgcgcccgcg aactcctccg 25800

ggacggcctg cctggtctcg ctcgtgcaca gcagcacctc cggcgcggaa tccgccagga 25860

tgaagctgat gcgctcgcgc ggataatcgg gatccatcgg gacgaacacc ccgcccgccg 25920

aggacacccc gagcagtgcc accaccagct cggccgagcg tcccacgagc acgcccaccc 25980

gcgtctcacg gcgcacgccc agccccacca gcagccgcgc cagctcctcc gcctcgtcca 26040

gcagtccgct gtacgacagg ctccgggccg cgtccaccac cgccaccgca tccggcgagc 26100

gctccacctg ccggcggaac agcatcggca ccggctccgc ggcgggcggc acgccggtcc 26160

tgttccactc ctccaccacc aggcggcgct gctcaggacc gatcaggccc acgcgcccga 26220

ccggcacgcg cggctcggcc accacctgct ccagtgcccg gaggatcgac gcgagcatct 26280

cctcggcctc ggcccgatcg accacgtccg gccggtagat gaactcaccg tggacgcggc 26340

ccgccacgga cgcgcgcatg gacagcggat agtgccctgt gtcgttcggg atgcccgcgg 26400

gccgcatgac gagcgcgtcg gggccttcgg gccgaggcgg cgggggcggg tagttctcga 26460

acaccacgat cgtgtcgaac gccgcgcccg gaccggcgag ctggttgatc tcgctcagcc 26520

ccacgtgctg gtgcggcatg cacgcgacct gccgttcctg aaggtctgtc agcatgtcga 26580

ggaacggctc cgcaccggcc aggcgagccc ggaccggcaa catgttcatg aacaggccga 26640

cggcggactc cacaccgggg atctcgggcg ggcgcccggc caccgcggcg ccgaagacca 26700

cgtcgtcgcg tccggtcagc cgggccaggt gcagtgccca gatcccctgg aagagcgtgt 26760
tcgccgtcac gccgtgacga ccggtgaact ccaccacgcg ccggctcagc gcctcgtcga 26820

gttcgaaccc gacacgttcg ggctccaggg gagtggtgat cgtctccggc ggcacgacgt 26880
gggtcgcctc gtcgagcccc gccagctcgg cccgccacgc ctctcgggcg gccgccttgt 26940
cctggcgggc gatccaggcg agatagtcgc ggtacgacgt cgcggccgga agggcccggc 27000
cgtcaccgcc ggactcgtac acggtcagca cgtcctcggt gatcagcggc agggaccagc 27060
cgtcggccac gatgtgatgc gaggtcagga ccagccggtg ccggcgttcg ccgaggcgca 27120
ccaggtgcag ccgcagctgc ggcgcccggg tcaggtcgaa ccgctcggtg tgcagctgct 27180
ccgcgaggcg gtcgaactcc gccagcgcct cgtcctcggg cagccgggac agatcggtct 27240
cctgccagtc cagcggcacc tcgcgagcga tcgcctgcac ggccgcgccg gacccgagct 27300
ggtggaagct cgcccgcagg gcggggtgcc ggtcgagcag agcctgccag gaggcgcgga 27360
accgggcgac gtccaacgga ccgtcgaggg cgagcttgcg catccccgcg tagacgtccg 27420
ggccgcgctc gtcggcggcg tggaacagca ggccctcctg gagcggagac agcggccaga 27480
tgtcgagcag ggtcggtacg gcggcctcga cctccgccac gtcctgctgc gtcagcgaga 27540
tgagcgggaa gtccgacggc gtgtgcccgc cggcgccgcc gccaccgacg tgtgccgcaa 27600
ggccggtcag catggccagc caggcctgcg cgagcgactc cgcctcggcc tcgccgagca 27660
gccgccccgc ccaggtcacg gtcaggctca gctcaggtcc tgccgcaccg tccagcacgg 27720
ccgcgtcgat ctccacggcg tgccgcaacg ccgtgtcctg ctccgccgtg ccgccgatgg 27780
tgcccagcag ctgccagggc tccggggcgc ccgcggaccg ggacgggaag cggccgaggt 27840
agttgaaccc gatctccggc ttcggcgccg ccgcgagagc ctgccccgtc ccggcgttga 27900
gatagcgcag gatcccgtag ccgagcccgc cgtcgggcac ggcccgcacg ttctccttga 27960
cctgcttcag caggtgaccg gccgcaccgc cacccgcgat cacttcggcc ggatcgatcc 28020
ctgtcacatc cagccggagc ggatgcacgt cggtgaacca gccgaccgtc cgcgacaggt 28080
ccagctcgtc gatgggccgg cggccgtgac cttcgacgtc caccacgacc gcggtgccgc 28140
cgcgccagtg ggccaccgcg cctgccagcg tcgccagcaa cacctcgtgg acaccgcagt 28200
ggaaggcgga ggtggcctgc tccacgagca cgcccgcccg gtcatgcggc agcgtccacg 28260
atgtgcgtcc cgcggtcgag acggtgtcgc gcgccgggtc gagctcgccc agccgcgatc 28320
gcgccccgtc gaggatctcc gtccacgtct ccagctccgt ggcccgtgtc accgcctgat 28380
cggccagcgt tcgcgcccag cgccggaacg agacgtcgac ggggtcgagc accggccgcc 28440
ggccggcggc cacggcttcg caggccacct gcaggtccgg cagcaggatt cgccacgaca 28500
cgacgtccac cacgagatgg tgcgccgcca cgacgagccg tcccacccgc cctggccccg 28560
cgtccaccca gaccgcccgg atcatcacgc cggcgtgggg atccagccgt gcggccgcgt 28620
cgcgggcgca gcgatccgcg atctcatcca cgtcaccggt gccggcctcg acccgttcga 28680
ccagcgtcgc cgcgtccacc gctccgcggc cggccacgac cagccggggc tgcgcagccc 28740

cggtgcggac gatccggctg cgcagcatgt catgcgcgtc gatgaccgcg cccaatccgg 28800

ccgccagcac gtccaccgac aggtcgtcgg gcgcaccggc ggtcacccac tgggacaggg 28860

cgccccgggt catcgcgtcg ggatcgcgtt cgagcagtgc ccggatcacc ggcgtcgaca 28920

tcacctcacc gacgccgtcg tcgaggctcg cccgcgtcgc accgccgcgt tcggcgacca 28980

tcgcgatccc ggcgggcgtc ttgcgctcga agacgtcctt cgcgccgaag acgagctcct 29040

cacgtcgcgc gcgggcggcc aaccgcatgg agaggatcga gtcgccgccc agctcgaaga 29100

agctgtcctc ggctccggct cgcgccacgc ccaggacctc ggcgaacagt tcgcacaaca 29160

cccgctcggc ctcggtacgc ggctcccggc cggccgcctt cccggtgaac tcggggacgg 29220

gcagcgcggc acggtcgatc ttcccgttgg gcgtcagcgg gacgccgtcc agcagcacca 29280

ccgccgccgg caccatgaac tccggcagcc gtcccgcgag gtgctcgcgt accgcgtcgg 29340

gatccaggcc cgagccctcc tccgcggtca cgtaggcgat gagtctcttc tcgccgggac 29400

ggtcctcccg cgccacgacg accgcctgcg cgacgtgcgg aacctcggcc agcgccgcct 29460

cgatctcccc tggctcgacg cggtagcccc ggatcttcac ctgggagtcg gcccgcccgg 29520

cgaacagcag ctcgcctcgg tccgtccagc gcgccaggtc gccggtgcgg tacatccgct 29580

caccggaggc cgcggggttc accgaagcgg gcaccgcgat gaaccgctcc gaggtcgccg 29640

cgggggcgcc caggtaaccg tgtgccaggc ccgcgccggc gaggtagagc tcaccggtga 29700

cgttcggcgc caccggctgg aggaaggcat ccaggacgta cacctgccgg ccggccagcg 29760

gacggccgat gggcaaggtg tcgcccgttt ccgtgtgcgg ctcgatgagg tgccaggtgg 29820

cgcagagcgt gacctcggtg gggccgtaca actcccggac ccggacctcc gggcatgccc 29880

ggcgcacccg tgcgacggac tcgagcggca ccacgtcccc gccggtgagg acctcgcgca 29940

gcccgctgaa ggagtccggc gactcctccg ccagcacccg gaaggtcccc gccgtcagat 30000

ggacggtggt cgcgccccgt gcgatcacgt cccgcagccg ctgcgcgtcg atcgcgcccg 30060

gttccgcgac catcacgcag gctccgctga ccagcggcac ccagatctcg agcagcgacg 30120

cgtcgaacgc gtgcgacgcg tgcatcaaca cgcggtcgcc ggcgccctgc gaccagcccg 30180

ggtcgccggc cagagccgcg gcgctcccat gcggcaccgc gacgcccttc ggcaccccgg 30240

tcgatccgga cgtgtacatc acgtaggcca cgtcatgcgc tccgaccgcg agcggtggcg 30300

cctcgtgccg ctccgcctcg gtcgcgggtg cgtccatgac caccggctcg atcccgtccg 30360

ggaccgcgtg ccgggtcgct cccgcgcaca ccgccaccga cgcgccggcg tcggccagca 30420

tccgctcgat gcgctccgcc gggtagtcca cgttcaccgg gacttgcgcg gcccccgcct 30480

tccagaccgc gagcagggcg acgatcaggt ccgcgccgcg ttccatcagc acgccgacgc 30540

ggtcgccgcg ccgcacgccc ctcctggcca ggtgccccgc cagccggtcc gattcccggt 30600
cgaggccggc gtaggacagg gtgcgtccgt cgccgatgac cgccgtcgcg tccggcgccg 30660

cgtcggcctg gcgccggaac agctccggca ccgatgaacc gccggccgcc gcaccggtcg 30720

agttccagcg ctccgtcacg gagccgcggg tggatcggct ggtcacggcc aggcggccga 30780

cgggaagcga gggctccgcc accatccggg ccaggacccg cacgacctgc ccggtgatct 30840

cggcggccag gtccccgccg atccagtcgg gccggtagtc cagctggatc tgcaggcgcg 30900

cgcccgggat gacgctcacg gacagcggat aggtggtgcc ggtccgcgtg cggatcgagc 30960

tgatcgccac gccaccgtcg tcgagaccgt cggcgtccag cgggtagttg acgatcatca 31020

ggatcgtgtc gaagatcgag cccggccccg ccgccttctg gatctccggc agccccaggt 31080

gctgatgctc cgtcaaggac gactggcgcc gctgcaggtc ctggagcagg tccagcaccg 31140

gaacagcccc gtcgaggcgc acccggaccg gaacggtgtt gatgaacatc cccaccatcc 31200

gctcgacatc cggcaacgcg tccgccggac gcccggacac gaccgtgccg aacaccacat 31260

ccgtccgtct cgccagccgc gccagcacca gggcccaggc gccctgcacg accgtgctca 31320

acgtcagccc atgaccacgg gcgaagccgg tgagggcgcg ggtcgcctcc tcggacagcc 31380

attcggcatg cccgtccggc atcaccggcg ccttgcccgc gtcgaggccc accacggtcg 31440

gttcgtccag cccggcgagc tcggcccgcc acgccgatcg tgccgcgtcc tcgtcctgac 31500

ggctcagcca cgccacgtag tcccggtagg agggcggcgc cggcgagacc cgtccgtcgg 31560

cgtaggcggt cagcatctcg cccagcagga tcggcgtgga ccagccgtcc acgagcacat 31620

ggtgcgacgt caccacgagc cggtgccgcg ccgcaccgag acggatcagc agcaaccgca 31680

gcagcggcgc cctgctgacg tcgaaccgct ccgcctgatc cgccgcgagc aggcgttcca 31740

cctccgcgtc cggctcatcg agccgcgaca ggtccgcctc acgccacagg acctcggcct 31800

cgcccacgac gacctgcacc gtctcgccgg atcccagctg gtggaagccc gtccggagcg 31860

tctcgtgccg gtcgatcacc gactgccacg ccgcgtgcag ccgttgcgcg tcgagcggcc 31920

cgtcgaggtc caggatccgc tgggtctggt agacgtcgac gccgtcctcg tcgaaggctc 31980

tctcgaagag gatgccctcc tggagcggcg acagtggcca gacgtccgtc aggccgggcg 32040

ccacggcctc cagttcgtcc acgtcccgct gccgcacctc gaccagctcg aagtcggacg 32100

gtgtgtgtcc gcccgcgccg ggagtgtcgg cgagagcggc gaggccggcc agcgtgtcca 32160

gccacgcctc gccgagccgc tccaccgcgg cagggtcgag gtccctgccg tcgatggcga 32220

gtctcagccg ggggccggcc ggcgtgtcct gaacgtccgc gccgacctcc agggcgtggg 32280

actggacgag gtccggcccg gccgcctgtc cgccgagagc cccttcgcac acctgccacg 32340

cggtgtcctc ggaggcgacg ccggaccgtc cgagatagtt gaatccgatc tgggccgacg 32400

gcagctccgc cagccgggcg ccggtttcgg ggttgaggta gcgcaacagc ccgtagccga 32460

gcccgtcgcc cggcaccgct cgcgcctgtt ccttcacggc cttcagcaac tccccggccg 32520

ccgcagctcc tggaccgaca ccggagacat cgaggcggac cggatgaacg ctggtgaacc 32580
agcccacggt acgcagcaga tcctctccgt cggcggcatg gcggccgtgg ccttccacgt 32640
ccaccaggat cccggcgtca gcaccgcgcc accgcgccac cgcacccgcc aggcccgcca 32700
gcaggacgtc ctgaaccccg cagtggaagg ctgccggcac gcgcgccacc aggttgcgcg 32760
cttgggcatc ggacagtgtc cgcgaccacg acgccgactg cccggggtgc cgctccagcg 32820
gcaggtcgcc gccttcgagc acgccggccc aatggccggc ctcggccacg gtgctctcgc 32880
tgagcgcctg cccggccagc cgccgcgccc attgccggta cgacgtcacc gcgggttcga 32940
ggacgggggt tccgccggag accgcctcgt cgtaggccgc ccgcagatcc gacagcagga 33000
tcgcccacga gaccgcgtcg acgaccaggt gatgcaccac caaggccaac cggcccggct 33060
cggcgtcgcc cgcgtcgacc cacacggccc ggaccatgat cccttcggac gggtccagcg 33120
tgcccgccgc cgtcctggcc tcgcgctcgg cgcgctcagc gaggttcccg ttcccggccg 33180
ccacccgcgt caccaggccg gccgcgtcca cggcacccgg ctcggccacc atcagccgtc 33240
cgtcgggctc cacccgcgtc cgtagcagat cgtgcacatc caggacggcc tgcagggcgg 33300
tcaccagcgc gtccggggcg aagccggccg gggtgacgac gacccgcgcc tgcgcgaaac 33360
cggggcgcac cgcgtcatcg ccgagggcac gcatcaccgg cgtcctcggg atctcgccca 33420
cgcccggctc cactgaggag gctcgcctcc ccggggcctg ttgagccagc gccgccagcc 33480
gctcgggcgt gcggtgctcg aacacctgtc ggggggtcag cgggataccc tggcgccgcg 33540
cgcgggcggc gacctgcatc gacgagatcg agtccccgcc cagctcgaag aagctgtctg 33600
cgaccccgac ccgccccgcc cccaggacct cggcgaacac tccgcacagg atccgctcgg 33660
cgtcggtggc cggctcccgg tccaccgccc cggcagcgaa gtccggctcg ggcagggccc 33720
ggcggtccac ctttccgttg ccggtcagcg gcaacgcgtc cagcaccagc accgcggccg 33780
gaaccatgaa ctcgggcagc gtcgcggcga gctgctcgcg tatccgcacc gggtcgaggt 33840
ccccccctgt ttcggcgacc acgtaaccga tcaggcgctc ttcccgcgcg gacaccacgg 33900
cctgaccgac acctggaagg ccggcgagga ccgcctcgat ctcgccgggc tccacccggt 33960
acccgcggat cttcacctgg tcgtcggcac gcccggcgaa cgccagctca ccctgatccg 34020
tccagcgcgc caggtctccg gtccggtaca tccgcccacc gggcacgaac ggctcggcga 34080
cgaaccgctc ggccgtcaac gccgggcggc ccagatagcc ctgcgccacc ccggccccgg 34140
cgacgtacag ctcgcccgtc acccccgggg gcacgggccg caggaacgcg tcgaggacat 34200
agacccgccg ccccgcgagc ggacgcccga tcggcagcac cggccccgtc ggctcgcccg 34260
gctgcagcag ccaccatgtc gcacacagcg tggcctccgt cgggccgtag agatgccgca 34320
cgcggacgtc cgggcacgcc cgccgcaccc gttccaccgc cgcgagcggc accgcgtccc 34380
caccggtcag cacctcgcgc agcccggcga ccgactccgg tgactcctcg gccagcaccc 34440

ggaaggtccc cgccgtcagg tgagcgcagg tgacaccgcc ggccacgtac ccggccaggg 34500

cctcgccgtc caccgcgccc ggctcggcga gcacgacccg ggcgcccgac agcagcggca 34560

cccacagctc gaacagcgag atgtcgaacg cgtgcgaggc gtgcatcagc acggcgtcct 34620

cgggccccag cccccatccc ggctcgccgg ccagcgccgc gacgttgccg tgcgagaccg 34680

cgacgccctt cggcctgccc gtcgatcccg acgtgtacat cacgtacgcg aggtcgtccg 34740

cgtgcgcacc cgcggagaga cgggcgtgct ccgccaccgc ccgcagcgtg tccgggtcgt 34800

ccaggacgat cggatcgagc ccgccggccg gcacggcggc ctggcacgct cgctcggtca 34860

ccactgccgc cggctccgcg tccgcgagca tgaactgcac gcgctccgcc gggtaggcgg 34920

gatccaccgg cacgaacgcc gctcccgcct tccacaccgc cagcagcgtc gcgatcaggc 34980

cgggtgaccg gcccatcacc acggccaccc gatccccgcg ccggacgccc cggccgctca 35040

ggtagccggc gagcctctcc gcgtgctcgg ccagctcacc gaacgagacc gcccgcttcc 35100

cctcgacgac cgccacccgg tcacggccgc gctccacctg gcggtcgaag agatccggag 35160

ccagctcgcc cggcgccacg cggggtgccg cactccatgc gttcaccacc agcgcacgct 35220

cggccgcact cgtcacgtcg acctcggcca ccgtgaggtc gcccgcgccc gccagctgcc 35280

gcagaatccc ggtgaatcgc tccaggatgg cgagcgcggc ttcccggtcg aagaggtccg 35340

tcacatggtc gagattgagc agcatcgact cgccggggac ggcgaccagc gtcagcggat 35400

aatgggcggc ttcccggccc tggtctattc gaatatcgaa ggctgccgcg gcatccgatg 35460

ggcgaagctc acggggaaag ttctggaaaa cgagcaaggt gtcgaagacg gcgccggcgc 35520

cggccgtcct ctgaatatcc gccaatccca tgtactggtg ggggatgagc gccgactgcc 35580

gcttctgcaa atccgccagg aattcgatga ccggcgtcga accgctcagc cgcacgcgta 35640

cggggacggt gttgaggaac aaccccacca tcccctcgac gccgggcaga tccggcgggc 35700

gtgccgagac cgccgcaccg aacaccacgt ccgtccggcc cgcgagctgg gcgagcagca 35760

acgcccacgc gccctgcacc actgtgttca gcgtcagccc atgggtgcga gccagctcgc 35820

tcagggctcg cgtgaggtcc tcgggcagct cgaccgtgat gttctccggc atggcgggcg 35880

cccggttcgc atcggcgggc gccaccagcg tcggctcctc gacaccccgc agctccgccg 35940

cccatgccga cagcgtgcgc tccttgtcct gccggtccag ccacaccagg taatcccggt 36000

acgacggcac cgcgggcagg tccagcgggc tcccgtcggc cgcgtacagc atcgacagct 36060

cgtcgagcat gatgggcatc gaccagccat ccatgatcgc gtggtggcag gtcatcacca 36120

ggcggtggtc gtcgccggcg agacggatca gggtcagccg cagcaacggc gccttcgcga 36180

ggtcgaacct ccgcgtcctg tcctcctcgg ccaccgcgcg cacggcctcc tcgggctcgc 36240

tgaggtggga gaggtccacc acccgccacg gcagctccac ctgcctggcg atgagctgca 36300

ccgtctcacc tgatttgcgc tgccgaaaac aagcccggag agcggcgtgc cgcgccagga 36360

gcgcctccca tgcggcacgc agtctgtccg cttccaccgg accgttcagg ttcaggatcc 36420

aatggcccac ataaaggccg ggccagtcgt cgtcataggt cgtgtggaag agcaaccctt 36480

gctggagtgg tgacagcggc cagaaatctt cgatccgcga ctgagccatg gatgaatatc 36540

tccctcaatc agcaaagcgg cccgagaggg aatcatccat tgatgggtct gacccggaca 36600

atctgtccat ccgtgactgc cgtcaccgat ccgggtgggg tcgaaggagg ccgccgacgc 36660

ggaacgtggc ggcttgcggg cgagcaacat ggctacggcg cgccatccac agctggatgg 36720

cgcgccgtag ccaggttcac cgctcgatcg agcgcggcct cactcgaagg aaagccccgc 36780

ggccggcgtc acccggacgg cccggcgtgc cgggagaaca ctggccagga gccccgccag 36840

ggcggcgacg aggacgacga cggcgagcag tggccagggg acctgcatgg tggcgttgtc 36900

gagagcctgc ttcacgaagg tctcgtaacc gacccaggcg aacccgatgc cgatcacggt 36960

gccgagcacg gcggccacca gggagagcag cacggcctcg gcggccagca tccgccgcaa 37020

ctgcctgcga gtgagcccga gcgcgcgcag cagcgcgtgt tcgcgaacac gctcgagaac 37080

ggacaggccc agggtgttgg cgatcccgac gagggcgatc gccacggaga agccgagcag 37140

cgcgacgatg gcccaggtga ggatcatcag cggcgcgttc tccgtctcac gggcctccag 37200

ctggtcgttc acgttcgccc cggccgcggc cgccaggtcg cccagctcac cgacgagccg 37260

cgtcgagtcg gcgtcggcgg atgcgcggat ccagacggca cgcggcgcgg cggagtcggt 37320

gagccgggcc agcgtctccg gcgcgacgac ggcctgcagc ccccagccgg tggcgagcga 37380

gacctgcagc acggcccgcc ggtcgccgac cgtgaccctg accttgtcac cggcccgcag 37440

gcgcagctgg cggaatgcgg actcatcgag cctgagcacg cctggctcca cccgggcgaa 37500

cgacccgccg tcgtgggcca cccgctgggc atccggcgcg gtgaccaccg ggatcggctt 37560

gtcgaggccg gagaccgtgg cgacggcgcc gtccaccgcg atggcctgat ccaccccgga 37620

agtgccacgg accttgtcga ggaagtcggc ggagaacggc ttgccggtcg agaccagcgc 37680

ggcgtcgatg gggtgctggc cgtcgagtct ctcgttcagc gcctcggagg tgatggcgac 37740

gccggtcagg acggcggtga tcagggtgat accgaccagc agtgaggcgg cggtggtggc 37800

ggtccggcgc gggttgcgca cggcgttctt cgtcgcgagc cgcccgatgg tgccgagccg 37860

cgtaccggtg atctccagca gacgggggat gagcaccggc ccgaacagga gcacgccggt 37920

gaacaacgaa ccgccgccgg ccagcatgag caccgtgctg tgccaagcca tcgccgacgc 37980

gagcaggacg agcccggcga tcaacatgaa gacgccgagc accagccgtg cccgccccgt 38040

ggctgtacgc gggtcggtcg cggtgtcggg acgcagtgcc gccagcgggc tcacccgcac 38100

cacgcgccgg atcggcagcc aggccgcgac cagggtggcc gtcagcccga tggcgagccc 38160

gcccagcagc cacggcgcgg gcggtgccgg ggcggcgatc ggggtgatcg gtgagagggt 38220

cttgatcagg gcgatgagcc cgtagccgag tccggcgccg accagcacgc cggccagcga 38280
cgacaggagg ccgacgacgg ccgcctcccg gcgtaccgaa ctcaccacct ggcggcgggt 38340
cgcaccgacg cagcgcaaca gggcgaagtc gcgcatgcgc tgggccagca ggatggagaa 38400
ggtgttcgcg atgaccagga tcgagacgaa cacggcgatg ccggcgaaga gcagcagcag 38460
cagtgaccag gtgtccacgc cgttctggag ctgcgccgtc cgggccgcga tctcctgctc 38520
cggggtctgc accttcgcgg tctcgggcac cggaccgacc gcgccgcgca ccgtcaccgt 38580
gtagatgccg agggagggat cgtcggccca gcgcatgagc tgcggccagg tgacgtacac 38640
cgacgcctgc gccacaggag aaggcgcccg cacgatgccg accacggtga agtcggctgc 38700
cgtggcacgc tcacctatcc ggatgcggtc gccgacggcg acgtcccagt tctgggcgtc 38760
ccacaggtcc accacggcct cgcccttgcg ctcggggaaa cggcccgagg tgagctgctg 38820
ccagcgcagg tccttggact cggcgaccgg ccccacgccc atctcggggt aggaccggtc 38880
acccgcgcgc accgtcagca tggccctgcc gagcggtgac gcgttcgcgc catgacgctc 38940
gacgagctcg aacgcatcct cgttcgtcag cttggacacc acgtggtcgg agttgcggaa 39000
cggcgccccg aagccggcca tgatgccgct ctgcgccccg gaggtgagca cgccgacccc 39060
gacgacgaag gccacggcga cggtgaccgc gatcgccgcc gcgacgtacc tgcggacatg 39120
ggtgcgcagc gacgcgagaa agacggtgcg catcaggcga tccgtccgtc ttccagggtg 39180
accacgccgt cggcgtaggc cgcggcctca cgctcgtggg tgaccatcac gacggtctgg 39240
cccagctcgc gggtggattt gtgcaggtac cccaggacct ccgccgaggt ggtgctgtcg 39300
aggtttccgg tgggctcgtc ggcgaacagc agatccggcc cggtgatcag agcccgggcg 39360
atggccaccc gctgctgctg gccgccggac atctcggagg gccggtggcc gagccggtcg 39420
gccatgccga gggtttcggc gagcacgtgc acgcgctcgg tcgccgcgtc gtcgatgcgc 39480
cggccgccga gctcgagcgg gagcgtgatg ttctggaacg ccgtgagcat cggcagcagg 39540
ttgaaagact ggaacacgaa gccgatgtgc tcacggcgga agaccgtgag ctcgttgtcg 39600
tcgagtgatc cgagatcggt gccggccacg gtgacagtgc catcgctcgc ctgatcgagc 39660
ccggccaggc agtgcatcaa cgtggacttg cccgatccgc tcgaccccat gatcgcggtg 39720
aacttgccgc gcgggaggtc gaggtcgacg ccgcgcaggg catgcacgcg ggtttcaccc 39780
tggccgtaca ccttggtcag gtttcgcgcg ctggccgcca cggtttccag agcggctcgc 39840
tggccggtca tatagaagca cccttcgatt gtgcttgcgt acagtcggca tgcatgagca 39900
gaaagccatc attgacggct tcatggcgct attcttcgcg ccaaggctgg tagtcgtgct 39960
ggtactccgc aaagcgccac ccatcgtaga cgagcagtcg gccgggcacc aacggttcca 40020
aattgaagca gggcgccgtg cggagtaata gtcaagactg tggatgccga gttccttggc 40080
gactgtggga agggtgcttg caccggacgg gcggcattcc ctggtcagcc cccgggtgtc 40140

cgggccgctg gtccggcgtt ggcgtcgaag gaactgccgc cgtacctggg gtcgagcacg 40200

gatgcggacc acgtggcggg cgcggccagc atggccgcga cgccgatggt gagcccggcg 40260

ctgaccagcg aactacgacg gggcctgacc agccgcgcca gcgcgagcgc gacgacggcg 40320

accacgccga gcgcgaccgc gccccacatc gcccacggca gaaaagtggg gtagaaggac 40380

cacaaccaga cggcccaggc gagttcggcc acgatcgcga gcggaaatat ccacgccatc 40440

ctgcctccgc tccgatacgc ccgccagaac attacaatgc cgattccgga caaagcggct 40500

accggcggcg cgagtacggc cacatatgcg ctgtgcggga tgacaaagac cgcgctgtag 40560

ggcagggcga aggtgagaag ccacacgccc cacatcacca ttccgccgcg tgccgggtcg 40620

gtacgctcgg cccggcgcca ccaccacagc ccgcacagca gagccatcag cgcgagcgga 40680

tacagccaac cggacgcgac gccgaggcgg ccgccgaaca gcttgcccca gccgccccca 40740

tgctcgatgc ctatctcggg gatgaccatg ccagggcgtg gccggggcag ctgcgtcgat 40800

ctcttcggag gcgccgggcc gatcaccgag cccatgtagt tgggcggcag ggcgccgggc 40860

agattgatgc ccaggcgtcc gagaccgttg tacccgaaca ccatcgcggc ggcgctgctg 40920

ttcgtggtgc cgctgatgta gggccggtcg gcggccggag tgacgtggta gagcgtgatc 40980

cacgacagcg acaccacgag cgtcaccact ccggcgatcc ccaggtgctg cagccgacgg 41040

cgcagtccga tcggcgcgct caggagataa ccgatcgcca gggcgggcag gatcatccac 41100

gcctgcaaca tcttcgcctg gaaacccagc ccgacccaga cgccggccca gaccagcgac 41160

cgcagccgtc cttccagcac ggcccgctga taggagtcga cggcgagcac caggcacatg 41220

accagcgccc catcggccat gctgtgcccg aacatggacg cggccacggg ggtgatggtg 41280

aagacggcgg cggcgagcag acctggcacc acgcccgccc atcgccgcac gatccggtac 41340

atcaccagca ccgagatcac gccctcgatc acctgcggca aggcaagggc ccaggcgtgg 41400

aagccgaaga tcttgaccga gatggcctgc ggcacgaagg ccccggcgag cttgtcgagc 41460

gtgtaggtcg cctgcacgtc gacggtgccg tacaggaacg ccttccagtt ctcggacatg 41520

ctcttgacgg cgtccgagta tctcggtgcg tagtcgacca gcggcaggtt ccaggcgtag 41580

agcaccgctg ccgtggccgc gatgcagagc agcgccggcc gggcccacca cggctggccg 41640

ggcggcgagc gccacaccgc ccagcggggg aatctgccgg cgggtgcggg gtcccggcac 41700

gcggacggcg gagtcatggt gatgtgcgac atgaggaact cccaggcgtt tccttcggca 41760

gttccctgcc tttactcggc tgcgtagcga atgaccggcc aggtggtctc gttatatccg 41820

ccgtccggtg cggcgttcct ggcatgctca tcgagtttgg cgaacaggtt cttgttcggc 41880

ccgtcgagca ccgacaactg cgtcgcgtag tgcttcatcg cctggaattt ccgcgtccgc 41940

gcctcctcat cgacgaaact cagctccgga gagccgagcc ggaggccgtc aggcaattca 42000

gccaggtcct gggaatacgc cgcgtacggg agatcctgcc agagtcgcag cggaatacct 42060

cgctcgcgtg cggcgagcag cgtggcatcc cgcgtggcct tgtggtcggg gtgtttcccg 42120
atggccacac aggtgagaac gagcgtcgga tcgcactccg cgatcatgga ctcgatgtcc 42180
tccctgatcg cggcgaccag gtcgtggttg ttcgccggcg actgctggcg gaccatcgag 42240
ccttcgttgt ggtgcagcag ccactggcca tccggtgacc ttcgatagat ggcatcgaga 42300
aaacggccat gccgatggcc cgcaccgagc tgatcgaggg cggcgatgtc ctcatttcgt 42360
cggcgcagcg gcgcgtcctc ggtcggcgac agaccccagc gtgcgtggaa tcgctccgcc 42420
gccggggaat aagggggcgc cgcgctgccg gcgaacaccg tgaagacggt tacttttcca 42480
ccgtcctgct ccgcttgggc gaggctggct ccgacggaga ggacggcatc atccaaatga 42540
ggggagattg ctaatatccg ggttcggtcg gcgtcctgca acatggttgt cagtctggtg 42600
tcggccctgc cccgttgcaa taaagcggaa ctggacggga cctcgcacgc gtggagaatt 42660
tccgggcggg ccgggcacca cgatgagtca cccggtcacg tcgcagtcac acgttctctg 42720
accagcctgt caccgtctgc tgcgtacaac tggtgtcaac gccgacaccg ggcaggagaa 42780
gatgagtggg aagagcgcag ccgcacgacc tcgcctcggt cgcatcgaac gacgccttcg 42840
ccgggctcac gtcggcgacc gcggcctcta ggatggcgcc agccggcgca gcgcgcgccg 42900
catgaggggg aagtggatct ctaccgagag gccaccctgc gggcgcgcgt gcgcggtcaa 42960
cgtggcgtcg tgggcgacgg cgatggcgcg gacgatggac agcccgaggc cgtggtggtc 43020
gtcggcgcgg gtgcggtcga gccgctggaa gggctcgaag aggcggtgga cctgctcggg 43080
gggcaccacc gggccggtgt tggcgatgga gacgacggcc ttcccggcct cggcccgggt 43140
ggagagctcc acctggccgc caggcacgtt gtagcgcatg gcgttgtcca gaaggttggt 43200
gatgagtcgt tcgaccagtg ccgggtcgcc cgtggtgggg gcatgagcga tcccggtcac 43260
caatcggggg tgtgggcaac cggcccggag ggacttcccg tcggcccagg aatcggttgc 43320
cgcgccggcc gtggggctgt tcccgtcagc cctccgatcg gcactcacgc cggccctggg 43380
gttgttcccg tcagccccgg ggccggtccc ctccatcgtg cggatcgtgt gctcggcgat 43440
ctccgccaga tccaggggct cgcggtgatc gaggccgccc tcgctcttgg ccagcgtcag 43500
cagcgattcc agcaggcgcc cctgctgccg gctgaggtcg agcagccgct ccatgatcga 43560
tcgcatggac ggggtgtcgg cgtcccgatg caggaggctc tcctccagca acgcgtgctc 43620
cagggtgagc ggggtgcgca gctcgtgggc cgcgttggcg acgaagcgct tctgcgcgtc 43680
gagggcgctg tggagacgtt ccagcagctc gtccaccgtg tcggcgaggt tgcgcagctc 43740
gtcgcgcggg ccgggcaggg cgagccgctc gtggacgttg cgggcggaga tccgcttgag 43800
cgtggtgttc atcgtccgta gcgggcgcag catcctgcct gccaccagcc agccgagcaa 43860
gaacgagatg accgtcatca gggccagcgc gatcagcgat tggaacagca ggttctccag 43920
aatggccgcc tgctgctgcc gcgcgaaggc gcggaacctg ccgccagggt caccgtccac 43980

cagcacgaag ggtctggagc cgcggaagag caggtaggtg atggcgagca ggaccacccc 44040
tgaggcggcg aacaacgcgc cgtagacgag cgtgaggcgc agccgtacgc tgcggaaggc 44100
tgttgtcagg cgtcggagac gatgggccac tgttccgatg gtatgagacc aggaccggcc 44160
ggaggagccc acggcgccga cgatcgcggc cccgggccga ggccgagatg ccgcgagtcg 44220
ggcgcctacc gagtcagcgc accgcgcacc gctgccgtga cctcgggcgc cagggtgcgg 44280
gcgaaggtgg tggtgaggtg gctgcggtcg gagtaggcga tcaggccgcc gatgacgggg 44340
ccgcaccgct cgccgcacac caggtgatcg acactcgcca cggagacgag gccggtgtcg 44400
tcggcgcggg cggcggcggc gagcggatcc ggccggagca cgacgccggc cgggccgccg 44460
caggagtcca gatcgtccgg gtgcttggcg atgcagtggg gcacgctgtc cggcatggcc 44520
ggggtgtcac gcaggacgag caccggaagg ccggcgccgg tgaaggcccg gagcgtgtcg 44580
cggtaggccc gctcggccgc ggcctgctgg ccggctggcg agacgccggc gagcggcaca 44640
tgcgtacggt tggacatgat caccaggtcg tagccgccgt tcacgatgga cccgaccgcc 44700
cacttgttga tcttctggca gttctccgag acccccgcgc cttcgaggac gagcggctga 44760
tcgacggtgt agcacgccag ctgtacgtag gtggtgagct gccagcgctc gctccacagc 44820
gccttctcca gggccgggac ccagtgtccc gcgtgggagt tcccgaccag ggcgatgcgc 44880
ctgccggcgg cgtcaggtcc gtacgtgcac gtgttccggg cgatgaacgg ttccttgttc 44940
acgcacccgt ccgcgtacac ggcgggcttg tccttcaacg cgacctgagg aggcatcagc 45000
aggcccagat cctggcacgc cgggtcgcgc acgacgccgg cacccaggca tgaccctgcc 45060
cgcgaggccg cggcctcgaa cgcggcactc tccgtacgct cggcggcgtc ggcgtaggcg 45120
acgacgcccg ctcccgctcc tgcgacgacc accacgcacg acgcgagcat cgcgaacgtg 45180
agcctgcggc tgcggaccag gaccgggtgc cagcgcagcc ggtcctcgac gaggtactgc 45240
gagagcgcgg cgaggaccag ggtcagcgcg atcacgccca cggactcgat cacggtcagc 45300
gagcggccca gcgcgtacgg gaggatcatg atcggcggcc aatgccacag gtacaccgcg 45360
taggaggcgt tgccgagcca ctggaccggc cgccacgcca gcgcccgccc gggtccgccg 45420
cgcagaccgt ccgcggccgc tgcgatcacc aggcaggccc ccactgtcgg caccagggcg 45480
gcggctccgg ggaaggccgt ctcggcgtcg aaccggacca cggcccaccc gatcatgccc 45540
aggccggccc acgcgagccc ggcccggacc gcccgcgcgc gcggcatcgc gcgtacggtg 45600
agaaccgcgg cgagcagacc gccgagcgcg agctcccaga agcgggtcgt cgacacgaag 45660
tacgcggcgg ccggatcggt cgccgtcttc tgcaccgacc aggcgaggga cgcggccacg 45720
accgcacccg tgaccaccac cgcgctccac ctcgtgaagt tctccggagg gcgacgcccc 45780
cgtgccaccc gggcggccag ccaggccgcc gaccccagca gcagcggcca gccgaggtag 45840

aactgttcct cgatggacag cgaccagtag tgctgcgcgg gccagtccgg ctggtcgacg 45900

tcgaggtagt tcgcctgcgt gagcgcgagt ctcaggttct ccacgtacac cgtggcggcg 45960
atcacctcac gcgccgccgt ccccagcacc gtgagcggca gccagaccac cgacgcggcg 46020

agcgtgacca ggagcgcgag gctcgcggcc gggatgaggc ggcggacgcg gcgtgcccag 46080

aagtccagca gtctcccgcc gccgtgtccc ggctgacgca gcaggtggct cgtgatgagg 46140

tacccggaga tgacgaagaa gacgtctacc cccacgtacc cgccggtcgg cccgccgggc 46200

cacaggtgga acgcgaccac cgccgccacc gcgatcgccc ggaggccctg aatgtccgtg 46260

cgggactcgg agctccggcc gccggcgtgc tcgcttcgcg gcgcacacga cggggcgtgc 46320

ggcgtcagcc cgcatgcgca ggtcggaagg gacatctgtt cggtgggtgc cgggcgctcc 46380

atggcaactc ccgcgtcatc gaggtgctgc gcagccctcg aaggtcgcac ccgcggacga 46440

gagcctgctt gatcgcaagc gtgctcaacg gactcgatgt ctacaagccg gtccaggtga 46500

acgcttggtc accaccaccg gtgaacgcgt ccaagcgcgc gaactgttca gttggaccca 46560

ctcgtggaca tcggctccgc tcagcacgat tgaggtcgct gacttgcgtg cgcgtgtgag 46620

aggagtcccg catggccata gtgtcgccgt tcggaggttt gctgaagggc gacggagagg 46680

atgatcccgc gccgtccagg atccgcccgg ggacgttgcg acgagtgctc ggatacttcc 46740

gcccgcacgt cggcaaggtg gcgctcttcg ttctcgtcac cgcattggat tcgatcttcg 46800

tcgtcgcgtc tccgttgatg ttgaaggacc tggtggacaa gggggttctg gggaacgatc 46860

tggagctcgt catcctgctg gcgtgcctgg ccgccggctt cgccgtgatg agcacgctgt 46920

tgcagctcgt gtcggcctac atctccggcc ggatcgggca gggggtcagt tacgacctgc 46980

gggttcaggc ccttgaccac gtccagcggc tgccgatcgc gttcttcacc cggacccaga 47040

cgggcgtgct ggtcggccgg ctgcacacgg agctggtcat gacgcagatg gcgttcaccc 47100

agatgctgac ggccgccgcc agcgcggtca cggtcctgct ggtgctggcc gagctgttct 47160

acctgtcgtg gatcgtcgcc ctcctcacgc tggtgctgat cccggtgttc ctggtgccct 47220

ggtcttacgt gggacggcgg atgcagcgct acaccagagg gctgatggag gagaacgccg 47280

gcctggccgg gctgctgcag gagcggttca acgtccaggg ggcgatgctc tccaagctct 47340

tcggccgtcc ggccgaggag atggccgagt acgagagcag ggccggccgg atccgcgggc 47400

tcgccgtgag cgtcaccctc tacggccgga tggcccccgc catcttcgcg ctgatggccg 47460

cgctcgccac ggcgctcgtc tacggggtcg gcggcgggct cgtgctctcg caggcgttcc 47520

agctcggcac gctggtcgcc ctggccaccc tgctcgggcg gctgttcggg ccgatcaccc 47580

agctggccag cattcaggag aacgcgctca cggtcctggt gagcttcgag cggatcttcg 47640

agctgctcga tctgaagccg ctgatcgagg aacgccccga cgcggtcgcg ctcaaggccg 47700

gcaaggcctc ggacgtccag ttcgagaacg tgtcgttccg ctaccccagc gcggacgagg 47760

tctcgctgcc gtcgctggaa cagaacgtgc gcaccgggca ggagcgtggt gaagcgacgc 47820
cggaggtgct gcgcgacgtg agcctgcacg tgccggccgg caccctcacc gcgctcgtgg 47880
gcccgtccgg cgccgggaag agcaccctca cgcacctggt gtcccggctg tacgacccga 47940
cctccggaac cgttcgcgtc ggcggacacg acctgcggga cctcaccttc gactcgctgc 48000
gcgaaacggt gggggtggtc agccaggaca cctacctctt ccatgacacg attcgggcga 48060
accttctcta cgcccgcccc gacgccaccg aggacgagct ggtcgaggcg tgccgagggg 48120
cgcagatctg ggacctgatc gcatccctgc cacgcgggct cgacaccgtc gtgggtgatc 48180
gcggttatcg cctgtcaggc ggggagaagc aacggctggc gatcgcccgg ctgctgctga 48240
aggcaccctc ggtcgtcgtt ctcgacgagg ccaccgccca cctggactcg gagtcggagg 48300
ccgccgtcca gcgggcactg acgacagccc tgcgcagccg tacctccctg gtgatcgccc 48360
accggttgtc cacgatccgc gaggccgacc acattctcgt gatcgacgac gggagggtca 48420
gggagcgcgg gacgcacgag gagttgctgg cggaaggcgg tctctacgcc gacctgtacc 48480
acacgcagtt cgccaagtca ggcgtcaacg ggacccggcc gggacagggc gacggggcgg 48540
agcccgtgca agaggtggtc ggtggagggg aacgatgagc gccggaacgc gggccacacc 48600
gacgacggtg ctggacctct tcgcccgcca ggtgggccgg gcacccgatg cggtggctct 48660
ggtcgacggg gaccgggtcc tgacctaccg gcggctggac gagctcgccg gagcgctgtc 48720
cgggcgcctg atcggccggg gtgtcggccg gggtgatcgc gtcgcggtga tgatggaccg 48780
ctcggcggac ctggtggtga cgctgctcgc cgtgtggcag gcgggggcgg cctacgtgcc 48840
ggtggacgcc gcccttcccg cccggcgggt ggcgttcatg gtggcggact ccggagcctg 48900
cctgatggtg tgctcggagg cgacgcgcga tgcggtaccg caaggggtcg agtcgatcgc 48960
gttgaccggc gagggcggat gcggcacgtc ggcggtcacg gtggacccgg gggatctggc 49020
gtacgtgatg tacacgtccg gctcgacggg caccccgaag ggggtggccg tcccgcatcg 49080
gagcgtcgcg gagctgacgg gaaaccccgg ctggggggtg gagcccggcg aggcggtgct 49140
catgcacgcg ccctacacct tcgacgcctc cctgttcgag atctgggtgc cgctcgtgtc 49200
gggcgcccgg gtggtgatcg ccgcaccggg tgcggtggac gcccggcgcc tgcgcgaggc 49260
ggtcgccgcc ggggtgacga gggtgcacct gaccgcgggc agcttccgcg cggtggcgga 49320
ggagtcgccg gagtcgttcg cgcacttccg tgaggtgctg accggtggtg acgtggtgcc 49380
cgcgtacgca gtgcagaagg tgcgggcggc ctgccctcac gtgcggatcc ggcatctgta 49440
cggcccgacg gagacgaccc tgtgcgcgac gtggcagctg ctggagccgg gtgacgtcgt 49500
ggggcccgtc ctgccgatcg gccgcccgct gccgggccgc cgggcctggg tgctcgacgc 49560
gtcattgcgg ccggtggagc ccggggtggt cggtgacctg tacctttccg gcgccggtct 49620
ggcggacggc tacctggacc gggcggggct gacggcggaa cggttcgtgg cggatccgtc 49680

cgcggcgggg aggcggatgt atcggacggg ggatctggct cagtggaccg cggacggtga 49740

gctgctgttc gcgggccggg ccgacgacca ggtgaaggtc cgcggattcc ggatcgagcc 49800
gggcgaggtc gaggccgcgc tgaccgctca gccgcacgtc cgcgaggccg tggtggtggc 49860

gatcgacggg cgcctgatcg gttacgtggt ggcggacggg gacgtggatc ccgtactgat 49920

gcgccggcgg ctggcggcgt cgctgccgga gtacatgatc ccggccgctc tggtcacgct 49980

ggacgcgttg ccgctgaccg gcagcggcaa ggtggacagg agggcgctgc ccgagcccga 50040

tttcgcgtcg gccgcgccgc gccgcgaacc cggcaccgag ccggagcgcg tcctgtgcga 50100

cctgttcgcg gagcttctgc aaccggaggg aaggggggta ggggtcgatg acggtttcgt 50160

cgagctgggc ggggactcga tcgtcgcgat ccggctggca gcacgtgcgt ccagggtggg 50220

gctgctggtg acgcccgccc agatcttcaa ggagaagact ccggcacggc tggcagccgt 50280

cgcgggtgcc gtaccggccg gcagacccgc cgacggcccg ctgatcaccc tcacggcgga 50340

ggaggaggcg gagctggcga ccgccgtccc gggggccgag gaggtctggc cactcgcacc 50400

gctccaggaa gggctctact tccaggccac cctcgacgac gagggtcacg acatctacca 50460

ggcgcaatgg atcctggagc tggcggggcc gctggacgcc gcccggctgc gggcctcgtg 50520

ggaagcggtc ttcgcccggc accccgagct tcgcgtgagc ttccaccggc gcgcgtcagg 50580

cacgatgctg caggtcgtcg ccgggcacgt cgtcctgccg tggcgagagg tggatctggc 50640

ggatgcgggc gatatcgacg cggccgtggc ggccctgatc agtgaggaac aggagcagcg 50700

gttcgacctc gccaaggcac cgctgttccg gctggtgctg gtccgtcacg gcgaggaccg 50760

gcaccgcctg ctggtcgtcc atcaccacat cctgaccgat ggctggtcgg tggcggtcat 50820

cctcaacgag gtggctgagg cgtacacgaa cggcggccgt ctcccggacc gcacgggcgc 50880

ggcctcctac cgggactacc tggcctggct ggaccggcag gacaaggacg ccgcacgtgc 50940

cgcctggcag gcggagctgt ccggcctcga agggcccgcg ccgatcgcga aggccgccac 51000

cacgaccggc gccgggacgg gctacgaata tcgcatcgcc ttcctgaccc ctgacctcca 51060

cacgcggctg acggagctgg cccgcgacca cgggctgacg ctgaacaccc tggcacaggg 51120

cgcatgggcg atggtgctgg cgcggctcgc gcggcgcact gacgtggtct tcggcaccac 51180

ggtcgcctgc cgtcccgccg agctccccga ggtggagtcg gtgccgggtc tcatgatgaa 51240

cacggttccg gtccgggtgc cgctgcaggg cgcgcaatcg gtcgtggacc tgctcaccgg 51300

cctgcaggaa cggcaggcgg ccttgctgcc gcaccagcat ctggggctga cggagatcca 51360

gcgggcggca ggacctggcg cgacgttcga cacgctgctg gtcttcgaga actacccgcg 51420

ggacttcgcc ggccagttca cctacctggg cacgatcgag gggacccact acccgctgac 51480

cctcggcatc atcccggggg atcacttcag gatccagctc gtctaccggc gcgggcaggt 51540

cggggagagc gtcgccgagt cgatcctggg atggttcacc ggcgctctca tgacgatggc 51600

cgctgatccg cacgggccgg tgggccggat cggtgtgggt gaggcccggg ccggcggctc 51660
ggaccgggcg atggcggcgg gggagccgct gccggtgctg ctacggcggg tggtgaagga 51720
ccggccggac gaggtggcgg tggtggacgg cgacggtgag ctgtcgttcg gggaattgtg 51780
ggaacgggcg acggcgctgg cggccgagct gagggctcac gggatcgggc cggagagccg 51840
ggtggccgtg atggtgggca ggtcggcgtg gtgggcggtc ggggtgctgg gcgtctgctt 51900
ggcgggcggc gcgttcatgc cggtggatcc ggcgtatccg gctgagcgcg tcaggtggat 51960
cctggccgac tccgacccac ggctggtgct gtgcgcgggg acgacgcggg aggcggtgcc 52020
ggaggagttc gcagaccggc tggtggtggt cgacgagctg gacctcgcgg ggagcgacga 52080
tgcgggcttg ccacgggtga gcccggatga cgcggcttat gtgatctata cgtcgggatc 52140
gacggggact cccaaggggg tcgtcgtctc gcacgcgggc ctcgggaatc tggcgatggc 52200
gcagatcgac cggttcgccg tgtcgccgtc gtcgcgagtc ctgcagttcg cggcgctggg 52260
cttcgacgcg atggtgtcgg agatgttgat ggcgctgttg tcgggggcga ggctggtgat 52320
ggcgccggag ccggccctgc caccgcgggt gtcgctggcc gaggcgttgc ggcggtggga 52380
ggtcacgcac gtcacggttc cgccgtcggt gctggccacc gccgatgcgc tgccggccgg 52440
gctggagacg gtggtggtgg cgggggaggc ctgcccgccg ggcctggccg aacgctggtc 52500
ggcgggacgg cggctggtca acgcgtacgg gccgaccgag gccacggtct gcgcggcgat 52560
gagcaggccg ttgacgggca gccgggaggt ggtcccgatc gggacaccca tcgccggcgg 52620
ccgttgctac gtgctggacg cgttcctgcg gccgttgccg ccggggatca ccggtgagct 52680
gtacgtggcc gggatcgggt tggcgcgcgg ctatctgggt cgtgcgtcgt tgacggctga 52740
gcggttcgtg gcggatccgt tcgtggctgg tgagcggatg tatcggacgg gggatctggc 52800
gtattggacg ggtgagggcg agctggtgtt cgccgggcgg gatgacgacc aggtgaagat 52860
ccgtgggtat cgggtggagc cgggtgaggt ggaggcggtg ctggcggggc agccgggggt 52920
ggatcaggcg gtggtggtgg cgcgtgaggg gcggttgctg ggttatgtcg tctccggtgg 52980
tggggtggat ccggtgcggt tgcgtgaggg ggtcgcgcgg gtgttgccgg agtacatggt 53040
gccggcggcg gtggtggtgc tgggtgcggt gccggtgacg gcgaacggga aggtggatcg 53100
ggaggcgttg ccggatccgg gcttcggcgg gcgggtttcg ggccgggagc cgcgtacgga 53160
ggtcgagcgg gcgttgtgcg ggctcttcgc cgaggtgctc gggctgccgg gggtgacggc 53220
ggtggggccg gacgacagct tcttcgagct gggcggggac tcgatcactt cgatgcagct 53280
ggcgtcgcgg gctcgccgcg aggggatgct cttcggcgcg cgggaggtgt tcgagcgcaa 53340
gacgcctgcg gggctcgcgg cgatcgtcga tgtgggcggc gagcttgcgg caggtccggc 53400
cgacggcgtg ggggagatcg cgtggacgcc gatcatgcgg gcgctcgggg acgggatcgt 53460

ggggtcgcgg ttcgcccagt gggtggtgct gggtgcgccg ccggacctac gggcggacgt 53520

ggtggccgcg ggattggcgg cggtggtgga cacgcacgac gtgttgcggc tgcgggtcgt 53580

cgatgaccgg gcgggccgcc ggctggcagt gggcgagcgc gggtcggtgg acacggccgg 53640
gctggtcacg cggctcgagt gcggcggccg tccgccggac gaggtcgtgg agcgcgcggt 53700

gcgggaggcc gtggggcggt tggacccggt ggcgggtgtg atggcgcagg cggtctgggt 53760

ggatgcgggg ccggcgcgga cggggcggtt ggtcgtcgtg gtgcatcatc tggcggtcga 53820

cgggatgtcg tggcggatcc tggtgcccga cctgcggctg gcgtgtgagg cggtggccga 53880

ggggcgggat ccggtgctgg agccggtgtg ggggtcgttc cggcgctggg cggctctgct 53940

ggaggagtcg gcgctctcgc gggagcgggt cggggagctg cacacgtggc ggacgatcgt 54000

cgatcaggag gatcggccgg tcggccggcg gcggctgagc gcaggggatg cggccggggg 54060

cgtgcgttca cggtcgtggg tgatgtcggg ggatgaggcg tcgctcctgg tggggaaggt 54120

tccggtggcg ttccactgcg gggtccacga ggtcctgctg gcgggcctgg cgggagcggt 54180

ggcgcgctgg cacggtgacg acggggtcct ggtggatgtg gaaggccacg ggcgtcatcc 54240

ggccgagggg atggatctgt ccaggacggt gggctggttc accagcatgc atccggtgcg 54300

cctggatgtg gcggggatcg agctggcggc ggtgccggcc ggtggccgtg cggccgggca 54360

gttgctgaag gcggtcaagg agcagtcgcg ggcggcgccc ggcgacgggc tcggttacgg 54420

gttgctgcgc catctcaatc ccgagacggg ccccgttctg gcggccctgc cgtcaccgca 54480

gatcgggttc aactacatgg gccggttcgt caccgtcgac cagggcggtg cgcggccgtg 54540

gcagccggtc ggggggatcg gcggttcgct ggaccccggc atgggcctgc cgcatgcgct 54600

ggaggtcaat gcgatcgtcc acgacaggct ggcgggcccg gagctggtgc tcacggtgga 54660

ctggcgggac gacctgctgg aggagaccga catcgaacga ctgtgccagg tgtggctgga 54720

catgttgtcc ggattgtccc gccaagcgga ggatccttcc gcaggcggac acaccgcgtc 54780

cgacttcgcc ctactcgacc tcgaccagga cgagatcgag ggcttcgaag ccatagcagc 54840

ggaactctct ggaggccaga catcgtgaac acgccgagca cacccgccgg atcggcgctt 54900

gaggaagtct ggccgctgtc accgatgcag gaggggatcc tctatcacgc cgcactcgat 54960

gaggcccctg acctctacct catccagcag tcgcagatca tcgaaggacc cttggacacc 55020

gagcggttcc gcctggcttg ggagagcctc ctcaaccggc atgcggcgct tcgcgcgtgc 55080

ttccaccggc ggaagtccgg tgagtcggtc cagctcatcc cccgtaaggt gccgctcccg 55140

tggtccgagc gcgacctgtc cggcctgtcc gaggaggacg cgctggccga ggcgagcgtg 55200

atcgcggaga aggagcgcgc cacgagattc gacccggcca agcctccgct gctgcggcag 55260

gtgctgatcc ggttcggtcc ggacaagcac tgtctggtga cgacgagcca tcacctggtc 55320

atggacgggt ggtcgcgggc gatcctcgag tcggagctcc tcgagctcta cgccgcgggt 55380

ggcgccgagc cggggctgcg gcccgccggc tcctaccggg actatctggc ctggctggag 55440

cggcaggaca aggaggccgc ccgcgcggca tggcgtgcgg agctggcggg cgccgaccgt 55500

tcgacactcg gcatccccga agcgtccagg aagacccagg ggcagcgggt gcgggaggtg 55560

ctcggctacg cgccggactt cacctccgct ctggtggact tcgcccgccg ccatgggctg 55620

acgctgaaca cgctggtgca gggggcgtgg gcgttggtgc tggcccggct cacgcgccgt 55680

cgtgacgtgg tgttcggcgc ggtggtctcg ggacgtccgg cggaggtgcc cggcgtggag 55740

caggccgtcg ggctgttcat caacaccgtg ccggtgcgcg tccggttgga cggcgggcag 55800

ccggtcatcc agctgctgac ggagctgcag gagcggcagt ccacgctcat ctcgcatcag 55860

catctcgggc tgcaggagat ccagaagctc tccggggtga gcttcgacac cgtcgtgtcg 55920

ttcgagaact acgtcgatcc gggggcgggt ccgggctccg atcgcgagct gcgcctgaga 55980

ctgaaggagt ttcaccagtc ggcgccgtac gcgctcctcc tcggcatcat gccaggtgag 56040

agcctccaga ccgacgtgga gtaccggccc gagctgctcg acgcccgcgt cgccaaggag 56100

gccctccacg ggctcgcccg cgtcctcgag cggatgatcg ccgagccgga gaccgcagtg 56160

ggccgcctgg acgtggtcgg tgacgcgggg cgcgagctgg tggtcgagcg gtggaacgag 56220

acgggcgacg cgatcggtgc gccgtccgcg gtggacctgt tccggcgcca ggttgcacgg 56280

gcacccgccg cgacggcggt gacggccggg gacctggcct ggtcgtacgc ggagctcgac 56340

gagcggtccg gccggctggc gcgggcactg acggaacgcg gcgtgcgacg cggcgaccgg 56400

gtgggcgtgg tgctggggcg gtcggcagag gtgctggcag cctggctcgg agtgtggaag 56460

gcaggcgcgg cgttcgtgcc ggtcgacccg gactacccgg cggaccgggt ggcgttcatg 56520

ctggccgact ccgccgtcgc gatggtggtg tgccaggagg cgacctcggg tgtggtgccc 56580

ccgggctacc agcagctcct ggtgaacgac gccgacgacg gcgaggccgc cctggtcccg 56640

atcggggcgg acgatctcgc ctacgtgatg tacacctccg gatcgaccgg gaccccgaag 56700

ggcgtggcga tcccgcacgg cggcgtggcg gcgctggcgg gagatccggg atggggcgtc 56760

ggacccggcg acgcggtgct gatgcacgcc ccgcacacct tcgacgcgtc gttgtacgac 56820

gtgtgggtgc cgctcgtctc cggcgcgcgg gtcatgatca ccgagccggg ggtcgtcgac 56880

gcggagcggc tcgccgggca tgtggccgac ggcctcaccg cggtcaactt caccgcgggg 56940

cacttccgcg cgctggcgca ggagtcgccg gagtcgttct ccgggctgcg cgaggtggcg 57000

gcgggtggcg acgtggtgcc gctcgatgtg gtggagcggg tacggcgggc gtgcccgcgg 57060

ctccgggtct ggcacaccta cggcccgacc gagaccacgc tgtgcgcgac gtggaaggcg 57120

atcgagcccg gtgacgaggt ggggccggtg ctgcccatcg gccgggcact gccgggccgg 57180

cggctgtacg tgctggacgc gttcctgcgg ccgttgccgc cgggcatcgc gggtgatctc 57240

tacctcgcag gcgccggagt ggcccacggc tatctgggtc gtgcgtcgtt gacggctgag 57300

cggttcgtgg cggatccgtt cgtggctggt gagcggatgt atcggacggg ggatctggcg 57360

tattggacgg gtgagggcga gctggtgttc gccgggcggg atgacgacca ggtgaagatc 57420

cgtgggtatc gggtggagcc gggtgaggtg gaggcggtgc tggcggggca gccgggggtg 57480
gatcaggcgg tggtggtggc gcgtgagggg cggttgctgg gttatgtcgt ctccggtggt 57540

ggggtggatc cggtgcggtt gcgtgagggg gtcgcgcggg tgttgccgga gtacatggtg 57600

ccggcggcgg tggtggtgct gggtgcggtg ccggtgacgg cgaacgggaa ggtggatcgg 57660

gaggcgttgc cggatccggg cttcggcggg cgggtttcgg gccgggagcc gcgtacggag 57720

gtcgagcggg cgttgtgcgg gctcttcgcc gaggtgctcg ggctgccggg ggtgacggcg 57780

gtggggccgg acgacagctt cttcgagctg ggcggggact ccatccattc ggtgaagctg 57840

gcagcgcggg ccacgcgtgc cggcatgccc ttcaccgtgg tcgaggtgtt cgagcacaag 57900

acgcctgcgg ggctcgcgac gatcgtcgac gtgggcggcg agcccgcggc aggtccggct 57960

gatcccccat cggactccga cctgctcggc ctggcgcagg acgagatagc ggagttcgag 58020

gccgaattcg acgacgaacg tcattctctg cgctgatcga aagcgggcgc cgcgcacggt 58080

gtgccggcag cctgcgagtt gtccaacatc ctgtcgtgcc aatgacgtat gcccatgagt 58140

aggttggctc aatgataagc aaagcaatgc atggaccgat tcggcccgcc cgcgcggata 58200

ccctgctggc ctcggtaggc gagcgaggca ttctgtgtga cttttacgac gagaacgcct 58260

cggaaatctt ccgtgatttg gaggcggacg cgggcggcac ggaagaagcc cacgggttcg 58320

cggcgctcgt ccgcccggag tcgggggcga tcctggagct cggggccgga acaggcaggc 58380

tgacgattcc gctcctggag ctcggctggg aggtgaccgc cctcgaactg tcgaccgcga 58440

tgctcaccac cctgcggacg cggctggcgg acgcgccggc ggacctccgg gatcggtgca 58500

ccctcgttca cgcggacatg accgccttca aactgggaga acgcttcgga acggcgattc 58560

tcagcccgtc cacgatcgac ctcctggacg atgccgacag accagggctg tactcgtcgg 58620

tccgtgagca tctgcggccc ggcgggagat tcctgctcgg catggccaac cccgacgcgt 58680

ccggcaggca ggagccgctg gagcgcaccc aggagttcac gggcaggagc ggccgccgat 58740

acgtgctgca cgccaaggtc tacccgtcgg aggagatccg cgacgtgacc attcatcctg 58800

cggatgaatc ggcggacccc ttcgtcatct gcgtcaatcg cttcagagtc atcaccccgg 58860

atcagatagc acgagagctg gagcaagccg gattcgacgt ggtcgcgcgg accccactgc 58920

ccggggtgcg taatcacgaa ctggtgctgg aagcgcaatg gggcagcgtg gaagacgcgc 58980

attagagccc tccggggaaa gcgcttgtgt acttttctgc agtcattcga cagtgaggaa 59040

cagaaatgag tgaggagctc ctcttcctcc ggcccgacac cattatcgaa ccgctggcca 59100

accggttcta cgcctcgatg tacgcgacgg ctcccgtcac ggccgccatg aatctcgcct 59160

tccgtaacct gccgatgctg gagtcctacc tcgcatcccc ggaatggcat ttcgcagccg 59220

ctcgcgatcc gaagttccgc ggcggattct tcgtcaacat cgaggagcag cggaagaacg 59280
aggtcgaggc gctgctcgct gcgatccggc gcgacagcgc ggacgtgctc cggttcgccg 59340
aggcgatcgc ggaggccgag aagatcatcc gcgaggaagc gaccggatac gatctcaggc 59400
cgctctaccc gaagctgcct cccgagctgt cgggtctggt ggagatcgcc tatgacaccg 59460
gcaacgcggc ctcgctgcac ttcctggagc cgctcatcta caagagcaag gcctacgccg 59520
aggactgcca gtccgttcag ctctccgtgg agaccgggat cgagcggccg ttcgtgatga 59580
gcaccccgcg actgccctca cccgacgtgc tcgagctgaa catcccgttc cggcatccgg 59640
gtctggagga gctcttcctg tccaggatcc ggcccaccac cctggccgcc ctccgcgagg 59700
cgctggagct cggcgacgcg gaagcggcgc ggctcgccga cctgctggtc ccggagccct 59760
cgctcgcctc cgaccgccat gtcgcggccg gagcccggat ccgctactgg gggcacgcct 59820
gcctgctcat gcagacgccc gacgtggcca tcatgacgga cccgttcatc agcgcggata 59880
ccgacgcgac cggccgctac acctacaacg acctgcctga ccgcctcgac tacgtcctca 59940
tcacgcacgg gcattccgac catctggtgc ccgagacgct gcttcaactg cgcggccggg 60000
tgggcacgtt cgtcgtgccg cgaacctcgc gcggcaacct gtgcgatcct tcgctggcgc 60060
tctatctcag aagcttcggg ctgcccgcga tcgaggtgga cgatttcgat gagatcgagt 60120
tccccggcgg gaagatcgtc tccaccccgt tcttcggcga gcacgccgat ctcgacatcc 60180
gggccaagtc gacgtattgg atcaacctcg gtggcaagtc gatctgggtg ggcgcggact 60240
cctcaggcct cgatccggtt ctctaccgcc atatccgccg gcatctcggc gcggtcaaca 60300
tcgccttcct cgggatggaa tgcgatggcg cgccgctgaa ctggcagtac cagccgttca 60360
tcaccaaggc gttgccgaag aagatgagcg acagccgcaa gatgtccggc tccaacgcgg 60420
agcaggcagg tgcgatcgtc accgagctgg gcgccgagga ggcgtacatc tacgccatgg 60480
gggaggagag ctggctgggg catgtcatgg ccaccagcta caacgaggac tcctaccagc 60540
tccagcagat cgccgagttc gaggcatggt gttcccgcaa gggtgtgaag gccgctcatc 60600
tgctcgacca gcatgagtgg cactggtcgt catccaggtg atcgcggtgg cccgccggtc 60660
ggccttcgct caggcgggca gggccgcggt cgcaagcagc tgccgaggcc gtgctcgccg 60720
aggccgtgct cgccgaggcc gtgcccgtgc tcgcccaggc cgtgcccgtg ctcgccgacg 60780
ccgtgctcgt cgaggccggt gccagagggc gcgtcaccgg cctctcagcg caaccggccg 60840
cgtgaaccgc ccggcggttc ggatcgttcg atatcagggc cggatcgaca acgcgtggtg 60900
gaagtggtta cgcgggtcgt aggcggcctt caccttgcgc agccgcgggt ggttcccctt 60960
gtagtagagg tcgtgccacg gcacgcccga ggtgttcaag cccggatccg cgaggtcgct 61020
gtcggggtaa ttgatgtacg ccccatcgct gacgtcgttc ggcaccggca ccccgccggt 61080

ctcggcgtac acatcggcat agagcttgcg gacccacgtc agatgcttgg cctcgttgcc 61140

gggattcgcc caaccggtga tgtagttcac cttgagtatc gcgtcgcgct gcggcagggc 61200

ggtggccgcc gggtcgacgg tgttcacctt cccgccgtag ccgatcagcc agacggcgcc 61260

gtagtcgatc ccgtccatgt gggtcatgtt ctcgtacacg gcctgaatct gccggtcggt 61320
cagccgcttg cgcaggtagc cggctttcgt cttcgacgcc gggcccctgc ctcctcgccc 61380

cggcgtcgag gccagccacc tctgttcgat cggctcgggc acctcggccg gcgggacgcc 61440

gtcgatcacc gcctcgatgt gcgcgtcgag cagtctccgg gcgtccggcc gggtggcgtc 61500

cacctggatg ggcatcatga agccgctctc acccatgccc gggacctcgt tcccgatcat 61560

gagctgactc cacagcccgg tgtacggcga gtcgggcccg ctgttccgct cgtaccactc 61620

cccgtggttg cgcagcagcc gggagaacgc cgcctccgtc atccccgccc agtcgaaggt 61680

caccgtgctc gtgagcaacg tcgcgggcgg cttcggcagc agccgctccg gattccggcc 61740

gacgtcctcc ggcaccctca tccagtactt cgtgaccacc ccgaagttcc cgccgccacc 61800

gccggtgtgc gcccaccaca ggtcgtgatg ggggtcgtcg cgctcacggg tcgccacgat 61860

cacgcgtgcc ttcccctgtt tgttgacgac gacgacctcg accgcgtaca agtagtcgac 61920

cacggagccg aactgccgtg acagcgggcc gtacccgcct ccgcagatgt gtccgccgac 61980

gccgaccccg ccgcagaccc cacccggtat ggtcacgttc cagcccaggt agagcttttc 62040

gtacacctct gagagcgtgt tgcccggctc gatcaggaac gcgttcatcg acgggtcgta 62100

cgcgatctcc gtcagcagcg acatgtcgat gatgaccttg acgtcggggt tgtcgacgaa 62160

gtcctcgaaa cagtgcccac cgctgcggac ggcgacccgc ttgccggtgc gcaccgtctc 62220

ctcgacggca tcggccacct gctgggtgga gccgaccagg tggatgtagt cgggctcgcc 62280

gttgaagcgg ctgttggcgc cacgcagctt caggttgagg tagcgcgggt cgtccggagt 62340

caccttgacc gggccggccg gcggtaagca gcgctcgccc cgcagctccg gtcgcgtgga 62400

cgaggcgccg gccgacgcgg cgtccgctcc ggtgccgccg gtcaccaccg ccgccgcgcc 62460

cccggcaagg gaggcactca gcaatctacg tcggttcagt tttgtcatgg cggcgacgtt 62520

actatcggtt cgattcgatc aactcgctgt ctgactggac gtaagcgatc tcttcacgcc 62580

gtggccgtac gtggctgtcc atcgcctaca gatttccgat ctctgaaggt acggtcacct 62640

gttgaagaac gcgtccgtca gcgcattcca ggtgacgccg ccgcgaacgc gcgctcacgg 62700

gaaaactcgc cgtcgaggcg ggtgacgacg gaatcgtggt gtccaaccac ggaggccgtc 62760

agttggacgg tgccgtcctg agccagtttc tgccaaacga ctgggaacac catgggacat 62820

ccacacgatc aagagcccga cggccaaatc cgtcctgcgg ctcagagccc ccgccggttg 62880

gtcgagatga cgagcacctc cgggcggcac ctgtatcacc gccaggtgcg attctccgat 62940

atcgacgccc acggccacgt caacaatgtg cgtttcctgg aatacctgga ggacgcctgg 63000

atcgccctct atctcgacaa tgcgggcccg ccgcaggagg accgcgacgg attgcccgcc 63060

gtggggttcg ccgtcgtgcg ccacgagatc ttctatcggc gcccgctcag gttccggcac 63120

gggtcggtgc gggtcgagtc gtgggtgacc aaggtgaaca gggtgacctg cgagatggcc 63180

gcgcagatct gctcggacgg ggaggtgttc gtcgaagccc gctcgatgat catggggttc 63240

gacacgcaca ccgccaagcc gcggcgcctc accctgcacg agcgcacctt tctcaagcgt 63300

tacctgcgct gatgtgactt ctccattgcc ggccgcggct ccgggcgttg gacgattttg 63360

accgccgaga tcggccgagc ctaccttcac ggtgttcgct gcgaccggaa aggtgaattc 63420

aatggccgcg tccgaggtca agcaagtgct ccggagcaag ctcaggacat gggggtggat 63480

gtatcgatga cgaccagcat cgcgtcggca gaagaccttt ccgtcctcac cggactgagc 63540

gagatcacca cgttcgccgg cgtggggaca gccgtttccg ccacgtccta ttcgcaagcc 63600

gagctgctcg aaatcctcga catacgcgat cccaggatcc gatcgctgtt cctgaacagc 63660

gcgatcgagc ggcgtttctt cgcgcttccg ccccagggcc gggacgggga gcgcgtggcg 63720

gaaccgcagg gtgatctcct ggacaagcac aaaaagctcg ccgtcgatat gggatgccgg 63780

gccctcgagt cctgcctgaa gtcggcgggc gcgacgctct cggatgtccg ccacctgtgc 63840

tgcgtcacct cgaccggttt tctcaccccc ggcctgagcg cactcatcat ccgcgagctc 63900

gggctcgacc cgcattgcag ccgcgccgac atcgtcggca tgggatgcaa cgcggggctg 63960

aacgcgctca acctggtcgc gggctggtcc gcggcgcacc cgggcgagct cgccgtcgtt 64020

ctgtgcagcg aggcgtgttc cgctgcttac gcactggacg gcaccatgcg caccgcggtg 64080

gtcaacagcc tgttcggcga cggatccgcc gccctcgccg tcgtctccgg tgacgggcgc 64140

gctgccggcc cgcgcgtcct gaagttcgcg agctacgtca tcaccgacgc gatcgaggcg 64200

atgcgctacg actgggaccg cgaccaggac cggttcagct tcttcctcga tccgcagatc 64260

ccctacgtgg tcggcgcgca cgccgagatc gtcgtcgaca agctgctgtc cggtacgggg 64320

ctgcgccgca gcgacatcgg ccattggctg gtgcactccg gcggcaagaa ggtgatcgac 64380

gccatcgtcg tcaacctcgg cctgagccgg catgacgtcc gccacacgac cgctgtgctc 64440

cgcgactacg ggaacctctc cagcggctcc ttcctcttct cctacgaacg gctcgccggc 64500

gagggcgtga ccaggcccgg agactacggg gtgctcatga ccatggggcc cggctccacg 64560

atcgaaacgg cgctgatcca atggtgagtg gcagtgacat gaacggcgaa ctggagctga 64620

gcctcgacgg cacccaggcg ctgaccgcgt cggtcgagga gctgaacggc ctctgcgacc 64680

gcgccgagga ccatcgagca cccggcccgg tcatcgtcca cgtcaccggc gtgccgcgcc 64740

ttggctggtc gaaggggctg acggtgggcc tggtctccaa gtgggagcgg gtggtgcgcc 64800

ggttcgaacg gctcggccgg ctcaccgtcg ccgtggcgtc aggcgactgc gcgggaccct 64860

ctctcgacct cctcctcgct gccgacgtgc ggatcgccgc tccggcgacc cggctgctgc 64920

cctcctgggc cggcggcgcc gcgtggccgg ggatggccgt ctaccggctc acccagcagg 64980

ccggtacggg cggcatccgg cgggccgtgc tgctcggggc acccatcgac gccgaccgcg 65040

cgctcgccct caacctgatc gacgaggtgt ccgcggaccc ggcggcgtcc ctggccggcc 65100

tggcgggtgc cggggacggc gcggagctgg cgattcgcag gcagctgatg ttcgaggcga 65160
gctcaaccac tttcgaggac gcgctcggtg ctcacctggc cgcggtggac cgggccctac 65220

gacgggagac cctctcgtga cgacggattg gccggcgctg ccgcccaggg cgccgctcgc 65280

cctctggacc ctgacggcgg aggcccagcg agtcgacgac ctgctcgccg ggctgccgga 65340

gcctcctgcc agaacctccg cccagcgcga tgccgcggcc tcggcactcg acaaggtgag 65400

gcggatgcgc gcggactaca tggaggcgca cgccgaggag atctacggcg agctcacctc 65460

cggccgcacc cggcacctgc gcatcgacga gctcgtacgg gccgccgccc gcgcctaccc 65520

cggcctggtg cccaccgatg agcagatggc ggccgagcgc gcgcggccac aggcggagaa 65580

ggaagggcgc gagatcgatc agggcatctt cctgcgcggg gtcctgcgtg ccccgaaggc 65640

gggcccgcac ctgctcgacg ccatgctccg gcccaccccc agggcccttg agctgctccc 65700

tgaattcatc gagtccggcg aggtgcggat ggaggcggtc ctgctgcggc gccgtgacgg 65760

tgtcgcgtac ctgaccctgt gccgggacga ctgcctcaac gccgaggacg cgcagcaggt 65820

ggacgacatg gagaccgcag tcgacctggc gctgctcgac ccccaggtcc gggtggggct 65880

cctgcggggc ggcgagatga gccatccccg gtaccggggg cgccgggtgt tctgcgcggg 65940

cgtcaacctc aagaagctga gctcgggcga catctccctc gtcgacttcc tcctacggcg 66000

cgagctgggc tacatccaca agatcgttcg cggcgtgtac acggacggtt cgtggcactc 66060

gaagctgacc gacaagccct ggatggcggt cgtcgactcc ttcgccatcg gcggtggggc 66120

tcagctcctc ctggtcttcg accaggtgct ggcggcgtcc gactcctaca tcagcctgcc 66180

tgcggcgacg gaggggatca ttccgggggt cgcgaactac cggctcaccc ggttcaccgg 66240

gccacgcgcg gcccggcaga tgatcctcgg cgggcggcgg atccgggcgg acgagccgga 66300

cgcacggttg atgatcgacg aggtcgtccc gccggaggag atggacgcgg cgatcgatcg 66360

cgcactggcc cgcctcgacg gagatgcggt gccggccaac cggcgcatgc tgaacctggc 66420

cgaggagccg cccgaggcgt tcggccggta cctggccgag ttcgccctgc agcaggcact 66480

gcgcatctac ggcagggacg tcatcggcaa ggtcggcagg ttcgcagcgg gatcggcatg 66540

agcgagcctc gcgtgcgcta cgagaagaag gaacacgtcg cccatgtgac gatgaaccgg 66600

ccccacgtgc tgaacgcgat ggatcgccgg atgcacgagg agctcgccga gatctgggac 66660

gacgtcgagg ccgacgacga cgtcaggacg gtcgtcctga ccggtgcggg aacgcgggcc 66720

ttctccgtcg gccaggacct caaggaacgc gcgctgctgg acgaggcggg cacgcaggcc 66780

tcgacgttcg gcagccgggg gcaggcaggt catccccggc tgaccgaccg cttcaccttg 66840

tccaagccgg tggtcgcccg ggtgcacggc tacgcgctgg gtggcggctt cgagctggtg 66900
ctcgcctgcg acctcgtcat cgcctccgag gaggcggtgt tcggcctgcc ggaggtccgg 66960
ctcggcctga tccccggggc gggaggcgtg ttccggctgc cgcggcagct gccgcagaag 67020
gtggcgatgg gccatctgct gaccgggcgc cggatggatg cggccacggc gttccggtac 67080
ggattggtga acgaggtcgt accgcttgat gagctggatc ggtgcgtggc cggatggacc 67140
gacgacctcg tacgcgccgc tccgctgtct gttcgcgcga tcaaggaggc cgccatgcgg 67200
tcgctcgaca ttcccctgga ggaggcgttc accacgtcct acccatggga agagcgtcgt 67260
cggcgtagcg gcgatgcgat cgagggcgtc cgggcgttcg tcgagaagag ggacccggtc 67320
tggacgtcga gatgatcccc ccgcacacgt tgctggtctt cttcgttcag gctgcggccc 67380
tcctgctgct cgcgttgctc ctgggccgcc tggccgtacg gctgggcctg gcggcggtcg 67440
tcggcgaact gtgtgccggc gtcatcctcg gcccctccgt gctggggcag gtcgcgcccg 67500
gggcggagca gtggctgttt ccctcgccgt cgtcacacat gctggacgcc gtcgggcagc 67560
tcggcgtgtt gttgctgatc ggcttgacgg gcgcgcatct ggatctgcgg ctgatccggc 67620
ggcagggcgc cacggcggtg cgggtgagcg ccttcgggtt ggtcgtgccg atggccctcg 67680
gcatcggcgc cggcctgttg ctgccggccg agttccgcgg gaccggcggc tcggccgtct 67740
tcgccctgtt cctgggggtg acgatgtgtg tcagctcgat ccccgtgatc gccaagacgc 67800
tgatggacat gaacctgctc catcgcaacg tcggccagct cacgctgacc gccggcatga 67860
tcgacgacgc cttcgggtgg gtgctgcttt cggtggtgac ggcgatggcc accgccggag 67920
ccggtgcggg gaccgtggtg ctgtcgatcg cgtcgctgct cggggtgatc gtcttcagcg 67980
tcgtcatcgg caggccggcg gtccgggtgg cgttgcggac gacggaggat cagggggtga 68040
tcgccggcca ggtcgtggtg ctggtgctcg cggccgcggc cgggacgcat gcgctgggcc 68100
tcgaaccgat cttcggggcc ttcgtcgccg ggctgctggt gagcacggcc atgccgaatc 68160
cggtcagact ggcaccgctg cgcacggtga cgctcggggt gctggctccc ctctatttcg 68220
ccaccatggg cctgcgcgtc gatctcacgg ccctggcgcg gccggaggtg ctcgccgtgg 68280
ggctgctggt cctggccctg gcgatcatcg gcaagttcct gggcgccttc ctgggcgcct 68340
ggaccagccg gctcagccga tgggaggcct tggcgctggg ggcggggatg aacgcccgtg 68400
gcgtcatcca gatgatcgtg gcgacggtcg gcctgcggct gggggtgatc actgacgaga 68460
tcttcacgat catcatcgtg gtggcggtga tcacctctct gctcgccccg ccactcctgc 68520
gcctggccat gaccaggatc gaggccaccg ccgaggagga ggcccgcctc ctcgcctacg 68580
ggctgcgccc cggcgaggcc cgggaagacg tacggtgacg acggctcggg atcgtcgtgc 68640
ccgacgacaa ggccggcagc cggacggtgg tggccggtgc cggctcagcc acagtgggcc 68700

ggggtcgcga tgcccagccg cgcgtgcagg tgcgcccaca gagcagcctg ctcgtgcccc 68760

aggaagaagt ggcctccggg caggacgtgg caggagaact cccgagccgt caactcggcc 68820

catcgcgcga ccgcgtcgag ccgtacgacg ggatcgtccg caccggtgaa cgccgtgatc 68880

ggcaccgtca ggggcggccc aggcgtgtgg cggtaggact ggacgagctg gaagtcgttg 68940

cgcacgtagg ggagggcgaa cgcccggaac tccgcgctcg cgagcgcctc ggcatcggtg 69000
ccgcccaaca ggcgcagcct gtcgatgagc gcctcctcgg aggccggcgc cacccgatgc 69060

gcgagacggc cacggtcgtg cgcggccaca cctccggaga cgaagagatg agccggcggg 69120

ataccggacc cggtgagaag ccgcgccgtc tcgtaagcga tcaaactgcc catactgtgc 69180

ccgaacagcg ccaccggccg gtcgaggagc ggcctcagct cacgccccac cgactccgcg 69240

agccggtggg catcaccgac gaggggttcg tgcaaccggt cggcgcggcc cggatactga 69300

accgcgtgca cttctatctc cggcgcggcc agccggtgcc aattccggta gaagaccgcc 69360

gaaccgcccg cgtgcggaaa acagatcagc cgcatcgtgg cgagcggccg cctgtcgaaa 69420

caccgaaacc aggtggacat gtagcctcgc ttcggcctca tatcatggtc ttgggtcaat 69480

cctggtgacc tgactatatg cctgcaccgc cataaagtat gtccgtccac tcatcggcgg 69540

gcatgcggca cgagtctgcc caggtcgcac ttgacgcctg gtcggcaaag ggaaaaccct 69600

tgcttccatg gactcccacg ttctcgccca tcaattgagc aaggaaacgc tgcacggatc 69660

gctgatggac ccggccatcg agtcgatgaa tctactgaac gagattgccg gcaactaccc 69720

cgacgccatt tccatggccg cgggccggcc gtacgaggag ttcttcgacg tcggcctcat 69780

ccacgactat ctggaggcct accgcgacca tctccgcaac gaccggcgga tggatgacgc 69840

cgggatcagc cgcatgcttt tccaatacgg gaccacgaag gggatcatct ccgaccttgt 69900

cgcccggcac ctcgccgagg acgagaacat cgaggccgac ccggcctccg tggtcatcac 69960

tgtgggcttc caggaggcca tgttcctggt gcttcgcgcg ctgcgagcga acgagcggga 70020

cgtcctgctc gcccccacgc ccacctacgt cggcctgacc ggagcggcgc tgctcaccga 70080

cacccctgtc tggccggtcc agtccaccga caacggcatc gacctcgacc accttgagca 70140

ccaactgaaa cgcgcccagg accagggcgc ccgggtccgg gcctgctacg tgaccccgaa 70200

cttcgccaac cccaccggca ccagcatgga cctgcccgcc cgccatcgcc tcctggaggt 70260

cgccgcggcc cacggcatcc tgatcctgga ggacaacgcg tacggactcc tcggccagga 70320

ccgcctcccc acgctgaagt ccctcgacca tgcggcgacc gtcgtctacc tcggctcctt 70380

cgccaagacc ggcatgcccg gcgcccgggt cggctacgtc gtggcggacc agcacgtagc 70440

ggggggcggc tcgctcgccg acgagctcgc gaagctcaag ggcatgctca ccgtgaacac 70500

ctcgcccatc gcccaggcgg tgatcgccgg caagctgctg cgccacgact tcagcctggc 70560

ccgggccaac gcccgcgaga ccgccatcta ccagcgcaac ctccacctca cgctggacga 70620

actcacccgc cggctcggcg ccgtcccggg agtcacctgg aacgcgccga cgggcgggtt 70680

cttcatcacc gtcaccgtgc ccttcgtcgt ggatgacgag ctgttggaac acgctgcccg 70740

cgatcatggc gttttgttca cgccgatgca tcacttctat ggtgggaagg atgggttcaa 70800

ccagcttcgg ctgtcgatca gcctgctcaa cccgcaactg atcgaggagg gtgtctcccg 70860

gcttgccggg ctcgtcaccg catgtctccc ctgaaccatg cctggggcct tgagtcggac 70920

ggccgggttg cgtgcggccg ggatgaaggt caaccacaag cgggtggtgc gcgagcacgg 70980

cctcgccggg cggtggccag cgaccaaggc ctcgacaaac gccatcgccg accctcccga 71040

gggaggatcg gcggttgaag atctgtgtgc cccctgcagg attcgaacct gcgcacccgg 71100

ctccggaggc cggtgctcta tcccctgagc taagggggg 71138

<210> 2
<211> 366
<212> PRT
<213> Nonomuria

<400> 2

Met His Glu Ser Pro Val Cys Leu Ala Glu Tyr Glu Glu Ile Ala Ala
1               5                   10                  15

Lys Val Leu Pro Ala Asp Val Arg Asp Phe Ile Asp Gly Gly Ser Gly
            20                  25                  30

Arg Glu Gln Thr Leu Arg Ala Asn Arg Ala Ala Phe Asp Arg Val Phe
        35                  40                  45

Leu Val Pro Arg Val Leu Gln Asp Val Ser Ala Cys Ser Thr Arg Ala
    50                  55                  60

Thr Leu Leu Gly His Pro Ala Thr Met Pro Val Ala Val Ala Pro Val
65                  70                  75                  80

Ala Tyr His Arg Leu Val His Pro Asp Gly Glu Leu Ala Thr Ala Arg
                85                  90                  95

Ala Ala Arg Asp Ala Gly Val Pro Phe Thr Val Ser Thr Leu Ser Ser
            100                 105                 110

Val Pro Val Glu Asp Val Thr Ala Leu Gly Gly His Val Trp Phe Gln
        115                 120                 125

Leu Tyr Cys Leu Arg Glu His Ala Ala Thr Leu Gly Leu Ile Arg Arg
    130                 135                 140

Ala Glu Asp Ala Gly Cys Arg Ala Leu Met Leu Thr Leu Asp Val Pro

145                 150                 155                 160

Trp Met Gly Arg Arg Pro Arg Asp Ile Arg Asn Arg Phe Arg Leu Pro
                165                 170                 175

Pro His Val Arg Pro Val His Leu Thr Ala Asn Ser Gly Thr Glu Ala
            180                 185                 190

His Arg Gly Ala Ser Gly Gly Ser Ala Leu Ala Ala His Thr Ala Met
        195                 200                 205

Glu Leu Ser Ala Ala Val Asp Trp Ser Tyr Leu Glu Thr Leu Arg Ala
    210                 215                 220

Ala Ser Gly Leu Pro Leu Val Val Lys Gly Ile Leu His Pro Glu Asp
225                 230                 235                 240

Ala Arg Arg Ala Ala Asp Leu Gly Ile Asp Gly Ile Val Val Ser Asn
                245                 250                 255

His Gly Gly Arg Gln Leu Asp Gly Ala Val Ala Ser Leu Asp Ala Leu
            260                 265                 270

Pro Gly Val Ala Glu Ser Val Gly Gly Arg Cys Glu Ile Met Leu Asp
        275                 280                 285

Gly Gly Val Arg Ser Gly Ala Asp Val Leu Lys Ala Leu Ala Leu Gly
    290                 295                 300

Ala Ser Gly Val Leu Val Gly Arg Pro Val Ile Trp Gly Leu Ala Ala
305                 310                 315                 320

Asp Gly Glu Arg Gly Val Arg Thr Val Leu Gly Leu Leu Gly Ala Glu
                325                 330                 335

Ile Glu Asp Gly Leu Gly Leu Ala Gly Cys Gly Asp Val Ala Ala Ala
            340                 345                 350

Gln Ala Leu Arg Thr Thr Arg Pro Gly Ala Gly Phe Val Ser
        355                 360                 365

<210>  3
<211>  356
<212>  PRT
<213>  Nonomuria

<400>  3

Met Glu Ser Leu Pro Pro Leu Ala Val Asp Tyr Val Glu Met Tyr Val

1               5                   10                  15

Ala Asp Leu Lys Val Ala Thr Leu Pro Trp Thr Asp Glu Tyr Arg Phe
            20                  25                  30

Ala Val Val Gly Thr Ala Asn Ala Ser Asp His Arg Ser Val Ala Leu
        35                  40                  45

Arg Gln Gly Arg Ile Thr Leu Val Leu Thr Gln Ala Thr Ser Asp Gly
    50                  55                  60

His Pro Ala Ser Ala Tyr Val Arg Thr His Gly Asp Gly Val Ala Asp
65                  70                  75                  80

Ile Ala Leu Arg Thr Pro Asp Val Asp Val Val Phe Thr His Ala Val
                85                  90                  95

Ala Ala Gly Ala Arg Pro Val Arg Ser Pro Ser Arg His Pro Gly Pro
            100                 105                 110

Gly Pro Ala Cys Ser Ala Ala Ile Gly Gly Phe Gly Asp Val Val His
        115                 120                 125

Thr Leu Val Gln Arg Asp Pro Gly Asp Asp Pro Gly Leu Pro Val Gly
    130                 135                 140

Phe Ser Glu Ala Pro Ser Ala Ala Glu Ser Gly Ala Asp Ala Ala Glu
145                 150                 155                 160

Leu Leu Asp Ile Asp His Phe Ala Val Cys Leu Pro Thr Gly Asp Leu
                165                 170                 175

Asp Ile Ile Thr Asp Phe Tyr Val Ala Thr Leu Gly Phe Ser Glu Thr
            180                 185                 190

Phe Lys Glu Arg Ile Glu Val Gly Thr Gln Ala Met Glu Ser Lys Val
        195                 200                 205

Val Gln Ser Ala Ser Gly Ala Val Thr Leu Thr Leu Ile Glu Pro Asp
    210                 215                 220

Pro Met Ala Glu Ala Gly Gln Ile Asp Met Phe Leu Glu Arg His Ala
225                 230                 235                 240

Gly Ala Gly Val Gln His Val Ala Phe Ser Ser Ser Asp Ala Val His
                245                 250                 255

Ala Val Asn Thr Leu Ser Glu Arg Gly Val Arg Phe Leu Ser Thr Pro

260                 265                 270

Gly Ser Tyr Tyr Asp Leu Leu Glu Ser Arg Ile Gln Ile Arg Gly His
        275                 280                 285

Thr Val Asp Gln Leu Arg Ala Thr Gly Leu Leu Ala Asp Glu Asp His
    290                 295                 300

Gly Gly Gln Leu Phe Gln Ile Phe Thr Ala Ser Thr His Pro Arg Glu
305                 310                 315                 320

Thr Leu Phe Phe Glu Val Ile Glu Arg Gln Gly Ala Arg Thr Phe Gly
                325                 330                 335

Gly Ala Asn Ile Lys Ala Leu Tyr Glu Ala Val Glu Val Ala Arg Ser
            340                 345                 350

Gln Gln Arg Ala
        355

<210>  4
<211>  867
<212>  PRT
<213>  Nonomuria

<400>  4

Met Leu Phe Gly Arg Asp Arg Glu Leu Lys Ser Leu Thr Arg Leu Leu
1               5                   10                  15

Asp Ser Thr Ala Ala Gly Arg Gly Gly Val Ala Val Ile Thr Gly Pro
            20                  25                  30

Val Val Gly Gly Lys Thr Ala Ile Leu His Glu Leu Gly Met Arg Ser
        35                  40                  45

Ile Ala Ala Gly Ile Arg Leu Val Thr Ala Arg Cys Thr Pro Ala Glu
    50                  55                  60

Gln Ser Leu Asp Trp Gly Val Ala Asp Gln Ile Leu Gly Arg Gly Ala
65                  70                  75                  80

Ala Glu Arg Leu Thr Ala Arg Arg Gly Gly Asp Ala Val Glu Asp Val
                85                  90                  95

Cys Val Ser Leu Phe Gln Met Ala Glu Ala Asn Pro Ile Leu Leu Thr
            100                 105                 110

Ile Asp Asp Val Asp Leu Ala Asp Asp Pro Ser Leu Leu Ala Ile Leu

        115                 120                 125

Ser Met Thr Pro Leu Leu Thr Asp Thr Arg Met Met Ile Ala Val Thr
    130                 135                 140

Ile Cys Gln Asp Arg Pro Pro Ala Pro Leu Pro His Val Ala Glu Ser
145                 150                 155                 160

Leu Leu Arg Leu Pro Gly Ile Glu Leu Val Glu Leu Pro Leu Leu Pro
                165                 170                 175

Arg Pro Ala Val Arg Gln Phe Ala Thr Glu His Leu Gly Ala Glu Thr
            180                 185                 190

Ala Asp Gln Leu Ala Asp Asp Leu Tyr Arg Phe Ser Gly Gly Ser Pro
        195                 200                 205

Leu Leu Val Arg Ala Leu Ile Glu Asp Gln Glu Ala Gly Ala Pro Gly
    210                 215                 220

Leu Val Val Gly Asp Ser Phe Met Ser Ala Val Ala Ala Cys Val His
225                 230                 235                 240

Gly Cys Glu Pro Glu Ala Val Arg Val Ala Glu Ala Val Ala Val Leu
                245                 250                 255

Gly Glu His Ala Thr Pro Asp Ala Val Gly Glu Leu Val Gly Ile Ala
            260                 265                 270

Pro Pro Ala Ala Thr Arg Ser Met Gly Met Leu Glu Arg Ala Gly Leu
        275                 280                 285

Leu Ala Gly Gly Arg Phe Arg His Glu Ala Gly Arg Leu Ala Val Leu
    290                 295                 300

Gly Arg Met Thr Ser Tyr Gly Arg Met Glu Ile Leu Arg Arg Ala Ala
305                 310                 315                 320

Glu Ile Leu His Arg Arg Gly Gly Pro Pro Ser Ala Val Ala Thr Arg
                325                 330                 335

Leu Leu Glu Ala Gly Trp Ser Gly Glu Glu Trp Ala Phe Asp Val Leu
            340                 345                 350

Val Glu Ala Gly Arg Gln Ala Phe Asp Glu Gly Asp Phe Val Ala Val
        355                 360                 365

Met Lys Cys Leu Arg Leu Ala Leu Ala Ser Gly Trp Gly Thr Pro Arg
    370                 375                 380

Arg Leu Asp Val Lys Val Met Leu Ala Ala Ala Glu Trp Arg Val Asp
385                 390                 395                 400

Pro Ala Val Ala Ala Arg His Val Pro Asp Leu Leu Asp Ala Thr Arg
                405                 410                 415

Ser Gly Ala Leu Arg Gly Ser His Gly Met Glu Leu Phe Arg Gln Leu
            420                 425                 430

Leu Trp Tyr Gly Arg Phe Ala Asp Ala Ala Glu Leu Ile Asp Arg Leu
        435                 440                 445

Arg Pro Ser Val Ala Asp Arg Asp Ala Asp Ala Ser Leu Ile Ala Met
    450                 455                 460

Cys His Val His Pro Val Leu Leu Asp Arg Leu Pro Arg Ser Ala Arg
465                 470                 475                 480

Gly Ser Met Gly Gln Thr Val Glu Asp Ala Arg Arg Ile Leu Arg Gln
                485                 490                 495

Ala Glu Pro Thr Asp Glu Ala Met Asp Ser Ile Ile Ser Ala Leu Met
            500                 505                 510

Ala Leu Leu Leu Gly Gly Val Ser Glu Val Ala Ala Ser Cys Glu Thr
        515                 520                 525

Leu Leu Lys Glu Pro Gly Val Thr Lys Ala Pro Thr Trp Lys Ala Ile
    530                 535                 540

Ile Ser Ala Ile Arg Ala Glu Thr Ala Trp Arg Lys Gly Asp Leu Ala
545                 550                 555                 560

Gly Ala Glu Ala His Ala Gln Glu Ala Leu Thr Ile Leu Gln Pro Ser
                565                 570                 575

Gly Trp Gly Val Ala Ile Gly Ala Pro Leu Ser Thr Leu Leu His Ala
            580                 585                 590

Gln Thr Ala Met Gly His Leu Asp Glu Ala Lys Ala Thr Val Ala Val
        595                 600                 605

Pro Met Pro Arg Glu Thr Ala Glu Thr Ala Phe Gly Ile Gly Tyr Glu
    610                 615                 620

Leu Ala Arg Ala His Tyr His Leu Val Thr Glu Gln Pro Arg Ala Ala
625                 630                 635                 640

Phe Ala Gly Phe Leu Ala Cys Gly Gln Ala Val Gln Arg Trp Gly Ser
                645                 650                 655

Ser Leu Ser Asp Val Val Pro Trp Arg Leu Gly Ala Ala Arg Ala Cys
            660                 665                 670

Leu Gln Leu Gly Trp Arg Arg Arg Ala Ala Asp Leu Val Thr Ala Gln
        675                 680                 685

Ile Ala His Thr Ser Ser Gly Asp Leu Arg Thr Tyr Gly Val Ala Leu
    690                 695                 700

Arg Leu His Ala Gln Leu Ser Lys Pro Ala Gln Arg Gln Arg Leu Leu
705                 710                 715                 720

Met Gln Ser Val Asp Ala Leu Glu Ala Ala Gln Asp Arg Tyr Gln Leu
                725                 730                 735

Ala Leu Ser Leu Cys Asp Leu Ala Gly Thr Pro Gln Leu Lys Gly Gly
            740                 745                 750

Lys Asp Glu Ala Arg Ala Tyr Trp Val Arg Ala Gln Glu Leu Ala Arg
        755                 760                 765

Glu Cys Asn Ala Lys Pro Leu Met Arg Arg Leu Ala Ala Gln His Asp
    770                 775                 780

His Gly Glu Thr Ala Pro Leu Ser Gly Ala Glu Arg Arg Val Ala Val
785                 790                 795                 800

Leu Ala Ala Arg Gly His Thr Asn Arg Glu Ile Ala Glu Ala Leu Tyr
                805                 810                 815

Ile Thr Arg Ser Thr Val Glu Gln His Leu Thr Arg Ile Tyr Arg Lys
            820                 825                 830

Leu His Val Gln Thr Arg Gly Asp Leu Gly Asn Leu Phe Ala Ala Asp
        835                 840                 845

Ile Ala Asp Lys Ala Thr Ala Thr Ala Gly Arg Glu Pro Arg Glu Ala
    850                 855                 860

Val Arg Leu
865

<210>  5
<211>  321
<212>  PRT
<213>  Nonomuria

<400>  5

Met Asp Pro Thr Gly Val Asp Ile Ala Thr Leu Pro Val Val Glu Ile
1               5                   10                  15

Glu Leu Ser Arg Leu Ser Ser Val Tyr Ser Pro Arg Thr Ser Gly Glu
            20                  25                  30

Asp Pro Glu His Val Glu Thr Leu Leu Ser Ala Gln Gly Glu Leu Pro
        35                  40                  45

Pro Ile Leu Val His Arg Pro Thr Met Arg Val Ile Asp Gly Leu His
    50                  55                  60

65                  70                  75                  80

Leu Ile Asp Gly Thr Glu Ser Asp Ala Phe Val Leu Ala Val Glu Ala
                85                  90                  95

Asn Val Arg His Gly Leu Pro Leu Ser Leu Ala Asp Arg Lys Arg Ala
            100                 105                 110

Ala Val Arg Ile Ile Gly Thr His Pro Gln Trp Ser Asp Arg Arg Val
        115                 120                 125

Ala Ser Ala Thr Gly Ile Ser Ala Gly Thr Val Ala Asp Leu Arg Arg
    130                 135                 140

Arg Arg Gly Gln Gly Gly Asp Glu Ala Arg Ile Gly Arg Asp Gly Arg
145                 150                 155                 160

Ile Arg Pro Val Asp Ser Ser Glu Gly Arg Arg Leu Ala Ala Glu Leu
                165                 170                 175

Ile Arg Ser His Pro Asp Leu Ser Leu Arg Gln Val Ala Lys Gln Val
            180                 185                 190

Gly Ile Ser Pro Glu Thr Val Arg Asp Val Arg Gly Arg Leu Glu His
        195                 200                 205

Gly Glu Ser Pro Ile Pro Asp Gly Ser Arg Arg Leu Arg Thr Lys Pro
    210                 215                 220

Glu Leu Leu Arg Arg Ala Glu Gln Asp Phe Gly His Val Asp Gly Arg
225                 230                 235                 240

Asp Arg Gln Ala Val Leu Glu Arg Leu Lys Ala Asp Pro Ala Leu Arg
                245                 250                 255

Leu Thr Glu Thr Gly Arg Ile Leu Leu Arg Met Leu Ser Leu His Ser
            260                 265                 270

Ile Asp Gly Gln Glu Trp Glu Arg Ile Leu Arg Gly Val Pro Pro His
        275                 280                 285

Trp Gly Thr Val Val Ala Arg Cys Ala Arg Asp His Ala Gln Ile Trp
    290                 295                 300

Ala Ala Phe Ala Asp Arg Leu Glu Gly Arg Ala Thr Asp Leu Ala Ala
305                 310                 315                 320

Gly

<210>  6
<211>  369
<212>  PRT
<213>  Nonomuria

<400>  6

Met Thr Leu Glu Arg Thr Leu Ile Val Gly Thr Gly Leu Ile Gly Thr
1               5                   10                  15

Ser Ala Ala Leu Ala Leu Arg Glu Lys Gly Val Ala Val Tyr Leu Ser
            20                  25                  30

Asp Val Asp Ala His Ala Val Arg Leu Ala Arg Ala Leu Gly Ala Gly
        35                  40                  45

Gln Glu Trp Thr Gly Gln Arg Val Asp Leu Ala Leu Ile Ala Val Pro
    50                  55                  60

Pro Pro Ser Val Gly Gln Arg Leu Ala Asp Leu Gln Gln Arg Arg Ala
65                  70                  75                  80

Ala Arg Ala Tyr Thr Asp Val Thr Ser Val Lys Val Asp Pro Ile Ala
                85                  90                  95

Asp Ala Glu Arg Leu Gly Cys Asp Leu Thr Ser Tyr Val Pro Gly His
            100                 105                 110

Pro Leu Ala Gly Arg Glu Arg Ser Gly Pro Ala Ala Ala Arg Ala Asp
        115                 120                 125

Leu Phe Leu Gly Arg Pro Trp Ala Leu Cys Pro Arg Pro Glu Thr Gly
    130                 135                 140

Ala Asp Ala Val Arg Leu Ala Arg Glu Leu Val Ser Met Cys Gly Ala
145                 150                 155                 160

Glu Pro Tyr Thr Val Ser Ala Gly Glu His Asp Thr Ala Val Ala Leu
                165                 170                 175

Val Ser His Ala Pro His Val Ala Ala Ser Ala Val Ala Ala Arg Leu
            180                 185                 190

Arg Asp Gly Asp Asp Val Ala Leu Ala Leu Ala Gly Gln Gly Leu Arg
        195                 200                 205

Asp Val Thr Arg Ile Ala Ala Gly Asp Pro Leu Leu Trp Arg Met Ile
    210                 215                 220

Leu Ala Ala Asn Ala Leu Pro Val Ala Gly Val Leu Glu Arg Ile Ala
225                 230                 235                 240

Ala Asp Leu Ala Ala Ala Ala Ser Ala Leu Arg Ser Gly Asp Leu Asp
                245                 250                 255

Asp Val Thr Asp Leu Leu Arg Arg Gly Val Asp Gly His Gly Arg Ile
            260                 265                 270

Pro Asp Lys His Gly Gly Pro Ala Arg Asp Tyr Thr Val Ile Gln Val
        275                 280                 285

Val Leu Gln Asp Arg Pro Gly Glu Leu Ala Arg Leu Phe Asn Ala Ala
    290                 295                 300

Gly Leu Ala Asp Val Asn Ile Glu Asp Ile Arg Leu Glu His Ser Ala
305                 310                 315                 320

Gly Leu Pro Val Gly Val Val Glu Val Ser Val Arg Pro Glu Asp Thr
                325                 330                 335

Gly Arg Leu Thr Glu Ala Leu Arg Phe His Gly Trp His Val Pro Pro
            340                 345                 350

Val Pro Asp Gly Asn Ser Arg Ile Asp Arg Thr Arg Ala Met Val Ser
        355                 360                 365

Asp

<210> 7
<211> 217
<212> PRT
<213> Nonomuria

<400> 7

Met Arg Val Leu Val Val Glu Asp Gln Val Asp Leu Ala Asp Ser Val
1               5                   10                  15

Ala Arg Val Leu Arg Arg Glu Gly Met Ala Val Asp Val Ser His Asp
            20                  25                  30

Gly Asp Asp Ala Gln Glu Arg Leu Ser Val Ile Asp Tyr Asp Val Val
        35                  40                  45

Val Leu Asp Arg Asp Ile Pro Gly Val His Gly Asp Glu Leu Cys Ala
    50                  55                  60

Glu Ile Ala Val Asp Asp Arg Arg Thr Arg Val Leu Met Leu Thr Ala
65                  70                  75                  80

Ser Gly Thr Thr Ala Asp Arg Val Ala Gly Leu Ser Leu Gly Ala Asp
                85                  90                  95

Asp Tyr Leu Pro Lys Pro Phe Ala Phe Ala Glu Leu Val Ala Arg Ile
            100                 105                 110

Arg Ala Leu Gly Arg Arg Ala His Pro Pro Ala Pro Pro Ile Leu Val
        115                 120                 125

His Gly Asp Leu Arg Leu Asp Pro Ala Gln Arg Val Ala Ile Arg Gly
    130                 135                 140

Gly Met Arg Leu Pro Leu Thr Thr Lys Glu Leu Ala Val Leu Glu His
145                 150                 155                 160

Leu Leu Thr Ala Arg Gly Arg Val Val Ser Ala Glu Glu Leu Leu Glu
                165                 170                 175

Arg Val Trp Asp Glu Gln Ala Asp Pro Phe Thr Thr Thr Val Lys Ala
            180                 185                 190

Thr Ile Asn Arg Leu Arg Ser Lys Leu Gly Gln Pro Pro Val Ile Glu
        195                 200                 205

Thr Val Pro Arg Glu Gly Tyr Arg Ile
    210                 215

<210>  8
<211>  196
<212>  PRT
<213>  Nonomuria

<400>  8

Met Arg Arg Ser Glu Gly Asp Asp Glu Pro Arg Thr Leu Pro Pro Arg
1               5                   10                  15

Ala Arg Asp Arg Val Tyr Thr Ala Val Thr Arg Val Leu Ala Val Leu
            20                  25                  30

Leu Leu Pro Val Ala Phe Val Arg Gln Pro Gly Arg Ala Arg Glu Leu
        35                  40                  45

Ala Cys Gly Trp Ala Leu Arg Met Arg Phe Pro Ala Glu Asp Leu Thr
    50                  55                  60

Gly Leu Thr Asp Gly Ala Arg Ala Ala Phe Thr Ala Ala Arg Ala Glu
65                  70                  75                  80

Ala Leu Trp Arg His Gly Gln Leu Val Gly Leu Thr Ser Gly Tyr Arg
                85                  90                  95

Asp Pro Arg Val Gln Gln Arg Met Phe Glu Glu Glu Val Arg Arg Ser
            100                 105                 110

Gly Ser Val Ala Ala Ala Arg Met Phe Val Ala Pro Pro Ala Glu Ser
        115                 120                 125

Asn His Val Lys Gly Met Ala Leu Asp Val Arg Pro His Glu Gly Ala
    130                 135                 140

Arg Trp Leu Glu Ala His Gly Ala Arg Tyr Asp Leu Tyr Arg Ile Tyr
145                 150                 155                 160

Asp Asn Glu Trp Trp His Phe Glu His Arg Pro Glu Cys Gly Gly Thr
                165                 170                 175

Pro Pro Arg Arg Leu Pro His Pro Gly Ala Ala Trp Ala Ser Arg Asn
            180                 185                 190

Gly Gly Arg Val
        195

<210> 9
<211> 319
<212> PRT
<213> Nonomuria

<400> 9

Met Asp Ala Glu Ser Val Arg Arg Gln Leu Arg Leu Gly Glu Asn Ala
1               5                   10                  15

Thr Ala Trp Leu Ser Arg Leu Glu Glu Leu Gly Pro Pro Pro Glu Pro
            20                  25                  30

Val Arg Leu Pro Gln Gly Asp Glu Ala Arg Asp Leu Leu His Arg Leu
        35                  40                  45

Glu Val Pro Ala Pro Asp Val Glu Glu Ile Val Ala Ala Thr Pro Gly
    50                  55                  60

Pro Asp Arg Asp Pro Ala Leu Trp Trp Leu Leu Glu Arg Ala His His
65                  70                  75                  80

Glu Leu Val Arg His Met Gly Asp Tyr Lys Val Lys Val Arg Gly Gly
                85                  90                  95

Pro Thr Leu Pro Tyr Glu Thr Gly Ala Ala Ala Arg Tyr Phe His Val
            100                 105                 110

Tyr Val Phe Leu Ala Thr Leu Pro Ala Leu Arg Arg Phe His Ala Thr
        115                 120                 125

Arg Asp Ile Pro Glu Ala Thr Thr Trp Glu Thr Leu Thr Gln Leu Gly
    130                 135                 140

Glu Ser Val Ala Ile His Arg Arg Lys Tyr Gly Glu Gly Gly Thr Asn
145                 150                 155                 160

Met Pro Trp Trp Leu Thr Leu Leu Val Arg Gly Leu Val Tyr Arg Leu
                165                 170                 175

Gly Arg Leu Gln Tyr Asn Leu Ala Val Ala Lys Asp Gly Thr Pro Val
            180                 185                 190

Leu Gly Leu His Ile Pro Glu Val Gly Gly Pro Leu Ile Pro Asp Ile
        195                 200                 205

Tyr Tyr Asp Ser Leu Arg Arg Ala Arg Pro Phe Phe Glu Arg His Phe
    210                 215                 220

Pro Glu His Gly Ala Arg Ala Ala Thr Gly Thr Ser Trp Leu Leu Asp
225                 230                 235                 240

Pro Gln Leu Ala Glu Tyr Leu Ala Glu Asp Ser His Ile Leu Gln Leu
                245                 250                 255

Arg Arg Gly Trp Thr Leu Leu Asp Ser Glu Pro Gln Asp Gly Asp Asp
            260                 265                 270

Ala Ile Leu Glu Phe Val Phe Arg Tyr Asn Gly Gln Pro Leu Glu Glu
        275                 280                 285

Leu Pro Gln Arg Ser Thr Leu Glu Lys Ala Val Val Thr His Leu Leu
    290                 295                 300

Ala Gly Arg His Trp Tyr Gln Arg Ser Gly Arg Ile Glu Leu Pro
305                 310                 315

<210>  10
<211>  408
<212>  PRT
<213>  Nonomuria

<400>  10

Met Arg Val Leu Leu Ser Thr Ser Gly Ser Arg Gly Asp Val Glu Pro
1               5                   10                  15

Leu Leu Gly Leu Ala Val Gln Leu Arg Glu Leu Gly Ala Glu Thr Arg
            20                  25                  30

Met Cys Ala Pro Pro Asp Cys Ala Glu Arg Leu Ala Glu Ala Gly Val
        35                  40                  45

Pro Leu Val Pro Val Gly Thr Ser Met Arg Ala Lys Leu His Gly Lys
    50                  55                  60

Arg Pro Pro Ser Leu Glu Asp Val Pro Arg Leu Asp Ala Glu Ala Ile
65                  70                  75                  80

Ala Thr Gln Leu Asp Gln Val Leu Pro Ala Ala Glu Gly Cys Glu Val
                85                  90                  95

Met Val Val Ser Gly Val Leu Ser Ala Ala Val Ala Val Arg Ser Val
            100                 105                 110

Ala Glu Lys Leu Gly Ile Pro Tyr Val Tyr Val Phe Tyr Cys Pro Ile
        115                 120                 125

Tyr Val Pro Ser Pro Tyr Tyr Pro Pro Pro Pro Pro Leu Gly Glu Gln
    130                 135                 140

Pro Ala Arg Asp Val Thr Asp Asn Arg Val Leu Trp Asp Arg Asn Asn
145                 150                 155                 160

Gln Gly Ala Tyr Gln Arg Phe Gly Ala Ala Leu Asn Ser Arg Arg Ala
                165                 170                 175

Ser Ile Gly Leu Pro Pro Val Asp Asp Ile Phe Ser Tyr Gly Tyr Thr
            180                 185                 190

Asp Arg Pro Phe Leu Ala Ala Asp Pro Val Leu Ala Pro Leu Gln Arg
        195                 200                 205

Thr Asp Leu Asp Val Val Gln Thr Gly Ala Trp Ile Met Pro Asp Glu
    210                 215                 220

Arg Pro Leu Pro Ala Glu Val Glu Ala Phe Leu Glu Ala Gly Pro Pro
225                 230                 235                 240

Pro Val His Val Glu Phe Gly Ser Gly Pro Ala Pro Thr Asp Ala Ala
                245                 250                 255

Arg Val Ala Ile Glu Ala Ile Arg Ala His Gly His Arg Val Ile Val
            260                 265                 270

Ser Arg Gly Trp Ala Gly Leu Ala Pro Pro Asp Asp Arg Ser Asp Cys
        275                 280                 285

Leu Thr Val Gly Glu Val Asn His Gln Val Leu Phe Gly Arg Val Ala
    290                 295                 300

Ala Val Val His Ala Gly Ser Ala Gly Ile Thr Thr Ala Val Thr Arg
305                 310                 315                 320

Ala Gly Ala Pro Gln Val Val Val Pro Gln Met Thr Asp Gln Pro Tyr
                325                 330                 335

His Ala Gly Arg Val Ala Glu Leu Gly Ile Gly Val Ala His Asp Gly
            340                 345                 350

Arg Val Pro Thr Val Glu Ser Leu Ser Ala Ala Leu Thr Thr Ala Leu
        355                 360                 365

Ala Pro Glu Thr Arg Ala Arg Ala Ile Asp Val Ala Gly Lys Ile Arg
    370                 375                 380

Ala Asp Gly Ala Ala Val Ala Ala Lys Leu Leu Leu Asp Thr Ala Ala
385                 390                 395                 400

Gly Ala Gly Arg Asn Arg Thr Glu
                405

<210>  11
<211>  489
<212>  PRT
<213>  Nonomuria

<400>  11

Met Glu Glu Phe Asp Val Val Val Ala Gly Gly Gly Pro Gly Gly Ser
1               5                   10                  15

Thr Val Ala Thr Leu Val Ala Met Gln Gly His Arg Val Leu Leu Val
            20                  25                  30

Glu Lys Glu Val Phe Pro Arg Tyr Gln Ile Gly Glu Ser Leu Leu Pro
        35                  40                  45

Ser Thr Val His Gly Val Cys Arg Met Leu Gly Val Thr Asp Glu Leu
    50                  55                  60

Ala Ala Ala Gly Phe Pro Val Lys Arg Gly Gly Thr Phe Arg Trp Gly
65                  70                  75                  80

Ala Arg Pro Glu Pro Trp Thr Phe Ser Phe Ser Val Ser Pro Arg Ile
                85                  90                  95

Thr Gly Pro Thr Thr Phe Ala Tyr Gln Val Glu Arg Ala Arg Phe Asp
            100                 105                 110

Glu Ile Leu Leu Gly Asn Ala Arg Arg Lys Gly Val Val Val Arg Glu
        115                 120                 125

Gly Cys Ser Val Thr Glu Val Ile Glu Asp Gly Asp Arg Val Thr Gly
    130                 135                 140

Leu Arg Tyr Val Asp Pro Asp Gly Gly Glu His Ala Val Ser Ala Arg
145                 150                 155                 160

Phe Val Ile Asp Ala Ser Gly Asn Lys Ser Arg Leu Tyr Ser Ser Val

Gly Gly Thr Arg Asn Tyr Ser Glu Phe Phe Arg Ser Leu Ala Leu Phe
            180                 185                 190

Gly Tyr Phe Glu Gly Gly Lys Arg Leu Ala Glu Pro Tyr Ser Gly Asn
        195                 200                 205

Ile Leu Ser Val Ala Phe Asp Ser Gly Trp Phe Trp Tyr Ile Pro Leu
    210                 215                 220

Ser Asp Thr Leu Thr Ser Val Gly Ala Val Val Arg Arg Glu Met Ala
225                 230                 235                 240

Glu Lys Ile Gln Gly Asp Arg Glu Lys Ala Leu Ala Ala Leu Ile Ala
                245                 250                 255

Glu Cys Pro Leu Ile Ser Glu Tyr Leu Ala Pro Ala Arg Arg Val Thr
            260                 265                 270

Thr Gly Lys Tyr Gly Gln Leu Arg Val Arg Lys Asp Tyr Ser Tyr His
        275                 280                 285

Gln Thr Lys Phe Trp Arg Pro Gly Met Ile Leu Val Gly Asp Ala Ala
    290                 295                 300

Cys Phe Val Asp Pro Val Phe Ser Ser Gly Val His Leu Ala Thr Tyr
305                 310                 315                 320

Ser Gly Leu Leu Ala Ala Arg Ser Ile Asn Ser Val Leu Ala Gly Asp
                325                 330                 335

Val Glu Glu Lys Ile Ala Leu His Glu Phe Glu Ala Arg Tyr Arg Arg
            340                 345                 350

Glu Tyr Ser Val Tyr Tyr Glu Phe Leu Leu Ala Phe Tyr Glu Met Asn
        355                 360                 365

Val Asn Glu Glu Ser Tyr Phe Trp His Ala Lys Lys Val Thr Asn Asn
    370                 375                 380

Lys Glu Tyr Thr Glu Leu Glu Ser Phe Val Asp Leu Val Gly Gly Leu
385                 390                 395                 400

Ser Ser Gly Glu Thr Ala Leu Ala Thr Ser Gly Arg Ile Ala Glu Arg
                405                 410                 415

Ser Ala Glu Phe Ala Ala Ala Val Asp Gln Met Ala Asp Gly Asp Asp
            420                 425                 430

Ser Ser Met Val Pro Leu Phe Lys Ser Gln Val Val Lys Gln Val Met
        435                 440                 445

Gln Glu Gly Gly Gln Glu Gln Met Arg Ala Val Leu Gly Ala Asp Ala
    450                 455                 460

Glu Pro Glu Gln Pro Leu Phe Pro Gly Gly Leu Val Thr Ser Pro Asp
465                 470                 475                 480

Gly Met Arg Trp Leu Thr His His Pro
                485

<210>  12
<211>  420
<212>  PRT
<213>  Nonomuria

<400>  12

Met Arg Ile Asp Ser Glu Trp Ser Phe Asp Pro Gly Met Asp Asp Asp
1               5                   10                  15

Ile Asp Ala Gly Ala Pro Val Leu Gln Pro Thr Ala Asn Tyr Met Met
            20                  25                  30

Arg Thr His Cys Asp Pro His Glu Asp Met Phe Ala Leu Arg Ala His
        35                  40                  45

Gly Pro Leu Val Arg Ile Gly Gly Asp Ala Ala Thr Gln Leu Arg Val
    50                  55                  60

Asp Tyr Val Trp Gln Ala Leu Gly Tyr Asp Val Val Arg Arg Ile Leu
65                  70                  75                  80

Gly Asp His Glu Asn Phe Thr Thr Arg Pro Arg Trp Ser Ser Ala Pro
                85                  90                  95

Ser Ile Ala Gly Glu Pro Ile Pro Pro Asn Leu Val Gly Gln Leu Ser
            100                 105                 110

Val Tyr Asp Pro Pro Glu His Thr Arg Leu Arg Gly Met Leu Thr Pro
        115                 120                 125

Glu Phe Thr Ala Arg Arg Ile Arg Arg Leu Glu Pro Ala Met Gln Asp
    130                 135                 140

Leu Ile Asp Asp Arg Ile Asp Glu Leu Glu Ala Ala Gly Pro Pro Ala
145                 150                 155                 160

Asp Val Gln Ala Leu Phe Ala Asp Pro Val Gly Gly Gly Val Leu Cys
                165                 170                 175

Glu Leu Leu Gly Ile Pro Arg Asp Asp Arg Ile Glu Phe Ile Arg Arg
            180                 185                 190

Val Arg Gln Asn Val Asp Leu Ser Arg Gly Phe Lys Ala Arg Ala Ala
        195                 200                 205

Asp Ser Ala Ala Phe Asn Arg Tyr Leu Asn Gly Leu Ile Ile Arg Gln
    210                 215                 220

Arg Lys Asp Pro Asp Glu Gly Phe Ile Gly Met Leu Val Arg Glu His
225                 230                 235                 240

Gly Asp Asp Val Thr Asp Glu Glu Leu Lys Gly Val Leu Thr Ala Leu
                245                 250                 255

Ile Leu Gly Gly Val Glu Thr Val Ala Gly Ser Ile Gly Phe Gly Val
            260                 265                 270

Leu Ala Leu Leu Asp His Pro Asp Gln Arg Gln Ser Leu Phe Ala Gly
        275                 280                 285

Arg Glu Glu Ala Asp Arg Val Val Gly Glu Leu Leu Arg Phe Leu Ser
    290                 295                 300

Pro Val Gln Gln Pro Asn Pro Arg Leu Ala Val Arg Asp Val Val Val
305                 310                 315                 320

Asp Gly Gln Leu Ile Lys Ala Gly Asp Tyr Val Leu Cys Ser Ile Leu
                325                 330                 335

Met Ala Asn Arg Asp Glu Ala Leu Thr Pro Asn Ala Asn Val Leu Asp
            340                 345                 350

Val Arg Arg Asp Cys Gly Ser His Val Gly Phe Gly His Gly Ile His
        355                 360                 365

Tyr Cys Ile Gly Ala Ala Ile Ala Arg Thr Leu Leu Arg Met Ala Tyr
    370                 375                 380

Gln Ser Leu Trp Arg Arg Phe Pro Gly Leu Arg Leu Ala Val Ser Ala
385                 390                 395                 400

Glu Glu Val Lys Phe Arg Asn Ala Phe Ile Asp Cys Pro Asp Glu Leu
                405                 410                 415

Pro Val Thr Trp

            420

<210> 13
<211> 398
<212> PRT
<213> Nonomuria

<400> 13

Met Ser Gly Asp Gly Ala Arg Pro Leu His Thr Arg Arg Gln Asp Leu
1               5                   10                  15

Asp Pro Ala Asp Glu Leu Arg Ala Ala Gly Thr Leu Thr Arg Ile Thr
            20                  25                  30

Ile Gly Ser Gly Ala Asp Ala Glu Thr Thr Trp Leu Ala Thr Gly Tyr
        35                  40                  45

Thr Val Val Arg Gln Val Leu Gly Asp His Arg Arg Phe Ser Thr Arg
    50                  55                  60

Arg Arg Trp Asn Glu Arg Asp Glu Ile Gly Gly Arg Gly Asn Phe Arg
65                  70                  75                  80

Pro Arg Glu Leu Val Gly Asn Leu Met Asp Tyr Asp Pro Pro Glu His
                85                  90                  95

Thr Arg Leu Arg Gln Lys Leu Thr Pro Gly Phe Thr Leu Arg Arg Ile
            100                 105                 110

Arg Arg Leu Lys Pro Tyr Ile Glu Gln Ile Val Thr Glu Arg Leu Asp
        115                 120                 125

Ala Leu Glu Arg Ala Gly Pro Pro Ala Asp Leu Val Glu Leu Val Ala
    130                 135                 140

145                 150                 155                 160

Asp Asp Arg Ala Met Phe Met Gln Leu Cys His Gly His Leu Asp Ala
                165                 170                 175

Ser Arg Ser Gln Lys Arg Arg Ala Ala Ala Gly Ala Ala Phe Ser Arg
            180                 185                 190

Tyr Leu Leu Ala Met Ile Ala Arg Glu Arg Lys Asp Pro Gly Glu Gly
        195                 200                 205

Leu Leu Gly Ala Val Leu Ala Glu Tyr Gly Asp Thr Ala Thr Asp Glu
    210                 215                 220

Glu Leu Arg Gly Phe Cys Val Gln Val Met Leu Ala Gly Asp Asp Asn
225                 230                 235                 240

Ile Ser Gly Met Ile Gly Leu Gly Val Leu Ala Leu Leu Arg His Pro
                245                 250                 255

Glu Gln Ile Ala Ala Leu Gln Gly Asp Asp Gln Ser Ala Asp Arg Ala
            260                 265                 270

Val Asp Glu Leu Ile Arg Tyr Leu Thr Val Pro Tyr Ala Pro Thr Pro
        275                 280                 285

Arg Val Ala Met Glu Asp Val Thr Ile Gly Gly Gln Val Ile Lys Glu
    290                 295                 300

Gly Glu Thr Val Ser Cys Ser Leu Pro Met Ala Asn Arg Asp Pro Ala
305                 310                 315                 320

Leu Leu Pro Asp Ala Gly Arg Leu Asp Val Arg Arg Glu Pro Val Pro
                325                 330                 335

His Val Ala Phe Gly His Gly Val His His Cys Leu Gly Ala Ala Leu
            340                 345                 350

Ala Arg Leu Glu Leu Arg Thr Val Tyr Thr Ala Leu Trp Arg Arg Phe
        355                 360                 365

Pro Thr Leu Arg Leu Ala Asp Pro Asp Arg Glu Pro Ser Phe Arg Leu
    370                 375                 380

Thr Thr Pro Ala Tyr Gly Leu Thr Ser Leu Met Val Ala Trp
385                 390                 395

<210>  14
<211>  384
<212>  PRT
<213>  Nonomuria

<400>  14

Met Val Val Pro Leu Pro His Gln Arg Leu Arg Leu Asp Pro Val Pro
1               5                   10                  15

Ala Leu Phe Asp Leu Gln Glu Asp Gly Pro Leu His Glu Tyr Asp Thr
            20                  25                  30

Glu Pro Gly Leu Asp Gly His Lys Gln Trp Leu Val Thr Gly Tyr Gly
        35                  40                  45

Glu Ile Arg Glu Ile Leu Ala Asp Ala Asn Arg Phe Ser Ser Met Arg
    50                  55                  60

Pro Val Glu Asp Glu Ala Glu Arg Ala Trp Leu Pro Gly Ile Leu Gln
65                  70                  75                  80

Ser Tyr Asp Ala Pro Asp His Thr Arg Leu Arg Arg Thr Val Thr Arg
                85                  90                  95

Ala Asn Thr Ala Arg Arg Ile Glu Ser Leu Arg Pro Val Val Glu Glu
            100                 105                 110

Thr Val Glu Asp Cys Leu Ala Asp Leu Glu Ser Met Gly Ser Pro Val
        115                 120                 125

Asp Phe Val Arg Asn Ala Ala Trp Pro Ile Pro Ala Leu Ile Ala Cys
    130                 135                 140

Asp Phe Leu Gly Val Pro Arg Asp Asp Gln Ala Glu Leu Ser Arg Met
145                 150                 155                 160

Phe Arg Asp Ser Arg Glu Ser Arg Val Pro Arg Gln Arg Asn Val Ser
                165                 170                 175

Gly Leu Gly Ile Val Asp Tyr Ala Arg Lys Leu Ala Ala Arg Glu Arg
            180                 185                 190

Leu Asp Pro Gly Thr Gly Met Ile Gly Gly Ile Val Arg Glu His Gly
        195                 200                 205

Gly Glu Val Thr Asp Glu Glu Leu Ala Gly Leu Val Glu Gly Ile Met
    210                 215                 220

Ile Gly Ala Val Glu Gln Met Ala Ser Gln Leu Ala Ile Ala Val Leu
225                 230                 235                 240

Leu Leu Val Thr His Pro Asp Gln Met Ala Leu Leu Arg Glu Arg Pro
                245                 250                 255

Glu Leu Ala Asp Ser Ala Ala Glu Glu Val Phe Arg Tyr Ala Ser Ile
            260                 265                 270

Val Glu Thr Pro Ser Pro Arg Thr Ala Leu Val Asp Thr Arg Leu Ala
        275                 280                 285

Gly Arg Asp Ile His Ala Gly Asp Val Leu Thr Cys Ser Ile Leu Ala

290                 295                 300

Gly Asn Arg Ala Arg Glu Asp Arg Phe Asp Leu Thr Arg Gly Asn Pro
305                 310                 315                 320

Glu His Leu Ala Phe Gly His Gly Val His Phe Cys Leu Gly Ala Pro
                325                 330                 335

Leu Ala Arg Leu Gln Ala Gln Val Ala Leu Pro Ala Leu Val Arg Arg
            340                 345                 350

Phe Pro Ser Leu Arg Leu Ala Val Pro Ala Glu Asp Leu Arg Phe Lys
        355                 360                 365

Pro Gly Lys Pro Ala Pro Phe Ala Val Glu Glu Leu Pro Val Glu Trp
    370                 375                 380

<210>  15
<211>  393
<212>  PRT
<213>  Nonomuria

<400>  15

Met Glu Val Phe Glu Glu Leu Asn Val Val Leu Pro Gly Glu Leu His
1               5                   10                  15

Trp Arg Asp Arg Phe Asp Pro Val Pro Gln Leu Arg Ser Phe Met Ala
            20                  25                  30

Glu Gly Pro Met Thr Glu Leu Gly Ala Glu Glu Gly Pro Gly Gly Arg
        35                  40                  45

Thr Ala Trp Leu Ala Thr Gly Phe Asp Glu Val Arg Gln Val Leu Gly
    50                  55                  60

Ser Asp Lys Phe Ser Ser Arg Leu Leu Tyr Gly Gly Thr Ala Ala Gly
65                  70                  75                  80

Ile Val Phe Pro Gly Phe Ile Thr Gln Tyr Asp Pro Pro Glu His Thr
                85                  90                  95

Arg Leu Arg Arg Val Val Ser Pro Ala Phe Thr Val Arg Arg Met Glu
            100                 105                 110

Arg Phe Arg Pro Gln Val Asp Gln Val Val Glu Asp Cys Leu Asp Ala
        115                 120                 125

Ile Glu Ser Ile Gly Gly Pro Leu Asp Phe Val Pro His Phe Gly Trp

130                 135                 140

Ser Ile Ala Thr Thr Ala Thr Cys Asp Phe Leu Gly Ile Pro Arg Asp
145                 150                 155                 160

Asp Gln Ala Glu Leu Ser Arg Ser Leu His Ala Ser Arg Ser Gln Arg
                165                 170                 175

Ala Ala Ser Arg Arg Gly Ala Ala Gly Asn Lys Phe Met Thr Tyr Met
            180                 185                 190

Gly Gln Val Val Ala Arg Thr Arg Arg Asp Pro Gly Asp Asp Met Leu
        195                 200                 205

Ser Val Val Val Arg Glu His Gly Asp Glu Ile Thr Asp Ala Glu Leu
    210                 215                 220

Thr Gly Leu Ala Ala Phe Val Met Gly Ala Gly Gly Asp Gln Val Ala
225                 230                 235                 240

Arg Phe Leu Ala Ala Gly Ala Trp Leu Met Ala Glu Val Pro Glu Gln
                245                 250                 255

Phe Ala Leu Leu Arg Asp Lys Pro Asp Val Val Pro Asp Trp Leu Glu
            260                 265                 270

Glu Met Val Arg Tyr Leu Thr Ile Asp Glu Lys Leu Thr Pro Arg Ile
        275                 280                 285

Ala Leu Glu Asp Val Arg Ile Gly Asp Arg Ile Val Lys Ala Gly Asp
    290                 295                 300

Thr Val Thr Cys Ser Leu Leu Gly Ala Asn Arg Arg His Phe Pro Gly
305                 310                 315                 320

Pro Asp Asp Gln Phe Asp Leu Thr Arg Asp Arg Ala Pro Asn Val Ala
                325                 330                 335

Phe Gly His Gly Ile His His Cys Leu Gly Arg Pro Leu Ala Glu Leu
            340                 345                 350

Ile Phe Arg Ser Ala Ile Pro Ala Leu Ala Arg Arg Phe Pro Ala Leu
        355                 360                 365

Arg Leu Ala Glu Pro Glu Gln Glu Ile Arg Leu Gly Pro Pro Pro Phe
    370                 375                 380

Asp Val Lys Ala Leu Leu Leu Asp Trp

385                 390

<210>  16
<211>  69
<212>  PRT
<213>  Nonomuria

<400>  16

Met Thr Asn Pro Phe Glu Asn Glu Asp Gly Ser Phe Leu Val Leu Val
1               5                   10                  15

Asn Asp Glu Gly Gln His Ser Leu Trp Pro Ser Phe Ala Glu Val Pro
            20                  25                  30

Pro Gly Trp Thr Arg Val His Gly Val Ala Thr Arg Gln Glu Cys Leu
        35                  40                  45

Ala Tyr Val Glu Glu Asn Trp Thr Asp Ile Arg Pro Lys Ser Leu Ile
    50                  55                  60

Ala Glu Ala Gly Ala
65

<210>  17
<211>  1863
<212>  PRT
<213>  Nonomuria

<400>  17

Met Thr Ile Asp Asp Thr Arg Ala Lys Pro Arg Ser Ser Val Glu Asp
1               5                   10                  15

Val Trp Pro Leu Ser Pro Leu Gln Glu Gly Met Leu Tyr His Thr Ala
            20                  25                  30

Leu Asp Asp Asp Gly Pro Asp Thr Tyr Thr Val Gln Thr Val Tyr Gly
        35                  40                  45

Ile Asp Gly Pro Leu Asp Ala Gly Arg Leu Arg Ala Ser Trp Gln Ala
    50                  55                  60

Leu Val Asp Arg His Ala Ala Leu Arg Ala Tyr Phe Arg Tyr Val Ser
65                  70                  75                  80

Gly Ala Gln Met Val Gln Val Ile Ala Arg Glu Ala Glu Ile Pro Trp
                85                  90                  95

Arg Glu Thr Asp Leu His Gly Leu Pro Asp Asp Leu Leu Asp Ser Glu
            100                 105                 110

Val Asp Arg Leu Ala Ala Asp Glu Leu Ala Glu Arg Leu Pro Leu Asp
        115                 120                 125

Ala Ala Pro Leu Met Lys Leu His Leu Ile Arg Leu Gly Pro Ala Ser
    130                 135                 140

His Arg Leu Val His Thr Leu His His Val Leu Leu Asp Gly Trp Ser
145                 150                 155                 160

Met Pro Ile Leu His Arg Glu Leu Ala Ala Ile Tyr Ala Ala Gly Gly
                165                 170                 175

Asp Ala Ser Gly Leu Pro Ala Ala Val Ser Tyr Arg Asp Tyr Leu Ala
            180                 185                 190

Trp Leu Gly Arg Gln Asp Lys Glu Ala Ala Arg Ala Ala Trp Arg Gln
        195                 200                 205

Glu Leu Ala Gly Leu Asp Thr Pro Thr Leu Val Ala Pro Ala Asp Pro
    210                 215                 220

Ala Arg Val Pro Asp Met Gly Thr Ala Val Ile Glu Leu Ser Ala Glu
225                 230                 235                 240

Leu Thr Asp Gly Leu Ala Arg Leu Ala Arg Gly His Gly Leu Thr Leu
                245                 250                 255

Asn Thr Val Val Gln Gly Ala Trp Ala Met Val Leu Ala Gln Leu Ala
            260                 265                 270

Gly Arg Thr Asp Val Val Phe Gly Ala Thr Ala Ser Gly Arg Pro Ala
        275                 280                 285

Glu Leu Ala Gly Val Glu Ser Met Val Gly Gln Leu Leu Gly Thr Leu
    290                 295                 300

Pro Val Arg Val Arg Leu Glu Gly Gly Arg Arg Val Val Glu Leu Leu
305                 310                 315                 320

Ala Glu Leu Gln Arg Ser Gln Ser Ala Leu Met Ala His Gln His Leu
                325                 330                 335

Gly Leu Gln Glu Met Gln Ala Ala Val Gly Pro Gly Ala Val Phe Asp
            340                 345                 350

Thr Leu Val Ile Tyr Glu Asn Phe Pro Arg Gln Gly Leu Gly Arg Ala
        355                 360                 365

Glu Glu Asp Gly Gly Leu Asp Leu Arg Pro Val Arg Arg Gly Arg Asn
    370                 375                 380

Ser Ser His Tyr Pro Phe Thr Leu Ile Thr Gly Pro Gly Ala Gln Met
385                 390                 395                 400

Pro Leu Ile Leu Asp Tyr Asp Arg Gly Leu Phe Asp Glu Ala Ala Ala
                405                 410                 415

Glu Ser Val Val Gly Ala Leu Ala Arg Val Leu Glu Arg Leu Val Ala
            420                 425                 430

Glu Pro Asp Val Leu Val Gly Arg Leu Thr Leu Leu Ser Glu Ala Glu
        435                 440                 445

Arg Ala Leu Val Val Glu Asp Trp Asn Ala Thr Ala Gly Pro Thr Pro
    450                 455                 460

Gly Gln Ser Val Leu Asp Leu Phe Gly Arg Arg Val Ala Thr Ala Pro
465                 470                 475                 480

Asp Ala Val Ala Ile Thr Asp Ala Gly Gly Ala Asp Leu Thr Tyr Ala
                485                 490                 495

Glu Val Asp Gln Ala Ala Asn Arg Leu Ala Arg His Leu Ala Ala Arg
            500                 505                 510

Gly Ile Gly Arg Gly Asp Arg Val Gly Val Val Met Asp Arg Ser Pro
        515                 520                 525

Asp Leu Leu Ile Ala Phe Leu Ala Ser Trp Lys Ala Gly Ala Ala Tyr
    530                 535                 540

Val Pro Val Asp Val Glu His Pro Ala Glu Arg Ile Glu Phe Val Leu
545                 550                 555                 560

Ala Asp Ser Gly Val Ser Ala Val Leu Cys Thr Arg Ala Thr Arg Glu
                565                 570                 575

Val Ala Pro Ala Asp Ala Ile Val Ile Asp Ala Pro Glu Thr Arg Ala
            580                 585                 590

Ala Ile Asp Ala Gly Ala Ala Thr Ala Pro Gln Ile Arg Leu Ser Ala
        595                 600                 605

Asp Asp Leu Ala Tyr Val Met Tyr Thr Ser Gly Ser Thr Gly Leu Pro

    610                 615                 620

Lys Gly Val Gly Val Pro His Gly Ala Val Ala Gly Leu Ala Gly Asp
625                 630                 635                 640

Glu Gly Trp Arg Ile Gly Pro Gly Asp Ala Val Leu Met His Ala Thr
                645                 650                 655

His Val Phe Asp Pro Ser Leu Tyr Ala Met Trp Val Pro Leu Ala Met
            660                 665                 670

Gly Gly Arg Val Val Leu Thr Glu Pro Gly Val Leu Asp Ala Leu Gly
        675                 680                 685

Met Arg Gln Ala Val Glu Arg Gly Val Thr Phe Val His Leu Thr Ala
    690                 695                 700

Gly Thr Phe Arg Ala Leu Ala Glu Ser Ser Pro Glu Cys Phe Ala Gly
705                 710                 715                 720

Leu Val Glu Val Gly Thr Gly Gly Asp Val Val Pro Ala Gln Ser Val
                725                 730                 735

Glu His Leu Arg Arg Ala Val Pro Gly Leu Arg Val Arg Asn Thr Tyr
            740                 745                 750

Gly Pro Thr Glu Thr Thr Leu Cys Ala Thr Trp Lys Pro Ile Glu Pro
        755                 760                 765

Gly Glu Glu Val Gly Arg Glu Leu Pro Ile Gly Arg Pro Met Thr Asn
    770                 775                 780

Arg Arg Ile Tyr Ile Leu Asp Ala Phe Leu Arg Pro Val Ala Pro Gly
785                 790                 795                 800

Val Ala Gly Glu Leu Tyr Ile Ala Gly Thr Gly Leu Ala Arg Gly Tyr
                805                 810                 815

Leu Gly Gly Pro Gly Leu Thr Ala Glu Arg Phe Val Ala Val Pro Ala
            820                 825                 830

Ser Val Asp Pro Ser Pro Gly Glu Arg Met Tyr Arg Thr Gly Asp Leu
        835                 840                 845

Ala Arg Trp Asn Arg Asp Gly Glu Val Val Phe Leu Gly Arg Thr Asp
    850                 855                 860

Asp Gln Val Lys Ile Arg Gly Tyr Arg Val Glu Leu Gly Glu Val Glu
865                 870                 875                 880

Ala Val Leu Ala Ala Gln Arg Gly Val Val Glu Ala Val Val Val Ala
                885                 890                 895

Arg Glu Asp Gln Pro Gly Glu Lys Arg Leu Val Gly Tyr Phe Ile Ser
            900                 905                 910

Asp Gly Thr Asp Ala Gly Pro Ala Glu Ile Arg Arg Glu Met Ala Leu
        915                 920                 925

Val Leu Pro Ala Tyr Met Val Pro Leu Ala Val Val Ala Leu Pro Ala
    930                 935                 940

Leu Pro Val Thr Pro Asn Gly Lys Val Asp Arg Leu Ala Leu Pro Ala
945                 950                 955                 960

Pro Asp Leu Val Gly Arg Ala Pro Asp Arg Ala Gln Glu Ser Glu Thr
                965                 970                 975

Glu Lys Val Leu Cys Ala Leu Phe Ala Glu Ile Leu Gly Val Asp Arg
            980                 985                 990

Val Gly Val Asp Asp Ala Phe His  Asp Leu Gly Gly Ser  Ser Ala Leu
        995                 1000                 1005

Ala Met  Arg Leu Ile Ala Arg  Ile Arg Glu Glu Leu  Gly Ala Asp
    1010                 1015                 1020

Leu Pro  Ile Arg Gln Leu Phe  Ser Ala Ala Thr Pro  Ala Gly Val
    1025                 1030                 1035

Ala Arg  Ala Leu Ala Ala Lys  Ser Arg Pro Ala Leu  Glu Pro Ala
    1040                 1045                 1050

Glu Arg  Pro Gly Arg Val Pro  Leu Thr Ala Gln Gln  Leu Ser Ala
    1055                 1060                 1065

Trp Leu  Leu Ala Ser Pro Gly  Glu Ala Ala Gly Leu  His Val Ser
    1070                 1075                 1080

Val Ala  Leu Arg Leu Arg Gly  Arg Leu Asp Val Pro  Ala Leu Glu
    1085                 1090                 1095

Ala Ala  Leu Gly Asp Val Ala  Ala Arg His Glu Ile  Leu Arg Thr
    1100                 1105                 1110

Thr Phe Pro Gly His Ala Gln Ser Val His Gln His Val His Asp
    1115                1120                1125

Ala Ser Pro Val Asp Leu Thr Pro Val Pro Ala Thr Glu Glu Ser
    1130                1135                1140

Leu Pro Gly Leu Leu Thr Glu Leu Arg Glu Ser Val Phe Asp Leu
    1145                1150                1155

Thr Arg Glu Val Pro Trp Arg Gly Asp Leu Phe Arg Leu Ser Asp
    1160                1165                1170

Gly Glu His Val Leu His Leu Met Val His Arg Ile Leu Ala Asp
    1175                1180                1185

Asp Glu Ser Leu Asp Val Phe Leu Arg Asp Leu Ser Ala Ala Tyr
    1190                1195                1200

Gly Ala Arg Arg Ala Gly Arg Ala Pro Glu Arg Ala Pro Leu Thr
    1205                1210                1215

Leu Gln Phe Ala Asp Tyr Ala Ile Trp Glu Arg Arg Leu Leu Glu
    1220                1225                1230

Gly Glu Arg Asp Ala Asp Gly Leu Ile Asn Glu Gln Leu Val Phe
    1235                1240                1245

Trp Arg Asp Asn Leu Ala Gly Ile His Gly Glu Thr Val Leu Pro
    1250                1255                1260

Phe Asp Arg Pro Arg Ser Ala Val Ala Ser Arg Arg Ala Gly Thr
    1265                1270                1275

Val Ser Leu Arg Leu Asp Ala Gly Pro His Ala Arg Leu Val Glu
    1280                1285                1290

Ala Val Asp Pro Ile Gly Ala His Pro Phe Gln Ile Val His Ala
    1295                1300                1305

Ala Leu Ala Met Leu Leu Thr Arg Leu Gly Ala Gly His Asp Leu
    1310                1315                1320

Val Ile Gly Thr Lys Leu Pro Arg Asp Asp Asp Leu Ile Asp Leu
    1325                1330                1335

Glu Pro Met Ile Gly Pro Phe Ala Arg Pro Leu Ala Leu Arg Thr
    1340                1345                1350

Asp Leu Ser Gly Asp Pro Thr Phe Leu Glu Val Val Thr Arg Ala
    1355                1360                1365

Gln Glu Ala Ile Arg Ser Ala Arg Gln His Leu Asp Val Pro Phe

Ala Arg Ile Val Glu Leu Leu Asp Leu Pro Val Ser Leu Ser Arg
    1385                1390                1395

His Pro Val Phe Gln Val Gly Leu Glu Val His Glu Glu Asp Leu
    1400                1405                1410

Gly Ala Trp Asp Ala Thr Glu Leu Pro Ala Leu Arg Thr Ser Val
    1415                1420                1425

Glu Pro Val Gly Pro Glu Ala Ile Glu Leu Asp Leu Ala Phe Arg
    1430                1435                1440

Leu Thr Glu Arg Arg Asp Glu Asp Gly Ile Glu Gly Thr Leu His
    1445                1450                1455

Tyr Ala Ala Asp Leu Phe Asp Gln Ala Thr Ala Glu Ser Leu Ala
    1460                1465                1470

Arg Arg Leu Val Ser Phe Leu Glu Gln Val Ala Glu Asp Pro Gln
    1475                1480                1485

Arg Arg Val Ser Asp Leu Asp Val Leu Leu Asp Asp Ala Glu Arg
    1490                1495                1500

Glu Arg Pro Ala Glu Ala Pro Ala Lys Trp Ser Glu Ala Val Pro
    1505                1510                1515

Pro Val Ala Ala Asp Leu Ala Glu Gly Gly Pro Leu Gly Ala Leu
    1520                1525                1530

Val Leu Asp Asp Arg Leu Arg Pro Ala Val Ala Val Gly Glu Leu
    1535                1540                1545

Tyr Leu Thr Gly Ala Ala Val Asp Ala Glu Pro Gly Asp Arg Thr
    1550                1555                1560

Leu Ala Cys Pro Phe Gly Ala Thr Gly Arg Arg Met Leu Pro Thr
    1565                1570                1575

Gly Leu Leu Ala Arg Trp Thr Ala Gly Gly Thr Leu Val Val Val
    1580                1585                1590

Gly Glu Arg Arg Gly Ser Ser Gly Ser Val Lys Thr Gly Thr Gly
    1595                1600                1605

Asp Phe Glu Val Leu Leu Pro Leu Arg Ala Gly Gly Asn Arg Pro
    1610                1615                1620

Pro Leu Tyr Cys Val His Ala Ser Gly Gly Leu Ser Trp Asn Tyr
    1625                1630                1635

Ala Pro Leu Leu Arg Ser Leu Pro Pro Asn Gln Pro Val Tyr Gly
    1640                1645                1650

Val Gln Ala Arg Gly Leu Ala Arg Thr Glu Pro Leu Ala Ala Gly
    1655                1660                1665

Val Glu Glu Met Ala Ala Asp Tyr Val Glu Gln Ile Arg Ala Val
    1670                1675                1680

Gln Pro Thr Gly Pro Tyr His Leu Leu Gly Trp Ser Leu Gly Gly
    1685                1690                1695

Arg Ile Ala Gln Glu Met Ala Arg Val Leu Glu Glu Gln Gly Glu
    1700                1705                1710

Gln Val Gly Leu Leu Ala Leu Leu Asp Ala Tyr Pro Thr Asp Val
    1715                1720                1725

Gly Arg Leu Arg Arg Pro Arg Gly Asp Ala Ala Asp Gln Glu Ala
    1730                1735                1740

Ala Asp Phe Asp Arg Gln Gln Glu Gln Gln Ala Gln Leu Ala Ala
    1745                1750                1755

Ala Val Ala Thr Glu Ala Gly Ala Arg Lys Arg Leu Asp Glu Val
    1760                1765                1770

Met Glu His Leu Ala Arg Val Gly Pro Leu His Thr Ser Arg Ser
    1775                1780                1785

Phe Gly Cys Asp Ile Leu Leu Phe Val Ala Thr Val Asn Arg Pro
    1790                1795                1800

Ser His Leu Pro Val Ala Asp Ala Ile Ala Ser Trp Arg Pro Leu
    1805                1810                1815

Thr Thr Gly Thr Val Glu Pro His Glu Ile Glu Ile Asp His Met
    1820                1825                1830

Gln Met Leu Gln Pro Ala Ala Leu Ala Arg Ile Gly Ala Val Val
    1835                1840                1845

Ala Glu Lys Leu Arg Pro Arg Pro Asp Gly Glu Arg Thr Gln Arg
    1850                1855                1860

<210> 18
<211> 4083
<212> PRT
<213> Nonomuria

<400> 18

Met Ala Gln Ser Arg Ile Glu Asp Phe Trp Pro Leu Ser Pro Leu Gln
1               5                   10                  15

Gln Gly Leu Leu Phe His Thr Thr Tyr Asp Asp Asp Trp Pro Gly Leu
            20                  25                  30

Tyr Val Gly His Trp Ile Leu Asn Leu Asn Gly Pro Val Glu Ala Asp
        35                  40                  45

Arg Leu Arg Ala Ala Trp Glu Ala Leu Leu Ala Arg His Ala Ala Leu
    50                  55                  60

Arg Ala Cys Phe Arg Gln Arg Lys Ser Gly Glu Thr Val Gln Leu Ile
65                  70                  75                  80

Ala Arg Gln Val Glu Leu Pro Trp Arg Val Val Asp Leu Ser His Leu
                85                  90                  95

Ser Glu Pro Glu Glu Ala Val Arg Ala Val Ala Glu Glu Asp Arg Thr
            100                 105                 110

Arg Arg Phe Asp Leu Ala Lys Ala Pro Leu Leu Arg Leu Thr Leu Ile
        115                 120                 125

Arg Leu Ala Gly Asp Asp His Arg Leu Val Met Thr Cys His His Ala
    130                 135                 140

Ile Met Asp Gly Trp Ser Met Pro Ile Met Leu Asp Glu Leu Ser Met
145                 150                 155                 160

Leu Tyr Ala Ala Asp Gly Ser Pro Leu Asp Leu Pro Ala Val Pro Ser
                165                 170                 175

Tyr Arg Asp Tyr Leu Val Trp Leu Asp Arg Gln Asp Lys Glu Arg Thr
            180                 185                 190

Leu Ser Ala Trp Ala Ala Glu Leu Arg Gly Val Glu Glu Pro Thr Leu
        195                 200                 205

Val Ala Pro Ala Asp Ala Asn Arg Ala Pro Ala Met Pro Glu Asn Ile
    210                 215                 220

Thr Val Glu Leu Pro Glu Asp Leu Thr Arg Ala Leu Ser Glu Leu Ala
225                 230                 235                 240

Arg Thr His Gly Leu Thr Leu Asn Thr Val Val Gln Gly Ala Trp Ala
                245                 250                 255

Leu Leu Leu Ala Gln Leu Ala Gly Arg Thr Asp Val Val Phe Gly Ala
            260                 265                 270

Ala Val Ser Ala Arg Pro Pro Asp Leu Pro Gly Val Glu Gly Met Val
        275                 280                 285

Gly Leu Phe Leu Asn Thr Val Pro Val Arg Val Arg Leu Ser Gly Ser
    290                 295                 300

Thr Pro Val Ile Glu Phe Leu Ala Asp Leu Gln Lys Arg Gln Ser Ala
305                 310                 315                 320

Leu Ile Pro His Gln Tyr Met Gly Leu Ala Asp Ile Gln Arg Thr Ala
                325                 330                 335

Gly Ala Gly Ala Val Phe Asp Thr Leu Leu Val Phe Gln Asn Phe Pro
            340                 345                 350

Arg Glu Leu Arg Pro Ser Asp Ala Ala Ala Ala Phe Asp Ile Arg Ile
        355                 360                 365

Asp Gln Gly Arg Glu Ala Ala His Tyr Pro Leu Thr Leu Val Ala Val
    370                 375                 380

Pro Gly Glu Ser Met Leu Leu Asn Leu Asp His Val Thr Asp Leu Phe
385                 390                 395                 400

Asp Arg Glu Ala Ala Leu Ala Ile Leu Glu Arg Phe Thr Gly Ile Leu
                405                 410                 415

Arg Gln Leu Ala Gly Ala Gly Asp Leu Thr Val Ala Glu Val Asp Val
            420                 425                 430

Thr Ser Ala Ala Glu Arg Ala Leu Val Val Asn Ala Trp Ser Ala Ala

435                 440                 445

Pro Arg Val Ala Pro Gly Glu Leu Ala Pro Asp Leu Phe Asp Arg Gln
    450                 455                 460

Val Glu Arg Gly Arg Asp Arg Val Ala Val Val Glu Gly Lys Arg Ala
465                 470                 475                 480

Val Ser Phe Gly Glu Leu Ala Glu His Ala Glu Arg Leu Ala Gly Tyr
                485                 490                 495

Leu Ser Gly Arg Gly Val Arg Arg Gly Asp Arg Val Ala Val Val Met
            500                 505                 510

Gly Arg Ser Pro Gly Leu Ile Ala Thr Leu Leu Ala Val Trp Lys Ala
        515                 520                 525

Gly Ala Ala Phe Val Pro Val Asp Pro Ala Tyr Pro Ala Glu Arg Val
    530                 535                 540

Gln Phe Met Leu Ala Asp Ala Glu Pro Ala Ala Val Val Thr Glu Arg
545                 550                 555                 560

Ala Cys Gln Ala Ala Val Pro Ala Gly Gly Leu Asp Pro Ile Val Leu
                565                 570                 575

Asp Asp Pro Asp Thr Leu Arg Ala Val Ala Glu His Ala Arg Leu Ser
            580                 585                 590

Ala Gly Ala His Ala Asp Asp Leu Ala Tyr Val Met Tyr Thr Ser Gly
        595                 600                 605

Ser Thr Gly Arg Pro Lys Gly Val Ala Val Ser His Gly Asn Val Ala
    610                 615                 620

Ala Leu Ala Gly Glu Pro Gly Trp Gly Leu Gly Pro Glu Asp Ala Val
625                 630                 635                 640

Leu Met His Ala Ser His Ala Phe Asp Ile Ser Leu Phe Glu Leu Trp
                645                 650                 655

Val Pro Leu Leu Ser Gly Ala Arg Val Val Leu Ala Glu Pro Gly Ala
            660                 665                 670

Val Asp Gly Glu Ala Leu Ala Gly Tyr Val Ala Gly Gly Val Thr Cys
        675                 680                 685

Ala His Leu Thr Ala Gly Thr Phe Arg Val Leu Ala Glu Glu Ser Pro
    690                 695                 700

Glu Ser Val Ala Gly Leu Arg Glu Val Leu Thr Gly Gly Asp Ala Val
705                 710                 715                 720

Pro Leu Ala Ala Val Glu Arg Val Arg Arg Ala Cys Pro Asp Val Arg
                725                 730                 735

Val Arg His Leu Tyr Gly Pro Thr Glu Ala Thr Leu Cys Ala Thr Trp
            740                 745                 750

Trp Leu Leu Gln Pro Gly Glu Pro Thr Gly Pro Val Leu Pro Ile Gly
        755                 760                 765

Arg Pro Leu Ala Gly Arg Arg Val Tyr Val Leu Asp Ala Phe Leu Arg
    770                 775                 780

Pro Val Pro Pro Gly Val Thr Gly Glu Leu Tyr Val Ala Gly Ala Gly
785                 790                 795                 800

Val Ala Gln Gly Tyr Leu Gly Arg Pro Ala Leu Thr Ala Glu Arg Phe
                805                 810                 815

Val Ala Glu Pro Phe Val Pro Gly Gly Arg Met Tyr Arg Thr Gly Asp
            820                 825                 830

Leu Ala Arg Trp Thr Asp Gln Gly Glu Leu Ala Phe Ala Gly Arg Ala
        835                 840                 845

Asp Asp Gln Val Lys Ile Arg Gly Tyr Arg Val Glu Pro Gly Glu Ile
    850                 855                 860

Glu Ala Val Leu Ala Gly Leu Pro Gly Val Gly Gln Ala Val Val Ser
865                 870                 875                 880

Ala Arg Glu Glu Arg Leu Ile Gly Tyr Val Val Ala Glu Thr Gly Gly
                885                 890                 895

Asp Leu Asp Pro Val Arg Ile Arg Glu Gln Leu Ala Ala Thr Leu Pro
            900                 905                 910

Glu Phe Met Val Pro Ala Ala Val Leu Val Leu Asp Ala Leu Pro Leu
        915                 920                 925

Thr Gly Asn Gly Lys Val Asp Arg Arg Ala Leu Pro Glu Pro Asp Phe
    930                 935                 940

Ala Ala Gly Ala Val Asp Arg Glu Pro Ala Thr Asp Ala Glu Arg Ile
945                 950                 955                 960

Leu Cys Gly Val Phe Ala Glu Val Leu Gly Ala Gly Arg Val Gly Val
                965                 970                 975

Ala Asp Ser Phe Phe Glu Leu Gly Gly Asp Ser Ile Ser Ser Met Gln
            980                 985                 990

Val Ala Ala Arg Ala Arg Arg Gln Gly Ile Pro Leu Thr Pro Arg Gln
        995                 1000                1005

Val Phe Glu His Arg Thr Pro Glu Arg Leu Ala Ala Leu Ala Gln
    1010                1015                1020

Gln Ala Pro Gly Arg Arg Ala Ser Ser Val Glu Pro Gly Val Gly
    1025                1030                1035

Glu Ile Pro Arg Thr Pro Val Met Arg Ala Leu Gly Asp Asp Ala
    1040                1045                1050

Val Arg Pro Gly Phe Ala Gln Ala Arg Val Val Val Thr Pro Ala
    1055                1060                1065

Gly Phe Ala Pro Asp Ala Leu Val Thr Ala Leu Gln Ala Val Leu
    1070                1075                1080

Asp Val His Asp Leu Leu Arg Thr Arg Val Glu Pro Asp Gly Arg
    1085                1090                1095

Leu Met Val Ala Glu Pro Gly Ala Val Asp Ala Ala Gly Leu Val
    1100                1105                1110

Thr Arg Val Ala Ala Gly Asn Gly Asn Leu Ala Glu Arg Ala Glu
    1115                1120                1125

Arg Glu Ala Arg Thr Ala Ala Gly Thr Leu Asp Pro Ser Glu Gly
    1130                1135                1140

Ile Met Val Arg Ala Val Trp Val Asp Ala Gly Asp Ala Glu Pro
    1145                1150                1155

Gly Arg Leu Ala Leu Val Val His His Leu Val Val Asp Ala Val
    1160                1165                1170

Ser Trp Ala Ile Leu Leu Ser Asp Leu Arg Ala Ala Tyr Asp Glu
    1175                1180                1185

Ala Val Ser Gly Gly Thr Pro Val Leu Glu Pro Ala Val Thr Ser
    1190                1195                1200

Tyr Arg Gln Trp Ala Arg Arg Leu Ala Gly Gln Ala Leu Ser Glu
    1205                1210                1215

Ser Thr Val Ala Glu Ala Gly His Trp Ala Gly Val Leu Glu Gly
    1220                1225                1230

Gly Asp Leu Pro Leu Glu Arg His Pro Gly Gln Ser Ala Ser Trp
    1235                1240                1245

Ser Arg Thr Leu Ser Asp Ala Gln Ala Arg Asn Leu Val Ala Arg
    1250                1255                1260

Val Pro Ala Ala Phe His Cys Gly Val Gln Asp Val Leu Leu Ala
    1265                1270                1275

Gly Leu Ala Gly Ala Val Ala Arg Trp Arg Gly Ala Asp Ala Gly
    1280                1285                1290

Ile Leu Val Asp Val Glu Gly His Gly Arg His Ala Ala Asp Gly
    1295                1300                1305

Glu Asp Leu Leu Arg Thr Val Gly Trp Phe Thr Ser Val His Pro
    1310                1315                1320

Val Arg Leu Asp Val Ser Gly Val Gly Pro Gly Ala Ala Ala Ala
    1325                1330                1335

Gly Glu Leu Leu Lys Ala Val Lys Glu Gln Ala Arg Ala Val Pro
    1340                1345                1350

Gly Asp Gly Leu Gly Tyr Gly Leu Leu Arg Tyr Leu Asn Pro Glu
    1355                1360                1365

Thr Gly Ala Arg Leu Ala Glu Leu Pro Ser Ala Gln Ile Gly Phe
    1370                1375                1380

Asn Tyr Leu Gly Arg Ser Gly Val Ala Ser Glu Asp Thr Ala Trp
    1385                1390                1395

Gln Val Cys Glu Gly Ala Leu Gly Gly Gln Ala Ala Gly Pro Asp
    1400                1405                1410

Leu Val Gln Ser His Ala Leu Glu Val Gly Ala Asp Val Gln Asp
    1415                1420                1425

Thr Pro Ala Gly Pro Arg Leu Arg Leu Ala Ile Asp Gly Arg Asp
    1430                1435                1440

Leu Asp Pro Ala Ala Val Glu Arg Leu Gly Glu Ala Trp Leu Asp
    1445                1450                1455

Thr Leu Ala Gly Leu Ala Ala Leu Ala Asp Thr Pro Gly Ala Gly
    1460                1465                1470

Gly His Thr Pro Ser Asp Phe Glu Leu Val Glu Val Arg Gln Arg
    1475                1480                1485

Asp Val Asp Glu Leu Glu Ala Val Ala Pro Gly Leu Thr Asp Val
    1490                1495                1500

Trp Pro Leu Ser Pro Leu Gln Glu Gly Ile Leu Phe Glu Arg Ala
    1505                1510                1515

Phe Asp Glu Asp Gly Val Asp Val Tyr Gln Thr Gln Arg Ile Leu
    1520                1525                1530

Asp Leu Asp Gly Pro Leu Asp Ala Gln Arg Leu His Ala Ala Trp
    1535                1540                1545

Gln Ser Val Ile Asp Arg His Glu Thr Leu Arg Thr Gly Phe His
    1550                1555                1560

Gln Leu Gly Ser Gly Glu Thr Val Gln Val Val Val Gly Glu Ala
    1565                1570                1575

Glu Val Leu Trp Arg Glu Ala Asp Leu Ser Arg Leu Asp Glu Pro
    1580                1585                1590

Asp Ala Glu Val Glu Arg Leu Leu Ala Ala Asp Gln Ala Glu Arg
    1595                1600                1605

Phe Asp Val Ser Arg Ala Pro Leu Leu Arg Leu Leu Leu Ile Arg
    1610                1615                1620

Leu Gly Ala Ala Arg His Arg Leu Val Val Thr Ser His His Val
    1625                1630                1635

Leu Val Asp Gly Trp Ser Thr Pro Ile Leu Leu Gly Glu Met Leu
    1640                1645                1650

Thr Ala Tyr Ala Asp Gly Arg Val Ser Pro Ala Pro Pro Ser Tyr
    1655                1660                1665

Arg Asp Tyr Val Ala Trp Leu Ser Arg Gln Asp Glu Asp Ala Ala
    1670                1675                1680

Arg Ser Ala Trp Arg Ala Glu Leu Ala Gly Leu Asp Glu Pro Thr
    1685                1690                1695

Val Val Gly Leu Asp Ala Gly Lys Ala Pro Val Met Pro Asp Gly
    1700                1705                1710

His Ala Glu Trp Leu Ser Glu Glu Ala Thr Arg Ala Leu Thr Gly
    1715                1720                1725

Phe Ala Arg Gly His Gly Leu Thr Leu Ser Thr Val Val Gln Gly
    1730                1735                1740

Ala Trp Ala Leu Val Leu Ala Arg Leu Ala Arg Arg Thr Asp Val
    1745                1750                1755

Val Phe Gly Thr Val Val Ser Gly Arg Pro Ala Asp Ala Leu Pro
    1760                1765                1770

Asp Val Glu Arg Met Val Gly Met Phe Ile Asn Thr Val Pro Val
    1775                1780                1785

Arg Val Arg Leu Asp Gly Ala Val Pro Val Leu Asp Leu Leu Gln
    1790                1795                1800

Asp Leu Gln Arg Arg Gln Ser Ser Leu Thr Glu His Gln His Leu
    1805                1810                1815

Gly Leu Pro Glu Ile Gln Lys Ala Ala Gly Pro Gly Ser Ile Phe
    1820                1825                1830

Asp Thr Ile Leu Met Ile Val Asn Tyr Pro Leu Asp Ala Asp Gly
    1835                1840                1845

Leu Asp Asp Gly Gly Val Ala Ile Ser Ser Ile Arg Thr Arg Thr
    1850                1855                1860

Gly Thr Thr Tyr Pro Leu Ser Val Ser Val Ile Pro Gly Ala Arg
    1865                1870                1875

Leu Gln Ile Gln Leu Asp Tyr Arg Pro Asp Trp Ile Gly Gly Asp
    1880                1885                1890

Leu Ala Ala Glu Ile Thr Gly Gln Val Val Arg Val Leu Ala Arg

1895                    1900                    1905

Met Val  Ala Glu Pro Ser Leu  Pro Val Gly Arg Leu  Ala Val Thr
    1910                    1915                    1920

Ser Arg  Ser Thr Arg Gly Ser  Val Thr Glu Arg Trp  Asn Ser Thr
    1925                    1930                    1935

Gly Ala  Ala Ala Gly Gly Ser  Ser Val Pro Glu Leu  Phe Arg Arg
    1940                    1945                    1950

Gln Ala  Asp Ala Ala Pro Asp  Ala Thr Ala Val Ile  Gly Asp Gly
    1955                    1960                    1965

Arg Thr  Leu Ser Tyr Ala Gly  Leu Asp Arg Glu Ser  Asp Arg Leu
    1970                    1975                    1980

Ala Gly  His Leu Ala Arg Arg  Gly Val Arg Arg Gly  Asp Arg Val
    1985                    1990                    1995

Gly Val  Leu Met Glu Arg Gly  Ala Asp Leu Ile Val  Ala Leu Leu
    2000                    2005                    2010

Ala Val  Trp Lys Ala Gly Ala  Ala Gln Val Pro Val  Asn Val Asp
    2015                    2020                    2025

Tyr Pro  Ala Glu Arg Ile Glu  Arg Met Leu Ala Asp  Ala Gly Ala
    2030                    2035                    2040

Ser Val  Ala Val Cys Ala Gly  Ala Thr Arg His Ala  Val Pro Asp
    2045                    2050                    2055

Gly Ile  Glu Pro Val Val Met  Asp Ala Pro Ala Thr  Glu Ala Glu
    2060                    2065                    2070

Arg His  Glu Ala Pro Pro Leu  Ala Val Gly Ala His  Asp Val Ala
    2075                    2080                    2085

Tyr Val  Met Tyr Thr Ser Gly  Ser Thr Gly Val Pro  Lys Gly Val
    2090                    2095                    2100

Ala Val  Pro His Gly Ser Ala  Ala Ala Leu Ala Gly  Asp Pro Gly
    2105                    2110                    2115

Trp Ser  Gln Gly Ala Gly Asp  Arg Val Leu Met His  Ala Ser His
    2120                    2125                    2130

Ala Phe Asp Ala Ser Leu Leu Glu Ile Trp Val Pro Leu Val Ser
    2135                2140                2145

Gly Ala Cys Val Met Val Ala Glu Pro Gly Ala Ile Asp Ala Gln
    2150                2155                2160

Arg Leu Arg Asp Val Ile Ala Arg Gly Ala Thr Thr Val His Leu
    2165                2170                2175

Thr Ala Gly Thr Phe Arg Val Leu Ala Glu Glu Ser Pro Asp Ser
    2180                2185                2190

Phe Ser Gly Leu Arg Glu Val Leu Thr Gly Gly Asp Val Val Pro
    2195                2200                2205

Leu Glu Ser Val Ala Arg Val Arg Arg Ala Cys Pro Glu Val Arg
    2210                2215                2220

Val Arg Glu Leu Tyr Gly Pro Thr Glu Val Thr Leu Cys Ala Thr
    2225                2230                2235

Trp His Leu Ile Glu Pro His Thr Glu Thr Gly Asp Thr Leu Pro
    2240                2245                2250

Ile Gly Arg Pro Leu Ala Gly Arg Gln Val Tyr Val Leu Asp Ala
    2255                2260                2265

Phe Leu Gln Pro Val Ala Pro Asn Val Thr Gly Glu Leu Tyr Leu
    2270                2275                2280

Ala Gly Ala Gly Leu Ala His Gly Tyr Leu Gly Ala Pro Ala Ala
    2285                2290                2295

Thr Ser Glu Arg Phe Ile Ala Val Pro Ala Ser Val Asn Pro Ala
    2300                2305                2310

Ala Ser Gly Glu Arg Met Tyr Arg Thr Gly Asp Leu Ala Arg Trp
    2315                2320                2325

Thr Asp Arg Gly Glu Leu Leu Phe Ala Gly Arg Ala Asp Ser Gln
    2330                2335                2340

Val Lys Ile Arg Gly Tyr Arg Val Glu Pro Gly Glu Ile Glu Ala
    2345                2350                2355

Ala Leu Ala Glu Val Pro His Val Ala Gln Ala Val Val Val Ala
    2360                2365                2370

Arg Glu Asp Arg Pro Gly Glu Lys Arg Leu Ile Ala Tyr Val Thr
    2375                2380                2385

Ala Glu Glu Gly Ser Gly Leu Asp Pro Asp Ala Val Arg Glu His
    2390                2395                2400

Leu Ala Gly Arg Leu Pro Glu Phe Met Val Pro Ala Ala Val Val
    2405                2410                2415

Leu Leu Asp Gly Val Pro Leu Thr Pro Asn Gly Lys Ile Asp Arg
    2420                2425                2430

Ala Ala Leu Pro Val Pro Glu Phe Thr Gly Lys Ala Ala Gly Arg
    2435                2440                2445

Glu Pro Arg Thr Glu Ala Glu Arg Val Leu Cys Glu Leu Phe Ala
    2450                2455                2460

Glu Val Leu Gly Val Ala Arg Ala Gly Ala Glu Asp Ser Phe Phe
    2465                2470                2475

Glu Leu Gly Gly Asp Ser Ile Leu Ser Met Arg Leu Ala Ala Arg
    2480                2485                2490

Ala Arg Arg Glu Glu Leu Val Phe Gly Ala Lys Asp Val Phe Glu
    2495                2500                2505

Arg Lys Thr Pro Ala Gly Ile Ala Met Val Ala Glu Arg Gly Gly
    2510                2515                2520

Ala Thr Arg Ala Ser Leu Asp Asp Gly Val Gly Glu Val Met Ser
    2525                2530                2535

Thr Pro Val Ile Arg Ala Leu Leu Glu Arg Asp Pro Asp Ala Met
    2540                2545                2550

Thr Arg Gly Ala Leu Ser Gln Trp Val Thr Ala Gly Ala Pro Asp
    2555                2560                2565

Asp Leu Ser Val Asp Val Leu Ala Ala Gly Leu Gly Ala Val Ile
    2570                2575                2580

Asp Ala His Asp Met Leu Arg Ser Arg Ile Val Arg Thr Gly Ala
    2585                2590                2595

Ala Gln Pro Arg Leu Val Val Ala Gly Arg Gly Ala Val Asp Ala
    2600                2605                2610

Ala Thr Leu Val Glu Arg Val Glu Ala Gly Thr Gly Asp Val Asp
    2615                2620                2625

Glu Ile Ala Asp Arg Cys Ala Arg Asp Ala Ala Ala Arg Leu Asp
    2630                2635                2640

Pro His Ala Gly Val Met Ile Arg Ala Val Trp Val Asp Ala Gly
    2645                2650                2655

Pro Gly Arg Val Gly Arg Leu Val Val Ala Ala His His Leu Val
    2660                2665                2670

Val Asp Val Val Ser Trp Arg Ile Leu Leu Pro Asp Leu Gln Val
    2675                2680                2685

Ala Cys Glu Ala Val Ala Ala Gly Arg Arg Pro Val Leu Asp Pro
    2690                2695                2700

Val Asp Val Ser Phe Arg Arg Trp Ala Arg Thr Leu Ala Asp Gln
    2705                2710                2715

Ala Val Thr Arg Ala Thr Glu Leu Glu Thr Trp Thr Glu Ile Leu
    2720                2725                2730

Asp Gly Ala Arg Ser Arg Leu Gly Glu Leu Asp Pro Ala Arg Asp
    2735                2740                2745

Thr Val Ser Thr Ala Gly Arg Thr Ser Trp Thr Leu Pro His Asp
    2750                2755                2760

Arg Ala Gly Val Leu Val Glu Gln Ala Thr Ser Ala Phe His Cys
    2765                2770                2775

Gly Val His Glu Val Leu Leu Ala Thr Leu Ala Gly Ala Val Ala
    2780                2785                2790

His Trp Arg Gly Gly Thr Ala Val Val Val Asp Val Glu Gly His
    2795                2800                2805

Gly Arg Arg Pro Ile Asp Glu Leu Asp Leu Ser Arg Thr Val Gly
    2810                2815                2820

Trp Phe Thr Asp Val His Pro Leu Arg Leu Asp Val Thr Gly Ile
    2825                2830                2835

Asp Pro Ala Glu Val Ile Ala Gly Gly Gly Ala Ala Gly His Leu

2840                2845                2850

Leu Lys Gln Val Lys Glu Asn Val Arg Ala Val Pro Asp Gly Gly
    2855                2860                2865

Leu Gly Tyr Gly Ile Leu Arg Tyr Leu Asn Ala Gly Thr Gly Gln
    2870                2875                2880

Ala Leu Ala Ala Ala Pro Lys Pro Glu Ile Gly Phe Asn Tyr Leu
    2885                2890                2895

Gly Arg Phe Pro Ser Arg Ser Ala Gly Ala Pro Glu Pro Trp Gln
    2900                2905                2910

Leu Leu Gly Thr Ile Gly Gly Thr Ala Glu Gln Asp Thr Ala Leu
    2915                2920                2925

Arg His Ala Val Glu Ile Asp Ala Ala Val Leu Asp Gly Ala Ala
    2930                2935                2940

Gly Pro Glu Leu Ser Leu Thr Val Thr Trp Ala Gly Arg Leu Leu
    2945                2950                2955

Gly Glu Ala Glu Ala Glu Ser Leu Ala Gln Ala Trp Leu Ala Met
    2960                2965                2970

Leu Thr Gly Leu Ala Ala His Val Gly Gly Gly Gly Ala Gly Gly
    2975                2980                2985

His Thr Pro Ser Asp Phe Pro Leu Ile Ser Leu Thr Gln Gln Asp
    2990                2995                3000

Val Ala Glu Val Glu Ala Ala Val Pro Thr Leu Leu Asp Ile Trp
    3005                3010                3015

Pro Leu Ser Pro Leu Gln Glu Gly Leu Leu Phe His Ala Ala Asp
    3020                3025                3030

Glu Arg Gly Pro Asp Val Tyr Ala Gly Met Arg Lys Leu Ala Leu
    3035                3040                3045

Asp Gly Pro Leu Asp Val Ala Arg Phe Arg Ala Ser Trp Gln Ala
    3050                3055                3060

Leu Leu Asp Arg His Pro Ala Leu Arg Ala Ser Phe His Gln Leu
    3065                3070                3075

Gly Ser Gly Ala Ala Val Gln Ala Ile Ala Arg Glu Val Pro Leu
    3080                3085                3090

Asp Trp Gln Glu Thr Asp Leu Ser Arg Leu Pro Glu Asp Glu Ala
    3095                3100                3105

Leu Ala Glu Phe Asp Arg Leu Ala Glu Gln Leu His Thr Glu Arg
    3110                3115                3120

Phe Asp Leu Thr Arg Ala Pro Gln Leu Arg Leu His Leu Val Arg
    3125                3130                3135

Leu Gly Glu Arg Arg His Arg Leu Val Leu Thr Ser His His Ile
    3140                3145                3150

Val Ala Asp Gly Trp Ser Leu Pro Leu Ile Thr Glu Asp Val Leu
    3155                3160                3165

Thr Val Tyr Glu Ser Gly Gly Asp Gly Arg Ala Leu Pro Ala Ala
    3170                3175                3180

Thr Ser Tyr Arg Asp Tyr Leu Ala Trp Ile Ala Arg Gln Asp Lys
    3185                3190                3195

Ala Ala Ala Arg Glu Ala Trp Arg Ala Glu Leu Ala Gly Leu Asp
    3200                3205                3210

Glu Ala Thr His Val Val Pro Pro Glu Thr Ile Thr Thr Pro Leu
    3215                3220                3225

Glu Pro Glu Arg Val Gly Phe Glu Leu Asp Glu Ala Leu Ser Arg
    3230                3235                3240

Arg Val Val Glu Phe Thr Gly Arg His Gly Val Thr Ala Asn Thr
    3245                3250                3255

Leu Phe Gln Gly Ile Trp Ala Leu His Leu Ala Arg Leu Thr Gly
    3260                3265                3270

Arg Asp Asp Val Val Phe Gly Ala Ala Val Ala Gly Arg Pro Pro
    3275                3280                3285

Glu Ile Pro Gly Val Glu Ser Ala Val Gly Leu Phe Met Asn Met
    3290                3295                3300

Leu Pro Val Arg Ala Arg Leu Ala Gly Ala Glu Pro Phe Leu Asp
    3305                3310                3315

Met Leu Thr Asp Leu Gln Glu Arg Gln Val Ala Cys Met Pro His
    3320                3325                3330

Gln His Val Gly Leu Ser Glu Ile Asn Gln Leu Ala Gly Pro Gly
    3335                3340                3345

Ala Ala Phe Asp Thr Ile Val Val Phe Glu Asn Tyr Pro Pro Pro
    3350                3355                3360

Pro Pro Arg Pro Glu Gly Pro Asp Ala Leu Val Met Arg Pro Ala
    3365                3370                3375

Gly Ile Pro Asn Asp Thr Gly His Tyr Pro Leu Ser Met Arg Ala
    3380                3385                3390

Ser Val Ala Gly Arg Val His Gly Glu Phe Ile Tyr Arg Pro Asp
    3395                3400                3405

Val Val Asp Arg Ala Glu Ala Glu Glu Met Leu Ala Ser Ile Leu
    3410                3415                3420

Arg Ala Leu Glu Gln Val Val Ala Glu Pro Arg Val Pro Val Gly
    3425                3430                3435

Arg Val Gly Leu Ile Gly Pro Glu Gln Arg Arg Leu Val Val Glu
    3440                3445                3450

Glu Trp Asn Arg Thr Gly Val Pro Pro Ala Ala Glu Pro Val Pro
    3455                3460                3465

Met Leu Phe Arg Arg Gln Val Glu Arg Ser Pro Asp Ala Val Ala
    3470                3475                3480

Val Val Asp Ala Ala Arg Ser Leu Ser Tyr Ser Gly Leu Leu Asp
    3485                3490                3495

Glu Ala Glu Glu Leu Ala Arg Leu Leu Val Gly Leu Gly Val Arg
    3500                3505                3510

Arg Glu Thr Arg Val Gly Val Leu Val Gly Arg Ser Ala Glu Leu
    3515                3520                3525

Val Val Ala Leu Leu Gly Val Ser Ser Ala Gly Gly Val Phe Val
    3530                3535                3540

Pro Met Asp Pro Asp Tyr Pro Arg Glu Arg Ile Ser Phe Ile Leu
    3545                3550                3555

Ala Asp Ser Ala Pro Glu Val Leu Leu Cys Thr Ser Glu Thr Arg
    3560                3565                3570

Gln Ala Val Pro Glu Glu Phe Ala Gly Ala Val Val Ala Leu Asp
    3575                3580                3585

Ala Pro Leu Ala Ala Asp Pro Arg Thr Ala Leu Pro Arg Val Glu
    3590                3595                3600

Ala Gly Asp Gly Ala Tyr Val Ile Tyr Thr Ser Gly Ser Thr Gly
    3605                3610                3615

Val Pro Lys Gly Val Leu Val Pro His Ala Gly Leu Gly Asn Leu
    3620                3625                3630

Ala Ser Ala Gln Ile Glu Arg Phe Gly Val Thr Ser Ala Ser Arg
    3635                3640                3645

Ile Leu Gln Phe Ala Ala Leu Gly Phe Asp Ala Ala Val Ser Glu
    3650                3655                3660

Leu Cys Met Ala Leu Leu Ser Gly Gly Thr Val Val Leu Ala Asp
    3665                3670                3675

Ala Glu Ser Met Pro Pro Arg Val Ser Leu Gly Asp Ala Val Arg
    3680                3685                3690

Arg Trp Gly Ile Thr His Val Thr Val Pro Pro Ser Val Pro Ala
    3695                3700                3705

Val Glu Asp Asp Leu Pro Asp Ser Leu Glu Thr Leu Val Val Ala
    3710                3715                3720

Gly Glu Ala Cys Pro Pro Ala Leu Val Asp Arg Trp Ser Pro Gly
    3725                3730                3735

Arg Arg Met Ile Asn Ala Tyr Gly Pro Thr Glu Thr Thr Val Cys
    3740                3745                3750

Ala Thr Met Ser Ser Pro Leu Ser Pro Gly Arg Asp Val Val Pro
    3755                3760                3765

Ile Gly Arg Pro Ile Thr Gly Leu Arg Ala Tyr Val Leu Asp Ala
    3770                3775                3780

Phe Leu Gln Pro Val Pro Pro Gly Val Thr Gly Glu Leu Tyr Val

3785                3790                3795

Ala Gly Ala Gly Leu Ala Arg Gly Tyr Leu Gly Arg Pro Gly Leu
    3800                3805                3810

Thr Ala Glu Arg Phe Val Ala Val Pro Ala Ser Val Ser Pro Ala
    3815                3820                3825

Arg Pro Gly Glu Arg Met Tyr Arg Thr Gly Asn Arg Ala Arg Trp
    3830                3835                3840

Thr Arg Asp Gly Glu Leu Val Phe Thr Gly Arg Ala Asp Ala Gln
    3845                3850                3855

Val Lys Val Arg Gly Tyr Arg Ile Glu Pro Gly Glu Ile Glu Ala
    3860                3865                3870

Val Leu Ala Asp His Pro Gly Val Ala Gln Val Ala Val Val Ala
    3875                3880                3885

Arg Glu Asp Gly Pro Gly Gln Lys Tyr Leu Val Ala Tyr Val Val
    3890                3895                3900

Pro Ala Ala Glu Gln Val Ala Gly Ala Pro Ser Glu Ala Gly Gln
    3905                3910                3915

Asp Gly Ala Leu Ile Ser Ala Leu Arg Glu Ser Ala Ala Gly Arg
    3920                3925                3930

Leu Pro Glu His Met Arg Pro Ala Ala Phe Val Pro Leu Asp Thr
    3935                3940                3945

Met Pro Leu Thr Pro Asn Gly Lys Val Asp His Arg Ala Leu Arg
    3950                3955                3960

Ala Pro Asp Phe Ala Arg Ser Ser Ser Gly Arg Asp Pro Arg Ser
    3965                3970                3975

Ala Met Glu Ala Lys Leu Cys Glu Leu Phe Ala Glu Val Leu Gly
    3980                3985                3990

Leu Glu Glu Val Gly Ala Gly Asp Ser Phe Phe Glu Leu Gly Gly
    3995                4000                4005

Asp Ser Ile Thr Ser Met Gln Leu Ser Ala Leu Ala Arg Arg Lys
    4010                4015                4020

Gly Leu Asp Leu Thr Pro Trp Gln Val Phe Asp Glu Lys Thr Ala
    4025                4030                4035

Glu Arg Leu Ala Ala Val Val Lys Glu Leu Pro Ala Asp Gly Glu
    4040                4045                4050

Gly Thr Gly Glu Pro Glu Pro Pro Ala Gly Thr Leu Val Asp Leu
    4055                4060                4065

Ser Pro Asp Gln Leu Asp Gln Leu Glu Ala Gly Pro Ala Gly Gly
    4070                4075                4080

<210> 19
<211> 753
<212> PRT
<213> Nonomuria

<400> 19

Met Ala Gly Phe Gly Ala Pro Phe Arg Asn Ser Asp His Val Val Ser
1               5                   10                  15

Lys Leu Thr Asn Glu Asp Ala Phe Glu Leu Val Glu Arg His Gly Ala
            20                  25                  30

Asn Ala Ser Pro Leu Gly Arg Ala Met Leu Thr Val Arg Ala Gly Asp
        35                  40                  45

Arg Ser Tyr Pro Glu Met Gly Val Gly Pro Val Ala Glu Ser Lys Asp
    50                  55                  60

Leu Arg Trp Gln Gln Leu Thr Ser Gly Arg Phe Pro Glu Arg Lys Gly
65                  70                  75                  80

Glu Ala Val Val Asp Leu Trp Asp Ala Gln Asn Trp Asp Val Ala Val
                85                  90                  95

Gly Asp Arg Ile Arg Ile Gly Glu Arg Ala Thr Ala Ala Asp Phe Thr
            100                 105                 110

Val Val Gly Ile Val Arg Ala Pro Ser Pro Val Ala Gln Ala Ser Val
        115                 120                 125

Tyr Val Thr Trp Pro Gln Leu Met Arg Trp Ala Asp Asp Pro Ser Leu
    130                 135                 140

Gly Ile Tyr Thr Val Thr Val Arg Gly Ala Val Gly Pro Val Pro Glu
145                 150                 155                 160

Thr Ala Lys Val Gln Thr Pro Glu Gln Glu Ile Ala Ala Arg Thr Ala
                165                 170                 175

Gln Leu Gln Asn Gly Val Asp Thr Trp Ser Leu Leu Leu Leu Leu Phe
            180                 185                 190

Ala Gly Ile Ala Val Phe Val Ser Ile Leu Val Ile Ala Asn Thr Phe
        195                 200                 205

Ser Ile Leu Leu Ala Gln Arg Met Arg Asp Phe Ala Leu Leu Arg Cys
    210                 215                 220

Val Gly Ala Thr Arg Arg Gln Val Val Ser Ser Val Arg Arg Glu Ala
225                 230                 235                 240

Ala Val Val Gly Leu Leu Ser Ser Leu Ala Gly Val Leu Val Gly Ala
                245                 250                 255

Gly Leu Gly Tyr Gly Leu Ile Ala Leu Ile Lys Thr Leu Ser Pro Ile
            260                 265                 270

Thr Pro Ile Ala Ala Pro Ala Pro Pro Ala Pro Trp Leu Leu Gly Gly
        275                 280                 285

Leu Ala Ile Gly Leu Thr Ala Thr Leu Val Ala Ala Trp Leu Pro Ile
    290                 295                 300

Arg Arg Val Val Arg Val Ser Pro Leu Ala Ala Leu Arg Pro Asp Thr
305                 310                 315                 320

Ala Thr Asp Pro Arg Thr Ala Thr Gly Arg Ala Arg Leu Val Leu Gly
                325                 330                 335

Val Phe Met Leu Ile Ala Gly Leu Val Leu Leu Ala Ser Ala Met Ala
            340                 345                 350

Trp His Ser Thr Val Leu Met Leu Ala Gly Gly Gly Ser Leu Phe Thr
        355                 360                 365

Gly Val Leu Leu Phe Gly Pro Val Leu Ile Pro Arg Leu Leu Glu Ile
    370                 375                 380

Thr Gly Thr Arg Leu Gly Thr Ile Gly Arg Leu Ala Thr Lys Asn Ala
385                 390                 395                 400

Val Arg Asn Pro Arg Arg Thr Ala Thr Thr Ala Ala Ser Leu Leu Val
                405                 410                 415

Gly Ile Thr Leu Ile Thr Ala Val Leu Thr Gly Val Ala Ile Thr Ser
            420                 425                 430

Glu Ala Leu Asn Glu Arg Leu Asp Gly Gln His Pro Ile Asp Ala Ala
        435                 440                 445

Leu Val Ser Thr Gly Lys Pro Phe Ser Ala Asp Phe Leu Asp Lys Val
    450                 455                 460

Arg Gly Thr Ser Gly Val Asp Gln Ala Ile Ala Val Asp Gly Ala Val
465                 470                 475                 480

Ala Thr Val Ser Gly Leu Asp Lys Pro Ile Pro Val Val Thr Ala Pro
                485                 490                 495

Asp Ala Gln Arg Val Ala His Asp Gly Gly Ser Phe Ala Arg Val Glu
            500                 505                 510

Pro Gly Val Leu Arg Leu Asp Glu Ser Ala Phe Arg Gln Leu Arg Leu
        515                 520                 525

Arg Ala Gly Asp Lys Val Arg Val Thr Val Gly Asp Arg Arg Ala Val
    530                 535                 540

Leu Gln Val Ser Leu Ala Thr Gly Trp Gly Leu Gln Ala Val Val Ala
545                 550                 555                 560

Pro Glu Thr Leu Ala Arg Leu Thr Asp Ser Ala Ala Pro Arg Ala Val
                565                 570                 575

Trp Ile Arg Ala Ser Ala Asp Ala Asp Ser Thr Arg Leu Val Gly Glu
            580                 585                 590

Leu Gly Asp Leu Ala Ala Ala Ala Gly Ala Asn Val Asn Asp Gln Leu
        595                 600                 605

Glu Ala Arg Glu Thr Glu Asn Ala Pro Leu Met Ile Leu Thr Trp Ala
    610                 615                 620

Ile Val Ala Leu Leu Gly Phe Ser Val Ala Ile Ala Leu Val Gly Ile
625                 630                 635                 640

Ala Asn Thr Leu Gly Leu Ser Val Leu Glu Arg Val Arg Glu His Ala
                645                 650                 655

Leu Leu Arg Ala Leu Gly Leu Thr Arg Arg Gln Leu Arg Arg Met Leu
            660                 665                 670

Ala Ala Glu Ala Val Leu Leu Ser Leu Val Ala Ala Val Leu Gly Thr
        675                 680                 685

Val Ile Gly Ile Gly Phe Ala Trp Val Gly Tyr Glu Thr Phe Val Lys
    690                 695                 700

Gln Ala Leu Asp Asn Ala Thr Met Gln Val Pro Trp Pro Leu Leu Ala
705                 710                 715                 720

Val Val Val Leu Val Ala Ala Leu Ala Gly Leu Leu Ala Ser Val Leu
                725                 730                 735

Pro Ala Arg Arg Ala Val Arg Val Thr Pro Ala Ala Gly Leu Ser Phe
            740                 745                 750

Glu

<210>  20
<211>  232
<212>  PRT
<213>  Nonomuria

<400>  20

Met Thr Gly Gln Arg Ala Ala Leu Glu Thr Val Ala Ala Ser Ala Arg
1               5                   10                  15

Asn Leu Thr Lys Val Tyr Gly Gln Gly Glu Thr Arg Val His Ala Leu
            20                  25                  30

Arg Gly Val Asp Leu Asp Leu Pro Arg Gly Lys Phe Thr Ala Ile Met
        35                  40                  45

Gly Ser Ser Gly Ser Gly Lys Ser Thr Leu Met His Cys Leu Ala Gly
    50                  55                  60

Leu Asp Gln Ala Ser Asp Gly Thr Val Thr Val Ala Gly Thr Asp Leu
65                  70                  75                  80

Gly Ser Leu Asp Asp Asn Glu Leu Thr Val Phe Arg Arg Glu His Ile
                85                  90                  95

Gly Phe Val Phe Gln Ser Phe Asn Leu Leu Pro Met Leu Thr Ala Phe
            100                 105                 110

Gln Asn Ile Thr Leu Pro Leu Glu Leu Gly Gly Arg Arg Ile Asp Asp
        115                 120                 125

Ala Ala Thr Glu Arg Val His Val Leu Ala Glu Thr Leu Gly Met Ala
    130                 135                 140

Asp Arg Leu Gly His Arg Pro Ser Glu Met Ser Gly Gly Gln Gln Gln
145                 150                 155                 160

Arg Val Ala Ile Ala Arg Ala Leu Ile Thr Gly Pro Asp Leu Leu Phe
                165                 170                 175

Ala Asp Glu Pro Thr Gly Asn Leu Asp Ser Thr Thr Ser Ala Glu Val
            180                 185                 190

Leu Gly Tyr Leu His Lys Ser Thr Arg Glu Leu Gly Gln Thr Val Val
        195                 200                 205

Met Val Thr His Glu Arg Glu Ala Ala Ala Tyr Ala Asp Gly Val Val
    210                 215                 220

Thr Leu Glu Asp Gly Arg Ile Ala
225                 230

<210>  21
<211>  535
<212>  PRT
<213>  Nonomuria

<400>  21

Met Ser His Ile Thr Met Thr Pro Pro Ser Ala Cys Arg Asp Pro Ala
1               5                   10                  15

Pro Ala Gly Arg Phe Pro Arg Trp Ala Val Trp Arg Ser Pro Pro Gly
            20                  25                  30

Gln Pro Trp Trp Ala Arg Pro Ala Leu Leu Cys Ile Ala Ala Thr Ala
        35                  40                  45

Ala Val Leu Tyr Ala Trp Asn Leu Pro Leu Val Asp Tyr Ala Pro Arg
    50                  55                  60

Tyr Ser Asp Ala Val Lys Ser Met Ser Glu Asn Trp Lys Ala Phe Leu
65                  70                  75                  80

Tyr Gly Thr Val Asp Val Gln Ala Thr Tyr Thr Leu Asp Lys Leu Ala
                85                  90                  95

Gly Ala Phe Val Pro Gln Ala Ile Ser Val Lys Ile Phe Gly Phe His
            100                 105                 110

Ala Trp Ala Leu Ala Leu Pro Gln Val Ile Glu Gly Val Ile Ser Val
        115                 120                 125

Leu Val Met Tyr Arg Ile Val Arg Arg Trp Ala Gly Val Val Pro Gly
    130                 135                 140

Leu Leu Ala Ala Ala Val Phe Thr Ile Thr Pro Val Ala Ala Ser Met
145                 150                 155                 160

Phe Gly His Ser Met Ala Asp Gly Ala Leu Val Met Cys Leu Val Leu
                165                 170                 175

Ala Val Asp Ser Tyr Gln Arg Ala Val Leu Glu Gly Arg Leu Arg Ser
            180                 185                 190

Leu Val Trp Ala Gly Val Trp Val Gly Leu Gly Phe Gln Ala Lys Met
        195                 200                 205

Leu Gln Ala Trp Met Ile Leu Pro Ala Leu Ala Ile Gly Tyr Leu Leu
    210                 215                 220

Ser Ala Pro Ile Gly Leu Arg Arg Arg Leu Gln His Leu Gly Ile Ala
225                 230                 235                 240

Gly Val Val Thr Leu Val Val Ser Leu Ser Trp Ile Thr Leu Tyr His
                245                 250                 255

Val Thr Pro Ala Ala Asp Arg Pro Tyr Ile Ser Gly Thr Thr Asn Ser
            260                 265                 270

Ser Ala Ala Ala Met Val Phe Gly Tyr Asn Gly Leu Gly Arg Leu Gly
        275                 280                 285

Ile Asn Leu Pro Gly Ala Leu Pro Pro Asn Tyr Met Gly Ser Val Ile
    290                 295                 300

Gly Pro Ala Pro Pro Lys Arg Ser Thr Gln Leu Pro Arg Pro Arg Pro
305                 310                 315                 320

Gly Met Val Ile Pro Glu Ile Gly Ile Glu His Gly Gly Gly Trp Gly
                325                 330                 335

Lys Leu Phe Gly Gly Arg Leu Gly Val Ala Ser Gly Trp Leu Tyr Pro
            340                 345                 350

Leu Ala Leu Met Ala Leu Leu Cys Gly Leu Trp Trp Trp Arg Arg Ala

355                 360                 365

Glu Arg Thr Asp Pro Ala Arg Gly Gly Met Val Met Trp Gly Val Trp
    370                 375                 380

Leu Leu Thr Phe Ala Leu Pro Tyr Ser Ala Val Phe Val Ile Pro His
385                 390                 395                 400

Ser Ala Tyr Val Ala Val Leu Ala Pro Pro Val Ala Ala Leu Ser Gly
                405                 410                 415

Ile Gly Ile Val Met Phe Trp Arg Ala Tyr Arg Ser Gly Gly Arg Met
            420                 425                 430

Ala Trp Ile Phe Pro Leu Ala Ile Val Ala Glu Leu Ala Trp Ala Val
        435                 440                 445

Trp Leu Trp Ser Phe Tyr Pro Thr Phe Leu Pro Trp Ala Met Trp Gly
    450                 455                 460

Ala Val Ala Leu Gly Val Val Ala Val Val Ala Leu Ala Leu Ala Arg
465                 470                 475                 480

Leu Val Arg Pro Arg Arg Ser Ser Leu Val Ser Ala Gly Leu Thr Ile
                485                 490                 495

Gly Val Ala Ala Met Leu Ala Ala Pro Ala Thr Trp Ser Ala Ser Val
            500                 505                 510

Leu Asp Pro Arg Tyr Gly Gly Ser Ser Phe Asp Ala Asn Ala Gly Pro
        515                 520                 525

Ala Ala Arg Thr Pro Gly Gly
    530                 535

<210>  22
<211>  270
<212>  PRT
<213>  Nonomuria

<400>  22

Met Leu Gln Asp Ala Asp Arg Thr Arg Ile Leu Ala Ile Ser Pro His
1               5                   10                  15

Leu Asp Asp Ala Val Leu Ser Val Gly Ala Ser Leu Ala Gln Ala Glu
            20                  25                  30

Gln Asp Gly Gly Lys Val Thr Val Phe Thr Val Phe Ala Gly Ser Ala

35                  40                  45

Ala Pro Pro Tyr Ser Pro Ala Ala Glu Arg Phe His Ala Arg Trp Gly
    50                  55                  60

Leu Ser Pro Thr Glu Asp Ala Pro Leu Arg Arg Arg Asn Glu Asp Ile
65                  70                  75                  80

Ala Ala Leu Asp Gln Leu Gly Ala Gly His Arg His Gly Arg Phe Leu
                85                  90                  95

Asp Ala Ile Tyr Arg Arg Ser Pro Asp Gly Gln Trp Leu Leu His His
            100                 105                 110

Asn Glu Gly Ser Met Val Arg Gln Gln Ser Pro Ala Asn Asn His Asp
        115                 120                 125

Leu Val Ala Ala Ile Arg Glu Asp Ile Glu Ser Met Ile Ala Glu Cys
    130                 135                 140

Asp Pro Thr Leu Val Leu Thr Cys Val Ala Ile Gly Lys His Pro Asp
145                 150                 155                 160

His Lys Ala Thr Arg Asp Ala Thr Leu Leu Ala Ala Arg Glu Arg Gly
                165                 170                 175

Ile Pro Leu Arg Leu Trp Gln Asp Leu Pro Tyr Ala Ala Tyr Ser Gln
            180                 185                 190

Asp Leu Ala Glu Leu Pro Asp Gly Leu Arg Leu Gly Ser Pro Glu Leu
        195                 200                 205

Ser Phe Val Asp Glu Glu Ala Arg Thr Arg Lys Phe Gln Ala Met Lys
    210                 215                 220

His Tyr Ala Thr Gln Leu Ser Val Leu Asp Gly Pro Asn Lys Asn Leu
225                 230                 235                 240

Phe Ala Lys Leu Asp Glu His Ala Arg Asn Ala Ala Pro Asp Gly Gly

Tyr Asn Glu Thr Thr Trp Pro Val Ile Arg Tyr Ala Ala Glu
            260                 265                 270

<210>  23
<211>  420
<212>  PRT
<213>  Nonomuria

<400> 23

Met Ala His Arg Leu Arg Arg Leu Thr Thr Ala Phe Arg Ser Val Arg
1               5                   10                  15

Leu Arg Leu Thr Leu Val Tyr Gly Ala Leu Phe Ala Ala Ser Gly Val
            20                  25                  30

Val Leu Leu Ala Ile Thr Tyr Leu Leu Phe Arg Gly Ser Arg Pro Phe
        35                  40                  45

Val Leu Val Asp Gly Asp Pro Gly Gly Arg Phe Arg Ala Phe Ala Arg
    50                  55                  60

Gln Gln Gln Ala Ala Ile Leu Glu Asn Leu Leu Phe Gln Ser Leu Ile
65                  70                  75                  80

Ala Leu Ala Leu Met Thr Val Ile Ser Phe Leu Leu Gly Trp Leu Val
                85                  90                  95

Ala Gly Arg Met Leu Arg Pro Leu Arg Thr Met Asn Thr Thr Leu Lys
            100                 105                 110

Arg Ile Ser Ala Arg Asn Val His Glu Arg Leu Ala Leu Pro Gly Pro
        115                 120                 125

Arg Asp Glu Leu Arg Asn Leu Ala Asp Thr Val Asp Glu Leu Leu Glu
    130                 135                 140

Arg Leu His Ser Ala Leu Asp Ala Gln Lys Arg Phe Val Ala Asn Ala
145                 150                 155                 160

Ala His Glu Leu Arg Thr Pro Leu Thr Leu Glu His Ala Leu Leu Glu
                165                 170                 175

Glu Ser Leu Leu His Arg Asp Ala Asp Thr Pro Ser Met Arg Ser Ile
            180                 185                 190

Met Glu Arg Leu Leu Asp Leu Ser Arg Gln Gln Gly Arg Leu Leu Glu
        195                 200                 205

Ser Leu Leu Thr Leu Ala Lys Ser Glu Gly Gly Leu Asp His Arg Glu
    210                 215                 220

Pro Leu Asp Leu Ala Glu Ile Ala Glu His Thr Ile Arg Thr Met Glu
225                 230                 235                 240

Gly Thr Gly Pro Gly Ala Asp Gly Asn Asn Pro Arg Ala Gly Val Ser

245                 250                 255

Ala Asp Arg Arg Ala Asp Gly Asn Ser Pro Thr Ala Gly Ala Ala Thr
            260                 265                 270

Asp Ser Trp Ala Asp Gly Lys Ser Leu Arg Ala Gly Cys Pro His Pro
        275                 280                 285

Arg Leu Val Thr Gly Ile Ala His Ala Pro Thr Thr Gly Asp Pro Ala
    290                 295                 300

Leu Val Glu Arg Leu Ile Thr Asn Leu Leu Asp Asn Ala Met Arg Tyr
305                 310                 315                 320

Asn Val Pro Gly Gly Gln Val Glu Leu Ser Thr Arg Ala Glu Ala Gly
                325                 330                 335

Lys Ala Val Val Ser Ile Ala Asn Thr Gly Pro Val Val Pro Pro Glu
            340                 345                 350

Gln Val His Arg Leu Phe Glu Pro Phe Gln Arg Leu Asp Arg Thr Arg
        355                 360                 365

Ala Asp Asp His His Gly Leu Gly Leu Ser Ile Val Arg Ala Ile Ala
    370                 375                 380

Val Ala His Asp Ala Thr Leu Thr Ala His Ala Arg Pro Gln Gly Gly
385                 390                 395                 400

Leu Ser Val Glu Ile His Phe Pro Leu Met Arg Arg Ala Leu Arg Arg
                405                 410                 415

Leu Ala Pro Ser
            420

<210>  24
<211>  709
<212>  PRT
<213>  Nonomuria

<400>  24

Met Ser Leu Pro Thr Cys Ala Cys Gly Leu Thr Pro His Ala Pro Ser
1               5                   10                  15

Cys Ala Pro Arg Ser Glu His Ala Gly Gly Arg Ser Ser Glu Ser Arg
            20                  25                  30

Thr Asp Ile Gln Gly Leu Arg Ala Ile Ala Val Ala Ala Val Val Ala

        35                  40                  45

Phe His Leu Trp Pro Gly Gly Pro Thr Gly Gly Tyr Val Gly Val Asp
    50                  55                  60

Val Phe Phe Val Ile Ser Gly Tyr Leu Ile Thr Ser His Leu Leu Arg
65                  70                  75                  80

Gln Pro Gly His Gly Gly Gly Arg Leu Leu Asp Phe Trp Ala Arg Arg
                85                  90                  95

Val Arg Arg Leu Ile Pro Ala Ala Ser Leu Ala Leu Leu Val Thr Leu
            100                 105                 110

115 120 125

Arg Glu Val Ile Ala Ala Thr Val Tyr Val Glu Asn Leu Arg Leu Ala
130 135 140

Leu Thr Gln Ala Asn Tyr Leu Asp Val Asp Gln Pro Asp Trp Pro Ala
145 150 155 160

Gln His Tyr Trp Ser Leu Ser Ile Glu Glu Gln Phe Tyr Leu Gly Trp
165 170 175

Pro Leu Leu Leu Gly Ser Ala Ala Trp Leu Ala Ala Arg Val Ala Arg
180 185 190

Gly Arg Arg Pro Pro Glu Asn Phe Thr Arg Trp Ser Ala Val Val Val
195 200 205

Thr Gly Ala Val Val Ala Ala Ser Leu Ala Trp Ser Val Gln Lys Thr
210 215 220

Ala Thr Asp Pro Ala Ala Ala Tyr Phe Val Ser Thr Thr Arg Phe Trp
225 230 235 240

Glu Leu Ala Leu Gly Gly Leu Leu Ala Ala Val Leu Thr Val Arg Ala
245 250 255

Met Pro Arg Ala Arg Ala Val Arg Ala Gly Leu Ala Trp Ala Gly Leu
260 265 270

Gly Met Ile Gly Trp Ala Val Val Arg Phe Asp Ala Glu Thr Ala Phe
275 280 285

Pro Gly Ala Ala Ala Leu Val Pro Thr Val Gly Ala Cys Leu Val Ile

290                 295                 300

Ala Ala Ala Ala Asp Gly Leu Arg Gly Gly Pro Gly Arg Ala Leu Ala
305                 310                 315                 320

Trp Arg Pro Val Gln Trp Leu Gly Asn Ala Ser Tyr Ala Val Tyr Leu
                325                 330                 335

Trp His Trp Pro Pro Ile Met Ile Leu Pro Tyr Ala Leu Gly Arg Ser
            340                 345                 350

Leu Thr Val Ile Glu Ser Val Gly Val Ile Ala Leu Thr Leu Val Leu
        355                 360                 365

Ala Ala Leu Ser Gln Tyr Leu Val Glu Asp Arg Leu Arg Trp His Pro
    370                 375                 380

Val Leu Val Arg Ser Arg Arg Leu Thr Phe Ala Met Leu Ala Ser Cys
385                 390                 395                 400

Val Val Val Val Ala Gly Ala Gly Ala Gly Val Val Ala Tyr Ala Asp
                405                 410                 415

Ala Ala Glu Arg Thr Glu Ser Ala Ala Phe Glu Ala Ala Ala Ser Arg
            420                 425                 430

Ala Gly Ser Cys Leu Gly Ala Gly Val Val Arg Asp Pro Ala Cys Gln
        435                 440                 445

Asp Leu Gly Leu Leu Met Pro Pro Gln Val Ala Leu Lys Asp Lys Pro
    450                 455                 460

Ala Val Tyr Ala Asp Gly Cys Val Asn Lys Glu Pro Phe Ile Ala Arg
465                 470                 475                 480

Asn Thr Cys Thr Tyr Gly Pro Asp Ala Ala Gly Arg Arg Ile Ala Leu
                485                 490                 495

Val Gly Asn Ser His Ala Gly His Trp Val Pro Ala Leu Glu Lys Ala
            500                 505                 510

Leu Trp Ser Glu Arg Trp Gln Leu Thr Thr Tyr Val Gln Leu Ala Cys
        515                 520                 525

Tyr Thr Val Asp Gln Pro Leu Val Leu Glu Gly Ala Gly Val Ser Glu
    530                 535                 540

Asn Cys Gln Lys Ile Asn Lys Trp Ala Val Gly Ser Ile Val Asn Gly
545                 550                 555                 560

Gly Tyr Asp Leu Val Ile Met Ser Asn Arg Thr His Val Pro Leu Ala
                565                 570                 575

Gly Val Ser Pro Ala Gly Gln Gln Ala Ala Ala Glu Arg Ala Tyr Arg
            580                 585                 590

Asp Thr Leu Arg Ala Phe Thr Gly Ala Gly Leu Pro Val Leu Val Leu
        595                 600                 605

Arg Asp Thr Pro Ala Met Pro Asp Ser Val Pro His Cys Ile Ala Lys
    610                 615                 620

625                 630                 635                 640

Arg Pro Asp Pro Leu Ala Ala Ala Ala Arg Ala Asp Asp Thr Gly Leu
                645                 650                 655

Val Ser Val Ala Ser Val Asp His Leu Val Cys Gly Glu Arg Cys Gly
            660                 665                 670

Pro Val Ile Gly Gly Leu Ile Ala Tyr Ser Asp Arg Ser His Leu Thr
        675                 680                 685

Thr Thr Phe Ala Arg Thr Leu Ala Pro Glu Val Thr Ala Ala Val Arg
    690                 695                 700

Gly Ala Leu Thr Arg
705

<210>  25
<211>  648
<212>  PRT
<213>  Nonomuria

<400>  25

Met Ala Ile Val Ser Pro Phe Gly Gly Leu Leu Lys Gly Asp Gly Glu
1               5                   10                  15

Asp Asp Pro Ala Pro Ser Arg Ile Arg Pro Gly Thr Leu Arg Arg Val
            20                  25                  30

Leu Gly Tyr Phe Arg Pro His Val Gly Lys Val Ala Leu Phe Val Leu
        35                  40                  45

Val Thr Ala Leu Asp Ser Ile Phe Val Val Ala Ser Pro Leu Met Leu

50                  55                  60

Lys Asp Leu Val Asp Lys Gly Val Leu Gly Asn Asp Leu Glu Leu Val
65                  70                  75                  80

Ile Leu Leu Ala Cys Leu Ala Ala Gly Phe Ala Val Met Ser Thr Leu
                85                  90                  95

Leu Gln Leu Val Ser Ala Tyr Ile Ser Gly Arg Ile Gly Gln Gly Val
            100                 105                 110

Ser Tyr Asp Leu Arg Val Gln Ala Leu Asp His Val Gln Arg Leu Pro
        115                 120                 125

Ile Ala Phe Phe Thr Arg Thr Gln Thr Gly Val Leu Val Gly Arg Leu
    130                 135                 140

His Thr Glu Leu Val Met Thr Gln Met Ala Phe Thr Gln Met Leu Thr
145                 150                 155                 160

Ala Ala Ala Ser Ala Val Thr Val Leu Leu Val Leu Ala Glu Leu Phe
                165                 170                 175

Tyr Leu Ser Trp Ile Val Ala Leu Leu Thr Leu Val Leu Ile Pro Val
            180                 185                 190

Phe Leu Val Pro Trp Ser Tyr Val Gly Arg Arg Met Gln Arg Tyr Thr
        195                 200                 205

Arg Gly Leu Met Glu Glu Asn Ala Gly Leu Ala Gly Leu Leu Gln Glu
    210                 215                 220

Arg Phe Asn Val Gln Gly Ala Met Leu Ser Lys Leu Phe Gly Arg Pro
225                 230                 235                 240

Ala Glu Glu Met Ala Glu Tyr Glu Ser Arg Ala Gly Arg Ile Arg Gly
                245                 250                 255

Leu Ala Val Ser Val Thr Leu Tyr Gly Arg Met Ala Pro Ala Ile Phe
            260                 265                 270

Ala Leu Met Ala Ala Leu Ala Thr Ala Leu Val Tyr Gly Val Gly Gly
        275                 280                 285

Gly Leu Val Leu Ser Gln Ala Phe Gln Leu Gly Thr Leu Val Ala Leu
    290                 295                 300

Ala Thr Leu Leu Gly Arg Leu Phe Gly Pro Ile Thr Gln Leu Ala Ser
305                 310                 315                 320

Ile Gln Glu Asn Ala Leu Thr Val Leu Val Ser Phe Glu Arg Ile Phe
                325                 330                 335

Glu Leu Leu Asp Leu Lys Pro Leu Ile Glu Glu Arg Pro Asp Ala Val
            340                 345                 350

Ala Leu Lys Ala Gly Lys Ala Ser Asp Val Gln Phe Glu Asn Val Ser
        355                 360                 365

Phe Arg Tyr Pro Ser Ala Asp Glu Val Ser Leu Pro Ser Leu Glu Gln
    370                 375                 380

Asn Val Arg Thr Gly Gln Glu Arg Gly Glu Ala Thr Pro Glu Val Leu
385                 390                 395                 400

Arg Asp Val Ser Leu His Val Pro Ala Gly Thr Leu Thr Ala Leu Val
                405                 410                 415

Gly Pro Ser Gly Ala Gly Lys Ser Thr Leu Thr His Leu Val Ser Arg
            420                 425                 430

Leu Tyr Asp Pro Thr Ser Gly Thr Val Arg Val Gly Gly His Asp Leu
        435                 440                 445

Arg Asp Leu Thr Phe Asp Ser Leu Arg Glu Thr Val Gly Val Val Ser
    450                 455                 460

Gln Asp Thr Tyr Leu Phe His Asp Thr Ile Arg Ala Asn Leu Leu Tyr
465                 470                 475                 480

Ala Arg Pro Asp Ala Thr Glu Asp Glu Leu Val Glu Ala Cys Arg Gly
                485                 490                 495

Ala Gln Ile Trp Asp Leu Ile Ala Ser Leu Pro Arg Gly Leu Asp Thr
            500                 505                 510

Val Val Gly Asp Arg Gly Tyr Arg Leu Ser Gly Gly Glu Lys Gln Arg
        515                 520                 525

Leu Ala Ile Ala Arg Leu Leu Leu Lys Ala Pro Ser Val Val Val Leu
    530                 535                 540

Asp Glu Ala Thr Ala His Leu Asp Ser Glu Ser Glu Ala Ala Val Gln
545                 550                 555                 560

Arg Ala Leu Thr Thr Ala Leu Arg Ser Arg Thr Ser Leu Val Ile Ala
                565                 570                 575

His Arg Leu Ser Thr Ile Arg Glu Ala Asp His Ile Leu Val Ile Asp
            580                 585                 590

Asp Gly Arg Val Arg Glu Arg Gly Thr His Glu Glu Leu Leu Ala Glu
        595                 600                 605

Gly Gly Leu Tyr Ala Asp Leu Tyr His Thr Gln Phe Ala Lys Ser Gly
    610                 615                 620

Val Asn Gly Thr Arg Pro Gly Gln Gly Asp Gly Ala Glu Pro Val Gln
625                 630                 635                 640

Glu Val Val Gly Gly Gly Glu Arg
                645

<210>  26
<211>  2097
<212>  PRT
<213>  Nonomuria

<400>  26

Met Ser Ala Gly Thr Arg Ala Thr Pro Thr Thr Val Leu Asp Leu Phe
1               5                   10                  15

Ala Arg Gln Val Gly Arg Ala Pro Asp Ala Val Ala Leu Val Asp Gly
            20                  25                  30

Asp Arg Val Leu Thr Tyr Arg Arg Leu Asp Glu Leu Ala Gly Ala Leu
        35                  40                  45

Ser Gly Arg Leu Ile Gly Arg Gly Val Gly Arg Gly Asp Arg Val Ala
    50                  55                  60

Val Met Met Asp Arg Ser Ala Asp Leu Val Val Thr Leu Leu Ala Val
65                  70                  75                  80

Trp Gln Ala Gly Ala Ala Tyr Val Pro Val Asp Ala Ala Leu Pro Ala
                85                  90                  95

Arg Arg Val Ala Phe Met Val Ala Asp Ser Gly Ala Cys Leu Met Val
            100                 105                 110

Cys Ser Glu Ala Thr Arg Asp Ala Val Pro Gln Gly Val Glu Ser Ile
        115                 120                 125

Ala Leu Thr Gly Glu Gly Gly Cys Gly Thr Ser Ala Val Thr Val Asp
    130                 135                 140

Pro Gly Asp Leu Ala Tyr Val Met Tyr Thr Ser Gly Ser Thr Gly Thr
145                 150                 155                 160

Pro Lys Gly Val Ala Val Pro His Arg Ser Val Ala Glu Leu Thr Gly
                165                 170                 175

Asn Pro Gly Trp Gly Val Glu Pro Gly Glu Ala Val Leu Met His Ala
            180                 185                 190

Pro Tyr Thr Phe Asp Ala Ser Leu Phe Glu Ile Trp Val Pro Leu Val
        195                 200                 205

Ser Gly Ala Arg Val Val Ile Ala Ala Pro Gly Ala Val Asp Ala Arg
    210                 215                 220

Arg Leu Arg Glu Ala Val Ala Ala Gly Val Thr Arg Val His Leu Thr
225                 230                 235                 240

Ala Gly Ser Phe Arg Ala Val Ala Glu Glu Ser Pro Glu Ser Phe Ala
                245                 250                 255

His Phe Arg Glu Val Leu Thr Gly Gly Asp Val Val Pro Ala Tyr Ala
            260                 265                 270

Val Gln Lys Val Arg Ala Ala Cys Pro His Val Arg Ile Arg His Leu
        275                 280                 285

Tyr Gly Pro Thr Glu Thr Thr Leu Cys Ala Thr Trp Gln Leu Leu Glu
    290                 295                 300

Pro Gly Asp Val Val Gly Pro Val Leu Pro Ile Gly Arg Pro Leu Pro
305                 310                 315                 320

Gly Arg Arg Ala Trp Val Leu Asp Ala Ser Leu Arg Pro Val Glu Pro
                325                 330                 335

Gly Val Val Gly Asp Leu Tyr Leu Ser Gly Ala Gly Leu Ala Asp Gly
            340                 345                 350

Tyr Leu Asp Arg Ala Gly Leu Thr Ala Glu Arg Phe Val Ala Asp Pro
        355                 360                 365

Ser Ala Ala Gly Arg Arg Met Tyr Arg Thr Gly Asp Leu Ala Gln Trp
    370                 375                 380

Thr Ala Asp Gly Glu Leu Leu Phe Ala Gly Arg Ala Asp Asp Gln Val
385                 390                 395                 400

Lys Val Arg Gly Phe Arg Ile Glu Pro Gly Glu Val Glu Ala Ala Leu
                405                 410                 415

Thr Ala Gln Pro His Val Arg Glu Ala Val Val Val Ala Ile Asp Gly
            420                 425                 430

Arg Leu Ile Gly Tyr Val Val Ala Asp Gly Asp Val Asp Pro Val Leu
        435                 440                 445

Met Arg Arg Arg Leu Ala Ala Ser Leu Pro Glu Tyr Met Ile Pro Ala
    450                 455                 460

Ala Leu Val Thr Leu Asp Ala Leu Pro Leu Thr Gly Ser Gly Lys Val
465                 470                 475                 480

Asp Arg Arg Ala Leu Pro Glu Pro Asp Phe Ala Ser Ala Ala Pro Arg
                485                 490                 495

Arg Glu Pro Gly Thr Glu Pro Glu Arg Val Leu Cys Asp Leu Phe Ala
            500                 505                 510

Glu Leu Leu Gln Pro Glu Gly Arg Gly Val Gly Val Asp Asp Gly Phe
        515                 520                 525

Val Glu Leu Gly Gly Asp Ser Ile Val Ala Ile Arg Leu Ala Ala Arg
    530                 535                 540

Ala Ser Arg Val Gly Leu Leu Val Thr Pro Ala Gln Ile Phe Lys Glu
545                 550                 555                 560

Lys Thr Pro Ala Arg Leu Ala Ala Val Ala Gly Ala Val Pro Ala Gly
                565                 570                 575

Arg Pro Ala Asp Gly Pro Leu Ile Thr Leu Thr Ala Glu Glu Glu Ala
            580                 585                 590

Glu Leu Ala Thr Ala Val Pro Gly Ala Glu Glu Val Trp Pro Leu Ala
        595                 600                 605

Pro Leu Gln Glu Gly Leu Tyr Phe Gln Ala Thr Leu Asp Asp Glu Gly
    610                 615                 620

His Asp Ile Tyr Gln Ala Gln Trp Ile Leu Glu Leu Ala Gly Pro Leu

625                 630                 635                 640

Asp Ala Ala Arg Leu Arg Ala Ser Trp Glu Ala Val Phe Ala Arg His
                645                 650                 655

Pro Glu Leu Arg Val Ser Phe His Arg Arg Ala Ser Gly Thr Met Leu
            660                 665                 670

Gln Val Val Ala Gly His Val Val Leu Pro Trp Arg Glu Val Asp Leu
        675                 680                 685

Ala Asp Ala Gly Asp Ile Asp Ala Ala Val Ala Ala Leu Ile Ser Glu
    690                 695                 700

Glu Gln Glu Gln Arg Phe Asp Leu Ala Lys Ala Pro Leu Phe Arg Leu
705                 710                 715                 720

Val Leu Val Arg His Gly Glu Asp Arg His Arg Leu Leu Val Val His
                725                 730                 735

His His Ile Leu Thr Asp Gly Trp Ser Val Ala Val Ile Leu Asn Glu
            740                 745                 750

Val Ala Glu Ala Tyr Thr Asn Gly Gly Arg Leu Pro Asp Arg Thr Gly
        755                 760                 765

Ala Ala Ser Tyr Arg Asp Tyr Leu Ala Trp Leu Asp Arg Gln Asp Lys
    770                 775                 780

Asp Ala Ala Arg Ala Ala Trp Gln Ala Glu Leu Ser Gly Leu Glu Gly
785                 790                 795                 800

Pro Ala Pro Ile Ala Lys Ala Ala Thr Thr Thr Gly Ala Gly Thr Gly
                805                 810                 815

Tyr Glu Tyr Arg Ile Ala Phe Leu Thr Pro Asp Leu His Thr Arg Leu
            820                 825                 830

Thr Glu Leu Ala Arg Asp His Gly Leu Thr Leu Asn Thr Leu Ala Gln
        835                 840                 845

Gly Ala Trp Ala Met Val Leu Ala Arg Leu Ala Arg Arg Thr Asp Val
    850                 855                 860

Val Phe Gly Thr Thr Val Ala Cys Arg Pro Ala Glu Leu Pro Glu Val
865                 870                 875                 880

Glu Ser Val Pro Gly Leu Met Met Asn Thr Val Pro Val Arg Val Pro
                885                 890                 895

Leu Gln Gly Ala Gln Ser Val Val Asp Leu Leu Thr Gly Leu Gln Glu
            900                 905                 910

Arg Gln Ala Ala Leu Leu Pro His Gln His Leu Gly Leu Thr Glu Ile
        915                 920                 925

Gln Arg Ala Ala Gly Pro Gly Ala Thr Phe Asp Thr Leu Leu Val Phe
    930                 935                 940

Glu Asn Tyr Pro Arg Asp Phe Ala Gly Gln Phe Thr Tyr Leu Gly Thr
945                 950                 955                 960

Ile Glu Gly Thr His Tyr Pro Leu Thr Leu Gly Ile Ile Pro Gly Asp
                965                 970                 975

His Phe Arg Ile Gln Leu Val Tyr Arg Arg Gly Gln Val Gly Glu Ser
            980                 985                 990

Val Ala Glu Ser Ile Leu Gly Trp  Phe Thr Gly Ala Leu  Met Thr Met
        995                 1000                1005

Ala Ala  Asp Pro His Gly Pro  Val Gly Arg Ile Gly  Val Gly Glu
    1010                1015                1020

Ala Arg  Ala Gly Gly Ser Asp  Arg Ala Met Ala Ala  Gly Glu Pro
    1025                1030                1035

Leu Pro  Val Leu Leu Arg Arg  Val Val Lys Asp Arg  Pro Asp Glu
    1040                1045                1050

    1055                1060                1065

Trp Glu  Arg Ala Thr Ala Leu  Ala Ala Glu Leu Arg  Ala His Gly
    1070                1075                1080

Ile Gly  Pro Glu Ser Arg Val  Ala Val Met Val Gly  Arg Ser Ala
    1085                1090                1095

Trp Trp  Ala Val Gly Val Leu  Gly Val Cys Leu Ala  Gly Gly Ala
    1100                1105                1110

Phe Met  Pro Val Asp Pro Ala  Tyr Pro Ala Glu Arg  Val Arg Trp
    1115                1120                1125

Ile Leu  Ala Asp Ser Asp Pro  Arg Leu Val Leu Cys  Ala Gly Thr
    1130                 1135                 1140

Thr Arg  Glu Ala Val Pro Glu  Glu Phe Ala Asp Arg  Leu Val Val
    1145                 1150                 1155

Val Asp  Glu Leu Asp Leu Ala  Gly Ser Asp Asp Ala  Gly Leu Pro
    1160                 1165                 1170

Arg Val  Ser Pro Asp Asp Ala  Ala Tyr Val Ile Tyr  Thr Ser Gly
    1175                 1180                 1185

Ser Thr  Gly Thr Pro Lys Gly  Val Val Val Ser His  Ala Gly Leu
    1190                 1195                 1200

Gly Asn  Leu Ala Met Ala Gln  Ile Asp Arg Phe Ala  Val Ser Pro
    1205                 1210                 1215

Ser Ser  Arg Val Leu Gln Phe  Ala Ala Leu Gly Phe  Asp Ala Met
    1220                 1225                 1230

Val Ser  Glu Met Leu Met Ala  Leu Leu Ser Gly Ala  Arg Leu Val
    1235                 1240                 1245

Met Ala  Pro Glu Pro Ala Leu  Pro Pro Arg Val Ser  Leu Ala Glu
    1250                 1255                 1260

Ala Leu  Arg Arg Trp Glu Val  Thr His Val Thr Val  Pro Pro Ser
    1265                 1270                 1275

Val Leu  Ala Thr Ala Asp Ala  Leu Pro Ala Gly Leu  Glu Thr Val
    1280                 1285                 1290

Val Val  Ala Gly Glu Ala Cys  Pro Pro Gly Leu Ala  Glu Arg Trp
    1295                 1300                 1305

Ser Ala  Gly Arg Arg Leu Val  Asn Ala Tyr Gly Pro  Thr Glu Ala
    1310                 1315                 1320

Thr Val  Cys Ala Ala Met Ser  Arg Pro Leu Thr Gly  Ser Arg Glu
    1325                 1330                 1335

Val Val  Pro Ile Gly Thr Pro  Ile Ala Gly Gly Arg  Cys Tyr Val
    1340                 1345                 1350

Leu Asp  Ala Phe Leu Arg Pro  Leu Pro Pro Gly Ile  Thr Gly Glu
    1355                 1360                 1365

Leu Tyr Val Ala Gly Ile Gly Leu Ala Arg Gly Tyr Leu Gly Arg
    1370                1375                1380

Ala Ser Leu Thr Ala Glu Arg Phe Val Ala Asp Pro Phe Val Ala
    1385                1390                1395

Gly Glu Arg Met Tyr Arg Thr Gly Asp Leu Ala Tyr Trp Thr Gly
    1400                1405                1410

Glu Gly Glu Leu Val Phe Ala Gly Arg Asp Asp Asp Gln Val Lys
    1415                1420                1425

Ile Arg Gly Tyr Arg Val Glu Pro Gly Glu Val Glu Ala Val Leu
    1430                1435                1440

Ala Gly Gln Pro Gly Val Asp Gln Ala Val Val Val Ala Arg Glu
    1445                1450                1455

Gly Arg Leu Leu Gly Tyr Val Val Ser Gly Gly Gly Val Asp Pro
    1460                1465                1470

Val Arg Leu Arg Glu Gly Val Ala Arg Val Leu Pro Glu Tyr Met
    1475                1480                1485

Val Pro Ala Ala Val Val Val Leu Gly Ala Val Pro Val Thr Ala
    1490                1495                1500

Asn Gly Lys Val Asp Arg Glu Ala Leu Pro Asp Pro Gly Phe Gly
    1505                1510                1515

Gly Arg Val Ser Gly Arg Glu Pro Arg Thr Glu Val Glu Arg Ala
    1520                1525                1530

Leu Cys Gly Leu Phe Ala Glu Val Leu Gly Leu Pro Gly Val Thr
    1535                1540                1545

Ala Val Gly Pro Asp Asp Ser Phe Phe Glu Leu Gly Gly Asp Ser
    1550                1555                1560

Ile Thr Ser Met Gln Leu Ala Ser Arg Ala Arg Arg Glu Gly Met
    1565                1570                1575

Leu Phe Gly Ala Arg Glu Val Phe Glu Arg Lys Thr Pro Ala Gly
    1580                1585                1590

Leu Ala Ala Ile Val Asp Val Gly Gly Glu Leu Ala Ala Gly Pro
    1595                1600                1605

Ala Asp Gly Val Gly Glu Ile Ala Trp Thr Pro Ile Met Arg Ala
1610 1615 1620

Leu Gly Asp Gly Ile Val Gly Ser Arg Phe Ala Gln Trp Val Val
1625 1630 1635

Leu Gly Ala Pro Pro Asp Leu Arg Ala Asp Val Val Ala Ala Gly
1640 1645 1650

Leu Ala Ala Val Val Asp Thr His Asp Val Leu Arg Leu Arg Val
1655 1660 1665

Val Asp Asp Arg Ala Gly Arg Arg Leu Ala Val Gly Glu Arg Gly
1670 1675 1680

Ser Val Asp Thr Ala Gly Leu Val Thr Arg Leu Glu Cys Gly Gly
1685 1690 1695

Arg Pro Pro Asp Glu Val Val Glu Arg Ala Val Arg Glu Ala Val
1700 1705 1710

Gly Arg Leu Asp Pro Val Ala Gly Val Met Ala Gln Ala Val Trp
1715 1720 1725

Val Asp Ala Gly Pro Ala Arg Thr Gly Arg Leu Val Val Val Val
1730 1735 1740

His His Leu Ala Val Asp Gly Met Ser Trp Arg Ile Leu Val Pro
1745 1750 1755

Asp Leu Arg Leu Ala Cys Glu Ala Val Ala Glu Gly Arg Asp Pro
1760 1765 1770

Val Leu Glu Pro Val Trp Gly Ser Phe Arg Arg Trp Ala Ala Leu
1775 1780 1785

Leu Glu Glu Ser Ala Leu Ser Arg Glu Arg Val Gly Glu Leu His
1790 1795 1800

Thr Trp Arg Thr Ile Val Asp Gln Glu Asp Arg Pro Val Gly Arg
1805 1810 1815

Arg Arg Leu Ser Ala Gly Asp Ala Ala Gly Gly Val Arg Ser Arg
1820 1825 1830

Ser Trp Val Met Ser Gly Asp Glu Ala Ser Leu Leu Val Gly Lys

1835                 1840                 1845

Val Pro  Val Ala Phe His Cys  Gly Val His Glu Val  Leu Leu Ala
    1850                 1855                 1860

Gly Leu  Ala Gly Ala Val Ala  Arg Trp His Gly Asp  Asp Gly Val
    1865                 1870                 1875

Leu Val  Asp Val Glu Gly His  Gly Arg His Pro Ala  Glu Gly Met
    1880                 1885                 1890

Asp Leu  Ser Arg Thr Val Gly  Trp Phe Thr Ser Met  His Pro Val
    1895                 1900                 1905

Arg Leu  Asp Val Ala Gly Ile  Glu Leu Ala Ala Val  Pro Ala Gly
    1910                 1915                 1920

Gly Arg  Ala Ala Gly Gln Leu  Leu Lys Ala Val Lys  Glu Gln Ser
    1925                 1930                 1935

Arg Ala  Ala Pro Gly Asp Gly  Leu Gly Tyr Gly Leu  Leu Arg His
    1940                 1945                 1950

Leu Asn  Pro Glu Thr Gly Pro  Val Leu Ala Ala Leu  Pro Ser Pro
    1955                 1960                 1965

Gln Ile  Gly Phe Asn Tyr Met  Gly Arg Phe Val Thr  Val Asp Gln
    1970                 1975                 1980

Gly Gly  Ala Arg Pro Trp Gln  Pro Val Gly Gly Ile  Gly Gly Ser
    1985                 1990                 1995

Leu Asp  Pro Gly Met Gly Leu  Pro His Ala Leu Glu  Val Asn Ala
    2000                 2005                 2010

Ile Val  His Asp Arg Leu Ala  Gly Pro Glu Leu Val  Leu Thr Val
    2015                 2020                 2025

Asp Trp  Arg Asp Asp Leu Leu  Glu Glu Thr Asp Ile  Glu Arg Leu
    2030                 2035                 2040

Cys Gln  Val Trp Leu Asp Met  Leu Ser Gly Leu Ser  Arg Gln Ala
    2045                 2050                 2055

Glu Asp  Pro Ser Ala Gly Gly  His Thr Ala Ser Asp  Phe Ala Leu
    2060                 2065                 2070

Leu Asp Leu Asp Gln Asp Glu Ile Glu Gly Phe Glu Ala Ile Ala
2075 2080 2085

Ala Glu Leu Ser Gly Gly Gln Thr Ser
2090 2095

<210> 27
<211> 1063
<212> PRT
<213> Nonomuria

<400> 27

Met Asn Thr Pro Ser Thr Pro Ala Gly Ser Ala Leu Glu Glu Val Trp
1               5                   10                  15

Pro Leu Ser Pro Met Gln Glu Gly Ile Leu Tyr His Ala Ala Leu Asp
            20                  25                  30

Glu Ala Pro Asp Leu Tyr Leu Ile Gln Gln Ser Gln Ile Ile Glu Gly
        35                  40                  45

Pro Leu Asp Thr Glu Arg Phe Arg Leu Ala Trp Glu Ser Leu Leu Asn
    50                  55                  60

Arg His Ala Ala Leu Arg Ala Cys Phe His Arg Arg Lys Ser Gly Glu
65                  70                  75                  80

Ser Val Gln Leu Ile Pro Arg Lys Val Pro Leu Pro Trp Ser Glu Arg
                85                  90                  95

Asp Leu Ser Gly Leu Ser Glu Glu Asp Ala Leu Ala Glu Ala Ser Val
            100                 105                 110

Ile Ala Glu Lys Glu Arg Ala Thr Arg Phe Asp Pro Ala Lys Pro Pro
        115                 120                 125

Leu Leu Arg Gln Val Leu Ile Arg Phe Gly Pro Asp Lys His Cys Leu
    130                 135                 140

Val Thr Thr Ser His His Leu Val Met Asp Gly Trp Ser Arg Ala Ile
145                 150                 155                 160

Leu Glu Ser Glu Leu Leu Glu Leu Tyr Ala Ala Gly Gly Ala Glu Pro
                165                 170                 175

Gly Leu Arg Pro Ala Gly Ser Tyr Arg Asp Tyr Leu Ala Trp Leu Glu
            180                 185                 190

Arg Gln Asp Lys Glu Ala Ala Arg Ala Ala Trp Arg Ala Glu Leu Ala
        195                 200                 205

Gly Ala Asp Arg Ser Thr Leu Gly Ile Pro Glu Ala Ser Arg Lys Thr
    210                 215                 220

Gln Gly Gln Arg Val Arg Glu Val Leu Gly Tyr Ala Pro Asp Phe Thr
225                 230                 235                 240

Ser Ala Leu Val Asp Phe Ala Arg Arg His Gly Leu Thr Leu Asn Thr
                245                 250                 255

Leu Val Gln Gly Ala Trp Ala Leu Val Leu Ala Arg Leu Thr Arg Arg
            260                 265                 270

Arg Asp Val Val Phe Gly Ala Val Val Ser Gly Arg Pro Ala Glu Val
        275                 280                 285

Pro Gly Val Glu Gln Ala Val Gly Leu Phe Ile Asn Thr Val Pro Val
    290                 295                 300

Arg Val Arg Leu Asp Gly Gly Gln Pro Val Ile Gln Leu Leu Thr Glu
305                 310                 315                 320

Leu Gln Glu Arg Gln Ser Thr Leu Ile Ser His Gln His Leu Gly Leu
                325                 330                 335

Gln Glu Ile Gln Lys Leu Ser Gly Val Ser Phe Asp Thr Val Val Ser
            340                 345                 350

Phe Glu Asn Tyr Val Asp Pro Gly Ala Gly Pro Gly Ser Asp Arg Glu
        355                 360                 365

Leu Arg Leu Arg Leu Lys Glu Phe His Gln Ser Ala Pro Tyr Ala Leu
    370                 375                 380

Leu Leu Gly Ile Met Pro Gly Glu Ser Leu Gln Thr Asp Val Glu Tyr
385                 390                 395                 400

Arg Pro Glu Leu Leu Asp Ala Arg Val Ala Lys Glu Ala Leu His Gly
                405                 410                 415

Leu Ala Arg Val Leu Glu Arg Met Ile Ala Glu Pro Glu Thr Ala Val
            420                 425                 430

Gly Arg Leu Asp Val Val Gly Asp Ala Gly Arg Glu Leu Val Val Glu
        435                 440                 445

Arg Trp Asn Glu Thr Gly Asp Ala Ile Gly Ala Pro Ser Ala Val Asp
    450                 455                 460

Leu Phe Arg Arg Gln Val Ala Arg Ala Pro Ala Ala Thr Ala Val Thr
465                 470                 475                 480

Ala Gly Asp Leu Ala Trp Ser Tyr Ala Glu Leu Asp Glu Arg Ser Gly
                485                 490                 495

Arg Leu Ala Arg Ala Leu Thr Glu Arg Gly Val Arg Arg Gly Asp Arg
            500                 505                 510

Val Gly Val Val Leu Gly Arg Ser Ala Glu Val Leu Ala Ala Trp Leu
        515                 520                 525

Gly Val Trp Lys Ala Gly Ala Ala Phe Val Pro Val Asp Pro Asp Tyr
    530                 535                 540

Pro Ala Asp Arg Val Ala Phe Met Leu Ala Asp Ser Ala Val Ala Met
545                 550                 555                 560

Val Val Cys Gln Glu Ala Thr Ser Gly Val Val Pro Pro Gly Tyr Gln
                565                 570                 575

Gln Leu Leu Val Asn Asp Ala Asp Asp Gly Glu Ala Ala Leu Val Pro
            580                 585                 590

Ile Gly Ala Asp Asp Leu Ala Tyr Val Met Tyr Thr Ser Gly Ser Thr
        595                 600                 605

Gly Thr Pro Lys Gly Val Ala Ile Pro His Gly Gly Val Ala Ala Leu
    610                 615                 620

Ala Gly Asp Pro Gly Trp Gly Val Gly Pro Gly Asp Ala Val Leu Met
625                 630                 635                 640

His Ala Pro His Thr Phe Asp Ala Ser Leu Tyr Asp Val Trp Val Pro
                645                 650                 655

Leu Val Ser Gly Ala Arg Val Met Ile Thr Glu Pro Gly Val Val Asp
            660                 665                 670

Ala Glu Arg Leu Ala Gly His Val Ala Asp Gly Leu Thr Ala Val Asn
        675                 680                 685

Phe Thr Ala Gly His Phe Arg Ala Leu Ala Gln Glu Ser Pro Glu Ser
    690                 695                 700

Phe Ser Gly Leu Arg Glu Val Ala Ala Gly Gly Asp Val Val Pro Leu
705                 710                 715                 720

Asp Val Val Glu Arg Val Arg Arg Ala Cys Pro Arg Leu Arg Val Trp
                725                 730                 735

His Thr Tyr Gly Pro Thr Glu Thr Thr Leu Cys Ala Thr Trp Lys Ala
            740                 745                 750

Ile Glu Pro Gly Asp Glu Val Gly Pro Val Leu Pro Ile Gly Arg Ala
        755                 760                 765

Leu Pro Gly Arg Arg Leu Tyr Val Leu Asp Ala Phe Leu Arg Pro Leu
    770                 775                 780

Pro Pro Gly Ile Ala Gly Asp Leu Tyr Leu Ala Gly Ala Gly Val Ala
785                 790                 795                 800

His Gly Tyr Leu Gly Arg Ala Ser Leu Thr Ala Glu Arg Phe Val Ala
                805                 810                 815

Asp Pro Phe Val Ala Gly Glu Arg Met Tyr Arg Thr Gly Asp Leu Ala
            820                 825                 830

Tyr Trp Thr Gly Glu Gly Glu Leu Val Phe Ala Gly Arg Asp Asp Asp
        835                 840                 845

Gln Val Lys Ile Arg Gly Tyr Arg Val Glu Pro Gly Glu Val Glu Ala
    850                 855                 860

Val Leu Ala Gly Gln Pro Gly Val Asp Gln Ala Val Val Val Ala Arg
865                 870                 875                 880

Glu Gly Arg Leu Leu Gly Tyr Val Val Ser Gly Gly Gly Val Asp Pro
                885                 890                 895

Val Arg Leu Arg Glu Gly Val Ala Arg Val Leu Pro Glu Tyr Met Val
            900                 905                 910

Pro Ala Ala Val Val Val Leu Gly Ala Val Pro Val Thr Ala Asn Gly
        915                 920                 925

Lys Val Asp Arg Glu Ala Leu Pro Asp Pro Gly Phe Gly Gly Arg Val
    930                 935                 940

Ser Gly Arg Glu Pro Arg Thr Glu Val Glu Arg Ala Leu Cys Gly Leu

945                 950                 955                 960

Phe Ala Glu Val Leu Gly Leu Pro Gly Val Thr Ala Val Gly Pro Asp
                965                 970                 975

Asp Ser Phe Phe Glu Leu Gly Gly Asp Ser Ile His Ser Val Lys Leu
            980                 985                 990

Ala Ala Arg Ala Thr Arg Ala Gly  Met Pro Phe Thr Val  Val Glu Val
        995                 1000                 1005

Phe Glu  His Lys Thr Pro Ala  Gly Leu Ala Thr Ile  Val Asp Val
    1010                 1015                 1020

Gly Gly  Glu Pro Ala Ala Gly  Pro Ala Asp Pro Pro  Ser Asp Ser
    1025                 1030                 1035

Asp Leu  Leu Gly Leu Ala Gln  Asp Glu Ile Ala Glu  Phe Glu Ala
    1040                 1045                 1050

Glu Phe  Asp Asp Glu Arg His  Ser Leu Arg
    1055                 1060

<210>  28
<211>  277
<212>  PRT
<213>  Nonomuria

<400>  28

Met Ile Ser Lys Ala Met His Gly Pro Ile Arg Pro Ala Arg Ala Asp
1               5                   10                  15

Thr Leu Leu Ala Ser Val Gly Glu Arg Gly Ile Leu Cys Asp Phe Tyr
            20                  25                  30

Asp Glu Asn Ala Ser Glu Ile Phe Arg Asp Leu Glu Ala Asp Ala Gly
        35                  40                  45

Gly Thr Glu Glu Ala His Gly Phe Ala Ala Leu Val Arg Pro Glu Ser
    50                  55                  60

Gly Ala Ile Leu Glu Leu Gly Ala Gly Thr Gly Arg Leu Thr Ile Pro
65                  70                  75                  80

Leu Leu Glu Leu Gly Trp Glu Val Thr Ala Leu Glu Leu Ser Thr Ala
                85                  90                  95

Met Leu Thr Thr Leu Arg Thr Arg Leu Ala Asp Ala Pro Ala Asp Leu

            100                 105                 110

Arg Asp Arg Cys Thr Leu Val His Ala Asp Met Thr Ala Phe Lys Leu
        115                 120                 125

Gly Glu Arg Phe Gly Thr Ala Ile Leu Ser Pro Ser Thr Ile Asp Leu
    130                 135                 140

Leu Asp Asp Ala Asp Arg Pro Gly Leu Tyr Ser Ser Val Arg Glu His
145                 150                 155                 160

Leu Arg Pro Gly Gly Arg Phe Leu Leu Gly Met Ala Asn Pro Asp Ala
                165                 170                 175

Ser Gly Arg Gln Glu Pro Leu Glu Arg Thr Gln Glu Phe Thr Gly Arg
            180                 185                 190

Ser Gly Arg Arg Tyr Val Leu His Ala Lys Val Tyr Pro Ser Glu Glu
        195                 200                 205

Ile Arg Asp Val Thr Ile His Pro Ala Asp Glu Ser Ala Asp Pro Phe
    210                 215                 220

Val Ile Cys Val Asn Arg Phe Arg Val Ile Thr Pro Asp Gln Ile Ala
225                 230                 235                 240

Arg Glu Leu Glu Gln Ala Gly Phe Asp Val Val Ala Arg Thr Pro Leu
                245                 250                 255

Pro Gly Val Arg Asn His Glu Leu Val Leu Glu Ala Gln Trp Gly Ser
            260                 265                 270

Val Glu Asp Ala His
        275

<210> 29
<211> 531
<212> PRT
<213> Nonomuria

<400> 29

Met Ser Glu Glu Leu Leu Phe Leu Arg Pro Asp Thr Ile Ile Glu Pro
1               5                   10                  15

Leu Ala Asn Arg Phe Tyr Ala Ser Met Tyr Ala Thr Ala Pro Val Thr
            20                  25                  30

Ala Ala Met Asn Leu Ala Phe Arg Asn Leu Pro Met Leu Glu Ser Tyr

35                  40                  45

Leu Ala Ser Pro Glu Trp His Phe Ala Ala Ala Arg Asp Pro Lys Phe
    50                  55                  60

Arg Gly Gly Phe Phe Val Asn Ile Glu Glu Gln Arg Lys Asn Glu Val
65                  70                  75                  80

Glu Ala Leu Leu Ala Ala Ile Arg Arg Asp Ser Ala Asp Val Leu Arg
                85                  90                  95

Phe Ala Glu Ala Ile Ala Glu Ala Glu Lys Ile Ile Arg Glu Glu Ala
            100                 105                 110

Thr Gly Tyr Asp Leu Arg Pro Leu Tyr Pro Lys Leu Pro Pro Glu Leu
        115                 120                 125

Ser Gly Leu Val Glu Ile Ala Tyr Asp Thr Gly Asn Ala Ala Ser Leu
    130                 135                 140

His Phe Leu Glu Pro Leu Ile Tyr Lys Ser Lys Ala Tyr Ala Glu Asp
145                 150                 155                 160

Cys Gln Ser Val Gln Leu Ser Val Glu Thr Gly Ile Glu Arg Pro Phe
                165                 170                 175

Val Met Ser Thr Pro Arg Leu Pro Ser Pro Asp Val Leu Glu Leu Asn
            180                 185                 190

Ile Pro Phe Arg His Pro Gly Leu Glu Glu Leu Phe Leu Ser Arg Ile
        195                 200                 205

Arg Pro Thr Thr Leu Ala Ala Leu Arg Glu Ala Leu Glu Leu Gly Asp
    210                 215                 220

Ala Glu Ala Ala Arg Leu Ala Asp Leu Leu Val Pro Glu Pro Ser Leu
225                 230                 235                 240

Ala Ser Asp Arg His Val Ala Ala Gly Ala Arg Ile Arg Tyr Trp Gly
                245                 250                 255

His Ala Cys Leu Leu Met Gln Thr Pro Asp Val Ala Ile Met Thr Asp
            260                 265                 270

Pro Phe Ile Ser Ala Asp Thr Asp Ala Thr Gly Arg Tyr Thr Tyr Asn
        275                 280                 285

Asp Leu Pro Asp Arg Leu Asp Tyr Val Leu Ile Thr His Gly His Ser
    290                 295                 300

Asp His Leu Val Pro Glu Thr Leu Leu Gln Leu Arg Gly Arg Val Gly
305                 310                 315                 320

Thr Phe Val Val Pro Arg Thr Ser Arg Gly Asn Leu Cys Asp Pro Ser
                325                 330                 335

Leu Ala Leu Tyr Leu Arg Ser Phe Gly Leu Pro Ala Ile Glu Val Asp
            340                 345                 350

Asp Phe Asp Glu Ile Glu Phe Pro Gly Gly Lys Ile Val Ser Thr Pro
        355                 360                 365

Phe Phe Gly Glu His Ala Asp Leu Asp Ile Arg Ala Lys Ser Thr Tyr
    370                 375                 380

Trp Ile Asn Leu Gly Gly Lys Ser Ile Trp Val Gly Ala Asp Ser Ser
385                 390                 395                 400

Gly Leu Asp Pro Val Leu Tyr Arg His Ile Arg Arg His Leu Gly Ala
                405                 410                 415

Val Asn Ile Ala Phe Leu Gly Met Glu Cys Asp Gly Ala Pro Leu Asn
            420                 425                 430

Trp Gln Tyr Gln Pro Phe Ile Thr Lys Ala Leu Pro Lys Lys Met Ser
        435                 440                 445

Asp Ser Arg Lys Met Ser Gly Ser Asn Ala Glu Gln Ala Gly Ala Ile
    450                 455                 460

Val Thr Glu Leu Gly Ala Glu Glu Ala Tyr Ile Tyr Ala Met Gly Glu
465                 470                 475                 480

Glu Ser Trp Leu Gly His Val Met Ala Thr Ser Tyr Asn Glu Asp Ser
                485                 490                 495

Tyr Gln Leu Gln Gln Ile Ala Glu Phe Glu Ala Trp Cys Ser Arg Lys
            500                 505                 510

Gly Val Lys Ala Ala His Leu Leu Asp Gln His Glu Trp His Trp Ser
        515                 520                 525

Ser Ser Arg
    530

<210>  30
<211>  523
<212>  PRT
<213>  Nonomuria

<400>  30

Met Thr Gly Gly Thr Gly Ala Asp Ala Ala Ser Ala Gly Ala Ser Ser
1               5                   10                  15

Thr Arg Pro Glu Leu Arg Gly Glu Arg Cys Leu Pro Pro Ala Gly Pro
            20                  25                  30

Val Lys Val Thr Pro Asp Asp Pro Arg Tyr Leu Asn Leu Lys Leu Arg
        35                  40                  45

Gly Ala Asn Ser Arg Phe Asn Gly Glu Pro Asp Tyr Ile His Leu Val
    50                  55                  60

Gly Ser Thr Gln Gln Val Ala Asp Ala Val Glu Glu Thr Val Arg Thr
65                  70                  75                  80

Gly Lys Arg Val Ala Val Arg Ser Gly Gly His Cys Phe Glu Asp Phe
                85                  90                  95

Val Asp Asn Pro Asp Val Lys Val Ile Ile Asp Met Ser Leu Leu Thr
            100                 105                 110

Glu Ile Ala Tyr Asp Pro Ser Met Asn Ala Phe Leu Ile Glu Pro Gly
        115                 120                 125

Asn Thr Leu Ser Glu Val Tyr Glu Lys Leu Tyr Leu Gly Trp Asn Val
    130                 135                 140

Thr Ile Pro Gly Gly Val Cys Gly Gly Val Gly Val Gly Gly His Ile
145                 150                 155                 160

Cys Gly Gly Gly Tyr Gly Pro Leu Ser Arg Gln Phe Gly Ser Val Val
                165                 170                 175

Asp Tyr Leu Tyr Ala Val Glu Val Val Val Val Asn Lys Gln Gly Lys
            180                 185                 190

Ala Arg Val Ile Val Ala Thr Arg Glu Arg Asp Asp Pro His His Asp
        195                 200                 205

Leu Trp Trp Ala His Thr Gly Gly Gly Gly Gly Asn Phe Gly Val Val
    210                 215                 220

Thr Lys Tyr Trp Met Arg Val Pro Glu Asp Val Gly Arg Asn Pro Glu
225                 230                 235                 240

Arg Leu Leu Pro Lys Pro Pro Ala Thr Leu Leu Thr Ser Thr Val Thr
                245                 250                 255

Phe Asp Trp Ala Gly Met Thr Glu Ala Ala Phe Ser Arg Leu Leu Arg
            260                 265                 270

Asn His Gly Glu Trp Tyr Glu Arg Asn Ser Gly Pro Asp Ser Pro Tyr
        275                 280                 285

Thr Gly Leu Trp Ser Gln Leu Met Ile Gly Asn Glu Val Pro Gly Met
    290                 295                 300

Gly Glu Ser Gly Phe Met Met Pro Ile Gln Val Asp Ala Thr Arg Pro
305                 310                 315                 320

Asp Ala Arg Arg Leu Leu Asp Ala His Ile Glu Ala Val Ile Asp Gly
                325                 330                 335

Val Pro Pro Ala Glu Val Pro Glu Pro Ile Glu Gln Arg Trp Leu Ala
            340                 345                 350

Ser Thr Pro Gly Arg Gly Gly Arg Gly Pro Ala Ser Lys Thr Lys Ala
        355                 360                 365

Gly Tyr Leu Arg Lys Arg Leu Thr Asp Arg Gln Ile Gln Ala Val Tyr
    370                 375                 380

Glu Asn Met Thr His Met Asp Gly Ile Asp Tyr Gly Ala Val Trp Leu
385                 390                 395                 400

Ile Gly Tyr Gly Gly Lys Val Asn Thr Val Asp Pro Ala Ala Thr Ala
                405                 410                 415

Leu Pro Gln Arg Asp Ala Ile Leu Lys Val Asn Tyr Ile Thr Gly Trp
            420                 425                 430

Ala Asn Pro Gly Asn Glu Ala Lys His Leu Thr Trp Val Arg Lys Leu
        435                 440                 445

Tyr Ala Asp Val Tyr Ala Glu Thr Gly Gly Val Pro Val Pro Asn Asp
    450                 455                 460

Val Ser Asp Gly Ala Tyr Ile Asn Tyr Pro Asp Ser Asp Leu Ala Asp
465                 470                 475                 480

Pro Gly Leu Asn Thr Ser Gly Val Pro Trp His Asp Leu Tyr Tyr Lys
                485                 490                 495

Gly Asn His Pro Arg Leu Arg Lys Val Lys Ala Ala Tyr Asp Pro Arg
            500                 505                 510

Asn His Phe His His Ala Leu Ser Ile Arg Pro
        515                 520

<210>  31
<211>  141
<212>  PRT
<213>  Nonomuria

<400>  31

Met Thr Ser Thr Ser Gly Arg His Leu Tyr His Arg Gln Val Arg Phe
1               5                   10                  15

Ser Asp Ile Asp Ala His Gly His Val Asn Asn Val Arg Phe Leu Glu
            20                  25                  30

Tyr Leu Glu Asp Ala Trp Ile Ala Leu Tyr Leu Asp Asn Ala Gly Pro
        35                  40                  45

Pro Gln Glu Asp Arg Asp Gly Leu Pro Ala Val Gly Phe Ala Val Val
    50                  55                  60

Arg His Glu Ile Phe Tyr Arg Arg Pro Leu Arg Phe Arg His Gly Ser
65                  70                  75                  80

Val Arg Val Glu Ser Trp Val Thr Lys Val Asn Arg Val Thr Cys Glu
                85                  90                  95

Met Ala Ala Gln Ile Cys Ser Asp Gly Glu Val Phe Val Glu Ala Arg
            100                 105                 110

Ser Met Ile Met Gly Phe Asp Thr His Thr Ala Lys Pro Arg Arg Leu
        115                 120                 125

Thr Leu His Glu Arg Thr Phe Leu Lys Arg Tyr Leu Arg
    130                 135                 140

<210>  32
<211>  372
<212>  PRT
<213>  Nonomuria

<400>  32

Met Gly Val Asp Val Ser Met Thr Thr Ser Ile Ala Ser Ala Glu Asp

1               5                   10                  15

Leu Ser Val Leu Thr Gly Leu Ser Glu Ile Thr Thr Phe Ala Gly Val
            20                  25                  30

Gly Thr Ala Val Ser Ala Thr Ser Tyr Ser Gln Ala Glu Leu Leu Glu
        35                  40                  45

Ile Leu Asp Ile Arg Asp Pro Arg Ile Arg Ser Leu Phe Leu Asn Ser
    50                  55                  60

Ala Ile Glu Arg Arg Phe Leu Ala Leu Pro Pro Gln Gly Arg Asp Gly
65                  70                  75                  80

Glu Arg Val Ala Glu Pro Gln Gly Asp Leu Leu Asp Lys His Lys Lys
                85                  90                  95

Leu Ala Val Asp Met Gly Cys Arg Ala Leu Glu Ser Cys Leu Lys Ser
            100                 105                 110

Ala Gly Ala Thr Leu Ser Asp Val Arg His Leu Cys Cys Val Thr Ser
        115                 120                 125

Thr Gly Phe Leu Thr Pro Gly Leu Ser Ala Leu Ile Ile Arg Glu Leu
    130                 135                 140

Gly Leu Asp Pro His Cys Ser Arg Ala Asp Ile Val Gly Met Gly Cys
145                 150                 155                 160

Asn Ala Gly Leu Asn Ala Leu Asn Leu Val Ala Gly Trp Ser Ala Ala
                165                 170                 175

His Pro Gly Glu Leu Ala Val Val Leu Cys Ser Glu Ala Cys Ser Ala
            180                 185                 190

Ala Tyr Ala Leu Asp Gly Thr Met Arg Thr Ala Val Val Asn Ser Leu
        195                 200                 205

Phe Gly Asp Gly Ser Ala Ala Leu Ala Val Val Ser Gly Asp Gly Arg
    210                 215                 220

Ala Ala Gly Pro Arg Val Leu Lys Phe Ala Ser Tyr Val Ile Thr Asp
225                 230                 235                 240

Ala Ile Glu Ala Met Arg Tyr Asp Trp Asp Arg Asp Gln Asp Arg Phe
                245                 250                 255

Ser Phe Phe Leu Asp Pro Gln Ile Pro Tyr Val Val Gly Ala His Ala
            260                 265                 270

Glu Ile Val Val Asp Lys Leu Leu Ser Gly Thr Gly Leu Arg Arg Ser
        275                 280                 285

Asp Ile Gly His Trp Leu Val His Ser Gly Gly Lys Lys Val Ile Asp
    290                 295                 300

Ala Ile Val Val Asn Leu Gly Leu Ser Arg His Asp Val Arg His Thr
305                 310                 315                 320

Thr Ala Val Leu Arg Asp Tyr Gly Asn Leu Ser Ser Gly Ser Phe Leu
                325                 330                 335

Phe Ser Tyr Glu Arg Leu Ala Gly Glu Gly Val Thr Arg Pro Gly Asp
            340                 345                 350

Tyr Gly Val Leu Met Thr Met Gly Pro Gly Ser Thr Ile Glu Thr Ala
        355                 360                 365

Leu Ile Gln Trp
    370

<210>  33
<211>  213
<212>  PRT
<213>  Nonomuria

<400>  33

Met Asn Gly Glu Leu Glu Leu Ser Leu Asp Gly Thr Gln Ala Leu Thr
1               5                   10                  15

Ala Ser Val Glu Glu Leu Asn Gly Leu Cys Asp Arg Ala Glu Asp His
            20                  25                  30

Arg Ala Pro Gly Pro Val Ile Val His Val Thr Gly Val Pro Arg Leu
        35                  40                  45

Gly Trp Ser Lys Gly Leu Thr Val Gly Leu Val Ser Lys Trp Glu Arg
    50                  55                  60

Val Val Arg Arg Phe Glu Arg Leu Gly Arg Leu Thr Val Ala Val Ala
65                  70                  75                  80

Ser Gly Asp Cys Ala Gly Pro Ser Leu Asp Leu Leu Leu Ala Ala Asp
                85                  90                  95

Val Arg Ile Ala Ala Pro Ala Thr Arg Leu Leu Pro Ser Trp Ala Gly
            100                 105                 110

Gly Ala Ala Trp Pro Gly Met Ala Val Tyr Arg Leu Thr Gln Gln Ala
        115                 120                 125

Gly Thr Gly Gly Ile Arg Arg Ala Val Leu Leu Gly Ala Pro Ile Asp
    130                 135                 140

Ala Asp Arg Ala Leu Ala Leu Asn Leu Ile Asp Glu Val Ser Ala Asp
145                 150                 155                 160

Pro Ala Ala Ser Leu Ala Gly Leu Ala Gly Ala Gly Asp Gly Ala Glu
                165                 170                 175

Leu Ala Ile Arg Arg Gln Leu Met Phe Glu Ala Ser Ser Thr Thr Phe
            180                 185                 190

Glu Asp Ala Leu Gly Ala His Leu Ala Ala Val Asp Arg Ala Leu Arg
        195                 200                 205

Arg Glu Thr Leu Ser
    210

<210>  34
<211>  434
<212>  PRT
<213>  Nonomuria

<400>  34

Met Thr Thr Asp Trp Pro Ala Leu Pro Pro Arg Ala Pro Leu Ala Leu
1               5                   10                  15

Trp Thr Leu Thr Ala Glu Ala Gln Arg Val Asp Asp Leu Leu Ala Gly
            20                  25                  30

Leu Pro Glu Pro Pro Ala Arg Thr Ser Ala Gln Arg Asp Ala Ala Ala
        35                  40                  45

Ser Ala Leu Asp Lys Val Arg Arg Met Arg Ala Asp Tyr Met Glu Ala
    50                  55                  60

His Ala Glu Glu Ile Tyr Gly Glu Leu Thr Ser Gly Arg Thr Arg His
65                  70                  75                  80

Leu Arg Ile Asp Glu Leu Val Arg Ala Ala Ala Arg Ala Tyr Pro Gly
                85                  90                  95

Leu Val Pro Thr Asp Glu Gln Met Ala Ala Glu Arg Ala Arg Pro Gln
            100                 105                 110

Ala Glu Lys Glu Gly Arg Glu Ile Asp Gln Gly Ile Phe Leu Arg Gly
        115                 120                 125

Val Leu Arg Ala Pro Lys Ala Gly Pro His Leu Leu Asp Ala Met Leu
    130                 135                 140

Arg Pro Thr Pro Arg Ala Leu Glu Leu Leu Pro Glu Phe Ile Glu Ser
145                 150                 155                 160

Gly Glu Val Arg Met Glu Ala Val Leu Leu Arg Arg Arg Asp Gly Val
                165                 170                 175

Ala Tyr Leu Thr Leu Cys Arg Asp Asp Cys Leu Asn Ala Glu Asp Ala
            180                 185                 190

Gln Gln Val Asp Asp Met Glu Thr Ala Val Asp Leu Ala Leu Leu Asp
        195                 200                 205

Pro Gln Val Arg Val Gly Leu Leu Arg Gly Gly Glu Met Ser His Pro
    210                 215                 220

Arg Tyr Arg Gly Arg Arg Val Phe Cys Ala Gly Val Asn Leu Lys Lys
225                 230                 235                 240

Leu Ser Ser Gly Asp Ile Ser Leu Val Asp Phe Leu Leu Arg Arg Glu
                245                 250                 255

Leu Gly Tyr Ile His Lys Ile Val Arg Gly Val Tyr Thr Asp Gly Ser
            260                 265                 270

Trp His Ser Lys Leu Thr Asp Lys Pro Trp Met Ala Val Val Asp Ser
        275                 280                 285

Phe Ala Ile Gly Gly Gly Ala Gln Leu Leu Leu Val Phe Asp Gln Val
    290                 295                 300

Leu Ala Ala Ser Asp Ser Tyr Ile Ser Leu Pro Ala Ala Thr Glu Gly
305                 310                 315                 320

Ile Ile Pro Gly Val Ala Asn Tyr Arg Leu Thr Arg Phe Thr Gly Pro
                325                 330                 335

Arg Ala Ala Arg Gln Met Ile Leu Gly Gly Arg Arg Ile Arg Ala Asp
            340                 345                 350

Glu Pro Asp Ala Arg Leu Met Ile Asp Glu Val Val Pro Pro Glu Glu
        355                 360                 365

Met Asp Ala Ala Ile Asp Arg Ala Leu Ala Arg Leu Asp Gly Asp Ala
    370                 375                 380

Val Pro Ala Asn Arg Arg Met Leu Asn Leu Ala Glu Glu Pro Pro Glu
385                 390                 395                 400

Ala Phe Gly Arg Tyr Leu Ala Glu Phe Ala Leu Gln Gln Ala Leu Arg
                405                 410                 415

Ile Tyr Gly Arg Asp Val Ile Gly Lys Val Gly Arg Phe Ala Ala Gly
            420                 425                 430

Ser Ala

<210>  35
<211>  265
<212>  PRT
<213>  Nonomuria

<400>  35

Met Ser Glu Pro Arg Val Arg Tyr Glu Lys Lys Glu His Val Ala His
1               5                   10                  15

Val Thr Met Asn Arg Pro His Val Leu Asn Ala Met Asp Arg Arg Met
            20                  25                  30

His Glu Glu Leu Ala Glu Ile Trp Asp Asp Val Glu Ala Asp Asp Asp
        35                  40                  45

Val Arg Thr Val Val Leu Thr Gly Ala Gly Thr Arg Ala Phe Ser Val
    50                  55                  60

Gly Gln Asp Leu Lys Glu Arg Ala Leu Leu Asp Glu Ala Gly Thr Gln
65                  70                  75                  80

Ala Ser Thr Phe Gly Ser Arg Gly Gln Ala Gly His Pro Arg Leu Thr
                85                  90                  95

Asp Arg Phe Thr Leu Ser Lys Pro Val Val Ala Arg Val His Gly Tyr
            100                 105                 110

Ala Leu Gly Gly Gly Phe Glu Leu Val Leu Ala Cys Asp Leu Val Ile
        115                 120                 125

Ala Ser Glu Glu Ala Val Phe Gly Leu Pro Glu Val Arg Leu Gly Leu
    130                 135                 140

Ile Pro Gly Ala Gly Gly Val Phe Arg Leu Pro Arg Gln Leu Pro Gln
145                 150                 155                 160

Lys Val Ala Met Gly His Leu Leu Thr Gly Arg Arg Met Asp Ala Ala
                165                 170                 175

Thr Ala Phe Arg Tyr Gly Leu Val Asn Glu Val Val Pro Leu Asp Glu
            180                 185                 190

Leu Asp Arg Cys Val Ala Gly Trp Thr Asp Asp Leu Val Arg Ala Ala
        195                 200                 205

Pro Leu Ser Val Arg Ala Ile Lys Glu Ala Ala Met Arg Ser Leu Asp
    210                 215                 220

Ile Pro Leu Glu Glu Ala Phe Thr Thr Ser Tyr Pro Trp Glu Glu Arg
225                 230                 235                 240

Arg Arg Arg Ser Gly Asp Ala Ile Glu Gly Val Arg Ala Phe Val Glu
                245                 250                 255

Lys Arg Asp Pro Val Trp Thr Ser Arg
            260                 265

<210>  36
<211>  428
<212>  PRT
<213>  Nonomuria

<400>  36

Met Ile Pro Pro His Thr Leu Leu Val Phe Phe Val Gln Ala Ala Ala
1               5                   10                  15

Leu Leu Leu Leu Ala Leu Leu Leu Gly Arg Leu Ala Val Arg Leu Gly
            20                  25                  30

Leu Ala Ala Val Val Gly Glu Leu Cys Ala Gly Val Ile Leu Gly Pro
        35                  40                  45

Ser Val Leu Gly Gln Val Ala Pro Gly Ala Glu Gln Trp Leu Phe Pro
    50                  55                  60

Ser Pro Ser Ser His Met Leu Asp Ala Val Gly Gln Leu Gly Val Leu
65                  70                  75                  80

Leu Leu Ile Gly Leu Thr Gly Ala His Leu Asp Leu Arg Leu Ile Arg
                85                  90                  95

Arg Gln Gly Ala Thr Ala Val Arg Val Ser Ala Phe Gly Leu Val Val
            100                 105                 110

Pro Met Ala Leu Gly Ile Gly Ala Gly Leu Leu Leu Pro Ala Glu Phe
        115                 120                 125

Arg Gly Thr Gly Gly Ser Ala Val Phe Ala Leu Phe Leu Gly Val Thr
    130                 135                 140

Met Cys Val Ser Ser Ile Pro Val Ile Ala Lys Thr Leu Met Asp Met
145                 150                 155                 160

Asn Leu Leu His Arg Asn Val Gly Gln Leu Thr Leu Thr Ala Gly Met
                165                 170                 175

Ile Asp Asp Ala Phe Gly Trp Val Leu Leu Ser Val Val Thr Ala Met
            180                 185                 190

Ala Thr Ala Gly Ala Gly Ala Gly Thr Val Val Leu Ser Ile Ala Ser
        195                 200                 205

Leu Leu Gly Val Ile Val Phe Ser Val Val Ile Gly Arg Pro Ala Val
    210                 215                 220

Arg Val Ala Leu Arg Thr Thr Glu Asp Gln Gly Val Ile Ala Gly Gln
225                 230                 235                 240

Val Val Val Leu Val Leu Ala Ala Ala Ala Gly Thr His Ala Leu Gly
                245                 250                 255

Leu Glu Pro Ile Phe Gly Ala Phe Val Ala Gly Leu Leu Val Ser Thr
            260                 265                 270

Ala Met Pro Asn Pro Val Arg Leu Ala Pro Leu Arg Thr Val Thr Leu
        275                 280                 285

Gly Val Leu Ala Pro Leu Tyr Phe Ala Thr Met Gly Leu Arg Val Asp
    290                 295                 300

Leu Thr Ala Leu Ala Arg Pro Glu Val Leu Ala Val Gly Leu Leu Val
305                 310                 315                 320

Leu Ala Leu Ala Ile Ile Gly Lys Phe Leu Gly Ala Phe Leu Gly Ala
                325                 330                 335

Trp Thr Ser Arg Leu Ser Arg Trp Glu Ala Leu Ala Leu Gly Ala Gly
            340                 345                 350

Met Asn Ala Arg Gly Val Ile Gln Met Ile Val Ala Thr Val Gly Leu
        355                 360                 365

Arg Leu Gly Val Ile Thr Asp Glu Ile Phe Thr Ile Ile Ile Val Val
    370                 375                 380

Ala Val Ile Thr Ser Leu Leu Ala Pro Pro Leu Leu Arg Leu Ala Met
385                 390                 395                 400

Thr Arg Ile Glu Ala Thr Ala Glu Glu Glu Ala Arg Leu Leu Ala Tyr
                405                 410                 415

Gly Leu Arg Pro Gly Glu Ala Arg Glu Asp Val Arg
            420                 425

<210>  37
<211>  251
<212>  PRT
<213>  Nonomuria

<400>  37

Met Ser Thr Trp Phe Arg Cys Phe Asp Arg Arg Pro Leu Ala Thr Met
1               5                   10                  15

Arg Leu Ile Cys Phe Pro His Ala Gly Gly Ser Ala Val Phe Tyr Arg
            20                  25                  30

Asn Trp His Arg Leu Ala Ala Pro Glu Ile Glu Val His Ala Val Gln
        35                  40                  45

Tyr Pro Gly Arg Ala Asp Arg Leu His Glu Pro Leu Val Gly Asp Ala
    50                  55                  60

His Arg Leu Ala Glu Ser Val Gly Arg Glu Leu Arg Pro Leu Leu Asp
65                  70                  75                  80

Arg Pro Val Ala Leu Phe Gly His Ser Met Gly Ser Leu Ile Ala Tyr
                85                  90                  95

Glu Thr Ala Arg Leu Leu Thr Gly Ser Gly Ile Pro Pro Ala His Leu
            100                 105                 110

Phe Val Ser Gly Gly Val Ala Ala His Asp Arg Gly Arg Leu Ala His
        115                 120                 125

Arg Val Ala Pro Ala Ser Glu Glu Ala Leu Ile Asp Arg Leu Arg Leu
    130                 135                 140

Leu Gly Gly Thr Asp Ala Glu Ala Leu Ala Ser Ala Glu Phe Arg Ala
145                 150                 155                 160

Phe Ala Leu Pro Tyr Val Arg Asn Asp Phe Gln Leu Val Gln Ser Tyr
                165                 170                 175

Arg His Thr Pro Gly Pro Pro Leu Thr Val Pro Ile Thr Ala Phe Thr
            180                 185                 190

Gly Ala Asp Asp Pro Val Val Arg Leu Asp Ala Val Ala Arg Trp Ala
        195                 200                 205

Glu Leu Thr Ala Arg Glu Phe Ser Cys His Val Leu Pro Gly Gly His
    210                 215                 220

Phe Phe Leu Gly His Glu Gln Ala Ala Leu Trp Ala His Leu His Ala
225                 230                 235                 240

Arg Leu Gly Ile Ala Thr Pro Ala His Cys Gly
                245                 250

<210>  38
<211>  428
<212>  PRT
<213>  Nonomuria

<400>  38

Met Asp Ser His Val Leu Ala His Gln Leu Ser Lys Glu Thr Leu His
1               5                   10                  15

Gly Ser Leu Met Asp Pro Ala Ile Glu Ser Met Asn Leu Leu Asn Glu
            20                  25                  30

Ile Ala Gly Asn Tyr Pro Asp Ala Ile Ser Met Ala Ala Gly Arg Pro
        35                  40                  45

Tyr Glu Glu Phe Phe Asp Val Gly Leu Ile His Asp Tyr Leu Glu Ala
    50                  55                  60

Tyr Arg Asp His Leu Arg Asn Asp Arg Arg Met Asp Asp Ala Gly Ile
65                  70                  75                  80

Ser Arg Met Leu Phe Gln Tyr Gly Thr Thr Lys Gly Ile Ile Ser Asp
                85                  90                  95

Leu Val Ala Arg His Leu Ala Glu Asp Glu Asn Ile Glu Ala Asp Pro
            100                 105                 110

Ala Ser Val Val Ile Thr Val Gly Phe Gln Glu Ala Met Phe Leu Val
        115                 120                 125

Leu Arg Ala Leu Arg Ala Asn Glu Arg Asp Val Leu Leu Ala Pro Thr
    130                 135                 140

Pro Thr Tyr Val Gly Leu Thr Gly Ala Ala Leu Leu Thr Asp Thr Pro
145                 150                 155                 160

Val Trp Pro Val Gln Ser Thr Asp Asn Gly Ile Asp Leu Asp His Leu
                165                 170                 175

Glu His Gln Leu Lys Arg Ala Gln Asp Gln Gly Ala Arg Val Arg Ala
            180                 185                 190

Cys Tyr Val Thr Pro Asn Phe Ala Asn Pro Thr Gly Thr Ser Met Asp
        195                 200                 205

Leu Pro Ala Arg His Arg Leu Leu Glu Val Ala Ala Ala His Gly Ile
    210                 215                 220

Leu Ile Leu Glu Asp Asn Ala Tyr Gly Leu Leu Gly Gln Asp Arg Leu
225                 230                 235                 240

Pro Thr Leu Lys Ser Leu Asp His Ala Ala Thr Val Val Tyr Leu Gly
                245                 250                 255

Ser Phe Ala Lys Thr Gly Met Pro Gly Ala Arg Val Gly Tyr Val Val
            260                 265                 270

Ala Asp Gln His Val Ala Gly Gly Gly Ser Leu Ala Asp Glu Leu Ala
        275                 280                 285

Lys Leu Lys Gly Met Leu Thr Val Asn Thr Ser Pro Ile Ala Gln Ala
    290                 295                 300

Val Ile Ala Gly Lys Leu Leu Arg His Asp Phe Ser Leu Ala Arg Ala
305                 310                 315                 320

Asn Ala Arg Glu Thr Ala Ile Tyr Gln Arg Asn Leu His Leu Thr Leu
                325                 330                 335

Asp Glu Leu Thr Arg Arg Leu Gly Ala Val Pro Gly Val Thr Trp Asn

340                 345                 350

Ala Pro Thr Gly Gly Phe Phe Ile Thr Val Thr Val Pro Phe Val Val
        355                 360                 365

Asp Asp Glu Leu Leu Glu His Ala Ala Arg Asp His Gly Val Leu Phe
    370                 375                 380

Thr Pro Met His His Phe Tyr Gly Gly Lys Asp Gly Phe Asn Gln Leu
385                 390                 395                 400

Arg Leu Ser Ile Ser Leu Leu Asn Pro Gln Leu Ile Glu Glu Gly Val
                405                 410                 415

Ser Arg Leu Ala Gly Leu Val Thr Ala Cys Leu Pro
            420                 425

<210>  39
<211>  18
<212>  DNA
<213>  synthetic

<400>  39
atgcgcgtgt tgatctcg                                                    18

<210>  40
<211>  18
<212>  DNA
<213>  synthetic

<400>  40
cggctgaccg cggcgaac                                                    18

<210>  41
<211>  20
<212>  DNA
<213>  synthetic

<400>  41
cgtgggggtg gatgtatcga                                                  20

<210>  42
<211>  17
<212>  DNA
<213>  synthetic

<400>  42
tcaccattgg atcagcg                                                     17

<210>  43
<211>  18
<212>  DNA

<400>  43
   tcaggagacg aaccccgc                                                    18

<210>  44
<211>  18
<212>  DNA
<213>  synthetic

<400>  44
gtgcacgaaa gtcccgtc                                                    18

<210>  45
<211>  18
<212>  DNA
<213>  synthetic

<400>  45
atggactccc acgttctc                                                    18

<210>  46
<211>  18
<212>  DNA
<213>  synthetic

<400>  46
tcaggggaga catgcggt                                                    18

<210>  47
<211>  29
<212>  DNA
<213>  synthetic

<400>  47
ttttgaattc tcaggcgatc cgtccgtct                                        29

<210>  48
<211>  31
<212>  DNA
<213>  synthetic

<400>  48
tttttctaga gcccggacac ccgggggctg a                                     31

<210>  49
<211>  31
<212>  DNA
<213>  synthetic

<400>  49
tttttctaga agtcatggtg atgtgcgaca t                                     31

<210>  50
<211>  30
<212>  DNA
<213>  synthetic

<400>  50

ttttaagctt atgttgcagg acgccgaccg 30

## Description

### BACKGROUND OF THE INVENTION

Actinomycetes are well known for their ability to produce structurally diverse and biologically active secondary metabolites, many of which have found co mmercial application (e.g. antibiotics). Important metabolites are not only produced by *Streptomyces* spp. (studied in most detail) but also by lesser known genera of actinomycetes: e.g. rifamycins, teicoplanin and erythromycin are currently produced industrially by *Amycolatopsis, Actinoplanes* and *Saccharopolyspora* species, respectively. The genetic elements governing the biosynthesis of secondary metabolites are organized in gene clusters, which contain all the genes required for synthesis of the metabolites, regulation and resistance.

Many different secondary metabolites share a common biosynthetic route, where similar enzymes intervene. This has been thoroughly documented for polyketides (Katz and McDaniel 1999), non-ribosomally synthesized peptides (Marahiel 1997) and deoxysugars (Rodriguez et al. 2000). However, despite this similarity, the organization of the gene cluster involved in the synthesis of a particular secondary metabolite in a given microorganism cannot be defined *a priori*. In fact, the synthesis of very similar secondary metabolites may be governed by differently organized clusters, especially when the corresponding producer strains do not belong to the same genus. Example of this sort can be found among the macrolide antibiotics (Katz and McDaniel 1999). Furthermore, the identification of a desired cluster within a producer strain is complicated in actinomycetes by the occurrence of multiple clusters specifying enzymes for the same pathway. This has been shown for polyketides (e.g. Ruan et al. 1997) and peptides (e.g. Sosio et al. 2000a), and confirmed by genome sequencing (Omura et al. 2001; Bentley et al. 2002). Consequently, one cannot know a *priori* the organization, nucleotide sequence, or extent of identity of a new cluster as compared to those already known.

Glycopeptides, also known as dalbaheptides because of their mechanism of action (Parenti and Cavalleri 1989), are an important class of antibiotics, interfering with cross-linking of the bacterial cell wall, with vancomycin and teicoplanin currently in clinical use. They are often last choice antibiotics in treating life- threatening infections. On the other hand, the emergence of resistance to glycopeptides among enterococci and the fear that this high-level resistance may eventually become widespread in methicillin-resistant *Staphylococcus aureus* has prompted the search for second-generation drugs of this class. Promising results have been obtained with the development of semi-synthetic derivatives with improved activity, expanded antibacterial spectrum or better pharmacokinetics (Malabarba and Ciabatti 2001).

Therefore, there exists the potential and the utility to obtain improved glycopeptides by manipulation of occurring natural compounds. However, glycopeptides are structurally complex molecules and their accessibility to chemistry is limited to a few positions in the molecule. For example, while the sugars can be easily removed chemically from a glycopeptide, generating the corresponding aglycone, the regioselective attachment of a different sugar to a particular position by chemical means is extremely difficult. It has been shown that the extent of chlorination in glycopeptides influences antibiotic activity. Similarly, the chemical dechlorination of aromatic rings in glycopeptides can be easily achieved, while the selected halogenation of desired rings in the structure is relatively complex. As a final example, glycopeptides of the teicoplanin family contain an acyl chain linked to the glucosamine attached to the arylamino acid at position 4, while compounds of the vancomycin class do not. Acylation and deacylation of glycopeptides has been reported either chemically or by biotransformation (Lancini and Cavalleri 1997), but it usually results in overall low yields. In light of the above, it would be desirable to have genes and enzymes useful for redirecting these steps in glycopeptide formation, in order to obtain derivatives that are hard or impossible to make by chemical means. This is particularly relevant, since it has been shown that the extent of chlorination influences the biological activity of glycopeptides, as well as that improved derivatives can be obtained by altering the glycosylation or acylation pattern of glycopeptides (Malabarba and Ciabatti 2001). One of the major limitations for chemistry is to change the type or order of amino acids present in the peptide backbone. Chemically, it has been shown to be possible to intervene only on amino acids 1 and 3 with relatively low yield (Malabarba et al. 1997). General methods for the design of novel glycopeptide derivatives directly by fermentation processes with precisely engineered strains would thus be highly desirable.

An attractive alternative would be to generate improved antibiotics by engineering of biosynthetic processes for naturally occurring glycopeptides. Examples of this sort have been reported. Indeed, it has been possible to selectively glycosylate glycopeptide aglycons both in vitro and in vivo after the expression of glycosyltransferases from the vancomycin and chloroeremomycin gene clusters (Solenberg et al. 1997; Loosey et al. 2001). However, none of the enzymes described so far is able to attach a glucosamine residue at desired positions. Similarly, inactivation of selected genes in the balhimycin producer *A. mediterranei* has led to the obtainment of balhimycin derivatives (Pelzer et al. 1999). However, no such experiments have been described for strains producing glycopeptides of the teicoplanin family.

The antibiotic A40926 belongs to the teicoplanin family of glycopeptides (Parenti and Cavalleri 1989). It consists of a complex of closely related molecules, whose core structure can be reconducted to a heptapeptide skeleton with a rigid scaffold determined by ether bonds between amino acids 1-3, 2-4 and 4-6, and a C-C bond between amino acids 5-7. In addition two sugar residues and two chlorine atoms are present on the molecule. The structure of the components of A40926 complex is represented by the formula shown below, wherein R represents [C₉-C₁₂] alkyl with the factors A₁(R= n-decyl), factor B₀ (R= 9-methyldecyl) and factor B₁ (R=n-undecyl) being the main components.

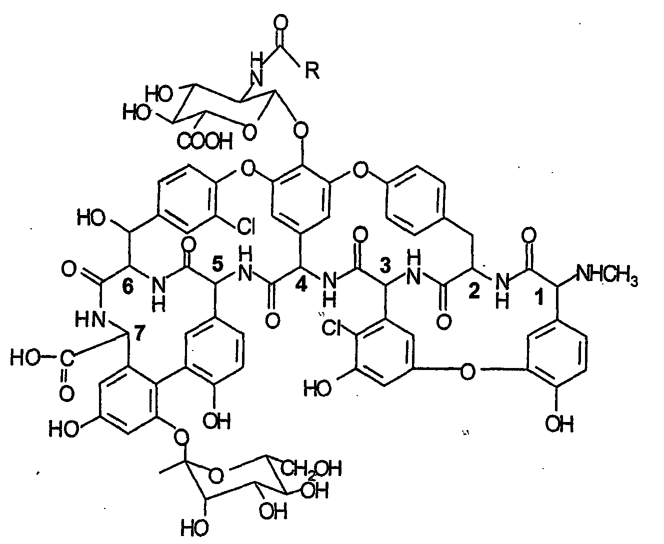

The producer strain, formerly known as *Actinomadura* sp. ATCC39727, has been recently reclassified as *Nonomuria* sp. ATCC39727 (Zhang et al. 1998). Besides showing an intrinsic antibacterial activity, A40926 is also the precursor of the semi-synthetic glycopeptide dalbavancin (formerly known as BI397 or MDL 62397; Malabarba and Ciabatti 2001). Therefore, additional tools for manipulating the structure of A40926 and for increasing its yield would be highly desirable. However, there are no examples of clusters described from other members of the genus *Nonomuria*. Therefore, the genes required for and regulating the formation of A40926 in *Nonomuria* can also be useful in optimizing the production process.

Recently, gene clusters involved in the formation of the glycopeptides chloroeremomycin (van Wageningen et al. 1998), balhimycin (Pelzer et al. 1999), complestatin (Chiu et al. 2001) and A47934 (Pootoolal et al. 2002) have been described. These clusters, designated *cep, bal, com* and *sta*, respectively, were obtained from *Amycolatopsis orientalis, Amycolatopsis mediterranei, Streptomyces lavendulae* and *Streptomyces toyocaensis,* respectively. These clusters have provided several genes useful for manipulating glycopeptide pathways. However, certain steps cannot be performed with the described clusters. For example, the available gene clusters do not encode functions capable of changing the oxidation state of sugars, of attaching a fatty acid chain, or of providing a chlorine atom at the aromatic moiety of amino acid 3. All these functions are also described in the present invention.

The design of industrial processes for antibiotic production has been relatively successful, resulting in large size fermentations with antibiotic titers reaching levels of several grams per liter. This has been achieved largely by following empirical, trial and error approaches, and lacks a rational basis. Development of new processes and improvement of current technology thus remains time consuming and may result in bacterial cultures that are unstable, perform inconsistently and accumulate unwanted by-products. In recent years, rational methods have been applied successfully to increase the level of antibiotic produced by *Streptomyces* spp., which have often involved the manipulation of key regulatory elements present within the gene cluster of interest or the overexpression of rate-limiting steps in the pathway. Therefore, the genes encoding such cluster-associated regulators or limiting steps in the synthesis can be effective tools for yield improvement. However, the cluster-associated regulators so far identified in actinomycetes belong to several different protein families (Chater and Bibb 1997). Even within one family, there is considerable variation in sequence identity. Therefore, the existence, nature, number and sequence of cluster-associated regulators cannot be predicted by comparison to other cluster, even those specifying a related antibiotic. As an example, the tylosin gene cluster encodes four distinct regulators, while none has been found in the cluster specifying the related macrolide antibiotic erythromycin (Bate et al. 1999). Similarly, the nature and reason for a rate-limiting step in a biosynthetic pathway cannot be established *a priori*.

### SUMMARY OF THE INVENTION

The present invention provides a set of isolated polynucleotide molecules required for the biosynthesis of the glycopeptide A40926 in microorganisms. In one form of the invention, polynucleotide molecules are selected from the contiguous DNA sequence (SEQ ID NO: 1), which represents the *dbv* gene cluster as isolated from *Nonomuria* sp. ATCC39727 and consists of 37 ORFs encoding the polypeptides required for A40926 formation. The amino acid sequences of the polypeptide encoded by said 37 ORFs are provided in SEQ ID NOS: 2 to 38.

The present invention provides an isolated nucleic acid comprising a nucleotide sequence selected from a group consisting of:
a) the *dbv* gene cluster encoding the polypeptides required for the synthesis of A40926 (SEQ ID NO: 1);
b) a nucleotide sequence encoding the same polypeptides encoded by the *dbv* gene cluster (SEQ ID NO. 1), other than the nucleotide sequence of the *dbv* gene cluster itself;
c) any nucleotide sequence of *dbv* ORFs 1 to 37, encoding the polypeptides of SEQ ID NOS: 2 to 38;
d) a nucleotide sequence encoding the same polypeptide encoded by any of *dbv* ORFs 1 to 37 (SEQ ID NOS: 2 to 38), other than the nucleotide sequence of said ORF.
   A further object of this invention is to provide an isolated nucleic acid comprising a nucleotide sequence selected from the group consisting of:
e) a nucleotide sequence of any of *dbv* ORFs 3 to 4, 6 to 10, 18 to 20, 22 to 23, 29 to 30, and 36, encoding the polypeptides specified in SEQ ID NOS: 4 to 5, 7 to 11, 19 to 21, 23 to 24, 30 to 31, and 37;
f) a nucleotide sequence encoding the same polypeptide encoded by any of *dbv* ORFs 3 to 4, 6 to 10, 18 to 20, 22 to 23, 29 to 30, and 36 (SEQ ID NOS: 4 to 5, 7 to 11, 19 to 21, 23 to 24, 30 to 31, and 37) other than the nucleotide sequence of said *dbv* ORF;
g) a nucleotide sequence encoding a polypeptide that is at least 80%, preferably 86%, more preferably 90%, most preferably 95% or more, identical in amino acid sequence to a polypeptide encoded by any of *dbv* ORFs 3, 6 to 9, 18 to 20, 22 to 23, 29 to 30, and 36 (SEQ ID NOS: 4, 7 to 10, 19 to 21, 23 to 24, 30 to 31, and 37);
h) a nucleotide sequence encoding a polypeptide that is at least 87%, preferably 90%; more preferably 95% or more, identical in amino acid sequence to a polypeptide encoded by any of *dbv* ORFs 4 and 10 (SEQ ID NOS: 5 and 11).

In one embodiment the isolated nucleic acids of this invention comprise combinations of ORFs selected from ORFs 1 to 37 (SEQ ID NOS: 2 to 38), which encode polypeptides required for the synthesis of 4-hydroxyphenylglycine (HPG) residues of A40926. In another embodiment, the nucleic acid comprises combinations of ORFs selected from ORFs 1 to 37 (SEQ ID NOS: 2 to 38), which encode the polypeptides required for the synthesis of 3,5-dihydroxyphenylglycine (DPG) residues of A40926. In yet another embodiment, the nucleic acid comprises combinations of ORFs selected from ORFs 1 to 37 (SEQ ID NOS: 2 to 38), which encode the polypeptides required for the synthesis of the heptapeptide skeleton of A40926. According to another embodiment, in a nucleic acid of this invention, combinations of ORFs selected from ORFs 1 to 37 (SEQ ID NOS: 2 to 38) are provided which encode a polypeptide required for the chlorination of the aromatic residues of amino acids 3 and 6 of A40926. In yet another embodiment, nucleic acid comprising combinations of ORFs selected from ORFs 1 to 37 (SEQ ID NOS: 2 to 38) are provided, which encode a polypeptide required for the b-hydroxylation of the tyrosine residue of aminoacid 6 of A40926. In yet another embodiment, nucleic acid comprising combinations of ORFs selected from ORFs 1 to 37 (SEQ ID NOS: 2 to 38) are provided, which encode polypeptides required for the cross-linking of the aromatic residues of amino acids at positions 2 and 4, 4 and 6, 1 and 3, and 5 and 7 of A40926. According to another embodiment, in the nucleic acid of this invention, combinations of ORFs selected from ORFs 1 to 37 (SEQ ID NOS: 2 to 38) are provided which encode the polypeptides required for the addition and formation of the N-acylglucuronamine residue. In yet another embodiment, nucleic acids are provided which comprise combinations of ORFs selected from ORFs 1 to 37 (SEQ ID NOS: 2 to 38), encoding a polypeptide required for the attachment of the mannosyl residue. In yet another embodiment, nucleic acids are provided which comprise combinations of ORFs selected from ORFs 1 to 37 (SEQ ID NOS: 2 to 38), encoding a polypeptide required for the N-methylation of A40926. According to yet another embodiment, nucleic acids are provided which comprise combinations of ORFs selected from ORFs 1 to 37 (SEQ ID NOS: 2 to 38), encoding polypeptides required for the export of and resistance to A40926. In yet another embodiment, nucleic acids are provided which comprise combinations of ORFs selected from ORFs 1 to 37 (SEQ ID NOS: 2 to 38), encoding polypeptides required for regulating the expression of the *dbv* gene cluster. In yet another embodiment, nucleic acids are provided which comprise one or more DNA segments selected from SEQ ID NO: 1, enhancing the expression level of an ORF selected from ORFs 1 through 37 (SEQ ID NOS: 2 to 38).

Those skilled in the art understand that the present invention, having provided the nucleotide sequences encoding polypeptides of the A40926 biosynthetic pathway, also provides nucleotides encoding fragments derived from such polypeptides. In addition, those skilled in the art understand that, since the genetic code is degenerate, the same polypeptides specified in SEQ ID NOS: 2 to 38 can be encoded by natural or artificial variants of ORFs 1 to 37, i.e. by nucleotide sequences other than the genomic nucleotide sequences specified by ORFs 1 to 37 but which encode the same polypeptides. Furthermore, it is also understood that naturally occurring or artificially manufactured variants can occur of the polypeptides specified in SEQ ID NOS: 2 to 38, said variants having the same function(s) as the above mentioned original polypeptides but containing addition, deletion or substitution of amino acid not essential for folding or catalytic function, or conservative substitution of essential amino acids.

Those skilled in the art understand also that, having provided the nucleotide sequence of the entire cluster required for A40926 biosynthesis, the present invention also provides nucleotide sequences required for the expression of the genes present in said cluster. Such regulatory sequences include but are not limited to promoter and enhancer sequences, antisense sequences, transcription terminator and antiterminator sequences. These sequences are useful for regulating the expression of the genes present in the *dbv* gene cluster. Cells carrying said nucleotide sequences, alone or fused to other nucleotide sequences, fall also within the scope of the present invention.

In one aspect, the present invention provides isolated nucleic acids comprising nucleotide sequences encoding the ORF9 polypeptide (SEQ ID NO: 10), or naturally occurring variants or derivatives of said polypeptide, useful for the attachment of a glucosamine residue to the core structure of a glycopeptide antibiotic precursor. In another aspect, the present invention provides nucleic acids comprising nucleotide sequences encoding the ORF23 polypeptide (SEQ ID NO: 24), or naturally occurring variants or derivatives of said polypeptide, useful for the attachment of fatty acid residues to the core structure of a glycopeptide antibiotic precursor. In yet another aspect, the present invention provides a nucleic acid comprising nucleotide sequences encoding the ORF29 polypeptide (SEQ ID NO: 30), or naturally occurring variants or derivatives of said polypeptide, useful for the oxidation of sugar moieties attached to a glycopeptide antibiotic precursor. In another aspect, the present invention provides nucleic acids comprising nucleotide sequences encoding the ORF10 polypeptide (SEQ ID NO: 11), or naturally occurring variants or derivatives of said polypeptide, useful for the chlorination of b-hydroxytyrosine and DPG residues in a core glycopeptide antibiotic precursor. In another aspect, the present invention provides nucleic acids comprising nucleotide sequences encoding the ORF20 polypeptide (SEQ ID NO: 21), or naturally occurring variants or derivatives of said polypeptide, useful for the attachment of mannosyl residues to the core structure of a glycopeptide antibiotic precursor.

In another aspect, the present invention provides nucleic acids comprising nucleotide sequences encoding the polypeptides encoded by ORFs 18, 19, 24 and 35 (SEQ ID NOS: 19, 20, 25 and 36), or naturally or artificially occurring variants or derivatives of said polypeptides, useful for export out of the cells of a glycopeptide antibiotic or a glycopeptide antibiotic precursor. In another aspect, the present invention provides nucleic acids comprising nucleotide sequences encoding the ORF7 polypeptide (SEQ ID NO: 8), or naturally or artificially occurring variants or derivatives of said polypeptide, useful for conferring resistance to the producing strain to a glycopeptide antibiotic or a glycopeptide antibiotic precursor. In another aspect, the present invention provides nucleic acids comprising nucleotide sequences encoding the ORF36 polypeptide (SEQ ID NO: 37), or naturally or artificially occurring variants or derivatives of said polypeptide, useful for increasing the yield of a glycopeptide antibiotic precursor.

In one embodiment, the present invention provides a glycopeptide producing strain carrying extra copies of the nucleotide sequences specifying at least one ORF selected from any of ORFs 1 through 37 (SEQ ID NOS: 2 to 38). In one preferred embodiment, such glycopeptide producing strain is any strain belonging to the order *Actinomycetales*. In yet another preferred embodiment, such glycopeptide producing strain is a member of the genus *Nonomuria*. In one further aspect, the present invention provides a *Nonomuria* strain containing one or more variations in the nucleotide sequence specified in SEQ ID NO: 1, such variation resulting in an increased or decreased expression of one or more of ORFs 1 through 37 (SEQ ID NOS: 2 to 38).

In one preferred embodiment, the present invention provides nucleic acids comprising a nucleotide sequence specified by SEQ ID NO: 1, or a portion thereof, carried on one or more vectors, useful for the production of A40926, one or more of its precursors or a derivative thereof by another cell. In one preferred embodiment, said nucleotide sequence or portion thereof is carried on a single vector. In yet another preferred embodiment, such vector is a bacterial artificial chromosome. In yet another aspect, said bacterial artificial chromosome is an ESAC vector (as described in WO99/63674). In another preferred embodiment, the present invention provides a recombinant actinomycete strain other than *Nonomuria* sp. ATCC 39727 containing the gene cluster specified by SEQ ID NO: 1, said gene cluster being carried in an ESAC vector which is integrated into the chromosome of said recombinant actinomycete strain.

In one aspect, the present invention provides a method for increasing the production of A40926, said method comprising the following steps: (1) transforming with a recombinant DNA vector a microorganism that produces A40926 or a A40926 precursor by means of a biosynthetic pathway, said vector comprising a DNA sequence, chosen from any of ORFs 1 through 37 (SEQ ID NO: 2 through 38), that codes for an activity that is rate limiting in said pathway; (2) culturing said microorganism transformed with said vector under conditions suitable for cell growth, expression of said gene and production of said antibiotic or antibiotic precursor.

In another aspect, the present invention provides a method for producing derivatives of A40926, said method comprising the following steps: (1) cloning in a suitable vector a segment chosen from the nucleotide sequence defined by SEQ ID NO:1, said segment containing at least a portion of one of ORFs 1 through 37 (SEQ ID NO: 2 through 38), said ORF encoding a polypeptide that catalyzes a biosynthetic step that one wishes to bypass; (2) inactivating said ORF by removing or replacing one or more codons that specify for amino acids that are essential for the activity of said polypeptide; (3) transforming with said recombinant DNA vector a microorganism that produces A40926 or a A40926 precursor by means of a biosynthetic pathway; (4) screening the resulting transformants for those where said DNA sequence has been replaced by the mutated copy; and (5) culturing said mutant cells under conditions suitable for cell growth, expression of said pathway and production of said pathway analogue.

In yet another aspect, the present invention provides a method for producing novel glycopeptides, said method comprising the following steps: (1) transforming with a recombinant DNA vector a microorganism that produces a glycopeptide or a glycopeptide precursor by means of a biosynthetic pathway, said vector comprising one or more ORFs, chosen among ORFs 1 through 37 (SEQ ID NOS: 2 through 38), that codes for the expression of an enzymatic activity that modifies said glycopeptide or glycopeptide precursor; (2) culturing said microorganism transformed with said vector under conditions suitable for cell growth, expression of said gene and production of said antibiotic or antibiotic precursor.

In yet another aspect, the present invention provides a method for producing novel glycopeptides, said method comprising the following steps: (1) transforming with a recombinant DNA vector a microorganism, said vector comprising one or more ORFs, chosen among ORFs 1 through 37 (SEQ ID NOS: 2 through 38), that codes for the expression of an enzymatic activity that modifies a glycopeptide or glycopeptide precursor; (2) culturing said microorganism transformed with said vector under conditions suitable for cell growth, expression of said gene, in the presence of said glycopeptide or glycopeptide precursor.

In yet another aspect, the present invention provides a method for producing novel glycopeptides, said method comprising the following steps: (1) transforming with a recombinant DNA vector a microorganism, said vector comprising one or more ORFs, chosen among ORFs 1 through 37 (SEQ ID NOS: 2 through 38), that codes for one or more polypeptides that modify a glycopeptide or glycopeptide precursor; (2) preparing a cell extract or cell fraction of said microorganism under conditions suitable for the presence of active polypeptide(s), said cell extract or cell fraction containing at least said polypeptide(s); (3) adding a glycopeptide or glycopeptide precursor to said cell extract or cell fraction, and incubating said mixture under conditions where said polypeptide(s) can modify said glycopeptide or glycopeptide precursor.

A further aspect of this invention includes an isolated polypeptide comprising a polypeptide sequence involved in the biosynthetic pathway of A40926 selected from:
a) an ORF polypeptide encoded by any of *dbv* ORFs 1 to 37 (SEQ ID NOS: 2 through 38) and
b) a polypeptide which is at least 90%, preferably 95% or more, identical in amino acid sequence to a polypeptide encoded by any of *dbv* ORFs 1 to 37 (SEQ ID NOS: 2 through 38), preferably by any one of the *dbv* ORFs 3 to 4, 6 to 10, 18 to 20, 22 to 23, 29 to 30 (SEQ ID NOS: 4 to 5, 7 to 11, 19 to 21, 23 to 24, 30 to 31 and 37).

A preferred group of polypeptides comprises any ORF polypeptide encoded by any of *dbv* ORFs 3, 6 to 9, 18 to 20, 22 to 23, 29 to 30 and 36 (SEQ ID NOS: 4, 7 to 10, 19 to 21, 23 to 24, 30 to 31 and 37) or any polypeptide which is at least 80% preferably 86%, more preferably 90%, most preferably 95% or more, identical in amino acid sequence to a polypeptide encoded by any of said *dbv* ORFs.

A further preferred group of polypeptides comprises any ORF polypeptide encoded by any of the *dbv* ORFs 4 and 10 (SEQ ID NOS: 5 to 11), or any polypeptide which is at least 87%, preferably 90%, more preferably 95% or more, identical in amino acid sequence to a polypeptide encoded by any of said ORFs.

### DEFINITIONS

The term "isolated nucleic acid" refers to a DNA molecule, either as genomic DNA or a complementary DNA (cDNA), which can be single or double stranded, of natural and synthetic origin. This term refers also to an RNA molecule, of natural or synthetic origin.

The term "nucleotide sequence" refers to full length or partial length sequences of ORFs and intergenic regions as disclosed herein. Any one of the nucleotide sequences of the invention as shown in the sequence listing is (a) a coding sequence, (b) an RNA molecule derived from transcription of (a), (c) a coding sequence which uses the degeneracy of the genetic code to encode an identical polypeptide, or (d) an intergenic region, containing promoters, enhancers, terminator and antiterminator sequences.

The terms "gene cluster", "cluster" and "biosynthesis cluster" all designate a contiguous segment of a microorganism's genome that contains all the genes required for the synthesis of a secondary metabolite.

The term "*dbv*" refers to a genetic element responsible for A40926 biosynthesis in *Nonomuria* sp. ATCC39727.

The term "ORF" refers to a genomic nucleotide sequence that encodes one polypeptide. In the context of the present invention, the term ORF is synonymous with "gene".

The term "ORF polypeptide" refers to a polypeptide encoded by an ORF.

The term "*dbv* ORF" refers to an ORF comprised within the *dbv* gene cluster.

The term "NRPS" refers to a non-ribosomal peptide synthetase which is a complex of enzymatic activities responsible for the incorporation of amino acids into an oligopeptide skeleton of a secondary metabolite. A functional NRPS is one that catalyzes the incorporation of one or more amino acid into an oligopeptide.

The term "NRPS module", or "module", refers to a segment of a NRPS that directs the activation, incorporation and possible modification of one amino acid into an oligopeptide.

The term "NRPS gene" refers to a gene that encodes an NRPS.

The term "secondary metabolite" refers to a bioactive substance produced by a microorganism through the expression of a set of genes specified by a gene cluster.

The term "production host" is a microorganism where the formation of a secondary metabolite is directed by a gene cluster derived from a donor organism.
The term "ESAC" identifies an "*Escherichia coli-Streptomyces* Artificial Chromosome", i.e. a recombinant vector that carries and maintains large DNA inserts in an *Escherichia coli* host and that can be introduced and maintained in an actinomycete production host. Examples of ESACs are given in W099/67374.

### BRIEF DESCRIPTION OF THE DRAWINGS

**Figure 1.** Isolated DNA segments derived from the chromosome of *Nonomuria* sp. ATCC39727. The thick line denotes the segment described in SEQ ID NO: 1. The cosmids carrying said isolated DNA segments are designated 11A5, 7F3, 7E9, 1B1, 7A2, 11B9 and 7C7.
**Figure 2.** Genetic organization of the *dbv* cluster. Each ORF is represented by an arrow, and numbered as in Table 1. The orientation is the same as in Fig. 1. Numbers on the scale bars indicate sequence coordinates (in kb).

### DETAILED DESCRIPTION OF THE INVENTION

### A. THE dbv GENES FROM NONOMURIA

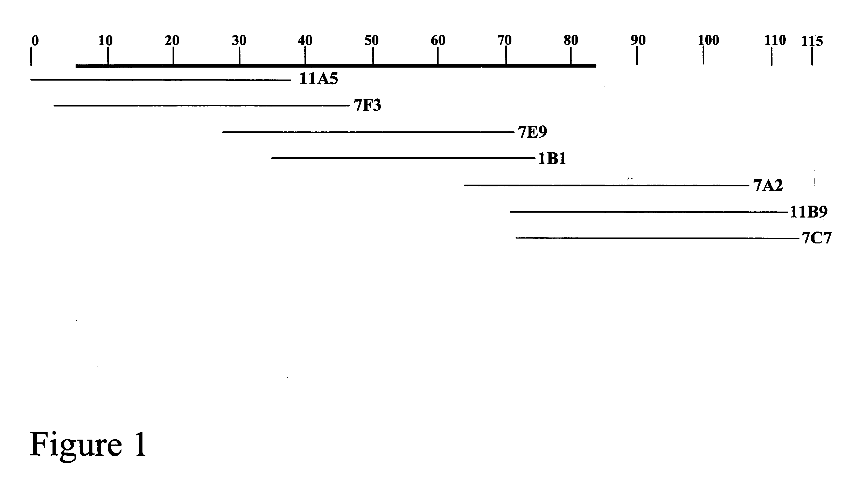

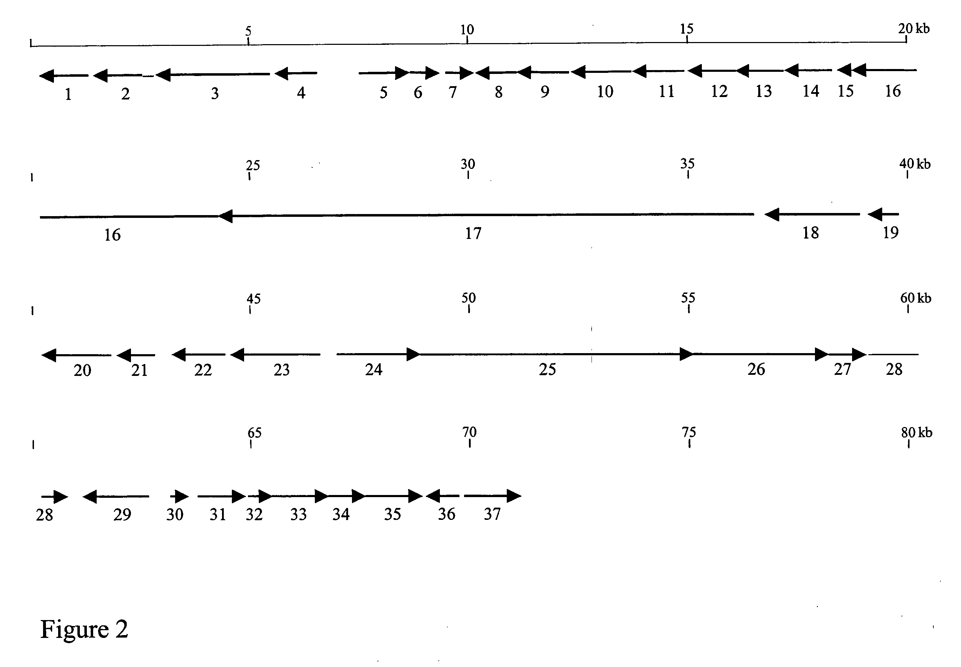

A40926 is a complex of closely related glycopeptide antibiotics produced by *Nonomuria* sp. ATCC39727. The present invention provides nucleic acid sequences and characterization of the gene cluster for the biosynthesis of A40926. The physical organization of the A40926 gene cluster, together with flanking DNA sequences, is reported in Fig. 1, which illustrates the physical map of a 90-kb genomic segment from the genome of *Nonomuria* sp. ATCC39727, together with a set of cosmids defining such segment. The genetic organization of the DNA segment governing A40926 biosynthesis, designated as the dbv cluster, is shown in Fig. 2 and its nucleotide sequence is reported as SEQ ID NO: 1.

The precise boundary of the cluster can be established by comparison with other glycopeptide clusters and from the functions of its gene products. Therefore, on the left end (Fig. 1) the *dbv* cluster is delimited by *dbv* ORF1, encoding the enzyme HmoS (SEQ ID No: 2), involved in the synthesis of HPG. On the right side, the *dbv* cluster is delimited by a remnant of an *attL* site, similar to the 3'-end of a tRNA gene, spanning nucleotides 71065 to 71138 of SEQ ID NO: 1. The *dbv* cluster spans approximately 71,100 base pairs and contains 37 ORFs, designated *dbv* ORF1 through *dbv* ORF37. The contiguous nucleotide sequence of SEQ ID NO: 1 (71138 base pairs) encodes the 37 deduced proteins listed in SEQ ID NOS: 2 to 38. ORF1 (SEQ ID NO: 2) represents 366 amino acids deduced from translating SEQ ID NO: 1 from nucleotides 1140 to 40 on the complementary strand. ORF2 (SEQ ID NO: 3) represents 356 amino acids deduced from translating SEQ ID NO: 1 from nucleotides 2329 to 1259 on the complementary strand. ORF3 (SEQ ID NO: 4) represents 867 amino acids deduced from translating SEQ ID NO: 1 from nucleotides 5161 to 2558 on the complementary strand. ORF4 (SEQ ID NO: 5) represents 321 amino acids deduced from translating SEQ ID NO: 1 from nucleotides 6231 to 5266 on the complementary strand. ORF5 (SEQ ID NO: 6) represents 369 amino acids deduced from translating SEQ ID NO: 1 from nucleotides 7183 to 8292. ORF6 (SEQ ID NO: 7) represents 217 amino acids deduced from translating SEQ ID NO: 1 from nucleotides 8320 to 8973. ORF7 (SEQ ID NO: 8) represents 196 amino acids deduced from translating SEQ ID NO: 1 from nucleotides 9069 to 9659. ORF8 (SEQ ID NO: 9) represents 319 amino acids deduced from translating SEQ ID NO: 1 from nucleotides 10667 to 9708 on the complementary strand. ORF9 (SEQ ID NO: 10) represents 408 amino acids deduced from translating SEQ ID NO: 1 from nucleotides 11896 to 10670 on the complementary strand. ORF10 (SEQ ID NO: 11) represents 489 amino acids deduced from translating SEQ ID NO: 1 from nucleotides 13419 to 11950 on the complementary strand. ORF11 (SEQ ID NO: 12) represents 420 amino acids deduced from translating SEQ ID NO: 1 from nucleotides 14741 to 13479 on the complementary strand. ORF12 (SEQ ID NO: 13) represents 398 amino acids deduced from translating SEQ ID NO: 1 from nucleotides 16019 to 14823 on the complementary strand. ORF13 (SEQ ID NO: 14) represents 384 amino acids deduced from translating SEQ ID NO: 1 from nucleotides 17163 to 16009 on the complementary strand. ORF14 (SEQ ID NO: 15) represents 393 amino acids deduced from translating SEQ ID NO: 1 from nucleotides 18366 to 17185 on the complementary strand. ORF15 (SEQ ID NO: 16) represents 69 amino acids deduced from translating SEQ ID NO: 1 from nucleotides 18671 to 18462 on the complementary strand. ORF16 (SEQ ID NO: 17) represents 1863 amino acids deduced from translating SEQ ID NO: 1 from nucleotides 24259 to 18668 on the complementary strand. ORF17 (SEQ ID NO: 18) represents 4083 amino acids deduced from translating SEQ ID NO: 1 from nucleotides 36529 to 24278 on the complementary strand. ORF18 (SEQ ID NO: 19) represents 753 amino acids deduced from translating SEQ ID NO: 1 from nucleotides 39021 to 36760 on the complementary strand. ORF19 (SEQ ID NO: 20) represents 232 amino acids deduced from translating SEQ ID NO: 1 from nucleotides 39851 to 39152 on the complementary strand. ORF20 (SEQ ID NO: 21) represents 535 amino acids deduced from translating SEQ ID NO: 1 from nucleotides 41732 to 40125 on the complementary strand. ORF21 (SEQ ID NO: 22) represents 270 amino acids deduced from translating SEQ ID NO: 1 from nucleotides 42584 to 41772 on the complementary strand. ORF22 (SEQ ID NO: 23) represents 420 amino acids deduced from translating SEQ ID NO: 1 from nucleotides 44130 to 42868 on the complementary strand. ORF23 (SEQ ID NO: 24) represents 709 amino acids deduced from translating SEQ ID NO: 1 from nucleotides 46355 to 44226 on the complementary strand. ORF24 (SEQ ID NO: 25) represents 648 amino acids deduced from translating SEQ ID NO: 1 from nucleotides 46632 to 48578. ORF25 (SEQ ID NO: 26) represents 2097 amino acids deduced from translating SEQ ID NO: 1 from nucleotides 48575 to 54868. ORF26 (SEQ ID NO: 27) represents 1063 amino acids deduced from translating SEQ ID NO: 1 from nucleotides 54865 to 58056. ORF27 (SEQ ID NO: 28) represents 277 amino acids deduced from translating SEQ ID NO: 1 from nucleotides 58152 to 58985. ORF28 (SEQ ID NO: 29) represents 531 amino acids deduced from translating SEQ ID NO: 1 from nucleotides 59046 to 60641. ORF29 (SEQ ID NO: 30) represents 523 amino acids deduced from translating SEQ ID NO: 1 from nucleotides 62445 to 60874 on the complementary strand. ORF30 (SEQ ID NO: 31) represents 141 amino acids deduced from translating SEQ ID NO: 1 from nucleotides 62887 to 63312. ORF31 (SEQ ID NO: 32) represents 372 amino acids deduced from translating SEQ ID NO: 1 from nucleotides 63469 to 64587. ORF32 (SEQ ID NO: 33) represents 213 amino acids deduced from translating SEQ ID NO: 1 from nucleotides 64599 to 65240. ORF33 (SEQ ID NO: 34) represents 434 amino acids deduced from translating SEQ ID NO: 1 from nucleotides 65237 to 66541. ORF34 (SEQ ID NO: 35) represents 265 amino acids deduced from translating SEQ ID NO: 1 from nucleotides 66538 to 67335. ORF35 (SEQ ID NO: 36) represents 428 amino acids deduced from translating SEQ ID NO: 1 from nucleotides 67332 to 68618. ORF36 (SEQ ID NO: 37) represents 251 amino acids deduced from translating SEQ ID NO: 1 from nucleotides 69423 to 68685 on the complementary strand. ORF37 (SEQ ID NO: 38) represents 428 amino acids deduced from translating SEQ ID NO: 1 from nucleotides 69608 to 70894.

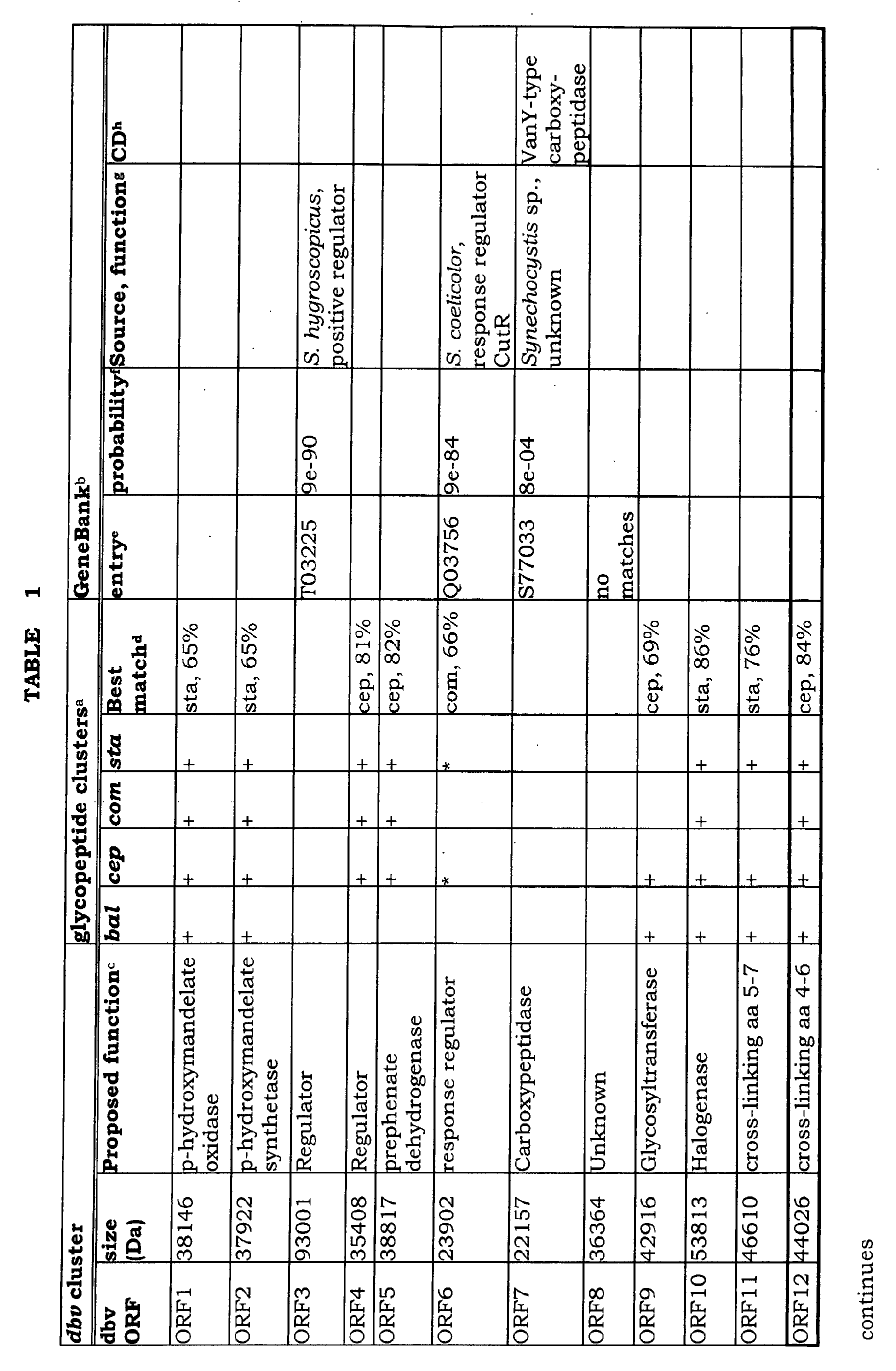

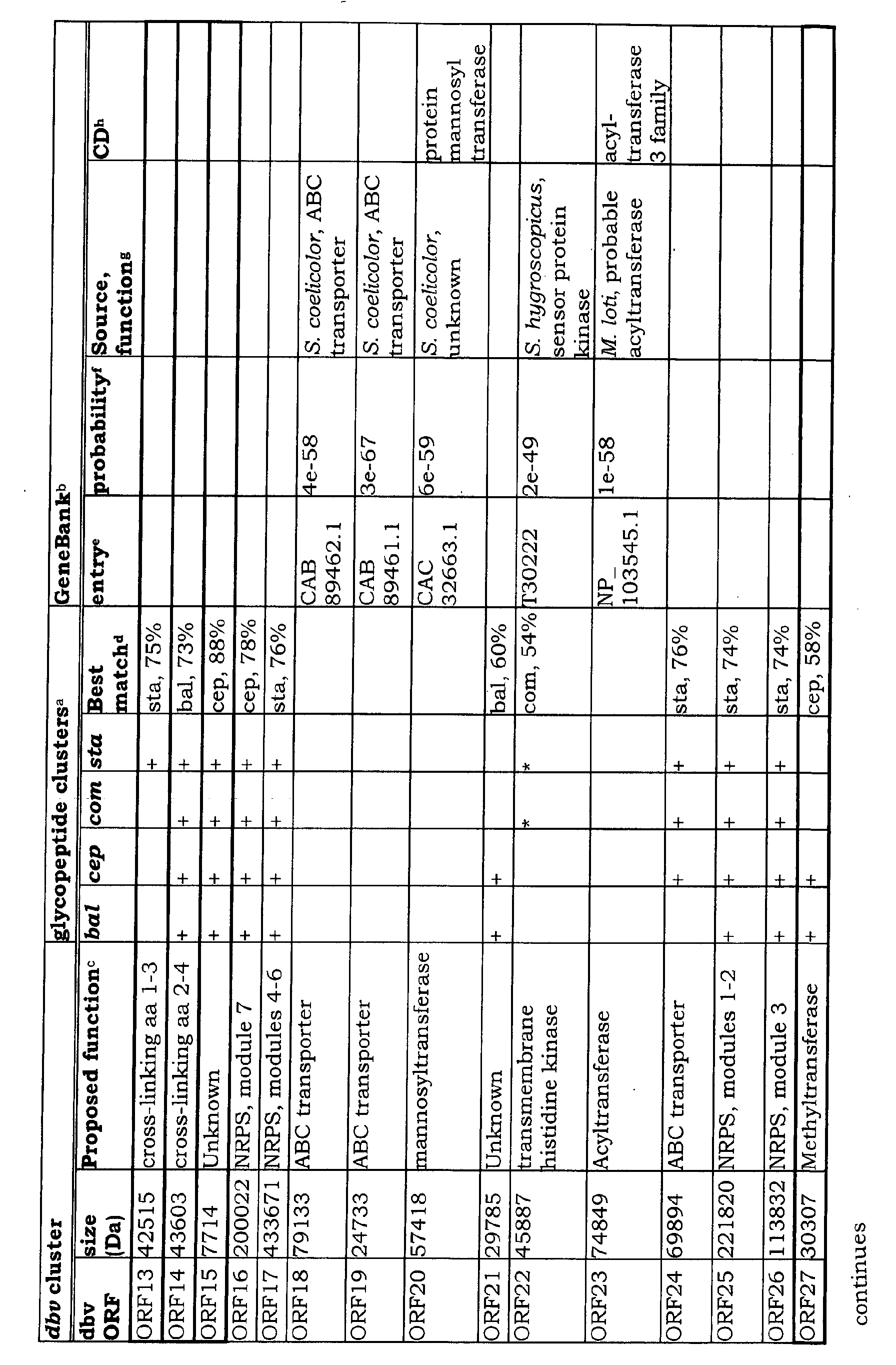

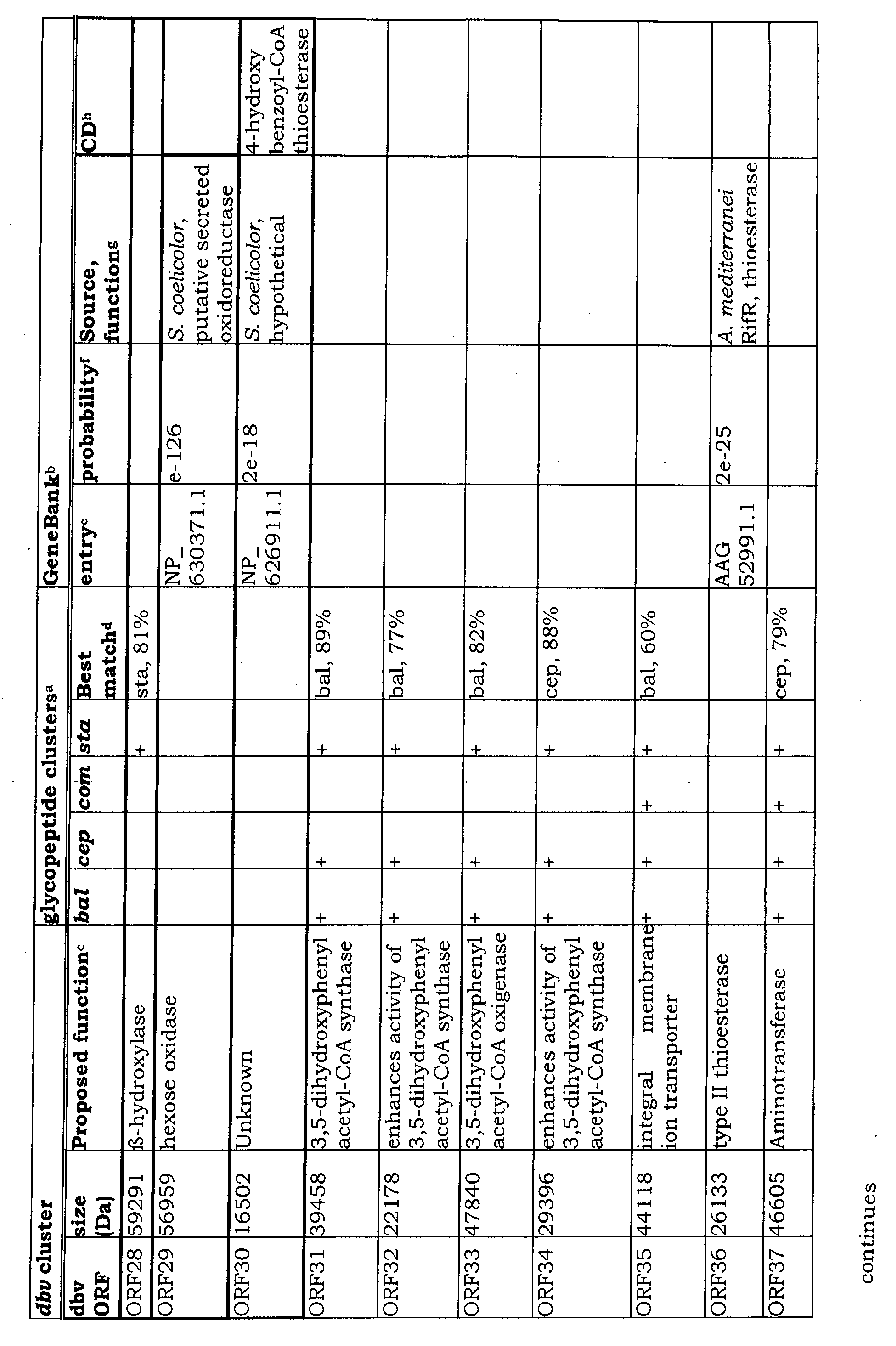

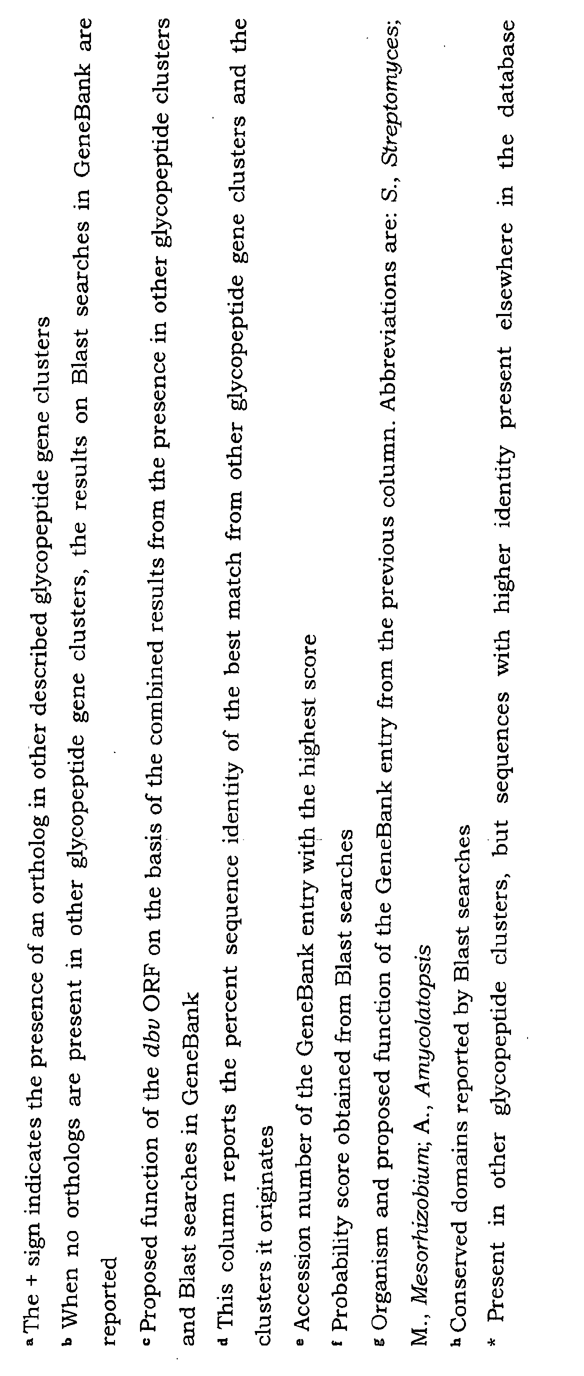

The *dbv* cluster presents an organization that substantially differs from those of other glycopeptide clusters. Indeed, the genes encoding the seven modules of NRPS are organized as two divergently transcribed regions, separated by a 12-kb segment (Fig. 2). This contrasts with the organizations of the *bal, cep, com* and *sta* clusters, where the seven modules of NRPS genes are present in a compact region and translated all in the same direction. Furthermore, while in the *bal, cep, com* and *sta* clusters all ORFs except one are transcribed in the same direction, only 22 of the 37 *dbv* ORFs are transcribed in one direction, while the remaining 15 are transcribed in the opposite direction. This indicates a transcriptional complexity of the *dbv* cluster.

The *dbv* cluster is also characterized by the presence of several ORFs that do not find homologs in the *bal, cep, com* and *sta* clusters. These include *dbv* ORFs 3, 6 through 8, 18 through 20, 22, 23, 29, 30 and 36 (SEQ ID NOS: 4, 7 through 9, 19 through 21, 23, 24, 30, 31 and 37). A comparison among the five *bal, cep, com, sta* and *dbv* clusters is summarized in Table 1. In conclusion, the genetic organization of the *dbv* cluster as described herein is substantially different from those of other clusters involved in the synthesis of other glycopeptides. It therefore represents the first example of a cluster with such a genetic organization.

### B. ROLES OF THE dbv GENES

The present invention discloses, in particular, the DNA sequence encoding the NRPS responsible for the synthesis of the heptapeptide precursor of A40926. The *dbv* NRPS consists of four polypeptides, each containing between 1 and 3 modules. These are designated *dbv* ORF16, ORF17, ORF25 and ORF26 (SEQ ID NOS: 17, 18, 26 and 27). Peptide synthesis by NRPSs is carried out by modular systems, where a loading module is followed by a series of elongating modules. In NRPSs, each elongating module is characterized by the presence of at least three domains: an adenylation (A) domain, responsible for substrate recognition and activation; a thiolation (T) domain, which covalently binds as thioesters amino acids and elongating peptides; and a condensation (C) domain, which catalyzes peptide bond formation. In addition to these core domains, the last module contains a thioesterase (Te) domain, which hydrolyzes the ester bond linking the completed peptide to the NRPS. Some modules convert an L-amino acid into the D-form through the action of an epimerization (E) domain. The *dbv* NRPS consists of seven modules, for a total of seven A domains, seven T domains, six C domains, three E domains and one Te domain. Specifically, *dbv* ORF26 (SEQ ID NO: 27) encodes NRPS modules 1 and 2, specifies the sequence of domains A-T-C-A-E-T and is required for the incorporation of a HPG and a Tyr residue (first two amino acids) in the heptapeptide core of A40926; *dbv* ORF25 (SEQ ID NO: 26) encodes NRPS module 3, specifies the sequence of domains C-A-T and is responsible for incorporating a DPG residue; *dbv* ORF17 (SEQ ID NO: 18) encodes NRPS modules 4 through 6, specifies the sequence of domains C-A-E-T-C-A-E-T-C-A-T and is responsible for incorporating two HPG and a Tyr residue in the A40926 heptapeptide core; and *dbv* ORF16 (SEQ ID NO: 17) encodes NRPS module 7, specifies the sequence of domains C-A-T-C*-T-Te (C* denotes an atypical condensation domain of unknown function) and is required for incorporation of the last DPG residue and in the release of the heptapeptide precursor of A40926.

Other genes present in the *dbv* cluster represent novel genetic elements useful for increasing production of A40926 or for synthesizing novel metabolites. Among these, *dbv* ORF9 (SEQ ID NO: 10) encodes the glycosyltransferase that attaches a glucosamine residue to the phenolic hydroxyl of the HPG residue at position 4 in the heptapeptide (Formula I). This gene can be cloned and expressed in a heterologous host to yield an active enzyme capable of attaching a glucosamine residue to other glycopeptide aglycones. Alternatively, *dbv* ORF9 can be inactivated in the producing strain, resulting in the formation of the A40926 aglycone. While this aglycone can be obtained by chemical means (Malabarba and Ciabatti 2001), it may be desirable to produce it through a single fermentation process, without the need for chemical intervention.

Yet other preferred nucleic acid molecules of the present invention include *dbv* ORF10 (SEQ ID NO: 11) that encodes a halogenase, responsible for the addition of chorine atoms at amino acid 3 and amino acid 6 of A40926. *dbv* ORF10 represents a novel genetic element, different from the halogenase genes present in the *cep, com, sta* and *bal* clusters. In fact, the A40926 chlorination pattern is rather unique among these glycopeptides. This gene can be cloned and expressed in a heterologous host to yield an active enzyme capable of chlorinating aromatic residues 3 and 6 of glycopeptides.

Yet other preferred nucleic acid molecules of the present invention include *dbv* ORF23 (SEQ ID NO: 24) that encodes an acyltransferase, responsible for N-acylation with a fatty acid of the glucosamine residue at amino acid 4. *dbv* ORF23 represents a novel genetic element, absent from the *cep, com, sta* and *bal* clusters. This gene can be cloned and expressed in a heterologous host to yield an active enzyme capable of N-acylating sugar moieties of different glycopeptides.

Yet other preferred nucleic acid molecules of the present invention include *dbv* ORF29 (SEQ ID NO: 30) that encodes a hexose oxidase, responsible for the oxidation to amino glucuronic acid of the D-glucosamine residue attached to amino acid 4 in A40926. *dbv* ORF29 represents a novel genetic element, absent from the *cep, com, sta* and *bal* clusters. This gene can be cloned and expressed in a heterologous host to yield an active enzyme capable of oxidizing D-glucosamine residues attached to a glycopeptide.

Yet other preferred nucleic acid molecules of the present invention include *dbv* ORF36 (SEQ ID NO: 37) that encodes a thioesterase, responsible for hydrolyzing aberrant intermediate peptides from the NRPS. Similarly to other thioesterases present as a polypeptide distinct from the NRPS (Kotowska et al. 2002), the product of *dbv* ORF36 is responsible for maintaining an efficient NRPS for A40926 biosynthesis, by hydrolyzing all those thioesters on the NRPS that are not processed further into heptapeptides. It thus represents a novel genetic element, absent from the *cep, sta, com* and *bal* clusters. This gene can be cloned and expressed in another glycopeptide producer strain to increase the yield of product formed. Host strains include but are not limited to strains belonging to the order *Actinomycetales,* to the families *Streptosporangiaceae,* *Micromonosporaceae, Pseudonocardiaceae* and *Streptomycetaceae,* to the genera *Nonomureae, Actinoplanes, Amycolatopsis, Streptomyces* and the like.

Yet other preferred nucleic acid molecules of the present invention include *dbv* ORF20 (SEQ ID NO: 21) that encodes a mannosyltransferase, responsible for attaching a mannosyl residue to amino acid 7. It thus represents a novel genetic element, absent from the *cep, sta, com* and *bal* clusters. This gene can be cloned and expressed in another glycopeptide producer strain to yield glycopeptides carrying a mannosyl residue attached to amino acid 7. Alternatively, *dbv* ORF20 can be inactivated in the producing strain, resulting in the formation of demannosyl-A40926. While this compound can be obtained by other means (Lancini and Cavalleri 1997), it may be desirable to produce it through a single fermentation process.

The *dbv* cluster also includes a number of genes responsible for the synthesis of the non-proteinogenic amino acids HPG and DPG. For the synthesis of the former, the products of *dbv* ORFs 1, 2, 5 and 37 (SEQ ID NOS: 2, 3, 6 and 38) are required. Synthesis of DPG requires the participation of *dbv* ORFs 31 to 34 (SEQ ID NOS: 32 to 35), in addition to ORF37 (SEQ ID NO: 38). Their roles are summarized in Table 1. Since HPG and DPG are non-proteinogenic amino acids, synthesis of the heptapeptide by the NRPS depends on their availability. Consequently, the activity of these enzymes is a limiting step in glycopeptide biosynthesis. Increased yield of glycopeptides can thus be obtained by increasing the expression of these ORFs. These genes can be overexpressed, individually or in any combination of them, in the A40926 producing strain to increase the yield of A40926.

The *dbv* cluster also includes a number of genes responsible for exporting glycopeptide intermediates or finished products out of the cytoplasm and for conferring resistance to the producer cell. These genes include *dbv* ORFs 7, 18 to 19, 24 and 35 (SEQ ID NOS: 8, 19 to 20, 25 and 36). *dbv* ORF7 encodes a carboxypeptidase responsible for removing the terminal D-alanine moiety from the growing peptidoglycan. It represents a novel genetic element, absent from the *cep, com, sta* and *bal* clusters. *dbv* ORFs 18 to 19 and 24 encode transporters of the ABC class (van Veen and Konings 1998), responsible for the ATP-dependent excretion of A40926 or its intermediates. *dbv* ORF35 encodes an Na/K ion-antiporter, responsible for exporting A40926 or its intermediates against a proton gradient. These genes can be cloned and expressed, either individually or in any combination of them, in another glycopeptide producer strain to increase the yield of product formed. Host strains include but are not limited to strains belonging to the order *Actinomycetales*, to the families *Streptosporangiaceae, Micromonosporaceae, Pseudonocardiaceae* and *Streptomycetaceae,* to the genera *Nonomureae, Actinoplanes, Amycolatopsis, Streptomyces* and the like. Alternatively, these genes can be overexpressed, individually or in any combination of them, in the A40926 producing strain to increase the yield of A40926.

The *dbv* cluster also includes a number of regulatory genes, responsible for activating, directly or indirectly, the expression of biosynthetic and resistance genes during A40926 production. These genes include *dbv* ORFs 3, 4, 6 and 22 (SEQ ID NOS: 4, 5, 7 and 23). *dbv* ORF3 is highly related to HygR, a positive regulator present in a gene cluster from *Streptomyces hygroscopicus* (Ruan et al. 1997). It represents a novel genetic element, absent from the *cep, com, bal* and *sta* clusters. *dbv* ORF4 is highly related to similar regulators present in other glycopeptide clusters. *dbv* ORFs 6 and 22 together encode a two-component signal transduction system. These four genes can be cloned and expressed, either individually or in any combination of them, in another glycopeptide producer strain to increase the yield of product formed. Host strains include but are not limited to strains belonging to the order *Actinomycetales,* to the families *Streptosporangiaceae, Micromonosporaceae, Pseudonocardiaceae* and *Streptomycetaceae,* to the genera *Nonomureae, Actinoplanes, Amycolatopsis, Streptomyces* and the like. Alternatively, these genes can be overexpressed, individually or in any combination of them, in the A40926 producing strain to increase the yield of A40926.

### C. USES OF THE dbv CLUSTER

The present invention provides also nucleic acids for the expression of the entire A40926 molecule, any of its precursors or a derivative thereof. Such nucleic acids include isolated gene cluster(s) comprising ORFs encoding polypeptides sufficient to direct the assembly of A40926. In one example, the entire *dbv* cluster (SEQ ID NO: 1) can be introduced into a suitable vector and used to transform a desired production host. In one aspect, this DNA segment is introduced into a suitable vector capable of carrying large DNA segments. Examples of such vectors include but are not limited to Bacterial Artificial Chromosome (BAC) vectors or specialized derivatives such as ESAC vectors (Shizuya et al. 1992; Ioannou et al. 1994; Sosio et al. 2000b). In another aspect, the *dbv* cluster is cloned as two separate segments into two distinct vectors, which can be compatible in the desired production host. In yet another aspect, the *dbv* cluster can be subdivided into three segments, each cloned into a separate, compatible vector. Examples of the use of one-, two- or three-vector systems have been described in the literature (e.g. Xue et al. 1999).

Once the *dbv* cluster has been suitably cloned into one or more vectors, it can be introduced into a number of suitable production hosts, where production of glycopeptide antibiotics might occur with greater efficiency than in the native host. Preferred host cells are those of species or strains that can efficiently express actinomycetes genes. Such host include but are not limited to *Actinomycetales, Streptosporangiaceae, Micromonosporaceae, Pseudonocardiaceae* and *Streptomycetaceae, Nonomuraea, Actinoplanes, Amycolatopsis* and *Streptomyces* and the like. Alternatively, a second copy of the *dbv* cluster, cloned into one or more suitable vectors, can be introduced the A40926 producing strain, where the second copy of *dbv* genes will increase the yield of A40926.

The transfer of the producing capability to a well characterized host can substantially improve several portions of the process of lead optimization and development: the titer of the natural product in the producing strain can be more effectively increased; the purification of the natural product can be carried out in a known background of possible interfering activities; the composition of the complex can be more effectively controlled; altered derivatives of the natural product can be more effectively produced through manipulation of the fermentation conditions or by pathway engineering.

Alternatively, the biosynthetic gene cluster can be modified, inserted into a host cell and used to synthesize or chemically modify a wide variety of metabolites: for example the open reading frames can be re-ordered, modified and combined with other glycopeptide biosynthesis gene cluster.
Using the information provided herein, cloning and expression of A40926 nucleic acids can be accomplished using routine and well known methods.

In another possible use, selected ORFs from the *dbv* gene cluster are isolated and inactivated by the use of routine molecular biology techniques. The mutated ORF, cloned in a suitable vector containing DNA segments that flank said ORF in the *Nonomuria* sp. ATCC39727 chromosome, is introduced into said *Nonomruria* strain, where two double cross-over events of homologous recombination result in the inactivation of said ORF in the producer strain. This procedure is useful for the production of precursors or derivatives of A40926 in an efficient manner.

In another possible use, selected ORFs from the *dbv* gene cluster are isolated and placed under the control of a desirable promoter. The engineered ORF, cloned in a suitable vector, is then introduced into *Nonomuria* sp. ATCC 39727, either by replacing the original ORF as described above, or as an additional copy of said ORF. This procedure is useful for increasing or decreasing the expression level of ORFs that are critical for production of the A40926 molecule, precursors or derivatives thereof.

### EXAMPLES

The following examples serve to illustrate the principles and methodologies through which the A40926 gene cluster is identified and the principles and methodologies through which all the *dbv* genes are identified and analyzed. These examples serve to illustrate the principles and methodologies of the present invention, but are not meant to limit its scope.

### General methods

Unless otherwise indicated, bacterial strains and cloning vectors can all be obtained from public collections or commercial sources. Standard procedures are used for molecular biology (e.g. Sambrook et al. 1989; Kieser et al. 2000). *Nonomuria* was grown in HT agar (Kieser et al. 2000) and in Rare3 medium (10 g/l glucose, 4 g/l yeast extract, 10 g/l malt extract, 2 g/l peptone, 2 g/l MgCl₂, 0.5% glycerol). Glycopeptides are isolated following published procedures (Lancini and Cavalleri, 1997). Sequence analyses are performed using the programs from the Wisconsin package, version 9.1 (Accelrys). Database searches are performed at with Blast or Fasta programs at public sites (http://www.ncbi.nlm.nih.gov/blast/index.html and http://www.ebi.ac.uk/fasta33).

### Example 1 - Isolation of A40926 biosynthesis genes

A genomic library is made with DNA from *Nonomuria* ATCC39727 in the cosmid vector Supercos (Stratagene, La Jolla, CA 92037). Total DNA from *Nonomuria* ATCC39727 was partially digested with *Sau*3AI in order to optimize fragment sizes in the 40 kb range. The partially digested DNA was treated with alkaline phosphatase and ligated to Supercos previously digested with *Bam*HI. The ligation mixture was packaged in vitro and used to transfect *E. coli* XL1Blue cells. The resulting cosmid library was screened by hybridization with two probes obtained from PCR amplification of segments from the *bal* cluster using *A. mediterranei* DSM 5908 genomic DNA as template. These probes were: *bgtfA*, obtained from amplification with oligos 5'-ATGCGCGTGTTGATCTCG-3' (SEQ ID NO: 39) and 5'-CGGCTGACCGCGGCGAAC-3' (SEQ ID NO: 40); and *dpgA*, obtained from amplification with oligos 5'-CGTGGGGGTG GATGTATCGA-3' (SEQ ID NO: 41) and 5'-TCACCATTGGATCAGCG-3' (SEQ ID NO: 42). All oligos were designed from the sequence deposited in GenBank with accession No. Y16952. Further hybridization was performed with the oligonucleotide Pep8 (Sosio et al. 2000a). The cosmids positive to one or more of these probes were isolated and physically mapped with restriction enzymes. From such experiments, the cosmids reported in Fig. 1 were identified. The segment thus identified from the genome of *Nonomuria* sp. ATCC39727 contains the *dbv* gene cluster responsible for the synthesis of the antibiotic A40926.

The above example serves to illustrate the principle and methodologies through which the *dbv* cluster can be isolated. It will occur to those skilled in the art that the *dbv* cluster can be cloned in a variety of vectors. However, those skilled in the art understand that, given the 72-kb size of the *dbv* cluster, preferred vectors are those capable of carrying large inserts, such as lambda, cosmid and BAC vectors. Those skilled in the art understand that other probes can be used to identify the *dbv* cluster from such a library. From the sequence reported in SEQ ID NO: 1, any fragment can be PCR-amplified from *Nonomuria* sp. ATCC39727 DNA and used to screen a library made with such DNA. One or more clones from said library can be identified that include any segment covered by SEQ ID NO: 1. Furthermore, it is also possible to identify the *dbv* cluster through the use of heterologous probes, such as those derived from the *cep, bal, com* and *sta* cluster, using the information provided in Table 1. Alternatively, other gene clusters directing the synthesis of secondary metabolites contain genes sufficiently related to the *dbv* genes as to allow heterologous hybridizations. All these variations fall within the scope of the present invention.

### Example 2 - Sequence analysis of A40926 gene cluster

The *dbv* cluster, identified as described under Example 1, was sequenced by the shotgun approach. The sequence of the *dbv* cluster is provided herein as SEQ ID NO: 1. The resulting DNA sequence was analyzed with Codonpreference [GCG, (Genetic Computer group, Madison, WI 53711) version 9.1] to identify likely coding sequences. Next, each coding sequence identified in this way was analyzed by comparison against the *bal, cep, com* and *sta* clusters using the program Tfasta (GCG, version 9.1, ). Coding sequences not identifying matches in any of these clusters were then searched against GenBank, employing the programs Blast, or against SwissProt, using Fasta. Finally, the exact start codon for each ORF was established by multiple alignment of related sequences with the program Pileup (GCG, version 9.1) or by searching for an upstream ribosomal binding site. In total, 37 ORFs, denominated *dbv*ORF1 through *dbv* ORF37, are identified. The results of these analyses are summarized in Table 1, and provided herein in the sequence listing as SEQ ID No: 2 through SEQ ID No: 38. Details are given below.

### 2A. Synthesis of specialized amino acids HPG and DPG

Seven proteins encoded by the *dbv* cluster participate in the synthesis of the specialized amino acids HPG and DPG. Namely, ORF1 and ORF2 (SEQ ID NOS: 2 and 3) are involved in the synthesis of the HPG residues required for A40926 formation and they encode the p-hydroxymandelate oxidase and the p-hydroxymandelate synthetase, respectively. Homologs of these ORFs are found in other glycopeptide clusters (Table 1) and their roles have been established experimentally (Li et al. 2001; Hubbard et al. 2000). ORFs 31 to 34 (SEQ ID NOS: 32 to 35) are involved in the synthesis of the DPG residues required for A40926 formation. Homologs of these ORFs are found in other glycopeptide clusters that direct the synthesis of heptapeptide containing DPG residues (Table 1) and the involvement of the corresponsing gene products has been determined experimentally (Pfeifer et al. 2001; Chen et al. 2001). ORF37 (SEQ ID NO: 38) encodes the amino transferase required for the transamination of both p-hydroxyphenylglyoxylate and 3,5-dihydroxyphenylglyoxylate, to yield HPG and DPG, respectively. Its role has been experimentally established (Pfeifer et al. 2001; Hubbard et al. 2000), and it utilizes preferentially tyrosine as an amino donor (Hubbard et al. 2000). This reaction results in the formation of p-hydroxyphenylpyruvate, which can then be converted into p-hydroxymandelate by the action of the gene product of ORF2 (SEQ ID NO: 3).

Other ORFs participating indirectly in the synthesis of HPG and DPG are also found in the *dbv* cluster, namely ORF5 and ORF 30 (SEQ ID NOS: 6 and 31). ORF5 (SEQ ID NO: 6) encodes a prephenate dehydrogenase that participates in the synthesis of p-hydroxyphenylpyruvate, the substrate for the product of ORF2 (SEQ ID NO: 3). This ORF therefore encodes the enzyme that primes the cycle converting tyrosine into HPG. The expression level of this ORF is therefore important in supplying adequate levels of HPG for A40926 formation. ORF30 (SEQ ID NO: 31) encodes a polypeptide highly similar to hypothetical polypeptides of unknown function identified from bacterial genome sequences, with the best matches being represented by NP_626911.1 from *S. coelicolor* (Table 1). However, all these proteins display the conserved domain typical of 4-hydroxybenzoyl-CoA thioesterases (Benning et al. 1998). Thus, the product of ORF30 (SEQ ID No: 31) is likely to facilitate the release of DPG or one of its precursors during synthesis of this small polyketide. ORF30 (SEQ ID NO: 31) is unique to the *dbv* cluster (Table 1).

### 2B. Synthesis of the heptapeptide precursor of A40926

Four proteins, encoded by ORFs 16, 17, 25 and 26 (SEQ ID NOS: 17, 18, 26 and 27) are involved in the synthesis of the heptapeptide core of A40926. All of these show significant similarity to other NRPS. Based on alignments with other NRPS systems, the proposed domain composition and specificities of the proteins encoded by these four ORFs are reported in Table 2.

**Table 2.**

| Domain composition and roles of *dbv* NRPS | | | | |
|---|---|---|---|---|
| dbv ORF | modules | domains | Amino acids | peptide bonds |
| ORF25 | 1-2 | AT-CATE | HPG, Tyr | 1-2 |
| ORF26 | 3 | CAT | DPG | 2-3 |
| ORF17 | 4-6 | CATE-CATE-CAT | HPG, HPG, Tyr | 3-4, 4-5, 5-6 |
| ORF16 | 7 | CATC*Te | DPG | 6-7 |

The assignment of the specific roles of the *dbv* NRPS genes could not be predicted by their genetic localization within the *dbv* cluster. In fact, while for all the glycopeptide clusters reported thus far there is a colinearity between the genetic order of the modules and the order in which the corresponding amino acids are incorporated into the polypeptide, this is not the case for the *dbv* cluster (Fig. 2), since its NRPS genes are divergently transcribed. However, their roles and specificities can be predicted on the basis of the following observations:
i) the domain composition of the protein specified by ORF16 (SEQ ID NO: 17), and the fact that it terminates with a thioesterase domain, is most consistent with a role in recognition of a DPG residue and formation of the last peptide bond of the heptapeptide, followed by cleavage of the enzyme bound thioester (Table 2);
ii) the module organization and domain composition of ORF 17 (SEQ ID NO: 18) is most consistent with this polypeptide containing modules 4 to 6, required for recognizing amino acids 4 to 6 of the heptapeptide and for their incorporation, as seen with other glycopeptide NRPS systems (van Wageningen et al 1998; Pelzer et al. 1999; Chiu et al. 2001; Pootoolal et al. 2002);
iii)the domain organization of the product of ORF25 (SEQ ID NO: 26) is most consistent with its role in starting heptapeptide synthesis and catalyzing formation of the first peptide bond, since this ORF encodes two NRPS modules but just one C domain (Table 2);
iv) the domain organization of ORF26 (SEQ ID NO: 27) is most consistent with this polypeptide containing module 3, responsible for the recognition and incorporation of the third amino acid in the heptapeptide, since this module does not contain an E domain (required by the role of modules 2, 4 and 5) and the presence and absence of a C and a Te domain, respectively (Table 2), excludes that this ORF encodes modules 1 and 7, respectively.

Other ORFs participating indirectly in the synthesis of the heptapeptide precursor of A40926 are also found in the *dbv* cluster, namely ORF15 and ORF36 (SEQ ID NOS: 16 and 37). ORF15 (SEQ ID NO: 16) encodes a short peptide of unknown function. Homologs of this gene product are found in many clusters encoding NRPS systems. ORF36 (SEQ ID NO: 37) encodes a type II thioesterase, a protein often encoded by other clusters containing NRPS or polyketide synthase genes. The proposed role for these thioesterases is to enhance the efficiency by which NRPS and PKS systems operate, by removing aberrant intermediates covalently attached to the enzymes (Heathcote et al. 2001). No orthologs of this protein are encoded by the other known glycopeptide clusters (Table 1).

### 2C. Cross-linking of the aromatic residues in the heptapeptide

Four proteins, encoded by ORFs 11 through 14 (SEQ ID NOS: 12 through 15) are involved in the cross-linking reactions that join together the aromatic residues of the A40926 heptapeptide precursors. These four proteins show significant homologies to P450 monooxygenases (Table 1). On the basis of the level of identities with the P450 monooxygenases found in other glycopeptide clusters, and on the basis of the roles predicted for the P450 monooxygenases encoded by the genes present in the *bal* cluster (Bischoff et al. 2001), the following predictions can be made. Namely, the product of ORF 14 (SEQ ID NO: 15) is likely to be involved in the cross-linking of the aromatic residues of amino acids 2 and 4; the product of ORF 12 (SEQ ID NO: 13) is likely to be involved in the cross-linking of the aromatic residues of amino acids 4 and 6; and the product of ORF 11 (SEQ ID NO: 12) is likely to be involved in the cross-linking of the aromatic residues of amino acids 5 and 7. An ortholog of ORF 13 (SEQ ID NO: 14) is not present in the *bal, cep* and *com* clusters, but it is found in the *sta* cluster (Table 1). Since the structure of A47934, like that of A40926, contains an extra cross-link between the aromatic residues of amino acids 1 and 3, the product of ORF13 (SEQ ID NO: 14) is likely to be involved in this cross-linking reactions.

### 2D. Formation of b-hydroxytyrosine and chlorination of aromatic residues

Two proteins, encoded by ORF10 and ORF28 (SEQ ID NOS: 11 and 29) are involved in the addition of a b-hydroxyl group to the tyrosine residue present as amino acid 6 in the heptapeptide and in the chlorination of the aromatic residues of amino acids 2 and 6. On the basis of the level of identities with the genes encoding halogenases found in other glycopeptide clusters, and on the basis of the roles predicted for the halogenase gene present in the *bal* cluster (Puk et al. 2002), the product of ORF 10 (SEQ ID NO: 11) is likely to be involved in the introduction of a chlorine atom into the aromatic residues of both amino acids 3 and 6. The product of ORF28 (SEQ ID NO: 29) is highly related a family of proteins that contain motifs typical of non-heme iron dioxygenases. One such protein is predicted from the *sta* cluster (Pootoolal et al. 2002) and is suggested to be involved in the b-hydroxylation of tyrosine. The exact timing of this hydroxylation reaction is not currently known. It could occur before incorporation of amino acid 6 into the heptapeptide, as it happens in the synthesis of balhimycin (Bischoff et al. 2001); it could occur during heptapeptide synthesis, or after completion of the heptapeptide skeleton.

### 2E. Addition and modification of sugars, and N-methylation

Five proteins, encoded by ORFs 9, 20, 23, 27 and 29 (SEQ ID NOS: 10, 21, 24, 28 and 30) are involved in some of the late steps in A40926 biosynthesis. Their predicted roles are as follows.
ORF9 (SEQ ID NO: 10) is highly related to proteins encoded by other glycopeptide clusters (Table 1), which have been demonstrated to be involved in the attachment of sugars to the p-hydroxyl group of the aromatic ring of the amino acid residue present at position 4 (Solenberg et al. 1997). Specifically, ORF9 (SEQ ID NO: 10) encodes a glycosyltransferase involved in the attachment of the glucosamine residue to the A40926 aglycone. No other glycosyltransferase with such a specificity is encoded by the other described glycopeptide clusters.

Homologs of ORF20 (SEQ ID NO: 21) are not found in the other described glycopeptide clusters. This protein contains motifs typical of the family of protein mannosyltransferases (Table 1). Furthermore, homologs of this ORF have been identified in the *S. coelicolor* genome (Table 1), as well as in the *Actinoplanes* spp. cluster specifying the synthesis of the antibiotic ramoplanin (WO0231155). Since ramoplanin contains a mannosyl residue attached to the peptide core, all these data point to a role for ORF20 (SEQ ID NO: 21) in attaching the mannosyl residue to the hydroxyl group of amino acid 7. This putative role is also demonstrated in Example 4 below.

Homologs of ORF23 (SEQ ID NO: 24) are not found in the other described glycopeptide clusters. This protein contains motifs typical of the family 3 of acyltransferases (Table 1). Since A40926 contains an acyl residue attached to the NH₂ group of the aminosugar residue, the product of this ORF is likely to be directly or indirectly involved in acylation of the A40926 precursor, resulting in the family of compounds that characterize the A40926 complex.

Homologs of ORF27 (SEQ ID NO: 28) are found in the *bal* and *cep* clusters (Table 1). It has been demonstrated that the homolog of ORF27 from the *cep* cluster is involved in the *N*-methylation of the terminal leucine residue of chloroeremomycin intermediates. An HPG residue is present at the N-terminal position in A40926. Consequently, the product of ORF27 (SEQ ID NO: 28) is likely to catalyze the *N*-methylation of an HPG residue in a glycopeptide precursor, and is thus endowed with a different specificity from the other described methyltransferases.

Homologs of ORF29 (SEQ ID NO: 30) are not found in other described glycopeptide clusters (Table 1). This protein contains motifs typical of FAD binding, and shows considerable matches to hexose oxidases (Table 1). Since A40926 contains a glucuronaminic residue attached to amino acid 4, the protein encoded by ORF29 (SEQ ID NO: 30) is likely to be involved in the oxidation of the glucosamine residue. Since this protein contains also a putative signal peptide sequence typical of proteins secreted out of the cytoplasm, it is likely that this oxidation occurs outside the cytoplasm, using as substrate a glucosamine residue attached to the glycopeptide core.

### 2F. Export and resistance

Five proteins, encoded by ORFs 7, 18, 19, 24 and 35 (SEQ ID NOS: 8, 19, 20, 25 and 36) are involved in exporting A40926 or some of its precursor outside the cytoplasm and in conferring resistance to the producing strain. Their predicted roles are as follows.

Homologs of ORF7 (SEQ ID NO: 8) are not found in the other described glycopeptide clusters. This protein contains motifs typical of the VanY family of carboxypeptidases (Table 1). This family is best studied in some vancomycin-resistant enterococci, where it is involved in the removal of the terminal alanyl residue from some of the pentapeptide chains in nascent peptidoglycan, thus reducing the extent of glycopeptide binding to its molecular target (Evers et al. 1996). ORF7 (SEQ ID NO: 8) is therefore likely to be involved in conferring some level of resistance to A40926 in the producing strain *Nonomuria* sp. ATCC38727.

Homologs of ORF24 and ORF35 (SEQ ID NOS: 25 and 36) are present in other glycopeptide clusters (Table 1). They are predicted to encode ABC-type and ion-dependent transmembrane transporters, respectively. They are thus likely to be involved in export or compartimentalization of A40926 or some of its precursors. Homologs of ORF18 and ORF19 (SEQ ID NOS: 19 and 20) are not found in other described glycopeptide clusters (Table 1). They are predicted to encode additional ABC-type transporters, and of these only ORF18 (SEQ ID NO: 19) is predicted to be a transmembrane protein. They are thus likely to be involved in export or compartimentalization of A40926 or some of its precursors.

### 2G. Regulation

Four proteins, encoded by ORFs 3, 4, 6 and 22 (SEQ ID NOS: 4, 5, 7 and 23) are involved in regulating the expression of one or more of the *dbv* genes. Homologs of ORF3 (SEQ ID NO: 4) are not found in the other described glycopeptide clusters. This protein contains motifs typical of positive regulators of the LuxR family, and is mostly related to one positive regulator found in a PKS cluster from *Streptomyces hygroscopicus* (Ruan et al. 1997). Homologs of ORF4 (SEQ ID NO: 5) are present in other glycopeptide clusters (Table 1), and belong to the family of LysR-type of positive transcriptional regulators. ORFs 3 and 4 (SEQ ID NOS: 4 and 5) are therefore likely to be required for the expression of one or more of the *dbv* genes. ORF6 and ORF22 (SEQ ID NOS: 7 and 23) encode the two members of a bacterial two-component signal transduction system. The former protein is a likely response regulators, with the best match found with the *S. coelicolor* CutR protein (Table 1). The latter protein is a likely transmembrane histidine kinase, mostly related to a putative sensor protein kinase from *S. hygroscopicus* (Table 1). ORFs 6 and 22 (SEQ ID NOS: 23) are therefore likely to be involved in sensing a signal that triggers the expression of one or more genes in the *dbv* cluster.

### Example 3 - Isolation of the dbv cluster in an ESAC vector

Using the information provided in Example 2, the *dbv* cluster was isolaetd in an ESAC vector as follows. A genomic library was made with DNA from *Nonomuria* ATCC39727 in the pPAC-S1 vector (Sosio et al. 2000b). DNA from *Nonomuria* ATCC39727 was prepared embedded in agarose plugs as described (Sosio et al. 2000b; WO99/67374), and partially digested with *Sau*3AI, in order to optimize fragment sizes in the 100-200 kb range. The resulting DNA fragments were briefly run on a PFGE gel, recovered and released from the agarose gel as described (Sosio et al. 2000b; WO99/67374). The resulting steps, including vector preparation, ligation and electroporation of *E. coli* DH10B competent cells, were performed as described (Sosio et al. 2000b; WO99/67374). The resulting colonies were arrayed onto nylon filters and screened by hybridization with two probes, PCR-amplified from *Nonomuria* ATCC39727 genomic DNA. Probe A was obtained using oligos 5'-TCAGGAGACGAACCCCGC-3' (SEQ ID NO: 43) and 5'-GTGCACGAAAGTCCCGTC-3' (SEQ ID NO: 44); and probe B with 5' -ATGGACTCCCACGTTCTC-3' (SEQ ID NO: 45) and 5' -TCAGGGGAGACATGCGGT-3' (SEQ ID NO: 46). All these sequences were derived from SEQ ID NO: 1. The ESAC clones positive to all these probes were then isolated and physically mapped by digestion with *Eco*RI and *Eco*RV. From one such experiment, the ESAC clone NmES1, containing an insert of about 84 kb, was isolated. NmES1 spans the entire *dbv* cluster (SEQ ID NO: 1) and extends it for about 5 kb 5' to nucleotide 1 of SEQ ID NO: 1, and for about 8 kb 3' to nt 71138 of SEQ ID NO: 1.

The above example serves to illustrate the principle and methodologies through which the *dbv* cluster can be obtained in an ESAC vector. It will occur to those skilled in the art that the vector pPAC-S1 is just one example of an ESAC vector that can be used for this purpose. Other vectors useful for cloning the entire *dbv* gene cluster and transferring into a suitable actinomycete host have been described (Sosio et al. 2000b; WO99/67374). Furthermore, other methods for preparing a large insert library of *Nonomuria* sp. ATCC39727 DNA, including but not limited to partial digestion, fragment separation and recovery, vector preparation, ligation and transformation of *E. coli* cells, also fall within the scope of the present invention. It will also occur to those skilled in the art that, once the boundaries of the *dbv* cluster are established as in SEQ ID NO: 1, any probe or probe combination other than probes A and B as described above, can be used to screen a library made with *Nonomuria* sp. ATCC39727 DNA to identify clones whose inserts span the entire *dbv* cluster. Alternatively, with the information provided in SEQ ID NO: 1 and in Table 1, other useful probes can be obtained from other gene clusters that contain genes sufficiently related to the *dbv* genes as to allow heterologous hybridizations. All these variations fall within the scope of the present invention.

### Example 4 - Manipulation of the A40926 pathway by gene replacement

Using the information provided in Example 2, an in frame deletion in ORF 20 was constructed as follows. Fragment A was obtained through amplification with oligos 5'-TTTTGAATTCTCAGGCGATCCGTCCGTCT-3' (SEQ ID NO: 47) and 5'-TTTTCTAGAGCCCGGACACCCGGGGGCTGA-3' (SEQ ID NO: 48); and fragment B with oligos 5'-TTTTCTAGAAGTCATGGTGATGTGCGACAT-3' (SEQ ID NO: 49) and 5'-TTTTAAGCTTATGTTGCAGGACGCCGACCG-3' (SEQ ID NO: 50). Next, fragment A was digested with *Eco*RI and *Xba*I, fragment B with *Xba*I and *Hin*dIII, and both were ligated to pSET152 (Bierman et al. 1992) previously digested with *Eco*RI and *Hind*III. After transformation of *E. coli* DH5a cells, the resulting plasmid, designated pSM4, was recognized by the presence of fragments of 4 kb and 1.5 kb after digestion with *Eco*RI and *Hin*dIII. An aliquot of pSM4 was transferred into *E. coli* ET12567(pUB307) (Kieser et al. 2000) cells, yielding strain SM4. Then, about 10⁸ CFU of SM4 cells, from an overnight culture in LB, were mixed with about 10⁷ CFU of *Nonomuria* ATCC39727 grown in Rare3 medium for about 80 h. The resulting mixture was spread onto HT plates, which were then incubated at 28 °C for about 20 h. After removing excess *E. coli* cells with a gentle wash with water, plates were overlaid with 3 ml soft agar containing 200 mg nalidixic acid and 15 mg/ml apramycin. After further incubation at 28 °C for 3-5 weeks, *Nonomuria* ex-conjugants were streaked onto fresh medium containing apramycin. One such ex-conjugant, named strain SS18, was further processed. Strain SS 18 was then grown for several passages in HT medium without apramycin and appropriate dilutions were plated on HT agar without apramycin. Individual colonies were then analyzed by PCR, using oligos 5'- TTTTGAATTCTCAGGCGATCCGTCCGTCT -3' (SEQ ID NO: 47) and 5'- TTTTAAGCTTATGTTGCAGGACGCCGACCG -3' (SEQ ID NO: 50). Colonies containing the deleted allele of ORF20 were recognized by the presence of a 1.5 kb band. One such colony, designated SSM18, was grown in HT medium and the formation of demannosyl-A40926 was confirmed by comparison with an authentic standard (Malabarba and Ciabatti 2001).

The above example serves to illustrate the principle and methodologies through which an ORF chosen among any of those specified by SEQ ID NOS: 2 to 38 can be replaced by a mutated copy in the A40926 producing strain *Nonomuria* sp. ATCC39727. It will occur to those skilled in the art that ORF20 (SEQ ID NO: 21) is just an example of the methodologies for creating in frame deletions in the cluster specified by SEQ ID NO: 1. Those skilled in the art understand also that in frame-deletions are just one method for generating mutations, and that other methods including but not limited to frame-shift mutations, insertions and site-directed mutations can also be used to generate null mutants in any of the ORFs specified by SEQ ID NOS: 2 to 38. Those skilled in the art also understand that, having established a method for generating mutations in any of the ORFs specified by SEQ ID NOS: 1, these same methodologies can be applied for altering the expression levels of these same ORFs. Examples for how this can be achieved include but are not limited to integration of multiple copies of said ORFs into any place in the *Nonomuria* sp. ATCC39727 genome, alteration in the promoters controlling the expression of said ORFs, removal of antisense RNAs or transcription terminators interfering with their expression.

Finally, variations in the vectors used for introducing the mutated alleles into *Nonomuria* sp. ATCC39727, in the conditions for conjugation and cultivation of the donor and recipient strain, in the method for selecting and screening ex-conjugants and their derivatives, all fall within the scope of the present invention.

### REFERENCES

Bate N, Butler AR, Gandecha AR, Cundliffe E (1999) Chem Biol 6: 617-624.
Benning MM, Wesenberg G, Liu R, Taylor KL, Dunaway-Mariano D, Holden HM (1998) J Biol Chem 273: 33572-33579.
Bentley SD, Chater KF, Cerdeno-Tarraga AM, Challis GL, Thomson NR, James KD, Harris DE, Quail MA, Kieser H, Harper D, Bateman A, Brown S, Chandra G, Chen CW, Collins M, Cronin A, Fraser A, Goble A, Hidalgo J, Hornsby T, Howarth S, Huang CH, Kieser T, Larke L, Murphy L, Oliver K, O'Neil S, Rabbinowitsch E, Rajandream MA, Rutherford K, Rutter S, Seeger K, Saunders D, Sharp S, Squares R, Squares S, Taylor K, Warren T, Wietzorrek A, Woodwardm J, Barrell BG, Parkhill J, Hopwood DA (2002) Nature 417: 141-147.
Bierman R, Logan K, O'Brien ET, Seno R, Nagaraja R, Schoner BE (1992) Gene 116: 43-49.
Bischoff D, Pelzer S, Holtzel A, Nicholson GJ, Stockert S, Wohlleben W, Jung G, Sussmuth RD (2001) Angew Chem Int Ed Engl 40: 1693-1696.
Chater KF, Bibb M (1997) in Biotechnology, vol 6, pp. 57-105, VCH, Weinheim, Germany.
(Kleinkauf H, von Dohren H eds), VCH, Weinheim, Germany.
Chen H, Tseng CC, Hubbard BK, Walsh CT (2001) Proc Natl Acad Sci USA 98: 14901-14906.
Chiu HT, Hubbard BK, Shah AN, Eide J, Fredenburg RA, Walsh CT, Khosla C (2001) Proc Natl Acad Sci USA 98: 8548-8553.
Evers S, Quintiliani R Jr, Courvalin P (1996) Microb Drug Resist 2: 219-223.
Heathcote ML, Staunton J, Leadlay PF (2001) Chem Biol 8: 207-220.
Hubbard BK ,Thomas MG, Walsh CT (2000) Chem Biol 7: 931-942.
Katz L, McDaniel R (1999) Med. Res. Rev. 19: 543-58.
Kieser T, Bibb MJ, Buttner MJ, Chater KF, Hopwood DA (2000) Practical *Streptomyces* Genetics, The John Innes Foundation, Norwich, UK.
Kotowska M, Pawlik K, Butler AR, Cundliffe E, Takano E, Kuczek K (2002) Microbiology 148: 1777-1883.
Ioannou PA. Amemiya CT, Garnes J, Kroisel PM, Shizuya H, Chen C, Batzer MA, de Jong PJ (1994) Nat Genet 6: 84-89.
Lancini GC, Cavalleri B (1990) In: Kleinkauf H, von Döhren H (eds), Biochemistry of Peptide Antibiotics pp. 159-178 Walter de Gruyter Berlin, New York.
Lancini GC, Cavalleri B (1997) Glycopeptide antibiotics (Dalbaaheptides) in Biotechnology, vol 7, pp.369-396, VCH, Weinheim, Germany.
Li TL, Choroba OW, Hong H, Williams DH, Spencer JB (2001) Chem Commun 20: 2156-2157.
Losey HC, Peczuh MW, Chen Z, Eggert US, Dong SD, Pelczer I, Kahne D, Walsh CT (2001) Biochemistry 40: 4745-4755.
Malabarba A, Ciabatti R (2001) Curr Med Chem 8: 1759-1773.
Malabarba A. Ciabatti R, Gerli E, Ripamonti F, Ferrari P, Colombo L, Olsufyeva EN, Pavlov AY, Reznikova MI, Lazhko EI, Preobrazhenskaya MN (1997) J Antibiot 50: 70-81.
Marahiel MA (1997) Chem Biol 4: 561-577.
Mendez C, Salas JA. (2001) Trends Biotechnol 19: 449-456.
Omura S, Ikeda H, Ishikawa J, Hanamoto A, Takahashi C, Shinose M, Takahashi Y, Horikawa H, Nakazawa H, Osonoe T, Kikuchi H, Shiba T, Sakaki Y, Hattori M (2001) Proc Natl Acad Sci USA 98: 12215-12220.
Parenti F, Cavalleri B (1989) J Antibiot 42:1882-1883.
Parenti F, Cavalleri B (1990) Drugs of the future 15: 57-72.
Pelzer S, Sussmuth R, Heckmann D, Recktenwald J, Huber P, Jung G, Wohlleben W (1999) Antimicrob Agents Chemother 43: 1565-1573.
Pfeifer V, Nicholson GJ, Ries J, Recktenwald J, Schefer AB, Shawky RM, Schroder J, Wohlleben W, Pelzer S (2001) J Biol Chem 276: 38370-38377.
Pootoolal J, Thomas MG, Marshall CG, Neu JM, Hubbard BK, Walsh CT, Wright GD (2002) Proc Natl Acad Sci USA 99: 8962-8967.
Puk O, Huber P, Bischoff D, Recktenwald J, Jung G, Sussmuth RD, van Pee KH, Wohlleben W, Pelzer S (2002) Chem Biol 9: 225-235.
Rodriguez L, Aguirrezabalaga I, Allende N, Brana AF, Mendez C, Salas JA (2002) Chem Biol. 9:721-729.
Ruan X, Stassi D, Lax SA, Katz L (1997) Gene 203: 1-9.
Sambrook J, Fritsch EF, Maniatis T (1989) Molecular Cloning: A laboratory Manual (Cold Spring Harbor Lab. Press, Cold Spring Harbor NY).
Shizuya H, Birren B, Kim UJ, Mancino V, Slepak T, Tachiri Y, Simon M (1992) Proc Natl Acad Sci USA 89: 8794-8797.
Solenberg PJ, Matsushima P, Stack DR, Wilkie SC, Thompson RC, Baltz RH (1997) Chem Biol 4:195-202.
Sosio M, Bossi E, Bianchi A, Donadio S (2000a) Mol Gen Genet 264: 213-221.
Sosio M, Giusino F, Cappellano C, Bossi E, Puglia AM, Donadio S (2000b) Nat Biotechnol 18: 343-345.
Steiert M, Schmitz FJ (2002) Curr Opin Investig Drugs 3: 229-233.
van Wageningen AM, Kirkpatrick PN, Williams DH, Harris BR, Kershaw JK, Lennard NJ, Jones M, Jones SJ, Solenberg PJ (1998) Chem Biol 5: 155-162.
van Veen HW, Konings WN (1998) Biochim Biophys Acta 1365: 31-36.
Xue Q, Ashley G, Hutchinson CR, Santi DV (1999) Proc Natl Acad Sci USA 96: 11740-11745.
Zhang Z, Wang Y. Ruan J (1998) Int J Syst Bacteriol 48:411-422.

## Claims

1. An isolated nucleic acid comprising a nucleotide sequence selected from the group consisting of:
a) the *dbv* gene cluster encoding the polypeptides required for the synthesis of A40926 (SEQ ID NO: 1);
b) a nucleotide sequence encoding the same polypeptides encoded by the *dbv* gene cluster (SEQ ID NO: 1), other than the nucleotide sequence of the *dbv* gene cluster;
c) any nucleotide sequence of *dbv* ORFs 1 to 37, encoding the polypeptides of SEQ ID NOS: 2 to 38;
d) a nucleotide sequence encoding the same polypeptides encoded by any of *dbv* ORFs 1 to 37 (SEQ ID NOS: 2 to 38), other than the nucleotide sequence of said ORF.

2. An isolated nucleic acid of claim 1 comprising a nucleotide sequence selected from the group consisting of:
e) a nucleotide sequence of any of *dbv* ORFs 3 to 4, 6 to 10, 18 to 20, 22 to 23, 29 to 30, and 36 (SEQ ID NOS: 4 to 5, 7 to 11, 19 to 21, 23 to 24, 30 to 31, and 37);
f) a nucleotide sequence encoding the same polypeptide encoded by any of *dbv* ORFs 3 to 4, 6 to 10, 18 to 20, 22 to 23, 29 to 30, and 36 (SEQ ID NOS: 4 to 5, 7 to 11, 19 to 21, 23 to 24, 30 to 31, and 37), other than the nucleotide sequence of said ORF.
g) a nucleotide sequence encoding a polypeptide that is at least 80%, preferably 86%, more preferably 90%, most preferably 95% or more, identical in amino acid sequence to a polypeptide encoded by any of *dbv* ORFs 3, 6 to 9, 18 to 20, 22 to 23, 29 to 30, and 36 (SEQ ID NOS: 4, 7 to 10, 19 to 21, 23 to 24, 30 to 31, and 37);
h) a nucleotide sequence encoding a polypeptide that is at least 87%, preferably 90%, more preferably 95% or more, identical in amino acid sequence to a polypeptide encoded by any of *dbv* ORFs 4 and 10 (SEQ ID NOS: 5 and 11).

3. An isolated nucleic acid according to claim 2 comprising a combination of nucleotide sequences which encode polypeptides required for the synthesis of the 4-hydroxy-phenylglycine residues of A40926 consisting of *dbv* ORFs_1, 2, 5 and 37 (SEQ ID NOS: 2, 3, 6 and 38), or nucleotide sequences encoding the same polypeptides, other than the nucleotide sequences of said ORFs.

4. An isolated nucleic acid according to claim 2 comprising a combination of nucleotide sequences which encode polypeptides required for the synthesis of the 3,5-dihydroxy-phenylglycine residues of A40926 consisting of *dbv* ORFs 30 to 34, and 37 (SEQ ID NOS: 31 to 35, and 38), or nucleotide sequences encoding the same polypeptides, other than the nucleotide sequences of said ORFs.

5. An isolated nucleic acid according to claim 2 comprising a combination of nucleotide sequences which encode polypeptides required for the synthesis of the heptapeptide skeleton of A40926 consisting of *dbv* ORFs 16, 17, 25, 26 and 36 (SEQ ID NOS: 17 to 18, 26 to 27, and 37), or nucleotide sequences encoding the same polypeptides, other than the nucleotide sequences of said ORFs.

6. An isolated nucleic acid according to claim 2 comprising a nucleotide sequence which encodes a polypeptide required for the chlorination of the aromatic residues of amino acids 3 and 6 of A40926 consisting of *dbv* ORF 10 (SEQ ID NO: 11), or nucleotide sequences encoding the same polypeptide, other than the nucleotide sequence of said ORF.

7. An isolated nucleic acid according to claim 2 comprising a nucleotide sequence which encodes a polypeptide required for the β-hydroxylation of the tyrosine residue of amino acid 6 of A40926 consisting of *dbv* ORF_28 (SEQ ID NO: 29), or nucleotide sequences encoding the same polypeptide, other than the nucleotide sequence of said ORF.

8. An isolated nucleic acid according to claim 2 comprising a combination of nucleotide sequences which encode polypeptides required for the cross-linking of the aromatic residues of amino acids at positions 2 and 4, 4 and 6, 1 and 3, and 5 and 7 of A40926 consisting of *dbv* ORFs_11 to 14 (SEQ ID NOS: 12 to 15), or nucleotide sequences encoding the same polypeptides, other than the nucleotide sequences of said ORFs.

9. An isolated nucleic acid according to claim 2 comprising a combination of nucleotide sequences which encode polypeptides required for the addition and formation of the N-acyl glucuronamine residue of A40926 consisting of ORFs 9, 23 and 29 (SEQ ID NOS: 10, 24 and 30), or nucleotide sequences encoding the same polypeptides, other than the nucleotide sequences of said ORFs.

10. An isolated nucleic acid according to claim 2 comprising a nucleotide sequence which encodes a polypeptide required for the attachment of the mannosyl residue of A40926 consisting of *dbv* ORF 20 (SEQ ID NO: 21), or nucleotide sequences encoding the same polypeptide, other than the nucleotide sequence of said ORF.

11. An isolated nucleic acid according to claim 2 comprising a nucleotide sequence which encodes a polypeptide required for the N-methylation of A40926 consisting of *dbv* ORF 27 (SEQ ID NO: 28), or nucleotide sequences encoding the same polypeptide, other than the nucleotide sequence of said ORF.

12. An isolated nucleic acid according to claim 2 comprising a combination of nucleotide sequences which encode polypeptides required for export of A40926 or some of its precursors outside of the cytoplasm and for conferring resistance to A40926 to the producing strain consisting of *dbv* ORFs 7, 18, 19, 24 and 35 (SEQ ID NOS: 8, 19 to 20, 25 and 36), or nucleotide sequences encoding the same polypeptides, other than the nucleotide sequences of said ORFs.

13. An isolated nucleic acid according to claim 2 comprising a combination of nucleotide sequences which encode polypeptides required for regulating the expression of one or more genes of the *dbv* gene clusterconsisting of *dbv* ORFs 3, 4, 6 and 22 (SEQ ID NOS: 4, 5, 7 and 23), or nucleotide sequences encoding the same polypeptides, other than the nucleotide sequences of said ORFs.

14. An isolated nucleic acid according to claim 1 comprising a nucleotide sequence consististing of the *dbv* gene cluster encoding the polypeptide required for the synthesis of a A40926 wherein an in frame deletion has been introduced in the nucleotide sequence encoding the polypeptides required for the attachment of the mannosyl residue.

15. An isolated nucleic acid according to claim 1 comprising a nucleotide sequence carrying at least one extra-copy of at least one of the *dbv* ORFs 1 to 37 (SEQ ID NOS: 2 to 38) or of a nucleotide sequence encoding the same polypeptides encoded by said *dbv* ORF, other than the nucleotide sequence of said *dbv* ORF.

16. An isolated nucleic acid of any of claims 1 to 15 wherein the nucleotide sequence is a DNA sequence.

17. A recombinant DNA vector which comprises a DNA sequence as defined in any of claims 1 to 15.

18. A recombinant vector according to claim 17 which is an ESAC vector.

19. A host cell transformed with a vector of any of claims 17 or 18.

20. A transformed host cell according to claim 19 which belongs to the order *Actinomycetales,* preferably to the family *Streptosporangiaceae, Micromonosporaceae, Pseudonocardiaceae* or *Streptomycetaceae,* more preferably to the genera *Nonomureae, Actinoplanes, Amycolatopsis, Streptomyces* or the like.

21. A method for increasing production of A40926 by a microorganism capable of producing A40926 or a precursor thereof by means of a biosynthetic pathway, said method comprising:
a) transforming with a recombinant DNA vector of claim 17 a microorganism that produces A40926 or a A40926 precursor by means of a biosynthetic pathway, wherein said DNA vector codes for the expression of an activity that is rate limiting in said pathway;
b) culturing said microorganism transformed with said vector under conditions suitable for cell growth, expression of said gene and production of said antibiotic or antibiotic presursor.

22. A transformed microorganism producing A40926 or a precursor or a derivative thereof, wherein the A40926 biosynthetic genes in its genome have been modified by insertion of a nucleotide sequence according to claim 15.

23. A process for producing A40926 or a precursor or a derivative thereof which comprises cultivating a transformed A40926-producing microorganism of claim 22.

24. A transformed A40926-producing microorganism having A40926 biosynthetic genes in its genome wherein at least one of the A40926 biosynthetic genes, selected from *dbv* ORFs 1 to 37 (SEQ ID NOS: 2 to 38), is disrupted.

25. A transformed microorganism according to claim 24, wherein the biosynthetic gene which is disrupted is the gene involved in the attachment of the mannosyl residue.

26. A process for producing a A40926 precursor or derivative which comprises a transformed A40926-producing microorganism of claim 24.

27. A method for producing a glycopeptide different from A40926 or a precursor thereof, which consists in:
a) transforming with a recombinant DNA vector a microorganism that produces a glycopeptide or a glycopeptide precursor different from A40926 or a precursor thereof by means of a biosynthetic pathway, said vector or portion thereof comprising a nucleotide sequence of any of claim 1 to 13, that codes for the expression of an enzymatic activity that modifies said glycopeptide or glycopeptide precursor;
b) culturing said microorganism transformed with said vector under conditions suitable for cell growth, expression of said gene and production of said antibiotic or antibiotic precursor

28. An isolated polypeptide comprising a polypeptide sequence involved in the biosynthetic pathway of A40926 selected from
a) an ORF polypeptide encoded by any of *dbv* ORFs 1 to 37 (SEQ ID NOS: 2 through 38) and
b) a polypeptide which is at least 90%, preferably 95% or more, identical in amino acid sequence to an ORF polypeptide encoded by any of *dbv* ORFs 1 to 37 (SEQ ID NOS: 2 through 38), preferably by any one of the *dbv* ORFs 3 to 4, 6 to 10, 18 to 20, 22 to 23, 29 to 30 (SEQ ID NOS: 4 to 5, 7 to 11, 19 to 21, 23 to 24, 30 to 31 and 37).

29. An isolated polypeptide of claim 28 comprising a *dbv* ORF polypeptide encoded by any of *dbv* ORFs 3, 6 to 9, 18 to 20, 22 to 23 , 29 to 30 and 36 (SEQ ID NOS.: 4, 7 to 10, 19 to 21, 23 to 24, 30 to 31 and 37), or any polypeptide which is at least 80%, preferably 86%, more preferably 90%, most preferably 95% or more, identical in amino acid sequence to a polypeptide encoded by any of said *dbv* ORFs.

30. An isolated polypeptide of claim 28 comprising a *dbv* ORF polypeptide encoded by any of *dbv* ORFs 4 and 10 (SEQ ID NOS: 5 and 11) or any polypeptide which is at least 87%, preferably 90%, more preferably 95% or more, identical in amino acid sequence to a polypeptide encoded by any of said *dbv* ORFs.

31. An isolated polypeptide comprising a polypeptide involved in the biosynthetic pathway of A40926 selected from the polypeptides encoded by any of the nucleic acids of any of claims 3 to 16.
